# EUROPÄISCHE PATENTSCHRIFT

(11) **EP 3 679 024 B1**
(45) Veröffentlichungstag und Bekanntmachung des Hinweises auf die Patenterteilung: **02.11.2022**
(21) Anmeldenummer: 18785253.8
(22) Anmeldetag: 05.09.2018
(51) Int. Cl.: C07C 217/94, C07F 5/02, H01L 51/00, C07D 333/76, C07C 211/54, C07C 211/61, C07D 405/12, C07D 307/91, C09K 11/06, H01L 51/50, C07D 209/86

(54) **MATERIALIEN FÜR ELEKTRONISCHE VORRICHTUNGEN**
MATERIALS FOR ELECTRONIC DEVICES
MATÉRIAUX POUR DISPOSITIFS ÉLECTRONIQUES

(30) Priorität: 08.09.2017 EP 17190206
(43) Veröffentlichungstag der Anmeldung: 15.07.2020
(73) Patentinhaber: Merck Patent GmbH, 64293 Darmstadt (DE)
(72) Erfinder: MONTENEGRO, Elvira, 69469 Weinheim (DE); MUJICA-FERNAUD, Teresa, 64283 Darmstadt (DE); MAIER-FLAIG, Florian, 69469 Weinheim (DE); VOGES, Frank, 67098 Bad Duerkheim (DE)
(86) Internationale Anmeldenummer: PCT/EP2018/073794
(87) Internationale Veröffentlichungsnummer: WO 2019/048443

(56) Entgegenhaltungen:
- WO-A1-2006/122630
- WO-A1-2017/036573
- YASUHIKO SHIROTA AND HIROSHI KAGEYAMA: "Charge Carrier Transporting Molecular Materials and Their Applications in Devices", CHEMICAL REV, AMERICAN CHEMICAL SOCIETY, US, Bd. 107, Nr. 4, 1. April 2007 (2007-04-01) , Seiten 953-1010, XP009141446, ISSN: 0009-2665, DOI: 10.1021/CR050143+ [gefunden am 2007-04-11]

## Beschreibung

Die vorliegende Anmeldung betrifft aromatische Verbindungen enthaltend eine Gruppe gewählt aus Aminogruppen, verbrückten Aminogruppen und Carbazolgruppen, gemäß der untenstehend definierten Formel (I). Diese Verbindungen eignen sich zur Verwendung in elektronischen Vorrichtungen.

Unter elektronischen Vorrichtungen im Sinne dieser Anmeldung werden sogenannte organische elektronische Vorrichtungen verstanden (organic electronic devices), welche organische Halbleitermaterialien als Funktionsmaterialien enthalten. Insbesondere werden darunter OLEDs (organische Elektrolumineszenzvorrichtungen) verstanden. Unter der Bezeichnung OLEDs werden elektronische Vorrichtungen verstanden, welche eine oder mehrere Schichten enthaltend organische Verbindungen aufweisen und unter Anlegen von elektrischer Spannung Licht emittieren. Der Aufbau und das allgemeine Funktionsprinzip von OLEDs sind dem Fachmann bekannt.

Bei elektronischen Vorrichtungen, insbesondere OLEDs, besteht großes Interesse an einer Verbesserung der Leistungsdaten, insbesondere Lebensdauer, Effizienz und Betriebsspannung. In diesen Punkten konnte noch keine vollständig zufriedenstellende Lösung gefunden werden.

Weiterhin werden Materialien mit einer hohen Glasübergangstemperatur, einer geringen Neigung zur Kristallisation und einem hohen Brechungsindex gesucht, insbesondere zur Verwendung in lochtransportierenden Schichten von OLEDs.

Einen großen Einfluss auf die Leistungsdaten von elektronischen Vorrichtungen haben Emissionsschichten und Schichten mit lochtransportierender Funktion. Zur Verwendung in diesen Schichten werden weiterhin neue Verbindungen gesucht, insbesondere lochtransportierende Verbindungen und Verbindungen, die als Matrixmaterial, insbesondere für phosphoreszierende Emitter, in einer emittierenden Schicht dienen können.

Im Stand der Technik (WO 2006/122630) sind verschiedene aromatische Verbindungen enthaltend eine Gruppe gewählt aus Aminogruppen, verbrückten Aminogruppen und Carbazolgruppen, als Lochtransportmaterialien und/oder Matrixmaterialien in elektronischen Vorrichtungen bekannt.

Es besteht jedoch weiterhin Bedarf an alternativen Verbindungen, die zur Verwendung in elektronischen Vorrichtungen geeignet sind. Es besteht auch Verbesserungsbedarf bezüglich der Leistungsdaten bei Verwendung in elektronischen Vorrichtungen, insbesondere bezüglich Lebensdauer, Betriebsspannung und Effizienz.

Nun wurde gefunden, dass sich bestimmte Verbindungen der oben genannten Strukturklasse hervorragend zur Verwendung in elektronischen Vorrichtungen eignen, insbesondere zur Verwendung in OLEDs, nochmals insbesondere darin zur Verwendung als Lochtransportmaterialien und zur Verwendung als Matrixmaterialien für phosphoreszierende Emitter. Die Verbindungen führen bevorzugt zu hoher Lebensdauer, hoher Effizienz und geringer Betriebsspannung der Vorrichtungen. Weiterhin bevorzugt weisen die Verbindungen eine geringe Kristallisationsneigung, eine hohe Glasübergangstemperatur und einen hohen Brechungsindex auf.

Die Verbindungen entsprechen der folgenden Formel (I) 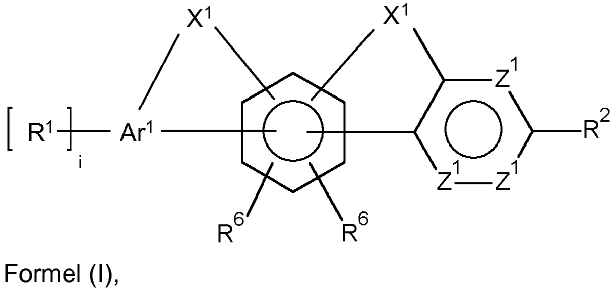 wobei für die auftretenden Variablen gilt:
Z¹ ist bei jedem Auftreten gleich oder verschieden gewählt aus CR¹ und CR³;
Ar¹ ist eine Arylgruppe mit 6 bis 20 aromatischen Ringatomen, die mit einem oder mehreren Resten R³ substituiert sein kann, oder eine Heteroarylgruppe mit 5 bis 20 aromatischen Ringatomen, die mit einem oder mehreren Resten R³ substituiert sein kann;
X¹ ist bei jedem Auftreten gleich oder verschieden eine divalente Gruppe gewählt aus -C(R⁴)₂-, -C(R⁴)₂-C(R⁴)₂-, -CR⁴=CR⁴- und -Si(R⁴)₂-;
R¹ ist eine Gruppe der Formel (N) 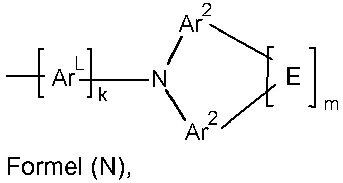
Ar^{L} ist gewählt aus aromatischen Ringsystemen mit 6 bis 40 aromatischen Ringatomen, die mit einem oder mehreren Resten R⁵ substituiert sein können, und heteroaromatischen Ringsystemen mit 5 bis 40 aromatischen Ringatomen, die mit einem oder mehreren Resten R⁵ substituiert sein können;
Ar² ist bei jedem Auftreten gleich oder verschieden gewählt aus aromatischen Ringsystemen mit 6 bis 40 aromatischen Ringatomen, die mit einem oder mehreren Resten R⁵ substituiert sein können, und heteroaromatischen Ringsystemen mit 5 bis 40 aromatischen Ringatomen, die mit einem oder mehreren Resten R⁵ substituiert sein können;
E ist eine Einfachbindung oder eine divalente Gruppe gewählt aus C(R⁵)₂, Si(R⁵)₂, N(R⁵), O, und S;
R² ist gewählt aus H, D, F, C(=O)R⁷, CN, Si(R⁷)₃, N(R⁷)₂, P(=O)(R⁷)₂, OR⁷, S(=O)R⁷, S(=O)₂R⁷, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁷ substituiert sein können; und wobei eine oder mehrere CH₂-Gruppen in den genannten Alkyl-, Alkoxy-, Alkenyl- und Alkinylgruppen durch -R⁷C=CR⁷-, -C=C-, Si(R⁷)₂, C=O, C=NR⁷, -C(=O)O-, -C(=O)NR⁷-, P(=O)(R⁷), -O-, -S-, SO oder SO₂ ersetzt sein können;
R³, R⁴, R⁵ bei jedem Auftreten gleich oder verschieden gewählt aus H, D, F, C(=O)R⁷, CN, Si(R⁷)₃, N(R⁷)₂, P(=O)(R⁷)₂, OR⁷, S(=O)R⁷, S(=O)₂R⁷, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R³ bzw. R⁴ bzw. R⁵ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁷ substituiert sein können; und wobei eine oder mehrere CH₂-Gruppen in den genannten Alkyl-, Alkoxy-, Alkenyl- und Alkinylgruppen durch-R⁷C=CR⁷-, -C≡C-, Si(R⁷)₂, C=O, C=NR⁷, -C(=O)O-, -C(=O)NR⁷-, NR⁷, P(=O)(R⁷), -O-, -S-, SO oder SO₂ ersetzt sein können;
R⁶ ist bei jedem Auftreten gleich oder verschieden gewählt aus H, D, F, C(=O)R⁷, CN, Si(R⁷)₃, P(=O)(R⁷)₂, OR⁷, S(=O)R⁷, S(=O)₂R⁷, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R⁶ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁷ substituiert sein können; und wobei eine oder mehrere CH₂-Gruppen in den genannten Alkyl-, Alkoxy-, Alkenyl- und Alkinylgruppen durch -R⁷C=CR⁷-, -C=C-, Si(R⁷)₂, C=O, C=NR⁷, -C(=O)O-, -C(=O)NR⁷-, NR⁷, P(=O)(R⁷), -O-, -S-, SO oder SO₂ ersetzt sein können;
R⁷ ist bei jedem Auftreten gleich oder verschieden gewählt aus H, D, F, C(=O)R⁸, CN, Si(R⁸)₃, N(R⁸)₂, P(=O)(R⁸)₂, OR⁸, S(=O)R⁸, S(=O)2R⁸, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R⁷ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁸ substituiert sein können; und wobei eine oder mehrere CH₂-Gruppen in den genannten Alkyl-, Alkoxy-, Alkenyl- und Alkinylgruppen durch -R⁸C=CR⁸-, -C=C-, Si(R⁸)₂, C=O, C=NR⁸, -C(=O)O-, -C(=O)NR⁸-, NR⁸, P(=O)(R⁸), -O-, -S-, SO oder SO₂ ersetzt sein können;
R⁸ ist bei jedem Auftreten gleich oder verschieden gewählt aus H, D, F, CN, Alkyl- oder Alkoxygruppen mit 1 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁸ miteinander verknüpft sein können und einen Ring bilden können; und wobei die genannten Alkyl-, Alkoxy-, Alkenyl- und Alkinylgruppen, aromatischen Ringsysteme und heteroaromatischen Ringsysteme mit F oder CN substituiert sein können;
k ist gleich 0 oder 1, wobei im Fall k=0 die Gruppe Ar^{L} wegfällt und das Stickstoffatom der Gruppe der Formel (N) die Anbindungsposition darstellt;
m ist gleich 0 oder 1, wobei im Fall m=0 die Gruppe E wegfällt und die Gruppen Ar² nicht miteinander verbunden sind;
i ist gleich 0 oder 1, wobei im Fall i=0 die Gruppe R¹ wegfällt; und
wobei mindestens eine Gruppe Z¹ vorliegt, die CR¹ ist.

Die Kreise, die in die Sechsringe der Formel (I) gezeichnet sind, bedeuten, dass die betreffenden Sechsringe Aromatizität aufweisen. Die in die Benzolringe hineingezeichneten Bindungen drücken aus, dass die betreffenden Bindungen an einer beliebigen Position am Benzolring lokalisiert sein können. Insbesondere können die Brücken X¹ nicht nur in trans-, sondern auch in cis-Stellung zueinander angeordnet sein.

Eine Arylgruppe im Sinne dieser Erfindung enthält 6 bis 40 aromatische Ringatome, von denen keines ein Heteroatom darstellt. Unter einer Arylgruppe im Sinne dieser Erfindung wird entweder ein einfacher aromatischer Cyclus, also Benzol, oder ein kondensierter aromatischer Polycyclus, beispielsweise Naphthalin, Phenanthren oder Anthracen, verstanden. Ein kondensierter aromatischer Polycyclus besteht im Sinne der vorliegenden Anmeldung aus zwei oder mehr miteinander kondensierten einfachen aromatischen Cyclen. Unter Kondensation zwischen Cyclen ist dabei zu verstehen, dass die Cyclen mindestens eine Kante miteinander teilen.

Eine Heteroarylgruppe im Sinne dieser Erfindung enthält 5 bis 40 aromatische Ringatome, von denen mindestens eines ein Heteroatom darstellt. Die Heteroatome der Heteroarylgruppe sind bevorzugt ausgewählt aus N, O und S. Unter einer Heteroarylgruppe im Sinne dieser Erfindung wird entweder ein einfacher heteroaromatischer Cyclus, beispielsweise Pyridin, Pyrimidin oder Thiophen, oder ein kondensierter heteroaromatischer Polycyclus, beispielsweise Chinolin oder Carbazol, verstanden. Ein kondensierter heteroaromatischer Polycyclus besteht im Sinne der vorliegenden Anmeldung aus zwei oder mehr miteinander kondensierten einfachen heteroaromatischen Cyclen. Unter Kondensation zwischen Cyclen ist dabei zu verstehen, dass die Cyclen mindestens eine Kante miteinander teilen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Triphenylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ein aromatisches Ringsystem im Sinne dieser Erfindung enthält 6 bis 40 C-Atome im Ringsystem und umfasst keine Heteroatome als aromatische Ringatome. Ein aromatisches Ringsystem im Sinne dieser Erfindung enthält daher keine Heteroarylgruppen. Unter einem aromatischen Ringsystem im Sinne dieser Erfindung soll ein System verstanden werden, das nicht notwendigerweise nur Arylgruppen enthält, sondern in dem auch mehrere Arylgruppen durch eine Einfachbindung oder durch eine nicht-aromatische Einheit, wie beispielsweise ein oder mehrere wahlweise substituierte C-, Si-, N-, O- oder S-Atome, verbunden sein können. Dabei umfasst die nicht-aromatische Einheit bevorzugt weniger als 10 % der von H verschiedenen Atome, bezogen auf die Gesamtzahl der von H verschiedenen Atome des Systems. So sollen beispielsweise auch Systeme wie 9,9'-Spirobifluoren, 9,9'-Diarylfluoren, Triarylamin, Diarylether und Stilben als aromatische Ringsysteme im Sinne dieser Erfindung verstanden werden, und ebenso Systeme, in denen zwei oder mehr Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Arylgruppen über Einfachbindungen miteinander verknüpft sind, als aromatische Ringsysteme im Sinne dieser Erfindung verstanden, wie beispielsweise Systeme wie Biphenyl und Terphenyl.

Bevorzugt wird unter einem aromatischen Ringsystem eine chemische Gruppe verstanden, in der die darin enthaltenen Arylgruppen miteinander konjugiert sind. Dies bedeutet, dass die enthaltenen Arylgruppen miteinander über Einfachbindungen oder über verbindende Einheiten, die ein freies pi-Elektronenpaar aufweisen, das an der Konjugation teilnehmen kann, verbunden sein müssen. Verbindende Einheiten sind dabei bevorzugt gewählt aus Stickstoffatomen, einzelnen C=C-Einheiten, einzelnen C=C-Einheiten, mehreren miteinander konjugierten C=C-Einheiten oder/oder C=C-Einheiten, -O-, und -S-.

Ein heteroaromatisches Ringsystem im Sinne dieser Erfindung enthält 5 bis 40 aromatische Ringatome, von denen mindestens eines ein Heteroatom darstellt. Die Heteroatome des heteroaromatischen Ringsystems sind bevorzugt ausgewählt aus N, O und/oder S. Ein heteroaromatisches Ringsystem entspricht der oben genannten Definition eines aromatischen Ringsystems, weist jedoch mindestens ein Heteroatom als eines der aromatischen Ringatome auf. Es unterscheidet sich dadurch von einem aromatischen Ringsystem im Sinne der Definition der vorliegenden Anmeldung, welches gemäß dieser Definition kein Heteroatom als aromatisches Ringatom enthalten kann.

Unter einem aromatischen Ringsystem mit 6 bis 40 aromatischen Ringatomen oder einem heteroaromatischen Ringsystem mit 5 bis 40 aromatischen Ringatomen, werden insbesondere Gruppen verstanden, die abgeleitet sind von den oben unter Arylgruppen und Heteroarylgruppen genannten Gruppen sowie von Biphenyl, Terphenyl, Quaterphenyl, Fluoren, Spirobifluoren, Dihydrophenanthren, Dihydropyren, Tetrahydropyren, Indenofluoren, Truxen, Isotruxen, Spirotruxen, Spiroisotruxen, Indenocarbazol, oder von Kombinationen dieser Gruppen.

Im Rahmen der vorliegenden Erfindung werden unter einer geradkettigen Alkylgruppe mit 1 bis 20 C-Atomen bzw. einer verzweigten oder cyclischen Alkylgruppe mit 3 bis 20 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden.

Unter einer Alkoxy- oder Thioalkylgruppe mit 1 bis 20 C-Atomen, in der auch einzelne H-Atome oder CH₂-Gruppen durch die oben bei der Definition der Reste genannten Gruppen substituiert sein können, werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methyl-butoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet.

Die Verbindung der Formel (I) weist bevorzugt nur eine einzige Diarylaminogruppe auf. Besonders bevorzugt weist sie nur eine einzige Aminogruppe auf.

Bevorzugt ist eine Gruppe Z¹ gleich CR¹, und die beiden anderen Gruppen Z¹ sind gleich CR³; oder zwei Gruppen Z¹ sind gleich CR¹, und die andere Gruppe Z¹ ist gleich CR³. Besonders bevorzugt ist eine Gruppe Z¹ gleich CR¹, und die beiden anderen Gruppen Z¹ sind gleich CR³.

Es ist bevorzugt, dass diejenige Gruppe Z¹, die in meta-Position zur Bindung zu X¹ steht, gleich CR¹ ist.

Bevorzugt bilden Reste R³, die Bestandteile einer Gruppe Z¹ sind, miteinander keine Ringe.

Bevorzugt ist Ar¹ eine Arylgruppe mit 6 bis 14 aromatischen Ringatomen, die mit einem oder mehreren Resten R³ substituiert sein kann, besonders bevorzugt eine Phenyl- oder Naphthylgruppe, die mit einem oder mehreren Resten R³ substituiert sein kann, am stärksten bevorzugt eine Phenylgruppe, die mit einem oder mehreren Resten R³ substituiert sein kann.

X¹ ist bevorzugt bei jedem Auftreten gleich oder verschieden gewählt aus C(R⁴)₂ und Si(R⁴)₂, besonders bevorzugt ist X¹ gleich C(R⁴)₂.

Gruppen Ar^{L} sind bevorzugt gewählt aus aromatischen Ringsystemen mit 6 bis 20 aromatischen Ringatomen, die mit einem oder mehreren Resten R⁵ substituiert sein können, und heteroaromatischen Ringsystemen mit 5 bis 20 aromatischen Ringatomen, die mit einem oder mehreren Resten R⁵ substituiert sein können. Besonders bevorzugte Gruppen Ar^{L} sind gewählt aus divalenten Gruppen abgeleitet von Benzol, Biphenyl, Terphenyl, Naphthalin, Fluoren, Indenofluoren, Spirobifluoren, Dibenzofuran, Dibenzothiophen, und Carbazol, die jeweils mit einem oder mehreren Resten R⁵ substituiert sein können. Ganz besonders bevorzugt ist Ar^{L} eine divalente Gruppe abgeleitet von Benzol, das jeweils mit einem oder mehreren Resten R⁵ substituiert sein kann. Gruppen Ar^{L} können bei jedem Auftreten gleich oder verschieden gewählt sein.

Bevorzugte Gruppen Ar^{L} entsprechen den folgenden Formeln:

| | | |
|---|---|---|
| 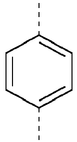 | 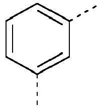 | 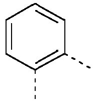 |
| Ar^{L}-1 | Ar^{L}-2 | Ar^{L}-3 |
| 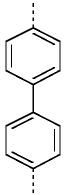 | 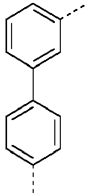 | 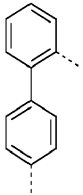 |
| Ar^{L}-4 | Ar^{L}-5 | Ar^{L}-6 |
| 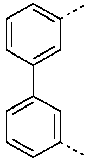 | 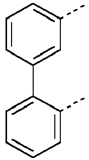 | 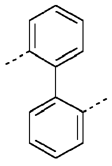 |
| Ar^{L}-7 | Ar^{L}-8 | Ar^{L}-9 |
| 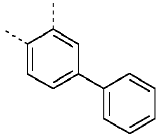 | 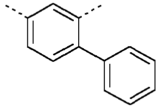 | 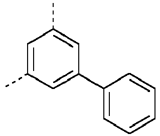 |
| Ar^{L}-10 | Ar^{L}-11 | Ar^{L}-12 |
| 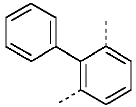 | 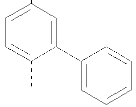 | 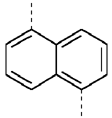 |
| Ar^{L}-13 | Ar^{L}-14 | Ar^{L}-15 |
| 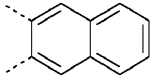 | 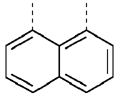 | 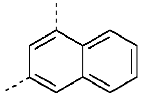 |
| Ar^{L}-16 | Ar^{L}-17 | Ar^{L}-18 |
| 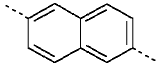 | 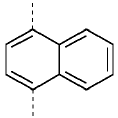 | 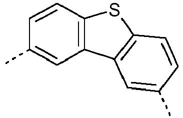 |
| Ar^{L}-19 | Ar^{L}-20 | Ar^{L}-21 |
| 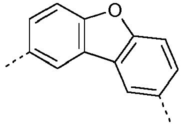 | 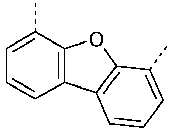 | 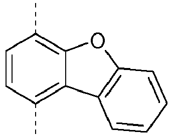 |
| Ar^{L}-22 | Ar^{L}-23 | Ar^{L}-24 |
| 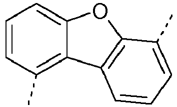 | 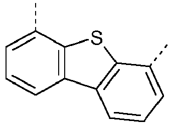 | 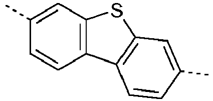 |
| Ar^{L}-25 | Ar^{L}-26 | Ar^{L}-27 |
| 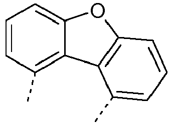 | 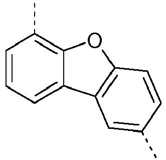 | 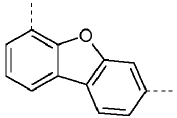 |
| Ar^{L}-28 | Ar^{L}-29 | Ar^{L}-30 |
| 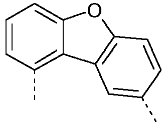 | 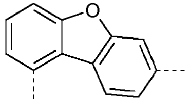 | 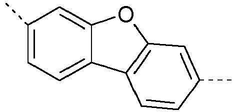 |
| Ar^{L}-31 | Ar^{L}-32 | Ar^{L}-33 |
| 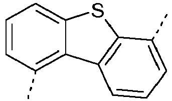 | 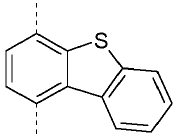 | 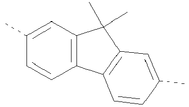 |
| Ar^{L}-34 | Ar^{L}-35 | Ar^{L}-36 |
| 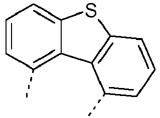 | 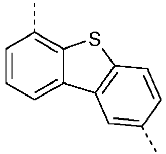 | 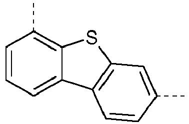 |
| Ar^{L}-37 | Ar^{L}-38 | Ar^{L}-39 |
| 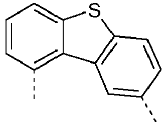 | 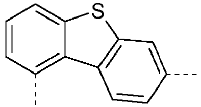 | 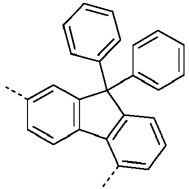 |
| Ar^{L}-40 | Ar^{L}-41 | Ar^{L}-42 |
| 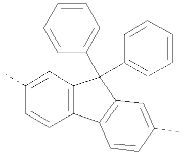 | 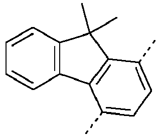 | 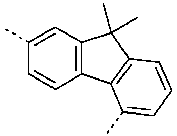 |
| Ar^{L}-43 | Ar^{L}-44 | Ar^{L}-45 |
| 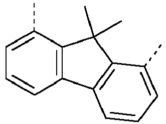 | 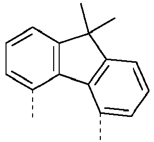 | 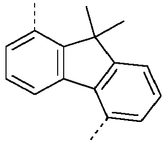 |
| Ar^{L}-46 | Ar^{L}-47 | Ar^{L}-48 |
| 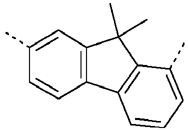 | 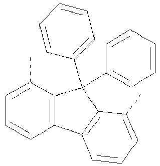 | 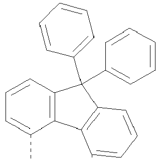 |
| Ar^{L}-49 | Ar^{L}-50 | Ar^{L}-51 |
| 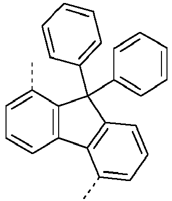 | 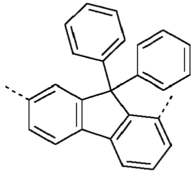 | 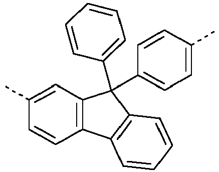 |
| Ar^{L}-52 | Ar^{L}-53 | Ar^{L}-54 |
| 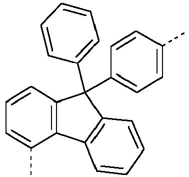 | 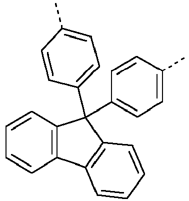 | 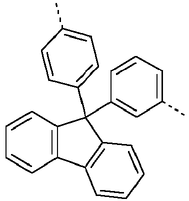 |
| Ar^{L}-55 | Ar^{L}-56 | Ar^{L}-57 |
| 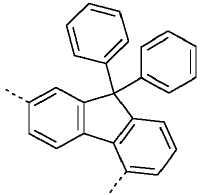 | 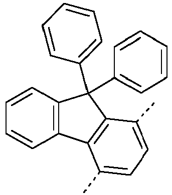 | 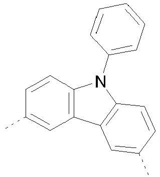 |
| Ar^{L}-58 | Ar^{L}-59 | Ar^{L}-60 |
| 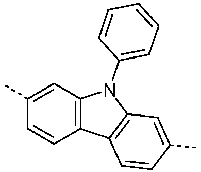 | 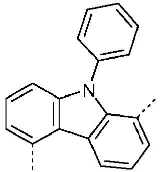 | 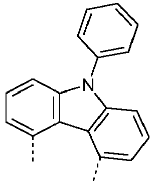 |
| Ar^{L}-61 | Ar^{L}-62 | Ar^{L}-63 |
| 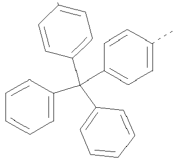 | 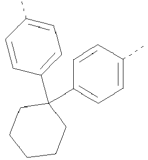 | 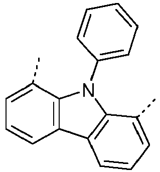 |
| Ar^{L}-64 | Ar^{L}-65 | Ar^{L}-66 |
| 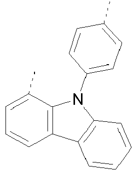 | 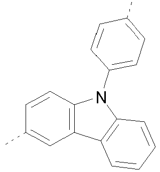 | 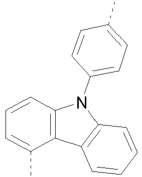 |
| Ar^{L}-67 | Ar^{L}-68 | Ar^{L}-69 |
| 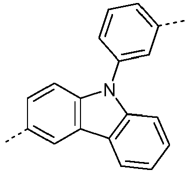 | 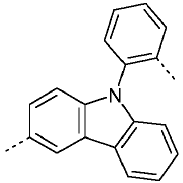 | 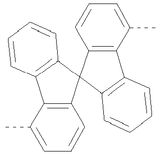 |
| Ar^{L}-70 | Ar^{L}-71 | Ar^{L}-72 |
| 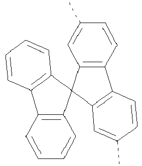 | 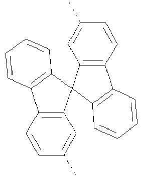 | 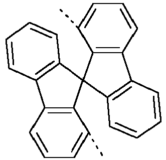 |
| Ar^{L}-73 | Ar^{L}-74 | Ar^{L}-75 |

wobei die gestrichelten Linien die Bindungen an den Rest der Formel (I) darstellen.

Bevorzugt sind die Gruppen Ar² bei jedem Auftreten gleich oder verschieden gewählt aus aromatischen Ringsystemen mit 6 bis 40 aromatischen Ringatomen, die mit einem oder mehreren Resten R⁵ substituiert sein können, und heteroaromatischen Ringsystemen mit 5 bis 40 aromatischen Ringatomen, die mit einem oder mehreren Resten R⁵ substituiert sein können.

Dabei ist es allgemein bevorzugt, dass die direkt an das Stickstoffatom bindende Gruppe von Ar² ein aromatisches Ringsystem ist.

Bevorzugt sind Gruppen Ar² bei jedem Auftreten gleich oder verschieden gewählt aus monovalenten Gruppen abgeleitet von Benzol, Biphenyl, Terphenyl, Quaterphenyl, Naphthalin, Fluoren, insbesondere 9,9'-Dimethylfluoren und 9,9'-Diphenylfluoren, Benzofluoren, Spirobifluoren, Indenofluoren, Dibenzofuran, Dibenzothiophen, Benzocarbazol, Carbazol, Benzofuran, Benzothiophen, Indol, Chinolin, Pyridin, Pyrimidin, Pyrazin, Pyridazin und Triazin, wobei die monovalenten Gruppen jeweils mit einem oder mehreren Resten R⁵ substituiert sein können. Alternativ bevorzugt können die Gruppen Ar² bei jedem Auftreten gleich oder verschieden gewählt sein aus Kombinationen von Gruppen, die abgeleitet sind von Benzol, Biphenyl, Terphenyl, Quaterphenyl, Naphthalin, Fluoren, insbesondere 9,9'-Dimethylfluoren und 9,9'-Diphenylfluoren, Benzofluoren, Spirobifluoren, Indenofluoren, Dibenzofuran, Dibenzothiophen, Carbazol, Benzofuran, Benzothiophen, Indol, Chinolin, Pyridin, Pyrimidin, Pyrazin, Pyridazin und Triazin, wobei die Gruppen jeweils mit einem oder mehreren Resten R⁵ substituiert sein können.

Besonders bevorzugte Gruppen Ar² sind bei jedem Auftreten gleich oder verschieden gewählt aus Phenyl, Biphenyl, Terphenyl, Quaterphenyl, Naphthyl, Fluorenyl, insbesondere 9,9'-Dimethylfluorenyl und 9,9'-Diphenylfluorenyl, Benzofluorenyl, Spirobifluorenyl, Indenofluorenyl, Dibenzofuranyl, Dibenzothiophenyl, Carbazolyl, Benzofuranyl, Benzothiophenyl, benzokondensiertes Dibenzofuranyl, benzokondensiertes Dibenzothiophenyl, Naphthyl-substituiertes Phenyl, Fluorenyl-substituiertes Phenyl, Spirobifluorenyl-substituiertes Phenyl, Dibenzofuranyl-substituiertes Phenyl, Dibenzothiophenyl-substituiertes Phenyl, Carbazolyl-substituiertes Phenyl, Pyridyl-substituiertes Phenyl, Pyrimidyl-substituiertes Phenyl, und Triazinyl-substituiertes Phenyl, wobei die genannten Gruppen jeweils mit einem oder mehreren Resten R⁵ substituiert sein können.

Besonders bevorzugte Gruppen Ar² sind gewählt aus den folgenden Formeln:

| | | |
|---|---|---|
| 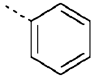 | 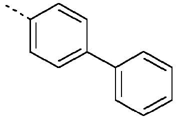 | 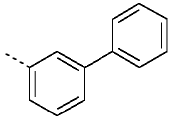 |
| Ar-1 | Ar-2 | Ar-3 |
| 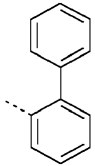 | 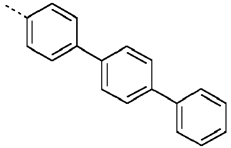 | 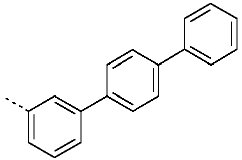 |
| Ar-4 | Ar-5 | Ar-6 |
| 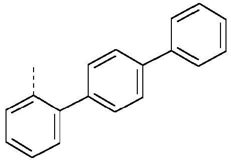 | 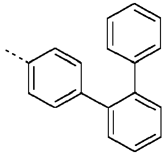 | 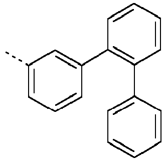 |
| Ar-7 | Ar-8 | Ar-9 |
| 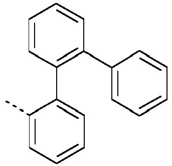 | 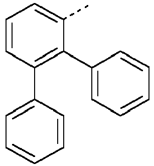 | 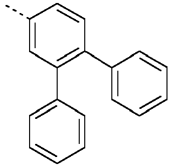 |
| Ar-10 | Ar-11 | Ar-12 |
| 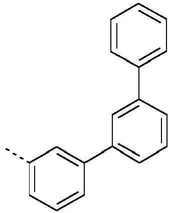 | 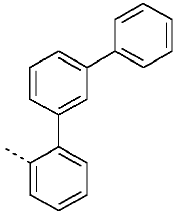 | 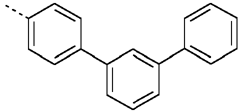 |
| Ar-13 | Ar-14 | Ar-15 |
| 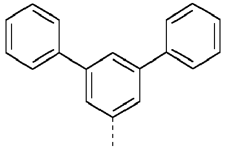 | 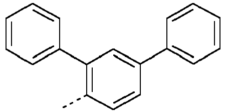 | 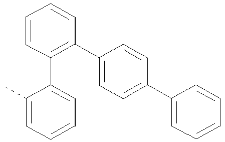 |
| Ar-16 | Ar-17 | Ar-18 |
| 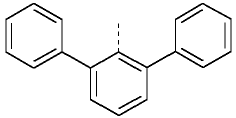 | | |
| Ar-19 | | |
| 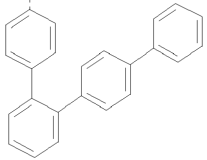 | 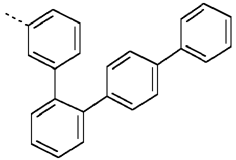 | 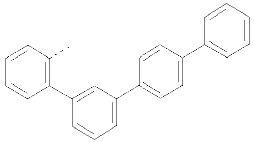 |
| Ar-20 | Ar-21 | Ar-22 |
| 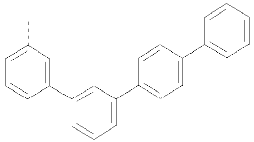 | 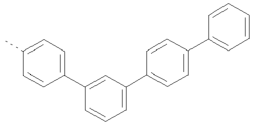 | 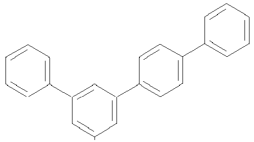 |
| Ar-23 | Ar-24 | Ar-25 |
| 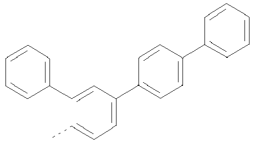 | 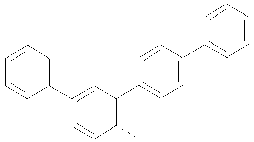 | 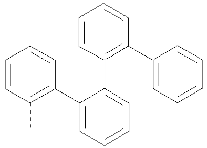 |
| Ar-26 | Ar-27 | Ar-28 |
| 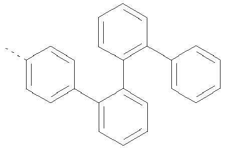 | 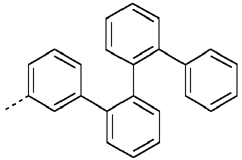 | 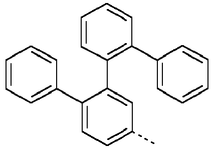 |
| Ar-29 | Ar-30 | Ar-31 |
| 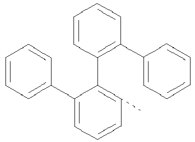 | 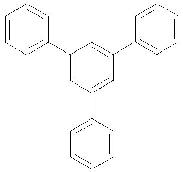 | 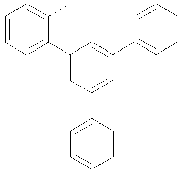 |
| Ar-32 | Ar-33 | Ar-34 |
| 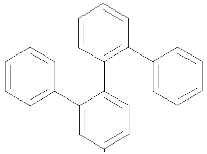 | 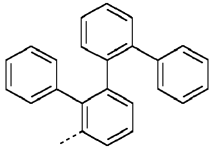 | 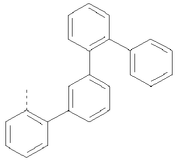 |
| Ar-35 | Ar-36 | Ar-37 |
| 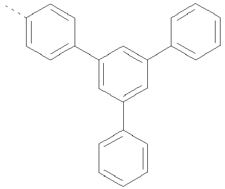 | 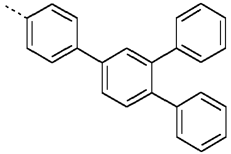 | 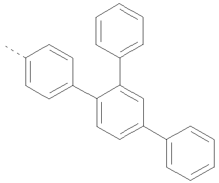 |
| Ar-38 | Ar-39 | Ar-40 |
| 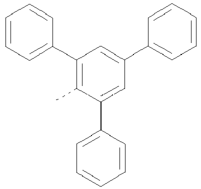 | 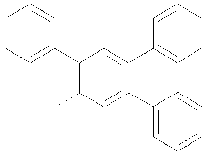 | 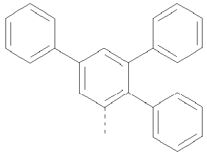 |
| Ar-41 | Ar-42 | Ar-43 |
| 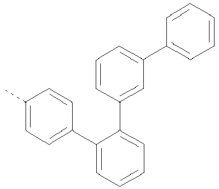 | 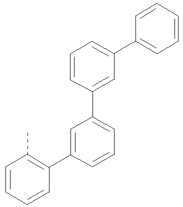 | 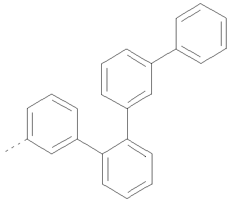 |
| Ar-44 | Ar-45 | Ar-46 |
| 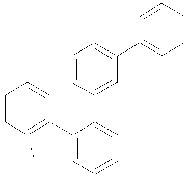 | | |
| | Ar-47 | |
| 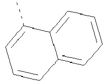 | 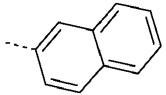 | 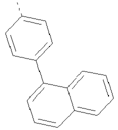 |
| Ar-48 | Ar-49 | Ar-50 |
| 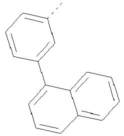 | 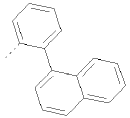 | 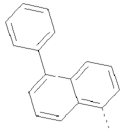 |
| Ar-51 | Ar-52 | Ar-53 |
| 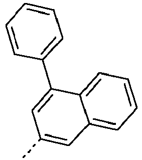 | 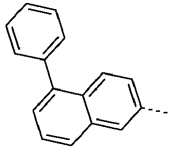 | 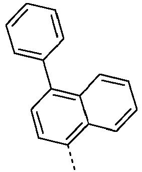 |
| Ar-54 | Ar-55 | Ar-56 |
| 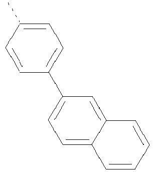 | 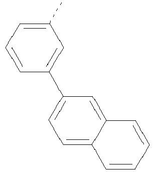 | 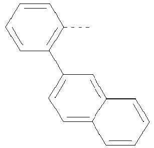 |
| Ar-57 | Ar-58 | Ar-59 |
| 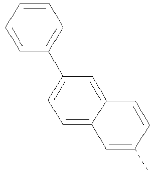 | 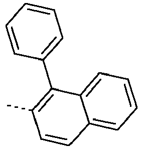 | 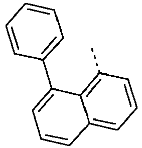 |
| Ar-60 | Ar-61 | Ar-62 |
| 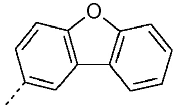 | 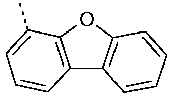 | 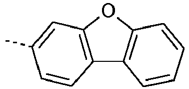 |
| Ar-63 | Ar-64 | Ar-65 |
| 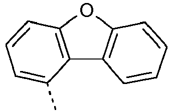 | 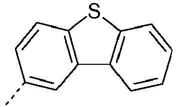 | 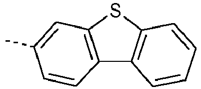 |
| Ar-66 | Ar-67 | Ar-68 |
| 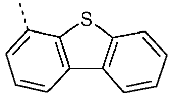 | 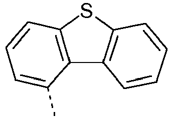 | 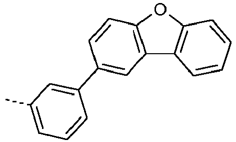 |
| Ar-69 | Ar-70 | Ar-71 |
| 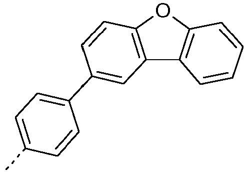 | 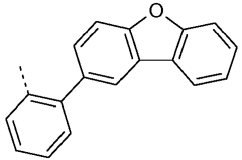 | 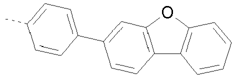 |
| Ar-72 | Ar-73 | Ar-74 |
| 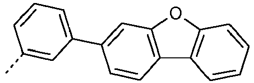 | 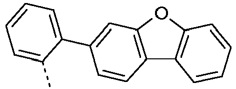 | 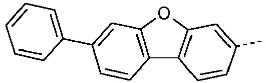 |
| Ar-75 | Ar-76 | Ar-77 |
| 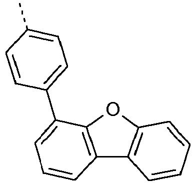 | 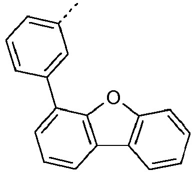 | 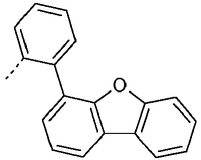 |
| Ar-78 | Ar-79 | Ar-80 |
| 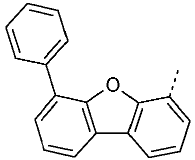 | 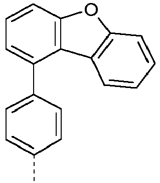 | 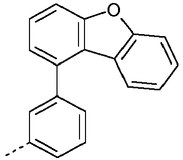 |
| Ar-81 | Ar-82 | Ar-83 |
| 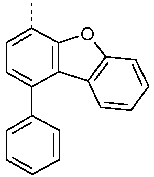 | 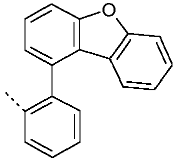 | 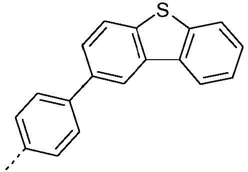 |
| Ar-84 | Ar-85 | Ar-86 |
| 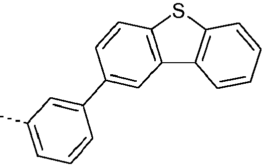 | 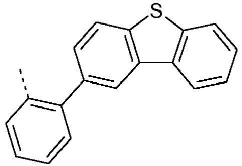 | 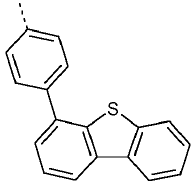 |
| Ar-87 | Ar-88 | Ar-89 |
| 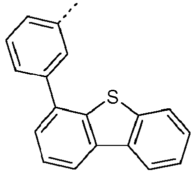 | 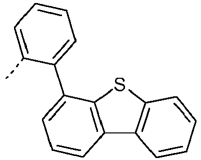 | 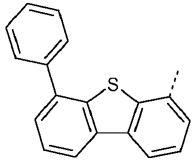 |
| Ar-90 | Ar-91 | Ar-92 |
| 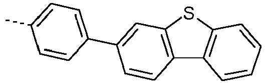 | 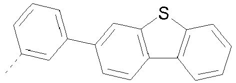 | 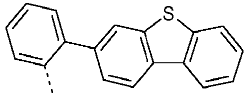 |
| Ar-93 | Ar-94 | Ar-95 |
| 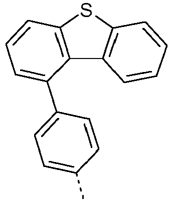 | 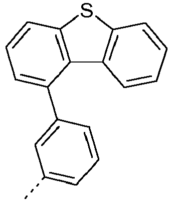 | 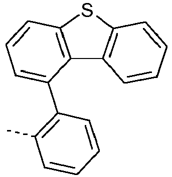 |
| Ar-96 | Ar-97 | Ar-98 |
| 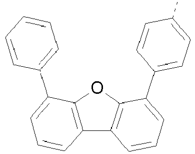 | 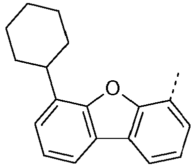 | 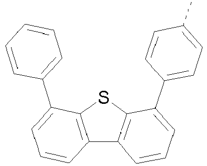 |
| Ar-99 | Ar-100 | Ar-101 |
| 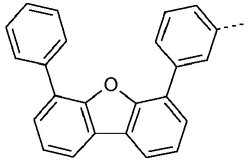 | 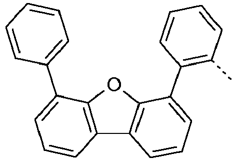 | 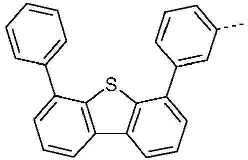 |
| Ar-102 | Ar-103 | Ar-104 |
| 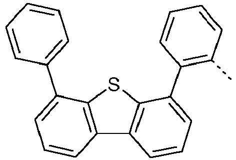 | | |
| Ar-105 | | |
| 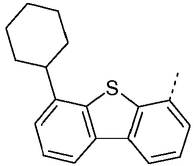 | 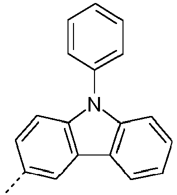 | 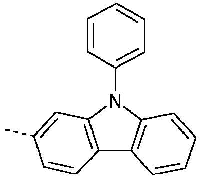 |
| Ar-106 | Ar-107 | Ar-108 |
| 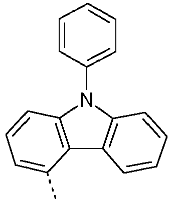 | 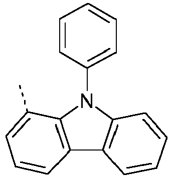 | 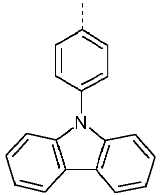 |
| Ar-109 | Ar-110 | Ar-111 |
| 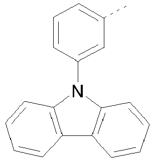 | 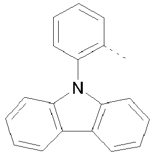 | |
| Ar-112 | Ar-113 | |
| 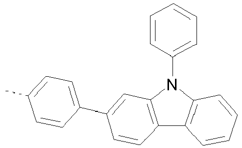 | 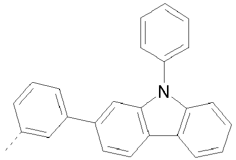 | 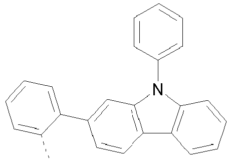 |
| Ar-114 | Ar-115 | Ar-116 |
| 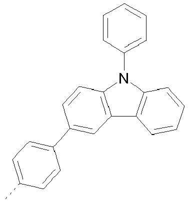 | 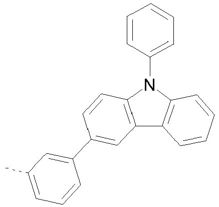 | 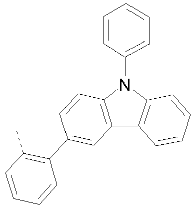 |
| Ar-117 | Ar-118 | Ar-119 |
| 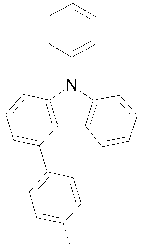 | 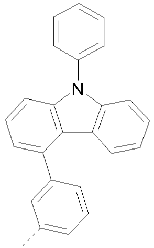 | 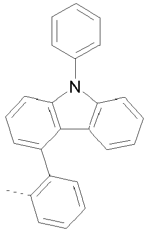 |
| Ar-120 | Ar-121 | Ar-122 |
| 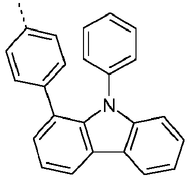 | 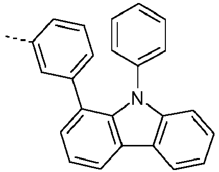 | 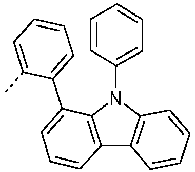 |
| Ar-123 | Ar-124 | Ar-125 |
| 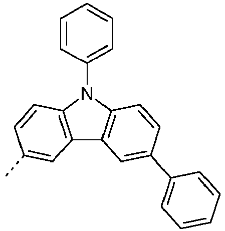 | 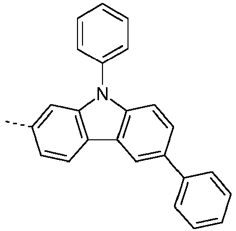 | 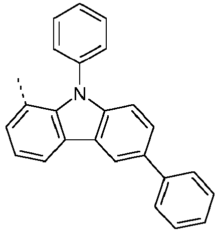 |
| Ar-126 | Ar-127 | Ar-128 |
| 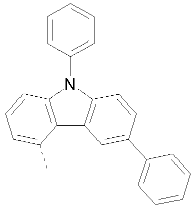 | 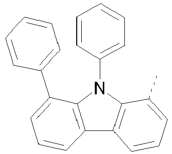 | 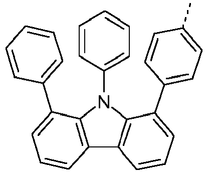 |
| Ar-129 | Ar-130 | Ar-131 |
| 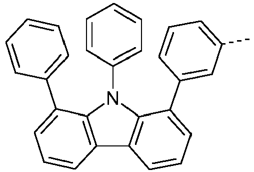 | 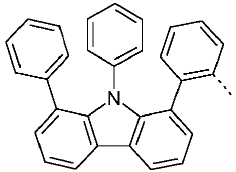 | |
| Ar-132 | Ar-133 | |
| 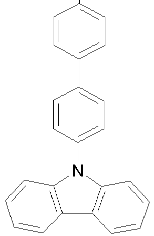 | 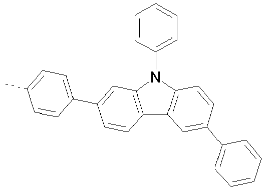 | 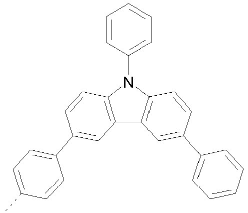 |
| Ar-134 | Ar-135 | Ar-136 |
| 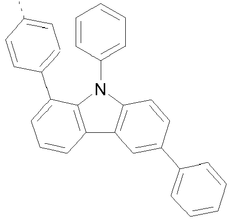 | 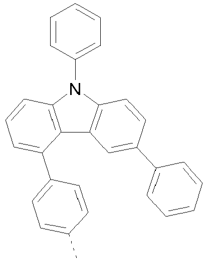 | 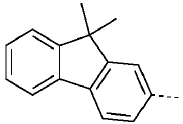 |
| Ar-137 | Ar-138 | Ar-139 |
| 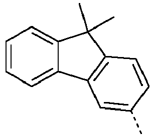 | 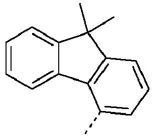 | 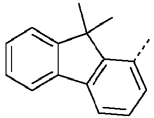 |
| Ar-140 | Ar-141 | Ar-142 |
| 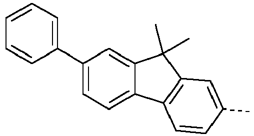 | 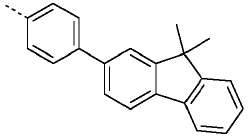 | 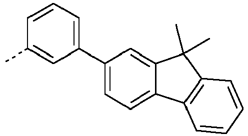 |
| Ar-143 | Ar-144 | Ar-145 |
| 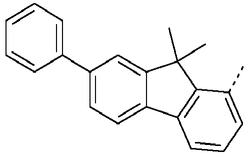 | 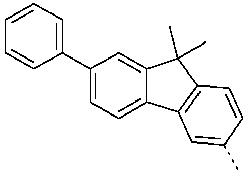 | 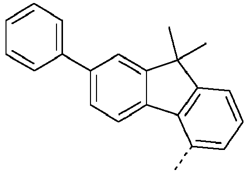 |
| Ar-146 | Ar-147 | Ar-148 |
| 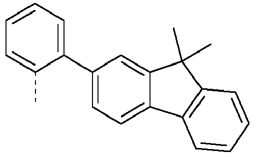 | 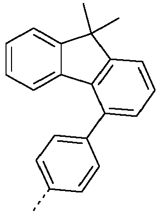 | 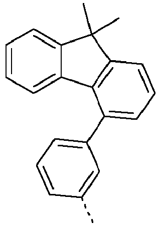 |
| Ar-149 | Ar-150 | Ar-151 |
| 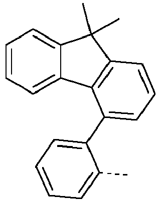 | 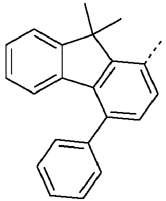 | 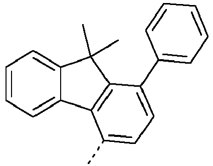 |
| Ar-152 | Ar-153 | Ar-154 |
| 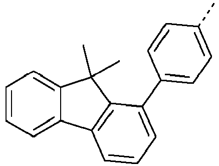 | 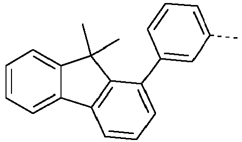 | 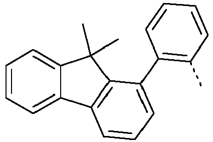 |
| Ar-155 | Ar-156 | Ar-157 |
| 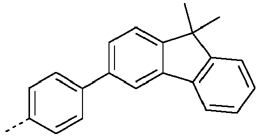 | 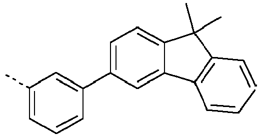 | 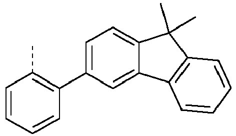 |
| Ar-158 | Ar-159 | Ar-160 |
| 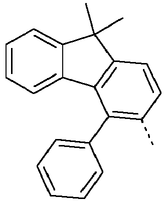 | 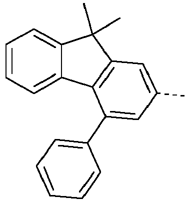 | 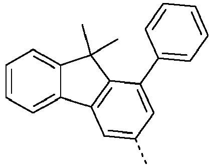 |
| Ar-161 | Ar-162 | Ar-163 |
| 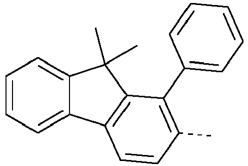 | 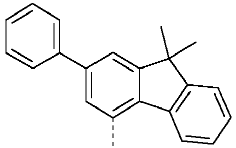 | 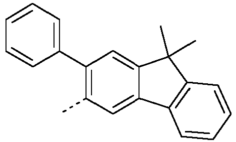 |
| Ar-164 | Ar-165 | Ar-166 |
| 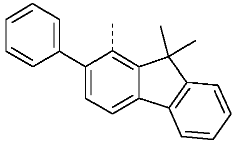 | 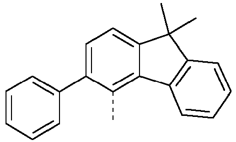 | 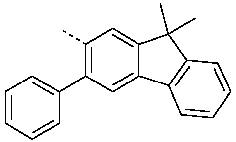 |
| Ar-167 | Ar-168 | Ar-169 |
| 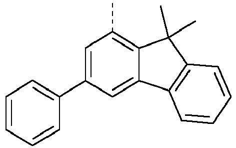 | 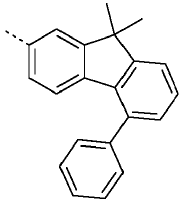 | 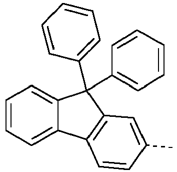 |
| Ar-170 | Ar-171 | Ar-172 |
| 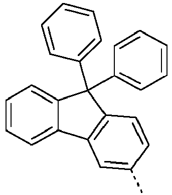 | 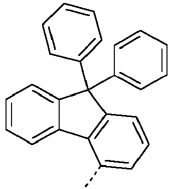 | 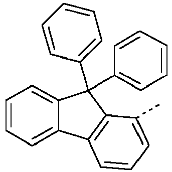 |
| Ar-173 | Ar-174 | Ar-175 |
| 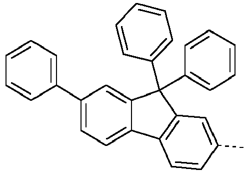 | 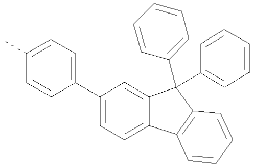 | 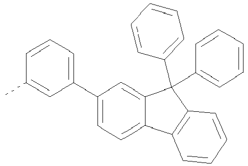 |
| Ar-176 | Ar-177 | Ar-178 |
| 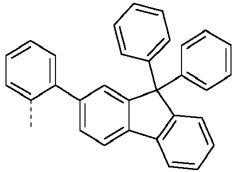 | 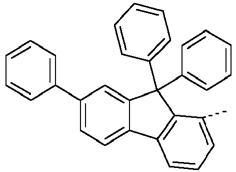 | 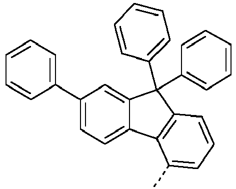 |
| Ar-179 | Ar-180 | Ar-181 |
| 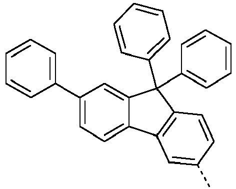 | 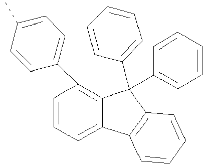 | 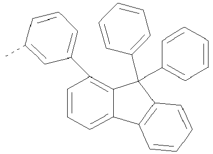 |
| Ar-182 | Ar-183 | Ar-184 |
| 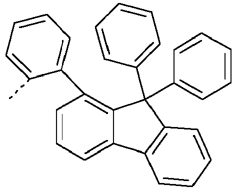 | 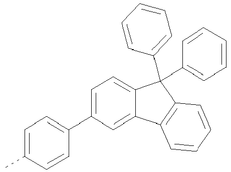 | 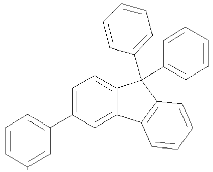 |
| Ar-185 | Ar-186 | Ar-187 |
| 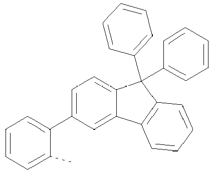 | 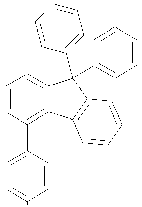 | 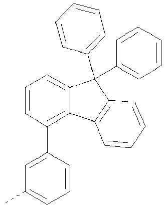 |
| Ar-188 | Ar-189 | Ar-190 |
| 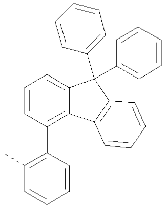 | | |
| Ar-191 | | |
| 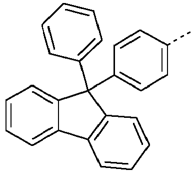 | 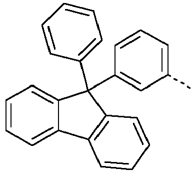 | 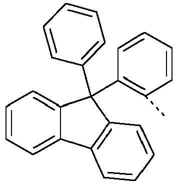 |
| Ar-192 | Ar-193 | Ar-194 |
| 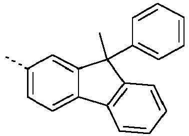 | 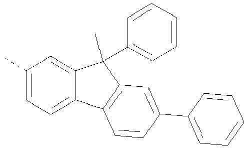 | 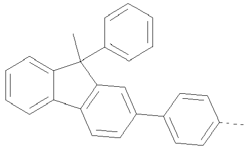 |
| Ar-195 | Ar-196 | Ar-197 |
| 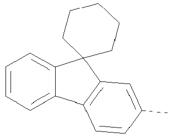 | 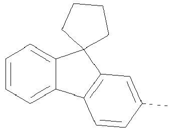 | 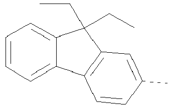 |
| Ar-198 | Ar-199 | Ar-200 |
| 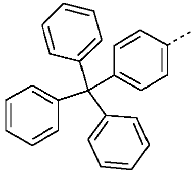 | 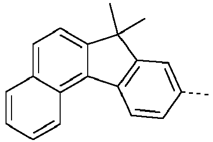 | 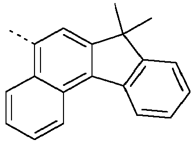 |
| Ar-201 | Ar-202 | Ar-203 |
| 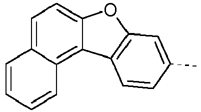 | 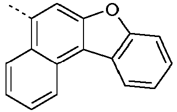 | 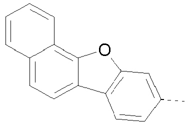 |
| Ar-204 | Ar-205 | Ar-206 |
| 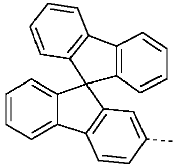 | 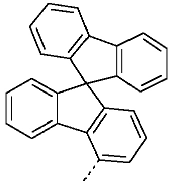 | 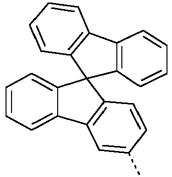 |
| Ar-207 | Ar-208 | Ar-209 |
| 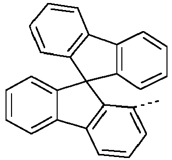 | 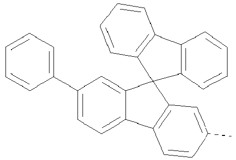 | 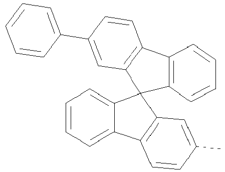 |
| Ar-210 | Ar-211 | Ar-212 |
| 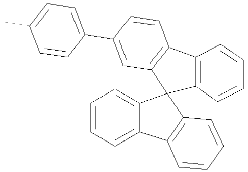 | 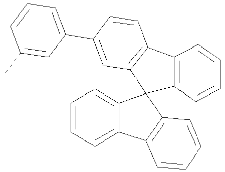 | 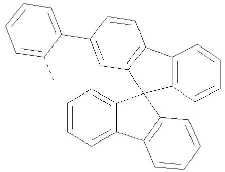 |
| Ar-213 | Ar-214 | Ar-215 |
| 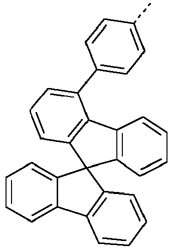 | 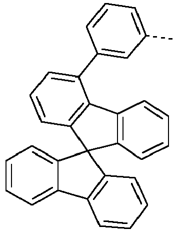 | 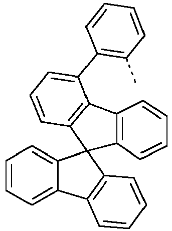 |
| Ar-216 | Ar-217 | Ar-218 |
| 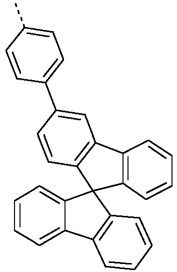 | 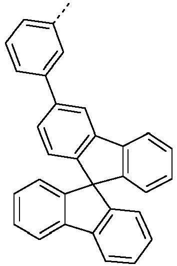 | 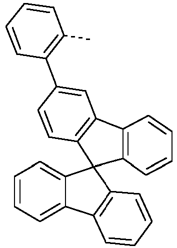 |
| Ar-219 | Ar-220 | Ar-221 |
| 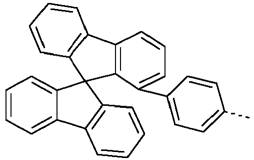 | 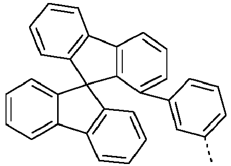 | 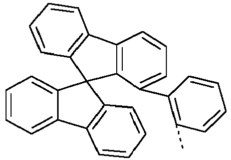 |
| Ar-222 | Ar-223 | Ar-224 |
| 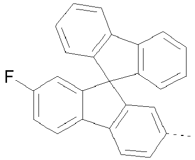 | 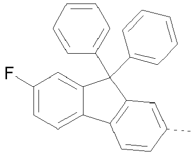 | 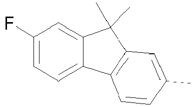 |
| Ar-225 | Ar-226 | Ar-227 |
| 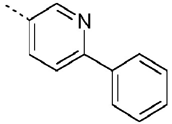 | 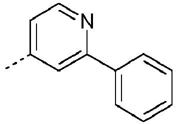 | 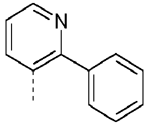 |
| Ar-228 | Ar-229 | Ar-230 |
| 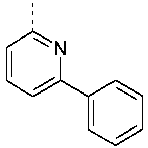 | 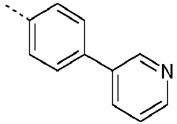 | 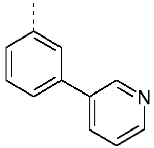 |
| Ar-231 | Ar-232 | Ar-233 |
| 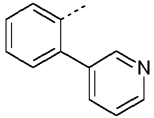 | 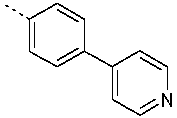 | 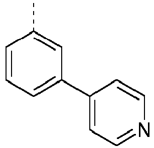 |
| Ar-234 | Ar-235 | Ar-236 |
| 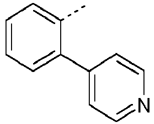 | 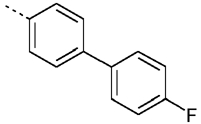 | 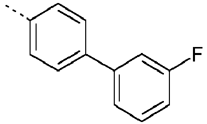 |
| Ar-237 | Ar-238 | Ar-239 |
| 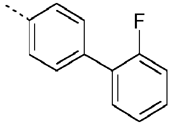 | 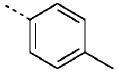 | 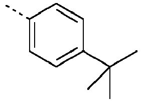 |
| Ar-240 | Ar-241 | Ar-242 |
| 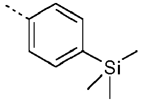 | 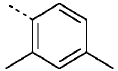 | 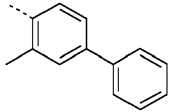 |
| Ar-243 | Ar-244 | Ar-245 |
| 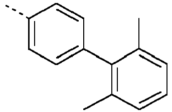 | 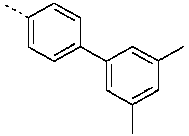 | 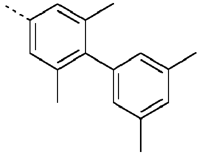 |
| Ar-246 | Ar-247 | Ar-248 |
| 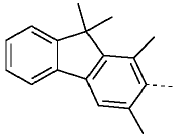 | 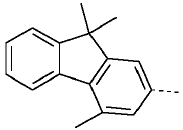 | 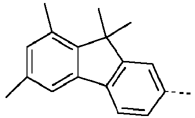 |
| Ar-250 | Ar-251 | Ar-252 |
| 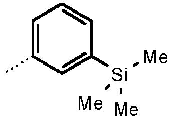 | 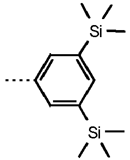 | 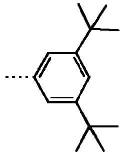 |
| Ar-253 | Ar-254 | Ar-255 |
| 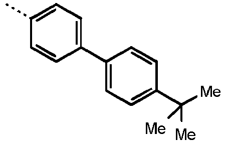 | 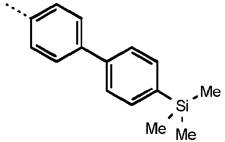 | 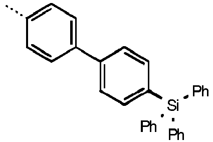 |
| Ar-256 | Ar-257 | Ar-258 |
| 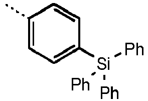 | 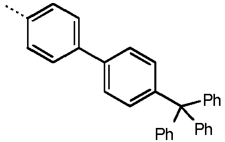 | |
| Ar-259 | Ar-260 | |

wobei die Gruppen an allen freien Positionen jeweils mit einem Rest R⁵ substituiert sein können, und wobei die gestrichelte Bindung die Bindung an das Stickstoffatom in Formel (N) darstellt.

Es ist bevorzugt, dass an ein Stickstoffatom in Formel (N) jeweils zwei unterschiedliche Gruppen Ar² gebunden sind.

Die Gruppe E ist bevorzugt eine Einfachbindung.

Bevorzugt ist m=0, so dass keine Gruppe E vorhanden ist.

Gemäß einer alternativen Ausführungsform, die ebenfalls bevorzugt ist, ist m=1, so dass die Gruppen Ar² miteinander über eine Gruppe E verbunden sind. In diesem Fall ist es bevorzugt, dass die Gruppen Ar² gewählt sind aus Phenyl und Fluorenyl, welche jeweils mit einem oder mehreren Resten R⁵ substituiert sein können. Weiterhin ist es in diesem Fall bevorzugt, dass die Gruppe E, welche die beiden Gruppen Ar² miteinander verbindet, an den betreffenden Gruppen Ar² in ortho-Position zur Bindung der Gruppe Ar² an die Aminogruppe in Formel (N) gebunden ist. Weiterhin ist es bevorzugt, dass die Gruppe E mit den Gruppen Ar² einen Sechsring bildet, falls E gewählt ist aus C(R⁵)₂, Si(R⁵)₂, NR⁵, O and S; und einen Fünfring bildet, falls E eine Einfachbindung ist.

Bevorzugte Ausführungsformen der Einheit 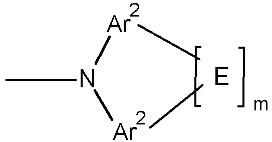 aus der Gruppe der Formel (N) für m=1 sind die im Folgenden abgebildeten Gruppen:

| | | |
|---|---|---|
| 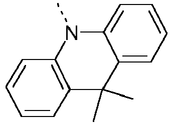 | 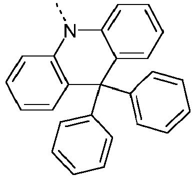 | 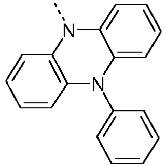 |
| N-1 | N-2 | N-3 |
| 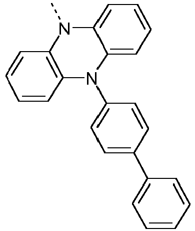 | 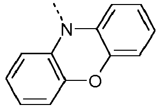 | 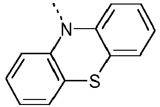 |
| N-4 | N-5 | N-6 |
| 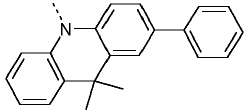 | 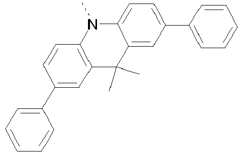 | 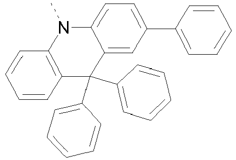 |
| N-7 | N-8 | N-9 |
| 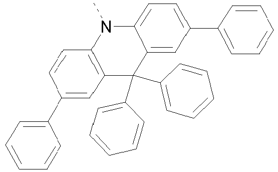 | 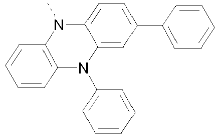 | 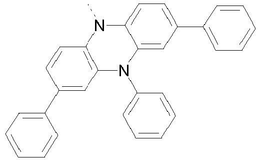 |
| N-10 | N-11 | N-12 |
| 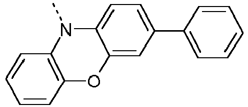 | 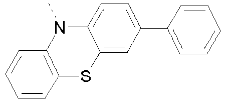 | 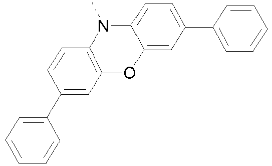 |
| N-13 | N-14 | N-15 |
| 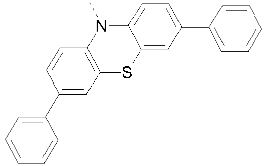 | 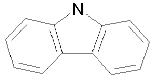 | 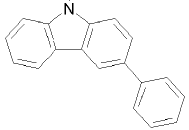 |
| N-16 | N-17 | N-18 |
| 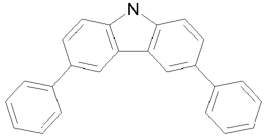 | 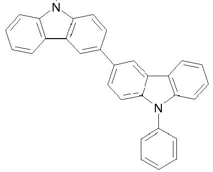 | 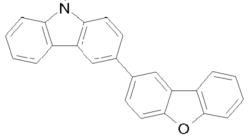 |
| N-19 | N-20 | N-21 |
| 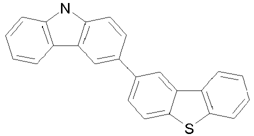 | 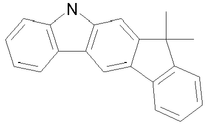 | 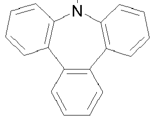 |
| N-22 | N-23 | N-24 |
| 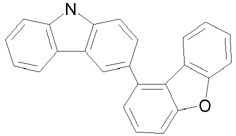 | 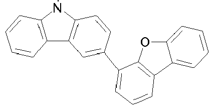 | 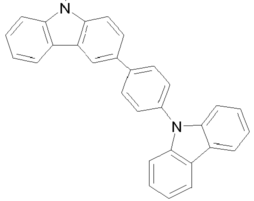 |
| N-25 | N-26 | N-27 |
| 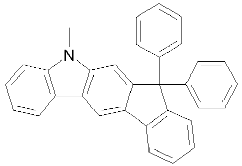 | 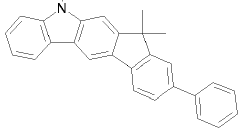 | 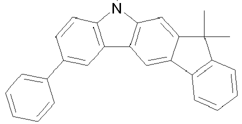 |
| N-28 | N-29 | N-30 |
| 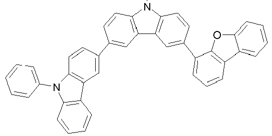 | 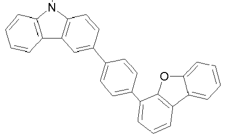 | |
| N-31 | N-32 | |

wobei die Gruppen an ihren freien Positionen jeweils mit einem Rest R⁵ substituiert sein können, und bevorzugt in den freien Positionen unsubstituiert sind, und wobei die gestrichelten Bindungen die Bindungen an den Rest der Formel darstellen.

Bevorzugte Ausführungsformen der Gruppe der Formel (N) für m=0 sind die im Folgenden abgebildeten Gruppen:

| | |
|---|---|
| 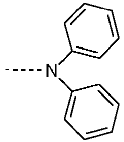 | 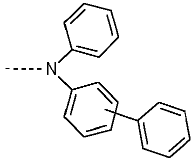 |
| A-1 | A-2 |
| 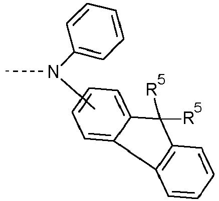 | 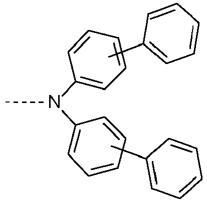 |
| A-3 | A-4 |
| 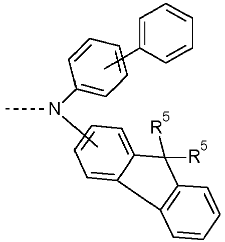 | 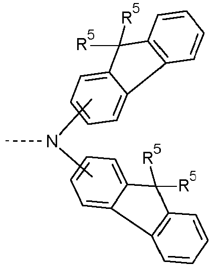 |
| A-5 | A-6 |
| 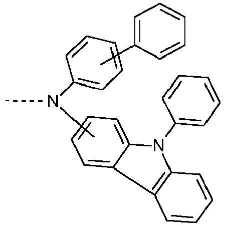 | 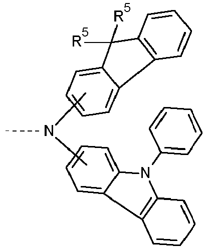 |
| A-7 | A-8 |
| 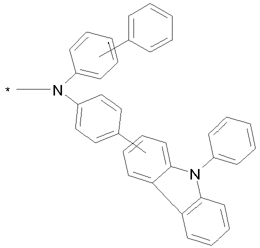 | 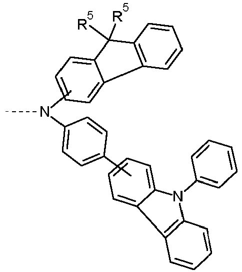 |
| A-9 | A-10 |
| 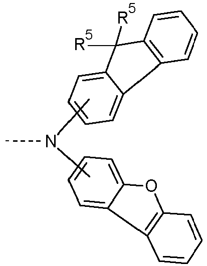 | 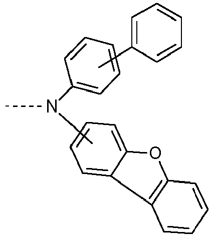 |
| A-11 | A-12 |
| 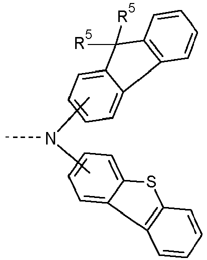 | 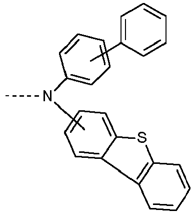 |
| A-13 | A-14 |
| 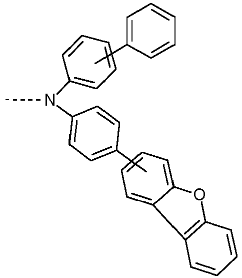 | 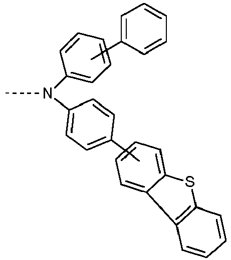 |
| A-15 | A-16 |
| 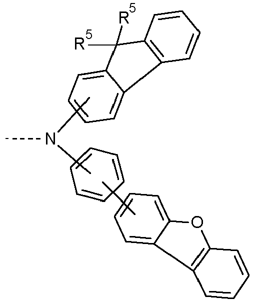 | 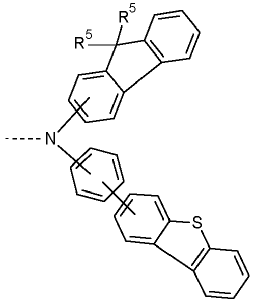 |
| A-17 | A-18 |
| 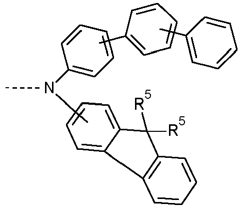 | 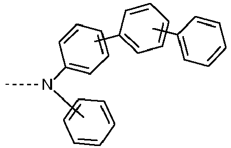 |
| A-19 | A-20 |
| 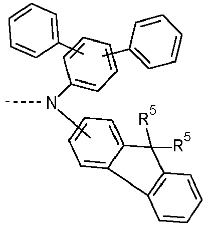 | 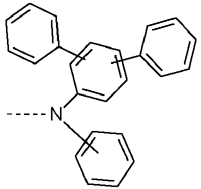 |
| A-21 | A-22 |
| 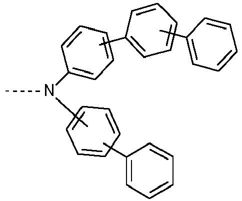 | 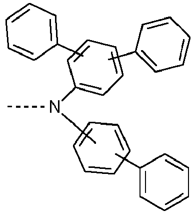 |
| A-23 | A-24 |
| 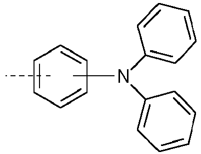 | 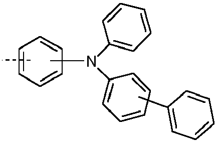 |
| A-25 | A-26 |
| 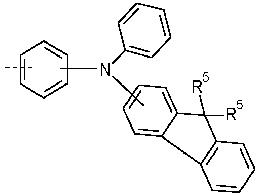 | 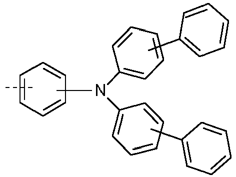 |
| A-27 | A-28 |
| 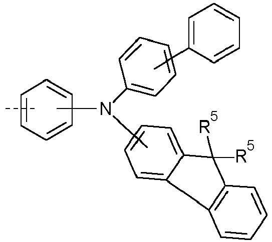 | 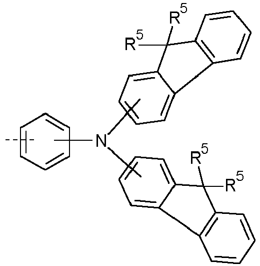 |
| A-29 | A-30 |
| 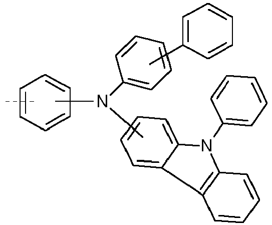 | 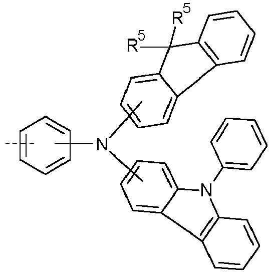 |
| A-31 | A-32 |
| 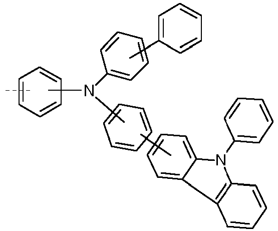 | 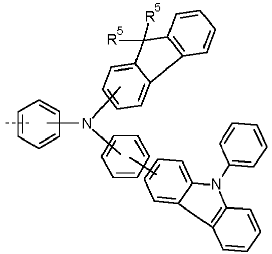 |
| A-33 | A-34 |
| 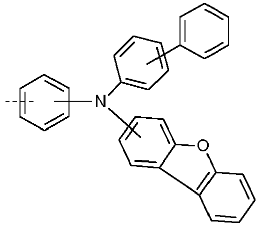 | 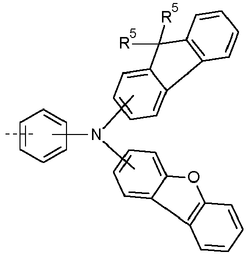 |
| A-35 | A-36 |
| 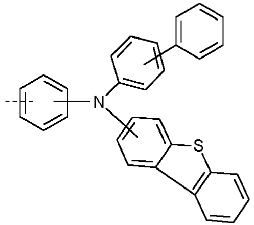 | 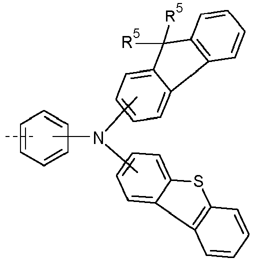 |
| A-37 | A-38 |
| 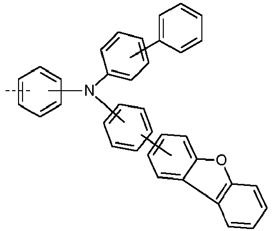 | 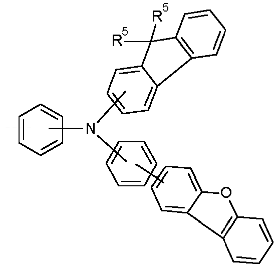 |
| A-39 | A-40 |
| 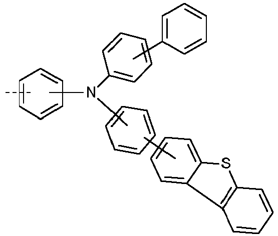 | 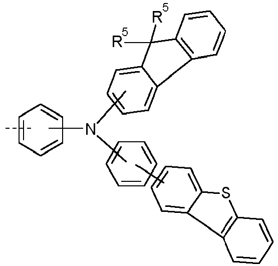 |
| A-41 | A-42 |
| 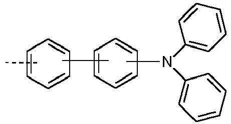 | 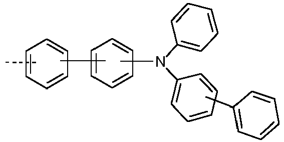 |
| A-43 | A-44 |
| 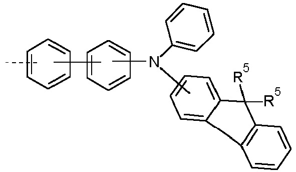 | 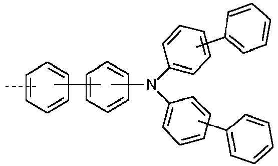 |
| A-45 | A-46 |
| 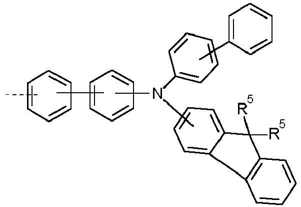 | 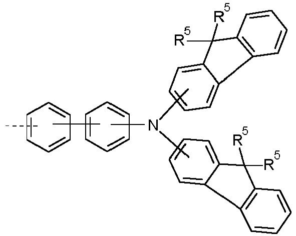 |
| A-47 | A-48 |

wobei die Gruppen an ihren freien Positionen jeweils mit einem Rest R⁵ substituiert sein können, und bevorzugt in den freien Positionen unsubstituiert sind, und wobei die gestrichelten Bindungen die Bindungen an den Rest der Formel darstellen.

R² ist bevorzugt gewählt aus H, D, F, CN, geradkettigen Alkyl- oder Alkoxygruppen mit 1 bis 10 C-Atomen, verzweigten oder cyclischen Alkyl- oder Alkoxygruppen mit 3 bis 10 C-Atomen, Alkenyl- oder Alkinylgruppen mit 2 bis 10 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁷ substituiert sein können. Besonders bevorzugt ist R² gewählt aus H, F, CN, geradkettigen Alkylgruppen mit 1 bis 10 C-Atomen, verzweigten oder cyclischen Alkylgruppen mit 3 bis 10 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen und die genannten aromatischen Ringsysteme und heteroaromatischen Ringsysteme jeweils mit einem oder mehreren Resten R⁷ substituiert sein können. Ganz besonders bevorzugt ist R² gleich H.

R³ ist bevorzugt bei jedem Auftreten gleich oder verschieden gewählt aus H, D, F, CN, Si(R⁷)₃, N(R⁷)₂, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen, die genannten aromatischen Ringsysteme und die genannten heteroaromatischen Ringsysteme jeweils mit einem oder mehreren Resten R⁷ substituiert sein können.

R⁴ ist bevorzugt bei jedem Auftreten gleich oder verschieden gewählt aus H, D, F, CN, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen, die genannten aromatischen Ringsysteme und die genannten heteroaromatischen Ringsysteme jeweils mit einem oder mehreren Resten R⁷ substituiert sein können. Besonders bevorzugt ist R⁴ bei jedem Auftreten gleich oder verschieden gewählt aus geradkettigen Alkylgruppen mit 1 bis 20 C-Atomen, verzweigten oder cyclischen Alkylgruppen mit 3 bis 20 C-Atomen, und aromatischen Ringsystemen mit 6 bis 40 aromatischen Ringatomen, wobei die genannten Alkylgruppen und die genannten aromatischen Ringsysteme jeweils mit einem oder mehreren Resten R⁷ substituiert sein können.

R⁵ ist bevorzugt bei jedem Auftreten gleich oder verschieden gewählt aus H, D, F, CN, Si(R⁷)₃, N(R⁷)₂, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen, die genannten aromatischen Ringsysteme und die genannten heteroaromatischen Ringsysteme jeweils mit einem oder mehreren Resten R⁷ substituiert sein können.

R⁶ ist bevorzugt bei jedem Auftreten gleich oder verschieden gewählt aus H, D, F, CN, Si(R⁷)₃, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen, die genannten aromatischen Ringsysteme und die genannten heteroaromatischen Ringsysteme jeweils mit einem oder mehreren Resten R⁷ substituiert sein können. Besonders bevorzugt ist R⁶ gleich H.

R⁷ ist bevorzugt bei jedem Auftreten gleich oder verschieden gewählt aus H, D, F, CN, Si(R⁸)₃, N(R⁸)₂, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen, die genannten aromatischen Ringsysteme und die genannten heteroaromatischen Ringsysteme jeweils mit einem oder mehreren Resten R⁸ substituiert sein können.

Index i ist bevorzugt gleich 0.

Bevorzugte Ausführungsformen der Formel (I) entsprechen einer der folgenden Formeln (1-1) bis (I-9)

| |
|---|
| 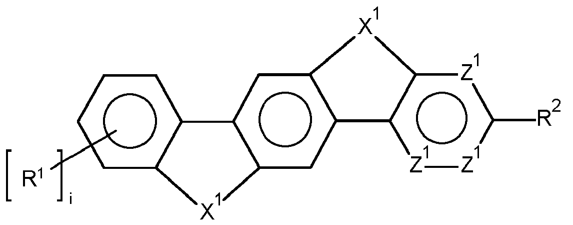 |
| Formel (I-1) |
| 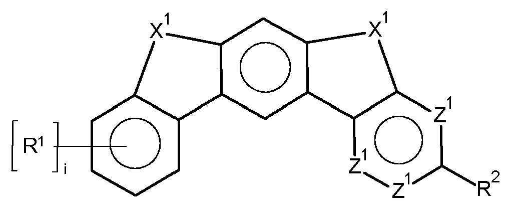 |
| Formel (I-2) |
| 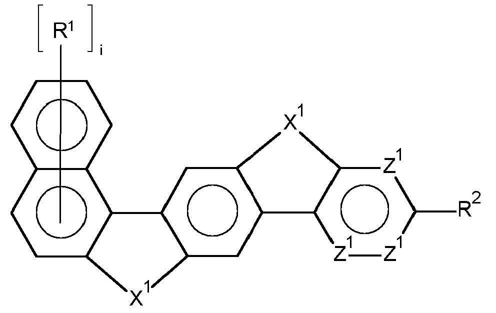 |
| Formel (I-3) |
| 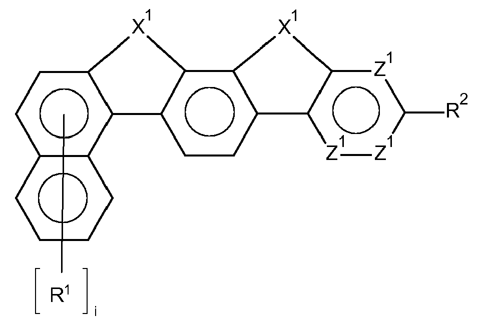 |
| Formel (I-4) |
| 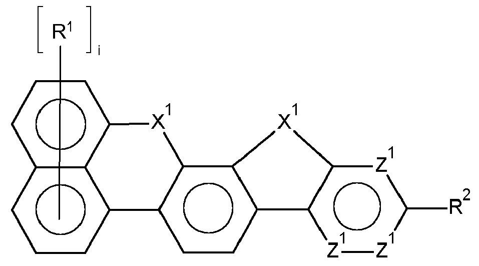 |
| Formel (I-5) |
| 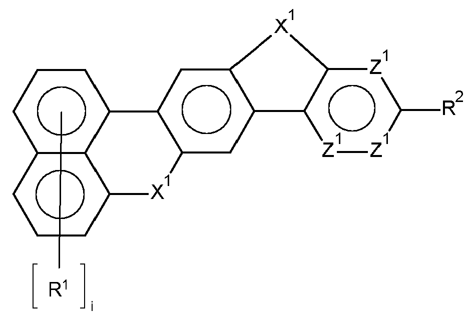 |
| Formel (I-6) |
| 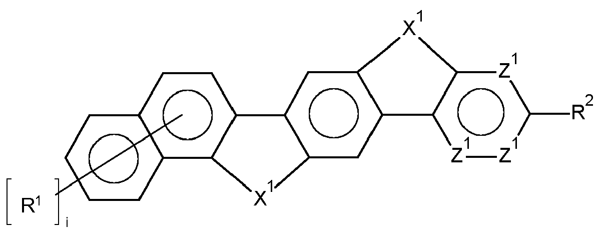 |
| Formel (I-7) |
| 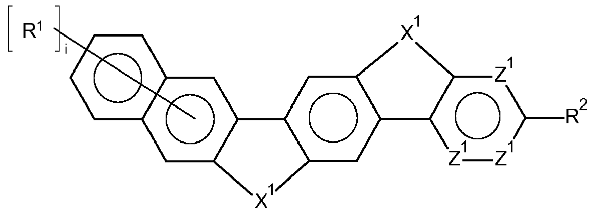 |
| Formel (I-8) |
| 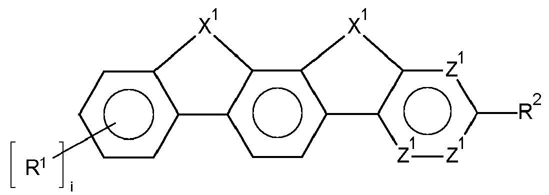 |
| Formel (I-9) |

wobei die auftretenden Variablen wie oben definiert sind, und bevorzugt X¹ gleich C(R⁴)₂ ist, und wobei die Verbindungen an den freien Positionen an den aromatischen Ringen jeweils mit einem Rest R³ bzw. R⁶ substituiert sein können, und bevorzugt an diesen Positionen unsubstituiert sind.

Bevorzugte Ausführungsformen der Formeln (I-1) bis (I-9) entsprechen den unten gezeigten Formeln

| |
|---|
| 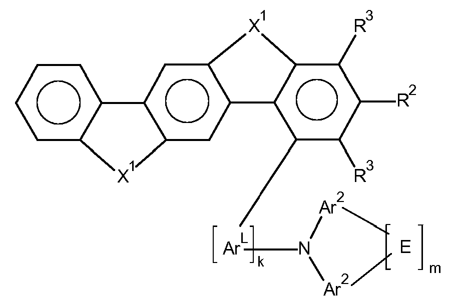 |
| Formel (I-1-A) |
| 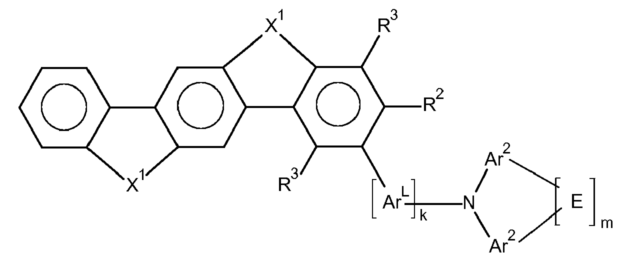 |
| Formel (I-1-B) |
| 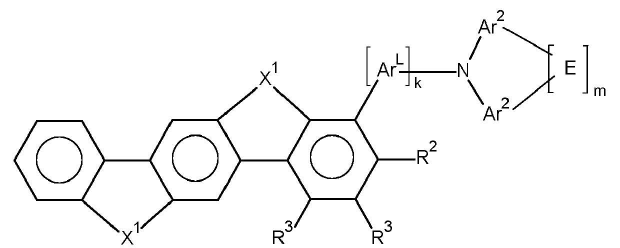 |
| Formel (I-1-A) |
| 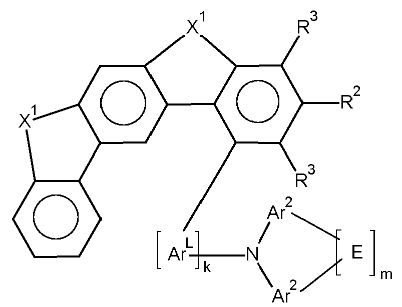 |
| Formel (I-2-A) |
| 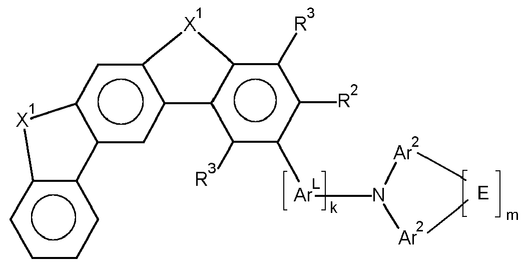 |
| Formel (I-2-B) |
| 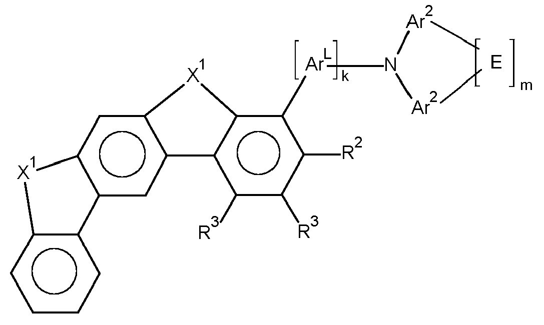 |
| Formel (I-2-C) |
| 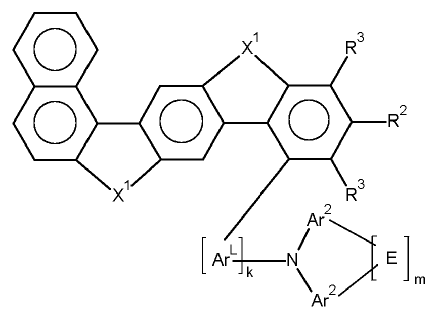 |
| Formel (I-3-A) |
| 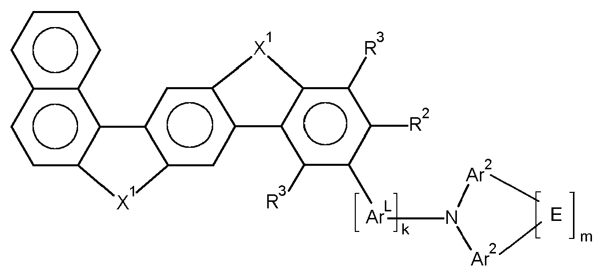 |
| Formel (I-3-B) |
| 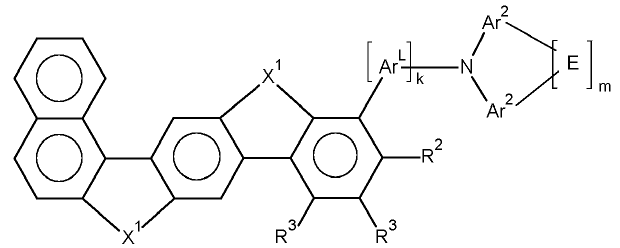 |
| Formel (I-3-C) |
| 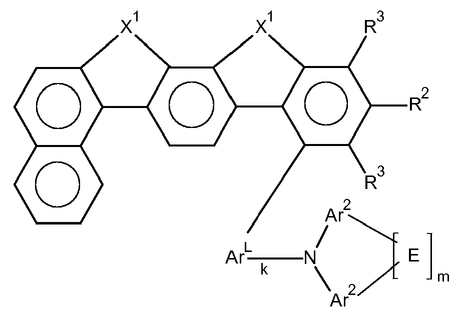 |
| Formel (I-4-A) |
| 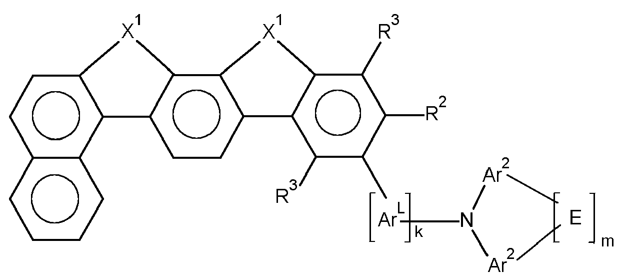 |
| Formel (I-4-B) |
| 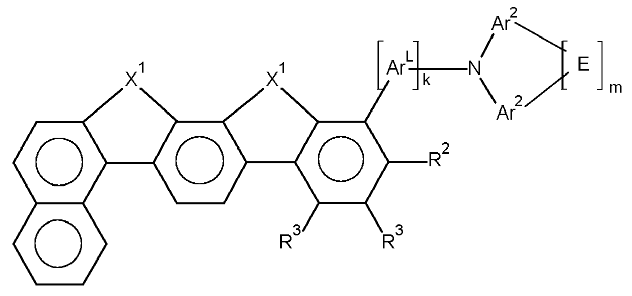 |
| Formel (I-4-C) |
| 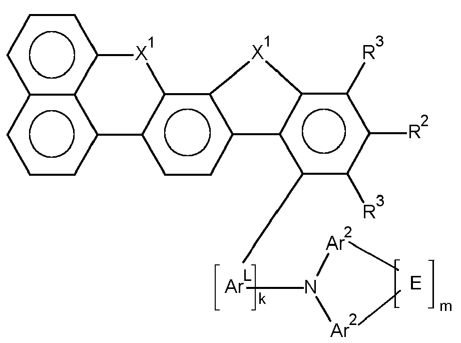 |
| Formel (I-5-A) |
| 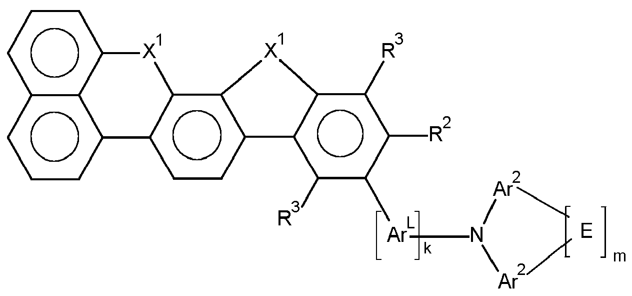 |
| Formel (I-5-B) |
| 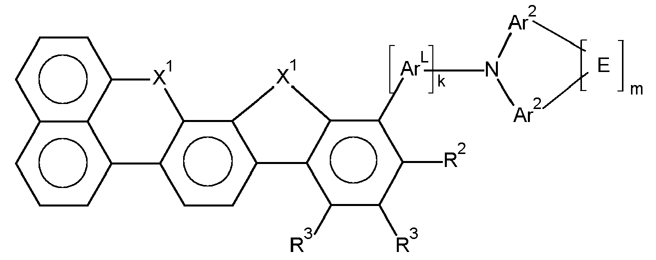 |
| Formel (I-5-C) |
| 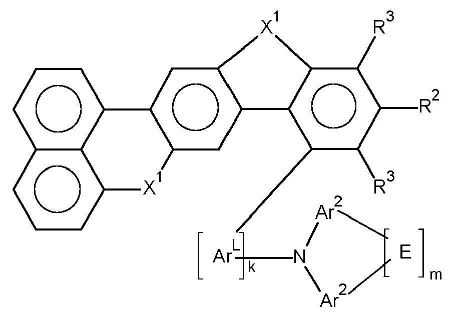 |
| Formel (I-6-A) |
| 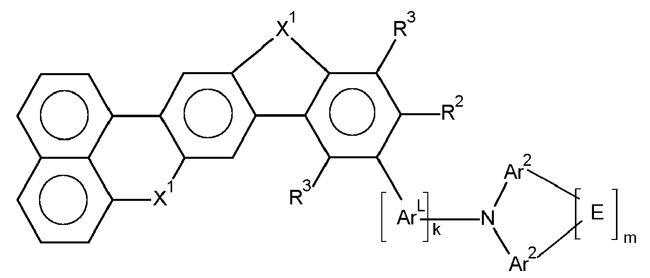 |
| Formel (I-6-B) |
| 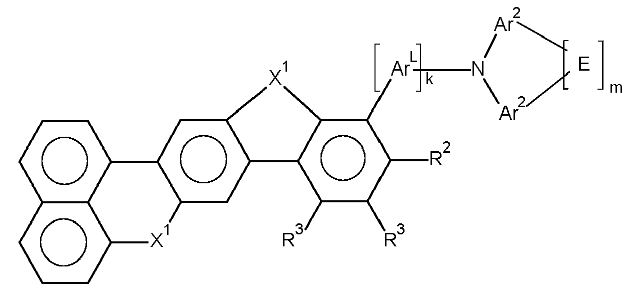 |
| Formel (I-6-C) |
| 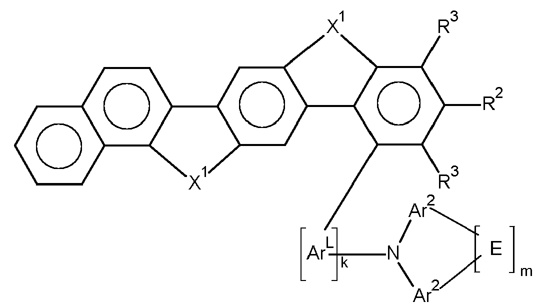 |
| Formel (I-7-A) |
| 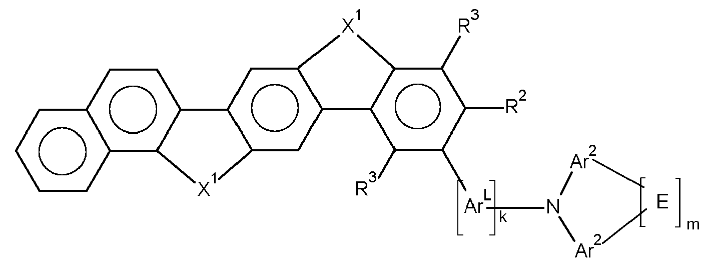 |
| Formel (I-7-B) |
| 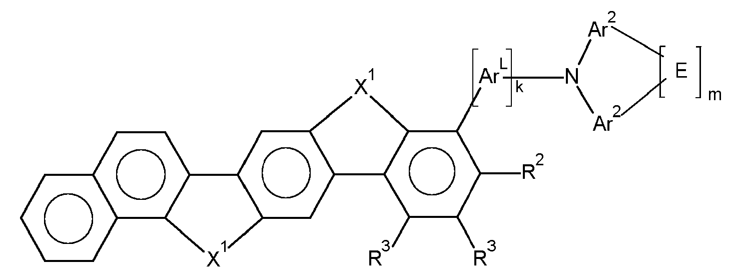 |
| Formel (I-7-C) |
| 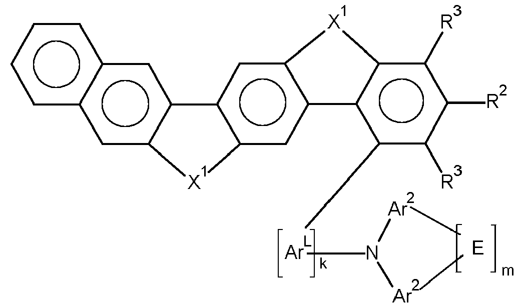 |
| Formel (I-8-A) |
| 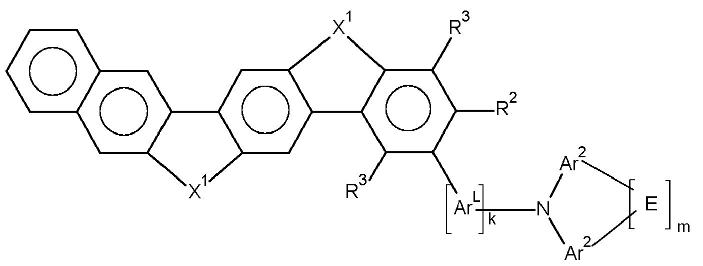 |
| Formel (I-8-B) |
| 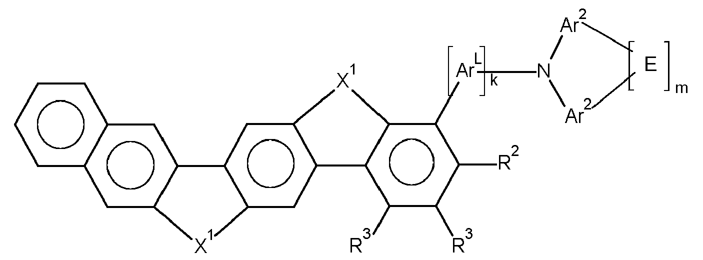 |
| Formel (I-8-C) |
| 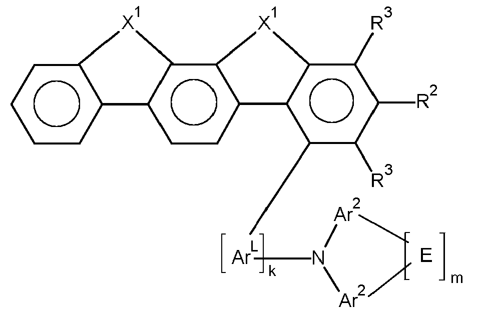 |
| Formel (I-9-A) |
| 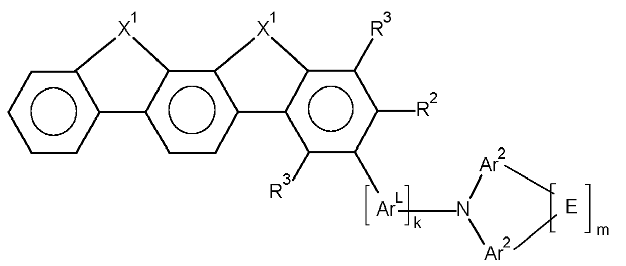 |
| Formel (I-9-B) |
| 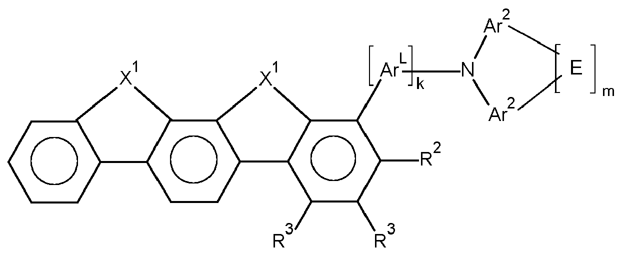 |
| Formel (I-9-C) |

wobei die auftretenden Variablen wie oben definiert sind, und bevorzugt X¹ gleich C(R⁴)₂ ist, und wobei die Verbindungen an den freien Positionen an den aromatischen Ringen jeweils mit einem Rest R³ bzw. R⁶ substituiert sein können, und bevorzugt an diesen Positionen unsubstituiert sind.

Bevorzugt unter den oben genannten Formeln sind die Formeln (1-1-A), (I-2-A), (I-3-A), (I-4-A), (I-5-A), (I-6-A), (I-7-A), (I-8-A) und (I-9-A). Besonders bevorzugt ist die Formel (I-1-A).

Weitere bevorzugte Ausführungsformen der Verbindungen der Formel (I) entsprechen den folgenden Formeln

| |
|---|
| 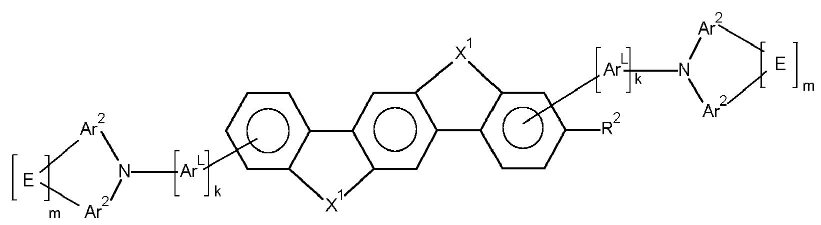 |
| Formel (I-1-D) |
| 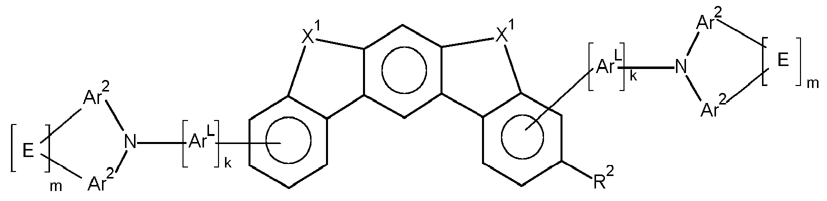 |
| Formel (I-2-D) |
| 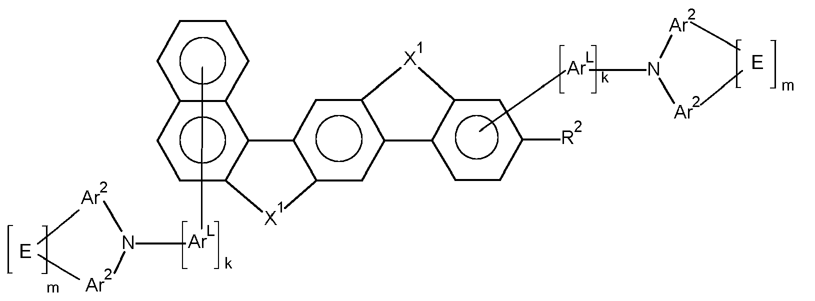 |
| Formel (I-3-D) |
| 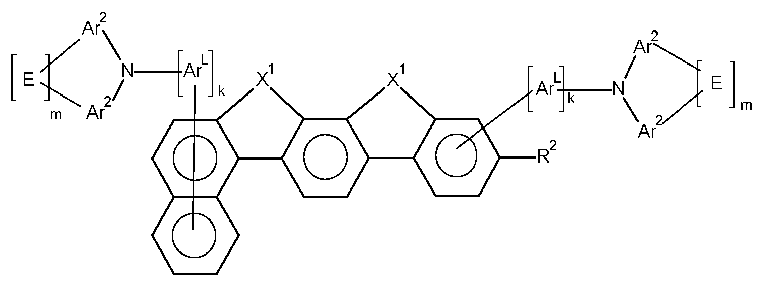 |
| Formel (I-4-D) |
| 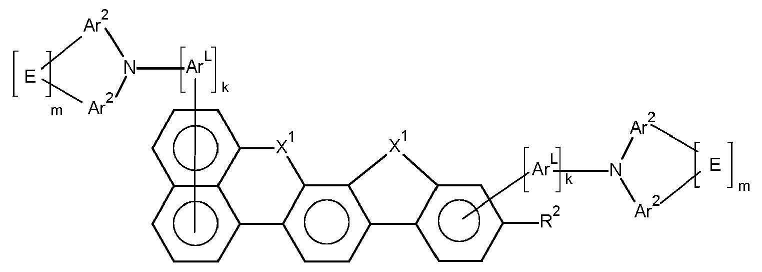 |
| Formel (I-5-D) |
| 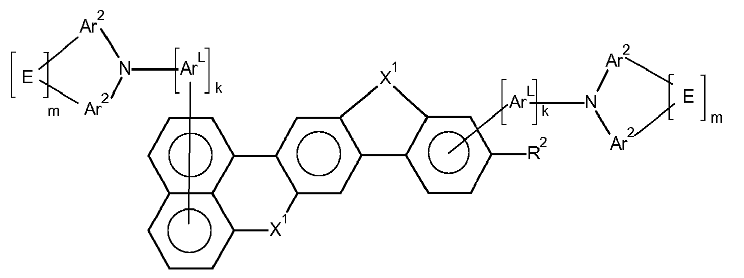 |
| Formel (I-6-D) |
| 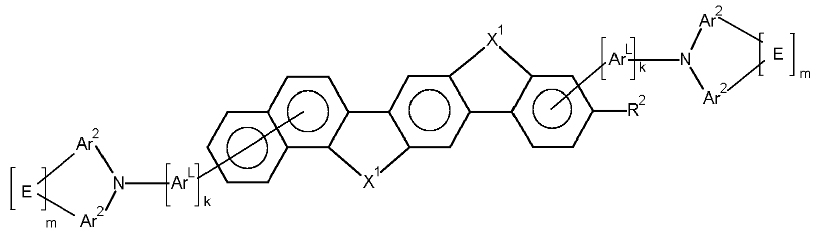 |
| Formel (I-7-D) |
| 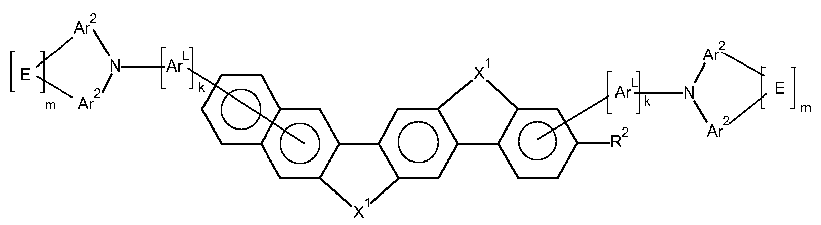 |
| Formel (I-8-D) |
| 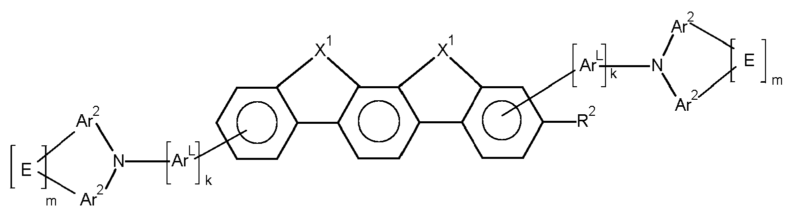 |
| Formel (I-9-D) |

wobei die auftretenden Variablen wie oben definiert sind, und bevorzugt X¹ gleich C(R⁴)₂ ist, und wobei die Verbindungen an den freien Positionen an den aromatischen Ringen jeweils mit einem Rest R³ bzw. R⁶ substituiert sein können, und bevorzugt an diesen Positionen unsubstituiert sind.

Bevorzugte Ausführungsformen der Formel (1-1-D) entsprechen den folgenden Formeln

| |
|---|
| 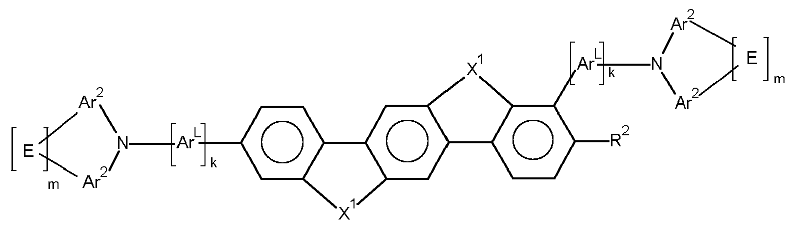 |
| Formel (I-1-D-1) |
| 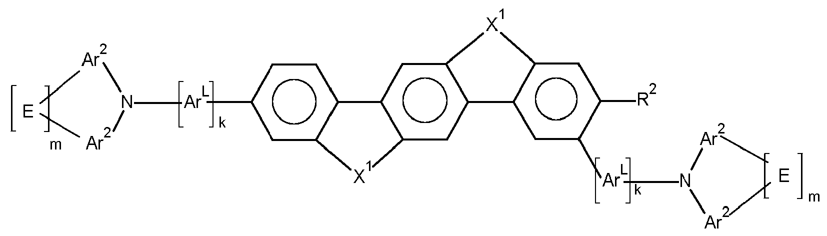 |
| Formel (I-1-D-2) |
| 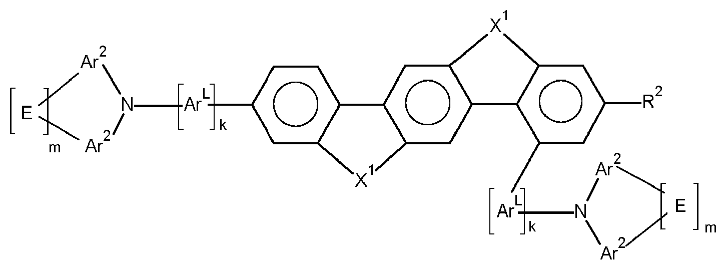 |
| Formel (I-1-D-3) |
| 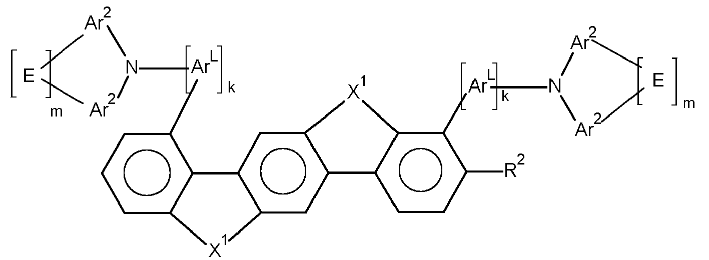 |
| Formel (I-1-D-4) |
| 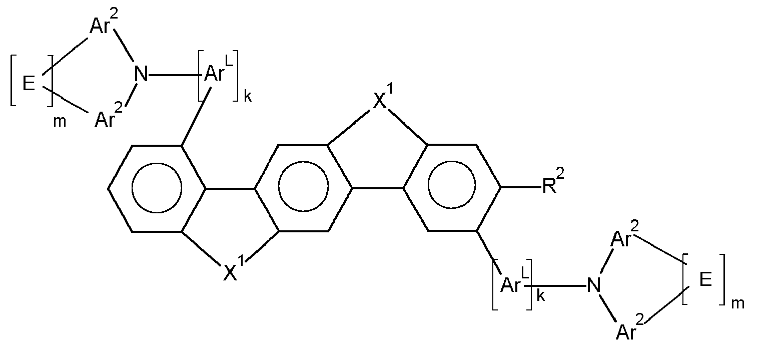 |
| Formel (I-1-D-5) |
| 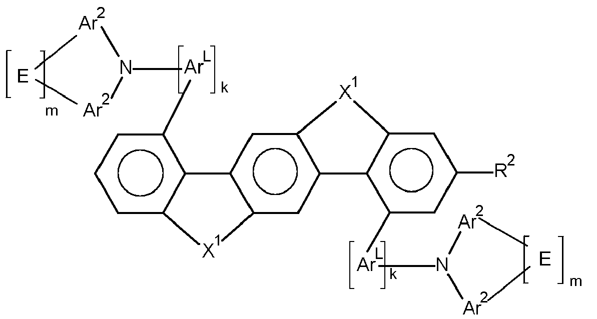 |
| Formel (I-1-D-6) |

wobei die auftretenden Variablen wie oben definiert sind, und bevorzugt X¹ gleich C(R⁴)₂ ist, und wobei die Verbindungen an den freien Positionen an den aromatischen Ringen jeweils mit einem Rest R³ bzw. R⁶ substituiert sein können, und bevorzugt an diesen Positionen unsubstituiert sind.

Weitere bevorzugte Ausführungsformen der Verbindungen der Formeln (I-1) bis (I-8) entsprechen den folgenden Formeln

| |
|---|
| 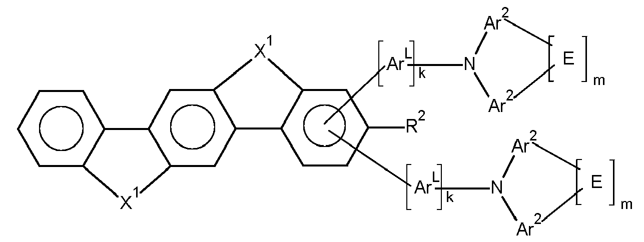 |
| Formel (I-1-E) |
| 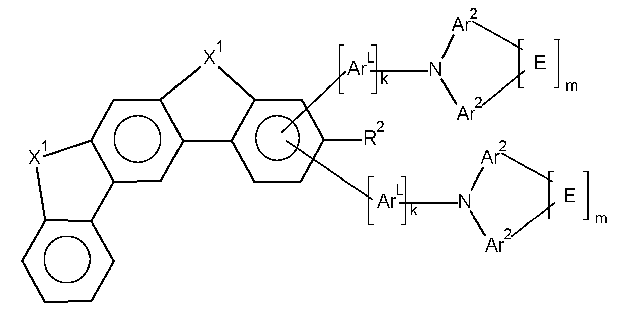 |
| Formel (I-2-E) |
| 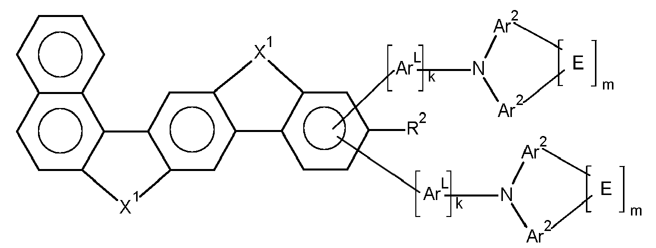 |
| Formel (I-3-E) |
| 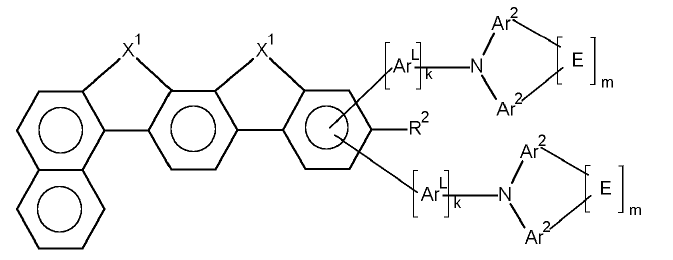 |
| Formel (I-4-E) |
| 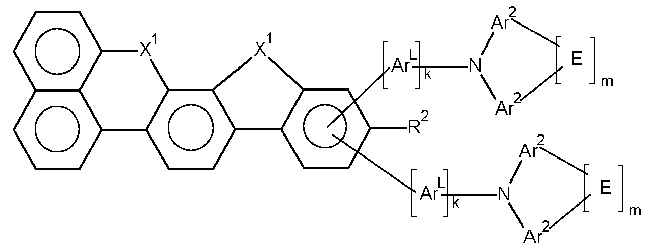 |
| Formel (I-5-E) |
| 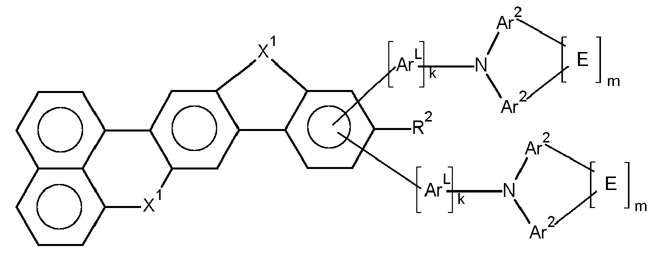 |
| Formel (I-6-E) |
| 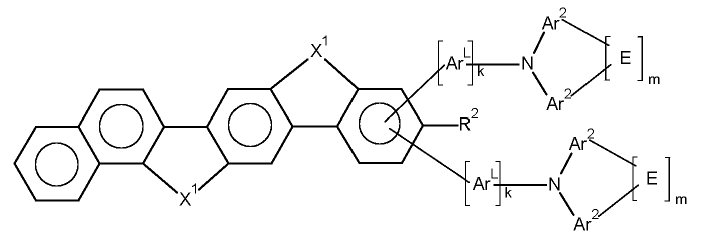 |
| Formel (I-7-E) |
| 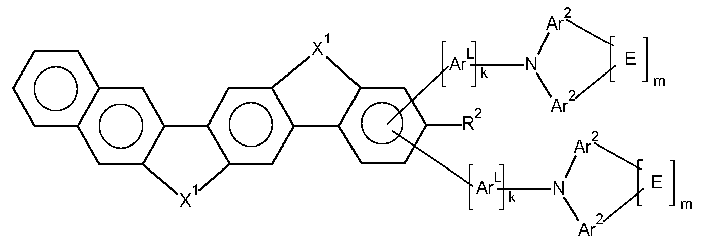 |
| Formel (I-8-E) |
| 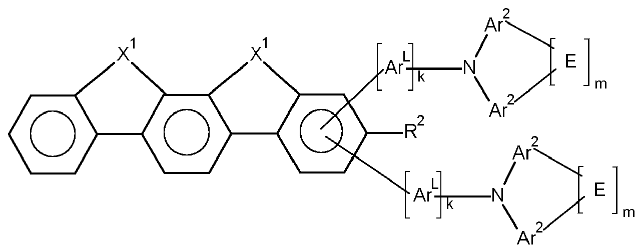 |
| Formel (I-9-E) |

wobei die auftretenden Variablen wie oben definiert sind, und bevorzugt X¹ gleich C(R⁴)₂ ist, und wobei die Verbindungen an den freien Positionen an den aromatischen Ringen jeweils mit einem Rest R³ bzw. R⁶ substituiert sein können, und bevorzugt an diesen Positionen unsubstituiert sind.

Besonders bevorzugt ist unter diesen Formeln die Formel (I-1-E).

Bevorzugte Ausführungsformen der Formeln (1-1-E) bis (I-9-E) entsprechen den folgenden Formeln

| |
|---|
| 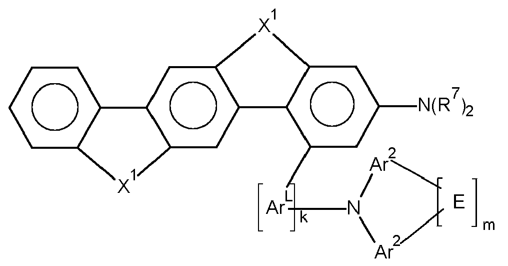 |
| Formel (I-1-E-1) |
| 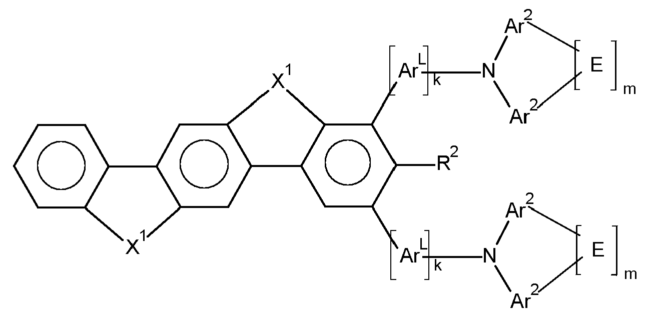 |
| Formel (I-1-E-2) |
| 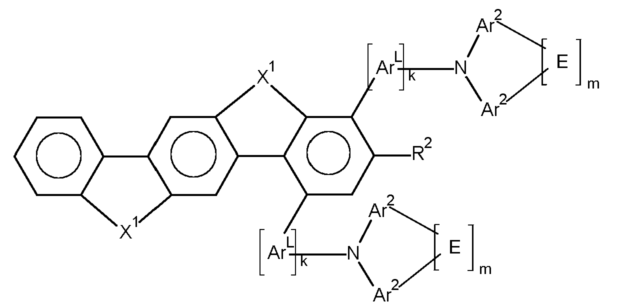 |
| Formel (I-1-E-3) |
| 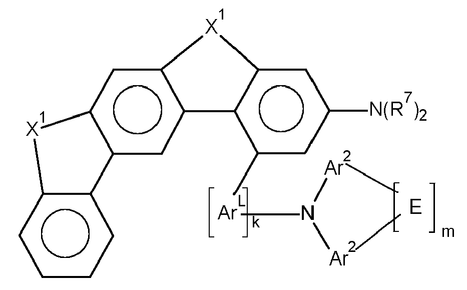 |
| Formel (I-2-E-1) |
| 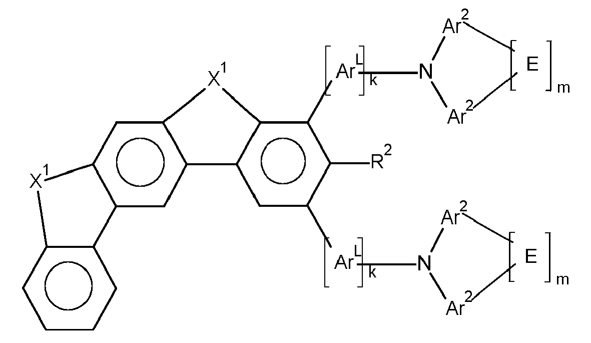 |
| Formel (I-2-E-2) |
| 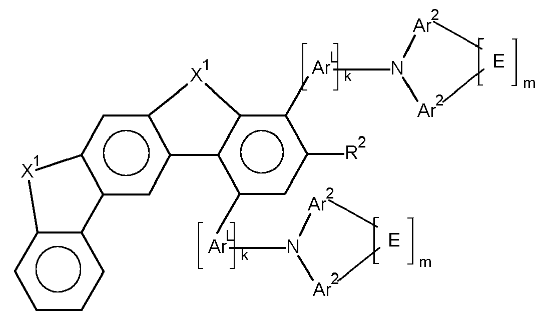 |
| Formel (I-2-E-3) |
| 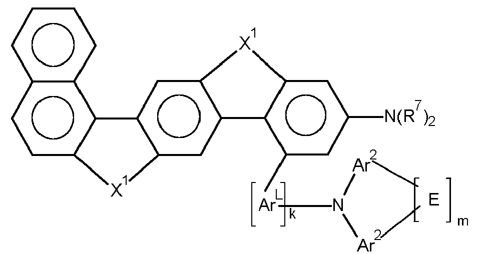 |
| Formel (I-3-E-3) |
| 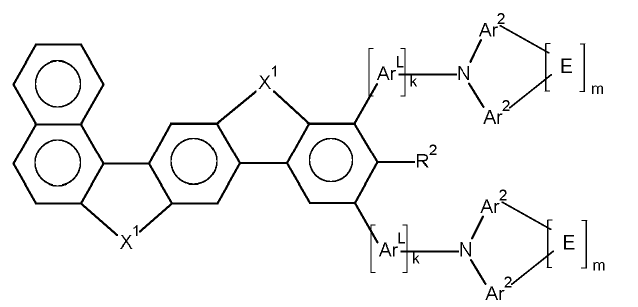 |
| Formel (I-3-E-2) |
| 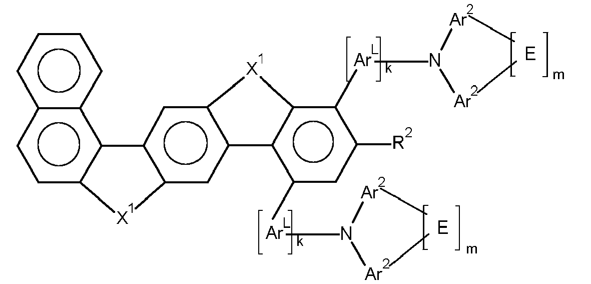 |
| Formel (I-3-E-3) |
| 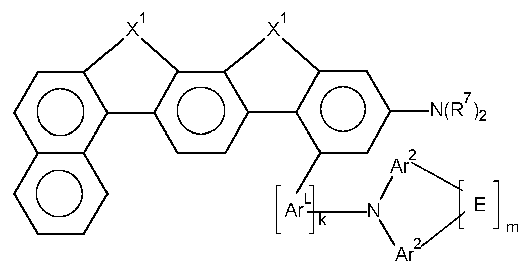 |
| Formel (I-4-E-1) |
| 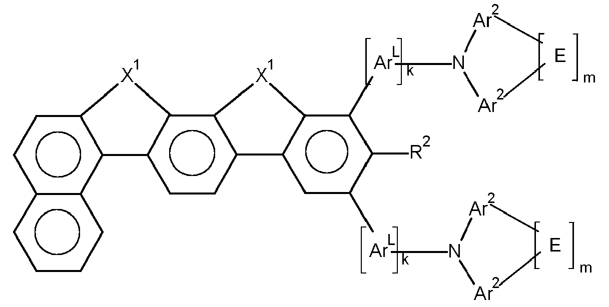 |
| Formel (I-4-E-2) |
| 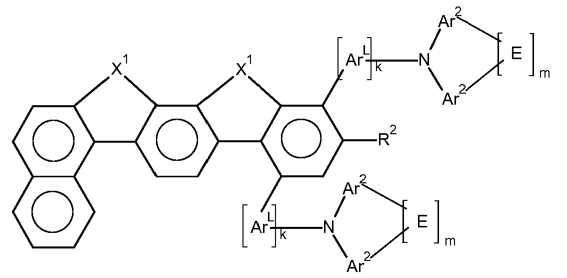 |
| Formel (I-4-E-3) |
| 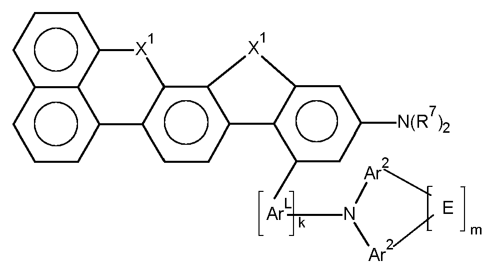 |
| Formel (I-5-E-1) |
| 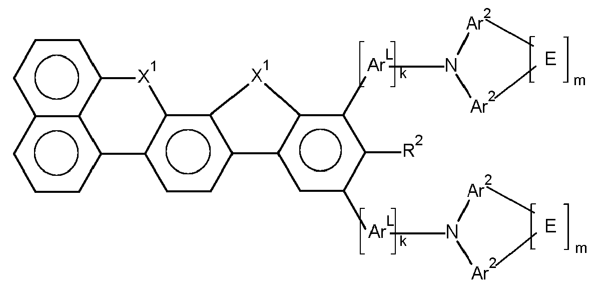 |
| Formel (I-5-E-2) |
| 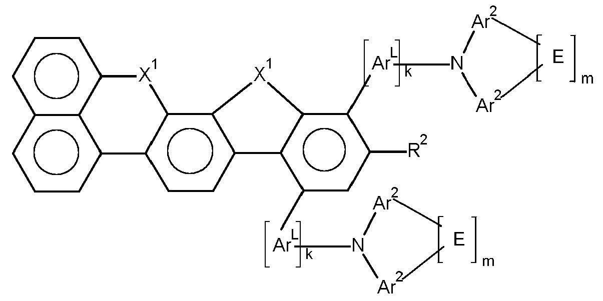 |
| Formel (I-5-E-3) |
| 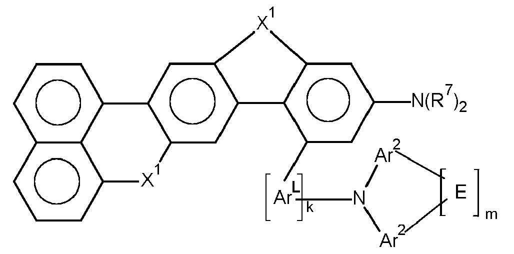 |
| Formel (I-6-E-1) |
| 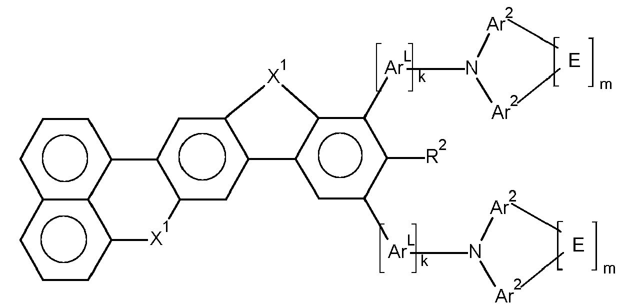 |
| Formel (I-6-E-2) |
| 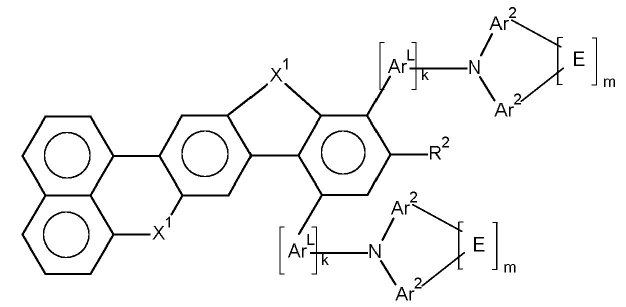 |
| Formel (I-6-E-3) |
| 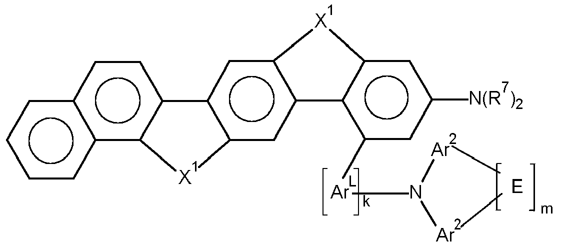 |
| Formel (I-7-E-1) |
| 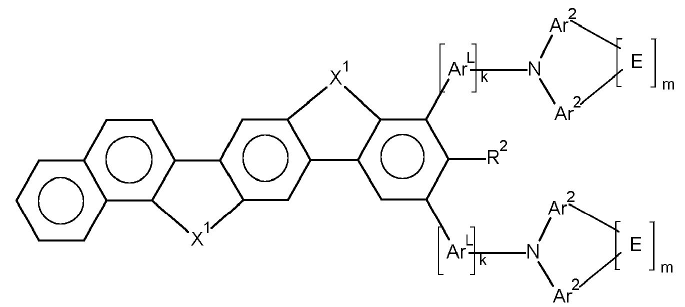 |
| Formel (I-7-E-2) |
| 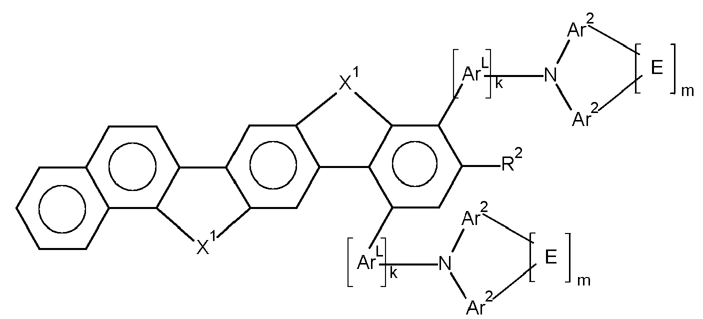 |
| Formel (I-7-E-3) |
| 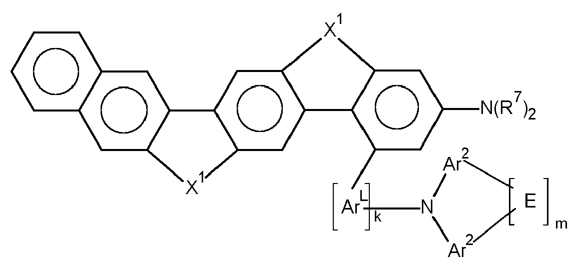 |
| Formel (I-8-E-1) |
| 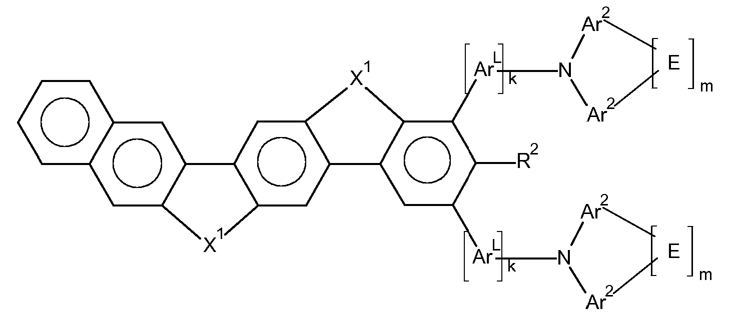 |
| Formel (I-8-E-2) |
| 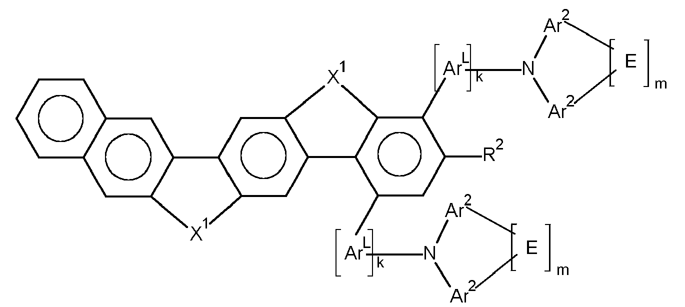 |
| Formel (I-8-E-3) |
| 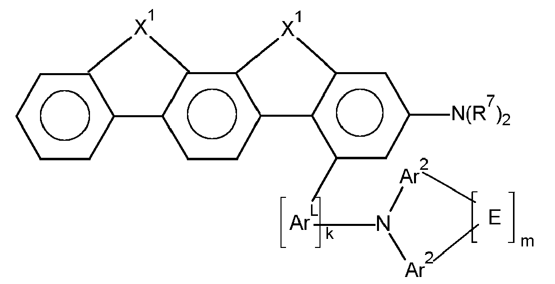 |
| Formel (I-9-E-1) |
| 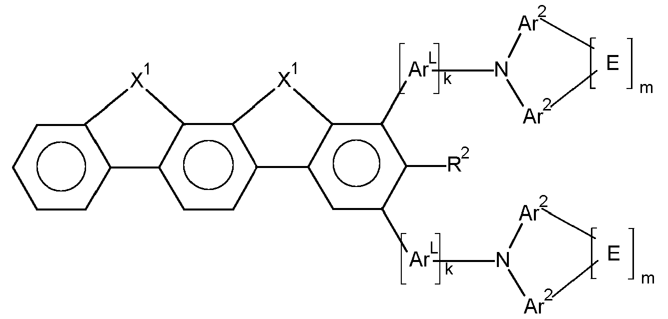 |
| Formel (I-9-E-2) |
| 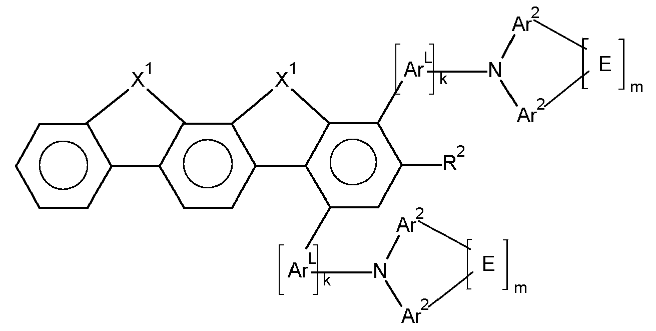 |
| Formel (I-9-E-3) |

wobei die auftretenden Variablen wie oben definiert sind, und bevorzugt X¹ gleich C(R⁴)₂ ist, und wobei die Verbindungen an den freien Positionen an den aromatischen Ringen jeweils mit einem Rest R³ bzw. R⁶ substituiert sein können, und bevorzugt an diesen Positionen unsubstituiert sind.

Besonders bevorzugt unter diesen Formeln sind die Formeln (I-1-E-1), (I-1-E-2) und (I-1-E-3).

Für die oben genannten bevorzugten Grundstrukturen gelten bevorzugt die oben genannten bevorzugten Ausführungsformen der Variablen.

Besonders bevorzugte konkrete Verbindungen entsprechen der folgenden Formel 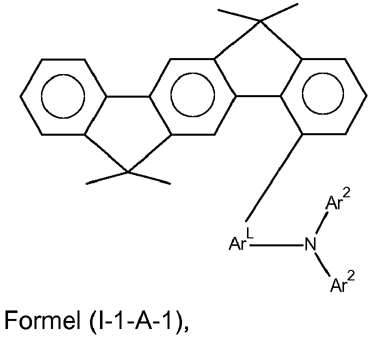 wobei die auftretenden Variablen Ar^{L} und Ar² wie folgt gewählt sind:

| **Verbindung** | **Gruppe Ar^{L}** | **Eine Gruppe Ar²** | **Andere Gruppe Ar²** |
|---|---|---|---|
| V-1 | Ar^{L}-1 | Ar-1 | Ar-1 |
| V-2 | " | " | Ar-2 |
| V-3 | " | " | Ar-4 |
| V-4 | " | " | Ar-5 |
| V-5 | " | " | Ar-74 |
| V-6 | " | " | Ar-78 |
| V-7 | " | " | Ar-82 |
| V-8 | " | " | Ar-108 |
| V-9 | " | " | Ar-117 |
| V-10 | " | " | Ar-139 |
| V-11 | " | " | Ar-150 |
| V-12 | " | " | Ar-172 |
| V-13 | " | " | Ar-207 |
| V-14 | " | Ar-2 | Ar-2 |
| V-15 | " | " | Ar-4 |
| V-16 | " | " | Ar-5 |
| V-17 | " | " | Ar-74 |
| V-18 | " | " | Ar-78 |
| V-19 | " | " | Ar-82 |
| V-20 | " | " | Ar-108 |
| V-21 | " | " | Ar-117 |
| V-22 | " | " | Ar-139 |
| V-23 | " | " | Ar-150 |
| V-24 | " | " | Ar-172 |
| V-25 | " | " | Ar-207 |
| V-26 | " | Ar-4 | Ar-4 |
| V-27 | " | " | Ar-5 |
| V-28 | " | " | Ar-74 |
| V-29 | " | " | Ar-78 |
| V-30 | " | " | Ar-82 |
| V-31 | " | " | Ar-108 |
| V-32 | " | " | Ar-117 |
| V-33 | " | " | Ar-139 |
| V-34 | " | " | Ar-150 |
| V-35 | " | " | Ar-172 |
| V-36 | " | " | Ar-207 |
| V-37 | " | Ar-5 | Ar-5 |
| V-38 | " | " | Ar-74 |
| V-39 | " | " | Ar-78 |
| V-40 | " | " | Ar-82 |
| V-41 | " | " | Ar-108 |
| V-42 | " | " | Ar-117 |
| V-43 | " | " | Ar-139 |
| V-44 | " | " | Ar-150 |
| V-45 | " | " | Ar-172 |
| V-46 | " | " | Ar-207 |
| V-47 | " | Ar-74 | Ar-74 |
| V-48 | " | " | Ar-78 |
| V-49 | " | " | Ar-82 |
| V-50 | " | " | Ar-108 |
| V-51 | " | " | Ar-117 |
| V-52 | " | " | Ar-139 |
| V-53 | " | " | Ar-150 |
| V-54 | " | " | Ar-172 |
| V-55 | " | " | Ar-207 |
| V-56 | " | Ar-78 | Ar-78 |
| V-57 | " | " | Ar-82 |
| V-58 | " | " | Ar-108 |
| V-59 | " | " | Ar-117 |
| V-60 | " | " | Ar-139 |
| V-61 | " | " | Ar-150 |
| V-62 | " | " | Ar-172 |
| V-63 | " | " | Ar-207 |
| V-64 | " | Ar-82 | Ar-82 |
| V-65 | " | " | Ar-108 |
| V-66 | " | " | Ar-117 |
| V-67 | " | " | Ar-139 |
| V-68 | " | " | Ar-150 |
| V-69 | " | " | Ar-172 |
| V-70 | " | " | Ar-207 |
| V-71 | " | Ar-108 | Ar-108 |
| V-72 | " | " | Ar-117 |
| V-73 | " | " | Ar-139 |
| V-74 | " | " | Ar-150 |
| V-75 | " | " | Ar-172 |
| V-76 | " | " | Ar-207 |
| V-77 | " | Ar-117 | Ar-117 |
| V-78 | " | " | Ar-139 |
| V-79 | " | " | Ar-150 |
| V-80 | " | " | Ar-172 |
| V-81 | " | " | Ar-207 |
| V-82 | " | Ar-139 | Ar-139 |
| V-83 | " | " | Ar-150 |
| V-84 | " | " | Ar-172 |
| V-85 | " | " | Ar-207 |
| V-86 | " | Ar-150 | Ar-150 |
| V-87 | " | " | Ar-172 |
| V-88 | " | " | Ar-207 |
| V-89 | " | Ar-172 | Ar-172 |
| V-90 | " | " | Ar-207 |
| V-91 | " | Ar-207 | Ar-207 |
| V-92 | Ar^{L}-2 | Ar-1 | Ar-1 |
| V-93 | " | " | Ar-2 |
| V-94 | " | " | Ar-4 |
| V-95 | " | " | Ar-5 |
| V-96 | " | " | Ar-74 |
| V-97 | " | " | Ar-78 |
| V-98 | " | " | Ar-82 |
| V-99 | " | " | Ar-108 |
| V-100 | " | " | Ar-117 |
| V-101 | " | " | Ar-139 |
| V-102 | " | " | Ar-150 |
| V-103 | " | " | Ar-172 |
| V-104 | " | " | Ar-207 |
| V-105 | " | Ar-2 | Ar-2 |
| V-106 | " | " | Ar-4 |
| V-107 | " | " | Ar-5 |
| V-108 | " | " | Ar-74 |
| V-109 | " | " | Ar-78 |
| V-110 | " | " | Ar-82 |
| V-111 | " | " | Ar-108 |
| V-112 | " | " | Ar-117 |
| V-113 | " | " | Ar-139 |
| V-114 | " | " | Ar-150 |
| V-115 | " | " | Ar-172 |
| V-116 | " | " | Ar-207 |
| V-117 | " | Ar-4 | Ar-4 |
| V-118 | " | " | Ar-5 |
| V-119 | " | " | Ar-74 |
| V-120 | " | " | Ar-78 |
| V-121 | " | " | Ar-82 |
| V-122 | " | " | Ar-108 |
| V-123 | " | " | Ar-117 |
| V-124 | " | " | Ar-139 |
| V-125 | " | " | Ar-150 |
| V-126 | " | " | Ar-172 |
| V-127 | " | " | Ar-207 |
| V-128 | " | Ar-5 | Ar-5 |
| V-129 | " | " | Ar-74 |
| V-130 | " | " | Ar-78 |
| V-131 | " | " | Ar-82 |
| V-132 | " | " | Ar-108 |
| V-133 | " | " | Ar-117 |
| V-134 | " | " | Ar-139 |
| V-135 | " | " | Ar-150 |
| V-136 | " | " | Ar-172 |
| V-137 | " | " | Ar-207 |
| V-138 | " | Ar-74 | Ar-74 |
| V-139 | " | " | Ar-78 |
| V-140 | " | " | Ar-82 |
| V-141 | " | " | Ar-108 |
| V-142 | " | " | Ar-117 |
| V-143 | " | " | Ar-139 |
| V-144 | " | " | Ar-150 |
| V-145 | " | " | Ar-172 |
| V-146 | " | " | Ar-207 |
| V-147 | " | Ar-78 | Ar-78 |
| V-148 | " | " | Ar-82 |
| V-149 | " | " | Ar-108 |
| V-150 | " | " | Ar-117 |
| V-151 | " | " | Ar-139 |
| V-152 | " | " | Ar-150 |
| V-153 | " | " | Ar-172 |
| V-154 | " | " | Ar-207 |
| V-155 | " | Ar-82 | Ar-82 |
| V-156 | " | " | Ar-108 |
| V-157 | " | " | Ar-117 |
| V-158 | " | " | Ar-139 |
| V-159 | " | " | Ar-150 |
| V-160 | " | " | Ar-172 |
| V-161 | " | " | Ar-207 |
| V-162 | " | Ar-108 | Ar-108 |
| V-163 | " | " | Ar-117 |
| V-164 | " | " | Ar-139 |
| V-165 | " | " | Ar-150 |
| V-166 | " | " | Ar-172 |
| V-167 | " | " | Ar-207 |
| V-168 | " | Ar-117 | Ar-117 |
| V-169 | " | " | Ar-139 |
| V-170 | " | " | Ar-150 |
| V-171 | " | " | Ar-172 |
| V-172 | " | " | Ar-207 |
| V-173 | " | Ar-139 | Ar-139 |
| V-174 | " | " | Ar-150 |
| V-175 | " | " | Ar-172 |
| V-176 | " | " | Ar-207 |
| V-177 | " | Ar-150 | Ar-150 |
| V-178 | " | " | Ar-172 |
| V-179 | " | " | Ar-207 |
| V-180 | " | Ar-172 | Ar-172 |
| V-181 | " | " | Ar-207 |
| V-182 | " | Ar-207 | Ar-207 |
| V-183 | Ar^{L}-3 | Ar-1 | Ar-1 |
| V-184 | " | " | Ar-2 |
| V-185 | " | " | Ar-4 |
| V-186 | " | " | Ar-5 |
| V-187 | " | " | Ar-74 |
| V-188 | " | " | Ar-78 |
| V-189 | " | " | Ar-82 |
| V-190 | " | " | Ar-108 |
| V-191 | " | " | Ar-117 |
| V-192 | " | " | Ar-139 |
| V-193 | " | " | Ar-150 |
| V-194 | " | " | Ar-172 |
| V-195 | " | " | Ar-207 |
| V-196 | " | Ar-2 | Ar-2 |
| V-197 | " | " | Ar-4 |
| V-198 | " | " | Ar-5 |
| V-199 | " | " | Ar-74 |
| V-200 | " | " | Ar-78 |
| V-201 | " | " | Ar-82 |
| V-202 | " | " | Ar-108 |
| V-203 | " | " | Ar-117 |
| V-204 | " | " | Ar-139 |
| V-205 | " | " | Ar-150 |
| V-206 | " | " | Ar-172 |
| V-207 | " | " | Ar-207 |
| V-208 | " | Ar-4 | Ar-4 |
| V-209 | " | " | Ar-5 |
| V-210 | " | " | Ar-74 |
| V-211 | " | " | Ar-78 |
| V-212 | " | " | Ar-82 |
| V-213 | " | " | Ar-108 |
| V-214 | " | " | Ar-117 |
| V-215 | " | " | Ar-139 |
| V-216 | " | " | Ar-150 |
| V-217 | " | " | Ar-172 |
| V-218 | " | " | Ar-207 |
| V-219 | " | Ar-5 | Ar-5 |
| V-220 | " | " | Ar-74 |
| V-221 | " | " | Ar-78 |
| V-222 | " | " | Ar-82 |
| V-223 | " | " | Ar-108 |
| V-224 | " | " | Ar-117 |
| V-225 | " | " | Ar-139 |
| V-226 | " | " | Ar-150 |
| V-227 | " | " | Ar-172 |
| V-228 | " | " | Ar-207 |
| V-229 | " | Ar-74 | Ar-74 |
| V-230 | " | " | Ar-78 |
| V-231 | " | " | Ar-82 |
| V-232 | " | " | Ar-108 |
| V-233 | " | " | Ar-117 |
| V-234 | " | " | Ar-139 |
| V-235 | " | " | Ar-150 |
| V-236 | " | " | Ar-172 |
| V-237 | " | " | Ar-207 |
| V-238 | " | Ar-78 | Ar-78 |
| V-239 | " | " | Ar-82 |
| V-240 | " | " | Ar-108 |
| V-241 | " | " | Ar-117 |
| V-242 | " | " | Ar-139 |
| V-243 | " | " | Ar-150 |
| V-244 | " | " | Ar-172 |
| V-245 | " | " | Ar-207 |
| V-246 | " | Ar-82 | Ar-82 |
| V-247 | " | " | Ar-108 |
| V-248 | " | " | Ar-117 |
| V-249 | " | " | Ar-139 |
| V-250 | " | " | Ar-150 |
| V-251 | " | " | Ar-172 |
| V-252 | " | " | Ar-207 |
| V-253 | " | Ar-108 | Ar-108 |
| V-254 | " | " | Ar-117 |
| V-255 | " | " | Ar-139 |
| V-256 | " | " | Ar-150 |
| V-257 | " | " | Ar-172 |
| V-258 | " | " | Ar-207 |
| V-259 | " | Ar-117 | Ar-117 |
| V-260 | " | " | Ar-139 |
| V-261 | " | " | Ar-150 |
| V-262 | " | " | Ar-172 |
| V-263 | " | " | Ar-207 |
| V-264 | " | Ar-139 | Ar-139 |
| V-265 | " | " | Ar-150 |
| V-266 | " | " | Ar-172 |
| V-267 | " | " | Ar-207 |
| V-268 | " | Ar-150 | Ar-150 |
| V-269 | " | " | Ar-172 |
| V-270 | " | " | Ar-207 |
| V-271 | " | Ar-172 | Ar-172 |
| V-272 | " | " | Ar-207 |
| V-273 | " | Ar-207 | Ar-207 |
| V-274 | Ar^{L}-4 | Ar-1 | Ar-1 |
| V-275 | " | " | Ar-2 |
| V-276 | " | " | Ar-4 |
| V-277 | " | " | Ar-5 |
| V-278 | " | " | Ar-74 |
| V-279 | " | " | Ar-78 |
| V-280 | " | " | Ar-82 |
| V-281 | " | " | Ar-108 |
| V-282 | " | " | Ar-117 |
| V-283 | " | " | Ar-139 |
| V-284 | " | " | Ar-150 |
| V-285 | " | " | Ar-172 |
| V-286 | " | " | Ar-207 |
| V-287 | " | Ar-2 | Ar-2 |
| V-288 | " | " | Ar-4 |
| V-289 | " | " | Ar-5 |
| V-290 | " | " | Ar-74 |
| V-291 | " | " | Ar-78 |
| V-292 | " | " | Ar-82 |
| V-293 | " | " | Ar-108 |
| V-294 | " | " | Ar-117 |
| V-295 | " | " | Ar-139 |
| V-296 | " | " | Ar-150 |
| V-297 | " | " | Ar-172 |
| V-298 | " | " | Ar-207 |
| V-299 | " | Ar-4 | Ar-4 |
| V-300 | " | " | Ar-5 |
| V-301 | " | " | Ar-74 |
| V-302 | " | " | Ar-78 |
| V-303 | " | " | Ar-82 |
| V-304 | " | " | Ar-108 |
| V-305 | " | " | Ar-117 |
| V-306 | " | " | Ar-139 |
| V-307 | " | " | Ar-150 |
| V-308 | " | " | Ar-172 |
| V-309 | " | " | Ar-207 |
| V-310 | " | Ar-5 | Ar-5 |
| V-311 | " | " | Ar-74 |
| V-312 | " | " | Ar-78 |
| V-313 | " | " | Ar-82 |
| V-314 | " | " | Ar-108 |
| V-315 | " | " | Ar-117 |
| V-316 | " | " | Ar-139 |
| V-317 | " | " | Ar-150 |
| V-318 | " | " | Ar-172 |
| V-319 | " | " | Ar-207 |
| V-320 | " | Ar-74 | Ar-74 |
| V-321 | " | " | Ar-78 |
| V-322 | " | " | Ar-82 |
| V-323 | " | " | Ar-108 |
| V-324 | " | " | Ar-117 |
| V-325 | " | " | Ar-139 |
| V-326 | " | " | Ar-150 |
| V-327 | " | " | Ar-172 |
| V-328 | " | " | Ar-207 |
| V-329 | " | Ar-78 | Ar-78 |
| V-330 | " | " | Ar-82 |
| V-331 | " | " | Ar-108 |
| V-332 | " | " | Ar-117 |
| V-333 | " | " | Ar-139 |
| V-334 | " | " | Ar-150 |
| V-335 | " | " | Ar-172 |
| V-336 | " | " | Ar-207 |
| V-337 | " | Ar-82 | Ar-82 |
| V-338 | " | " | Ar-108 |
| V-339 | " | " | Ar-117 |
| V-340 | " | " | Ar-139 |
| V-341 | " | " | Ar-150 |
| V-342 | " | " | Ar-172 |
| V-343 | " | " | Ar-207 |
| V-344 | " | Ar-108 | Ar-108 |
| V-345 | " | " | Ar-117 |
| V-346 | " | " | Ar-139 |
| V-347 | " | " | Ar-150 |
| V-348 | " | " | Ar-172 |
| V-349 | " | " | Ar-207 |
| V-350 | " | Ar-117 | Ar-117 |
| V-351 | " | " | Ar-139 |
| V-352 | " | " | Ar-150 |
| V-353 | " | " | Ar-172 |
| V-354 | " | " | Ar-207 |
| V-355 | " | Ar-139 | Ar-139 |
| V-356 | " | " | Ar-150 |
| V-357 | " | " | Ar-172 |
| V-358 | " | " | Ar-207 |
| V-359 | " | Ar-150 | Ar-150 |
| V-360 | " | " | Ar-172 |
| V-361 | " | " | Ar-207 |
| V-362 | " | Ar-172 | Ar-172 |
| V-363 | " | " | Ar-207 |
| V-364 | " | Ar-207 | Ar-207 |
| V-365 | Ar^{L-}7 | Ar-1 | Ar-1 |
| V-366 | " | " | Ar-2 |
| V-367 | " | " | Ar-4 |
| V-368 | " | " | Ar-5 |
| V-369 | " | " | Ar-74 |
| V-370 | " | " | Ar-78 |
| V-371 | " | " | Ar-82 |
| V-372 | " | " | Ar-108 |
| V-373 | " | " | Ar-117 |
| V-374 | " | " | Ar-139 |
| V-375 | " | " | Ar-150 |
| V-376 | " | " | Ar-172 |
| V-377 | " | " | Ar-207 |
| V-378 | " | Ar-2 | Ar-2 |
| V-379 | " | " | Ar-4 |
| V-380 | " | " | Ar-5 |
| V-381 | " | " | Ar-74 |
| V-382 | " | " | Ar-78 |
| V-383 | " | " | Ar-82 |
| V-384 | " | " | Ar-108 |
| V-385 | " | " | Ar-117 |
| V-386 | " | " | Ar-139 |
| V-387 | " | " | Ar-150 |
| V-388 | " | " | Ar-172 |
| V-389 | " | " | Ar-207 |
| V-390 | " | Ar-4 | Ar-4 |
| V-391 | " | " | Ar-5 |
| V-392 | " | " | Ar-74 |
| V-393 | " | " | Ar-78 |
| V-394 | " | " | Ar-82 |
| V-395 | " | " | Ar-108 |
| V-396 | " | " | Ar-117 |
| V-397 | " | " | Ar-139 |
| V-398 | " | " | Ar-150 |
| V-399 | " | " | Ar-172 |
| V-400 | " | " | Ar-207 |
| V-401 | " | Ar-5 | Ar-5 |
| V-402 | " | " | Ar-74 |
| V-403 | " | " | Ar-78 |
| V-404 | " | " | Ar-82 |
| V-405 | " | " | Ar-108 |
| V-406 | " | " | Ar-117 |
| V-407 | " | " | Ar-139 |
| V-408 | " | " | Ar-150 |
| V-409 | " | " | Ar-172 |
| V-410 | " | " | Ar-207 |
| V-411 | " | Ar-74 | Ar-74 |
| V-412 | " | " | Ar-78 |
| V-413 | " | " | Ar-82 |
| V-414 | " | " | Ar-108 |
| V-415 | " | " | Ar-117 |
| V-416 | " | " | Ar-139 |
| V-417 | " | " | Ar-150 |
| V-418 | " | " | Ar-172 |
| V-419 | " | " | Ar-207 |
| V-420 | " | Ar-78 | Ar-78 |
| V-421 | " | " | Ar-82 |
| V-422 | " | " | Ar-108 |
| V-423 | " | " | Ar-117 |
| V-424 | " | " | Ar-139 |
| V-425 | " | " | Ar-150 |
| V-426 | " | " | Ar-172 |
| V-427 | " | " | Ar-207 |
| V-428 | " | Ar-82 | Ar-82 |
| V-429 | " | " | Ar-108 |
| V-430 | " | " | Ar-117 |
| V-431 | " | " | Ar-139 |
| V-432 | " | " | Ar-150 |
| V-433 | " | " | Ar-172 |
| V-434 | " | " | Ar-207 |
| V-435 | " | Ar-108 | Ar-108 |
| V-436 | " | " | Ar-117 |
| V-437 | " | " | Ar-139 |
| V-438 | " | " | Ar-150 |
| V-439 | " | " | Ar-172 |
| V-440 | " | " | Ar-207 |
| V-441 | " | Ar-117 | Ar-117 |
| V-442 | " | " | Ar-139 |
| V-443 | " | " | Ar-150 |
| V-444 | " | " | Ar-172 |
| V-445 | " | " | Ar-207 |
| V-446 | " | Ar-139 | Ar-139 |
| V-447 | " | " | Ar-150 |
| V-448 | " | " | Ar-172 |
| V-449 | " | " | Ar-207 |
| V-450 | " | Ar-150 | Ar-150 |
| V-451 | " | " | Ar-172 |
| V-452 | " | " | Ar-207 |
| V-453 | " | Ar-172 | Ar-172 |
| V-454 | " | " | Ar-207 |
| V-455 | " | Ar-207 | Ar-207 |
| V-456 | Ar^{L}-19 | Ar-1 | Ar-1 |
| V-457 | " | " | Ar-2 |
| V-458 | " | " | Ar-4 |
| V-459 | " | " | Ar-5 |
| V-460 | " | " | Ar-74 |
| V-461 | " | " | Ar-78 |
| V-462 | " | " | Ar-82 |
| V-463 | " | " | Ar-108 |
| V-464 | " | " | Ar-117 |
| V-465 | " | " | Ar-139 |
| V-466 | " | " | Ar-150 |
| V-467 | " | " | Ar-172 |
| V-468 | " | " | Ar-207 |
| V-469 | " | Ar-2 | Ar-2 |
| V-470 | " | " | Ar-4 |
| V-471 | " | " | Ar-5 |
| V-472 | " | " | Ar-74 |
| V-473 | " | " | Ar-78 |
| V-474 | " | " | Ar-82 |
| V-475 | " | " | Ar-108 |
| V-476 | " | " | Ar-117 |
| V-477 | " | " | Ar-139 |
| V-478 | " | " | Ar-150 |
| V-479 | " | " | Ar-172 |
| V-480 | " | " | Ar-207 |
| V-481 | " | Ar-4 | Ar-4 |
| V-482 | " | " | Ar-5 |
| V-483 | " | " | Ar-74 |
| V-484 | " | " | Ar-78 |
| V-485 | " | " | Ar-82 |
| V-486 | " | " | Ar-108 |
| V-487 | " | " | Ar-117 |
| V-488 | " | " | Ar-139 |
| V-489 | " | " | Ar-150 |
| V-490 | " | " | Ar-172 |
| V-491 | " | " | Ar-207 |
| V-492 | " | Ar-5 | Ar-5 |
| V-493 | " | " | Ar-74 |
| V-494 | " | " | Ar-78 |
| V-495 | " | " | Ar-82 |
| V-496 | " | " | Ar-108 |
| V-497 | " | " | Ar-117 |
| V-498 | " | " | Ar-139 |
| V-499 | " | " | Ar-150 |
| V-500 | " | " | Ar-172 |
| V-501 | " | " | Ar-207 |
| V-502 | " | Ar-74 | Ar-74 |
| V-503 | " | " | Ar-78 |
| V-504 | " | " | Ar-82 |
| V-505 | " | " | Ar-108 |
| V-506 | " | " | Ar-117 |
| V-507 | " | " | Ar-139 |
| V-508 | " | " | Ar-150 |
| V-509 | " | " | Ar-172 |
| V-510 | " | " | Ar-207 |
| V-511 | " | Ar-78 | Ar-78 |
| V-512 | " | " | Ar-82 |
| V-513 | " | " | Ar-108 |
| V-514 | " | " | Ar-117 |
| V-515 | " | " | Ar-139 |
| V-516 | " | " | Ar-150 |
| V-517 | " | " | Ar-172 |
| V-518 | " | " | Ar-207 |
| V-519 | " | Ar-82 | Ar-82 |
| V-520 | " | " | Ar-108 |
| V-521 | " | " | Ar-117 |
| V-522 | " | " | Ar-139 |
| V-523 | " | " | Ar-150 |
| V-524 | " | " | Ar-172 |
| V-525 | " | " | Ar-207 |
| V-526 | " | Ar-108 | Ar-108 |
| V-527 | " | " | Ar-117 |
| V-528 | " | " | Ar-139 |
| V-529 | " | " | Ar-150 |
| V-530 | " | " | Ar-172 |
| V-531 | " | " | Ar-207 |
| V-532 | " | Ar-117 | Ar-117 |
| V-533 | " | " | Ar-139 |
| V-534 | " | " | Ar-150 |
| V-535 | " | " | Ar-172 |
| V-536 | " | " | Ar-207 |
| V-537 | " | Ar-139 | Ar-139 |
| V-538 | " | " | Ar-150 |
| V-539 | " | " | Ar-172 |
| V-540 | " | " | Ar-207 |
| V-541 | " | Ar-150 | Ar-150 |
| V-542 | " | " | Ar-172 |
| V-543 | " | " | Ar-207 |
| V-544 | " | Ar-172 | Ar-172 |
| V-545 | " | " | Ar-207 |
| V-546 | " | Ar-207 | Ar-207 |
| V-547 | Ar^{L}-36 | Ar-1 | Ar-1 |
| V-548 | " | " | Ar-2 |
| V-549 | " | " | Ar-4 |
| V-550 | " | " | Ar-5 |
| V-551 | " | " | Ar-74 |
| V-552 | " | " | Ar-78 |
| V-553 | " | " | Ar-82 |
| V-554 | " | " | Ar-108 |
| V-555 | " | " | Ar-117 |
| V-556 | " | " | Ar-139 |
| V-557 | " | " | Ar-150 |
| V-558 | " | " | Ar-172 |
| V-559 | " | " | Ar-207 |
| V-560 | " | Ar-2 | Ar-2 |
| V-561 | " | " | Ar-4 |
| V-562 | " | " | Ar-5 |
| V-563 | " | " | Ar-74 |
| V-564 | " | " | Ar-78 |
| V-565 | " | " | Ar-82 |
| V-566 | " | " | Ar-108 |
| V-567 | " | " | Ar-117 |
| V-568 | " | " | Ar-139 |
| V-569 | " | " | Ar-150 |
| V-570 | " | " | Ar-172 |
| V-571 | " | " | Ar-207 |
| V-572 | " | Ar-4 | Ar-4 |
| V-573 | " | " | Ar-5 |
| V-574 | " | " | Ar-74 |
| V-575 | " | " | Ar-78 |
| V-576 | " | " | Ar-82 |
| V-577 | " | " | Ar-108 |
| V-578 | " | " | Ar-117 |
| V-579 | " | " | Ar-139 |
| V-580 | " | " | Ar-150 |
| V-581 | " | " | Ar-172 |
| V-582 | " | " | Ar-207 |
| V-583 | " | Ar-5 | Ar-5 |
| V-584 | " | " | Ar-74 |
| V-585 | " | " | Ar-78 |
| V-586 | " | " | Ar-82 |
| V-587 | " | " | Ar-108 |
| V-588 | " | " | Ar-117 |
| V-589 | " | " | Ar-139 |
| V-590 | " | " | Ar-150 |
| V-591 | " | " | Ar-172 |
| V-592 | " | " | Ar-207 |
| V-593 | " | Ar-74 | Ar-74 |
| V-594 | " | " | Ar-78 |
| V-595 | " | " | Ar-82 |
| V-596 | " | " | Ar-108 |
| V-597 | " | " | Ar-117 |
| V-598 | " | " | Ar-139 |
| V-599 | " | " | Ar-150 |
| V-600 | " | " | Ar-172 |
| V-601 | " | " | Ar-207 |
| V-602 | " | Ar-78 | Ar-78 |
| V-603 | " | " | Ar-82 |
| V-604 | " | " | Ar-108 |
| V-605 | " | " | Ar-117 |
| V-606 | " | " | Ar-139 |
| V-607 | " | " | Ar-150 |
| V-608 | " | " | Ar-172 |
| V-609 | " | " | Ar-207 |
| V-610 | " | Ar-82 | Ar-82 |
| V-611 | " | " | Ar-108 |
| V-612 | " | " | Ar-117 |
| V-613 | " | " | Ar-139 |
| V-614 | " | " | Ar-150 |
| V-615 | " | " | Ar-172 |
| V-616 | " | " | Ar-207 |
| V-617 | " | Ar-108 | Ar-108 |
| V-618 | " | " | Ar-117 |
| V-619 | " | " | Ar-139 |
| V-620 | " | " | Ar-150 |
| V-621 | " | " | Ar-172 |
| V-622 | " | " | Ar-207 |
| V-623 | " | Ar-117 | Ar-117 |
| V-624 | " | " | Ar-139 |
| V-625 | " | " | Ar-150 |
| V-626 | " | " | Ar-172 |
| V-627 | " | " | Ar-207 |
| V-628 | " | Ar-139 | Ar-139 |
| V-629 | " | " | Ar-150 |
| V-630 | " | " | Ar-172 |
| V-631 | " | " | Ar-207 |
| V-632 | " | Ar-150 | Ar-150 |
| V-633 | " | " | Ar-172 |
| V-634 | " | " | Ar-207 |
| V-635 | " | Ar-172 | Ar-172 |
| V-636 | " | " | Ar-207 |
| V-637 | " | Ar-207 | Ar-207 |

Weitere besonders bevorzugte konkrete Verbindungen entsprechen der folgenden Formel 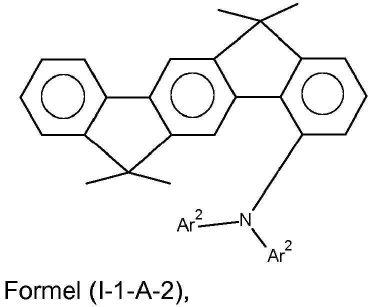 wobei die auftretenden Variablen Ar² wie folgt gewählt sind:

| **Verbindung** | **Eine Gruppe Ar²** | **Andere Gruppe Ar²** |
|---|---|---|
| V-638 | Ar-1 | Ar-1 |
| V-639 | " | Ar-2 |
| V-640 | " | Ar-4 |
| V-641 | " | Ar-5 |
| V-642 | " | Ar-74 |
| V-643 | " | Ar-78 |
| V-644 | " | Ar-82 |
| V-645 | " | Ar-108 |
| V-646 | " | Ar-117 |
| V-647 | " | Ar-139 |
| V-648 | " | Ar-150 |
| V-649 | " | Ar-172 |
| V-650 | " | Ar-207 |
| V-651 | Ar-2 | Ar-2 |
| V-652 | " | Ar-4 |
| V-653 | " | Ar-5 |
| V-654 | " | Ar-74 |
| V-655 | " | Ar-78 |
| V-656 | " | Ar-82 |
| V-657 | " | Ar-108 |
| V-658 | " | Ar-117 |
| V-659 | " | Ar-139 |
| V-660 | " | Ar-150 |
| V-661 | " | Ar-172 |
| V-662 | " | Ar-207 |
| V-663 | Ar-4 | Ar-4 |
| V-664 | " | Ar-5 |
| V-665 | " | Ar-74 |
| V-666 | " | Ar-78 |
| V-667 | " | Ar-82 |
| V-668 | " | Ar-108 |
| V-669 | " | Ar-117 |
| V-670 | " | Ar-139 |
| V-671 | " | Ar-150 |
| V-672 | " | Ar-172 |
| V-673 | " | Ar-207 |
| V-674 | Ar-5 | Ar-5 |
| V-675 | " | Ar-74 |
| V-676 | " | Ar-78 |
| V-677 | " | Ar-82 |
| V-678 | " | Ar-108 |
| V-679 | " | Ar-117 |
| V-680 | " | Ar-139 |
| V-681 | " | Ar-150 |
| V-682 | " | Ar-172 |
| V-683 | " | Ar-207 |
| V-684 | Ar-74 | Ar-74 |
| V-685 | " | Ar-78 |
| V-686 | " | Ar-82 |
| V-687 | " | Ar-108 |
| V-688 | " | Ar-117 |
| V-689 | " | Ar-139 |
| V-690 | " | Ar-150 |
| V-691 | " | Ar-172 |
| V-692 | " | Ar-207 |
| V-693 | Ar-78 | Ar-78 |
| V-694 | " | Ar-82 |
| V-695 | " | Ar-108 |
| V-696 | " | Ar-117 |
| V-697 | " | Ar-139 |
| V-698 | " | Ar-150 |
| V-699 | " | Ar-172 |
| V-700 | " | Ar-207 |
| V-701 | Ar-82 | Ar-82 |
| V-702 | " | Ar-108 |
| V-703 | " | Ar-117 |
| V-704 | " | Ar-139 |
| V-705 | " | Ar-150 |
| V-706 | " | Ar-172 |
| V-707 | " | Ar-207 |
| V-708 | Ar-108 | Ar-108 |
| V-709 | " | Ar-117 |
| V-710 | " | Ar-139 |
| V-711 | " | Ar-150 |
| V-712 | " | Ar-172 |
| V-713 | " | Ar-207 |
| V-714 | Ar-117 | Ar-117 |
| V-715 | " | Ar-139 |
| V-716 | " | Ar-150 |
| V-717 | " | Ar-172 |
| V-718 | " | Ar-207 |
| V-719 | Ar-139 | Ar-139 |
| V-720 | " | Ar-150 |
| V-721 | " | Ar-172 |
| V-722 | " | Ar-207 |
| V-723 | Ar-150 | Ar-150 |
| V-724 | " | Ar-172 |
| V-725 | " | Ar-207 |
| V-726 | Ar-172 | Ar-172 |
| V-727 | " | Ar-207 |
| V-728 | Ar-207 | Ar-207 |

Weitere besonders bevorzugte konkrete Verbindungen entsprechen der folgenden Formel 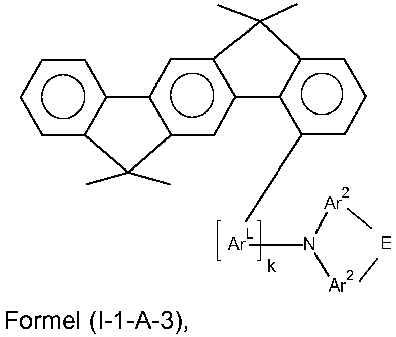 wobei die auftretende Variable k , die Gruppe Ar^{L} und die Einheit 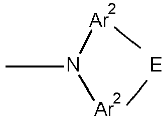 wie folgt gewählt sind:

| **Verbindung** | **k** | **Ar^{L}** | 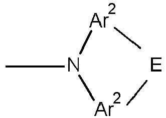 **Einheit** |
|---|---|---|---|
| V-729 | 0 | -- | N-17 |
| V-730 | 0 | -- | N-20 |
| V-731 | 1 | Ar^{L}-1 | N-17 |
| V-732 | 1 | Ar^{L}-1 | N-20 |
| V-733 | 1 | Ar^{L}-2 | N-17 |
| V-734 | 1 | Ar^{L}-2 | N-20 |
| V-735 | 1 | Ar^{L}-3 | N-17 |
| V-736 | 1 | Ar^{L}-3 | N-20 |
| V-737 | 1 | Ar^{L}-4 | N-17 |
| V-738 | 1 | Ar^{L}-4 | N-20 |
| V-739 | 1 | Ar^{L}-7 | N-17 |
| V-740 | 1 | Ar^{L}-7 | N-20 |
| V-741 | 1 | Ar^{L}-19 | N-17 |
| V-742 | 1 | Ar^{L}-19 | N-20 |
| V-743 | 1 | Ar^{L}-36 | N-17 |
| V-744 | 1 | Ar^{L}-36 | N-20 |

Bevorzugte konkrete Verbindungen gemäß Formel (I) sind in der folgenden Tabelle abgebildet:

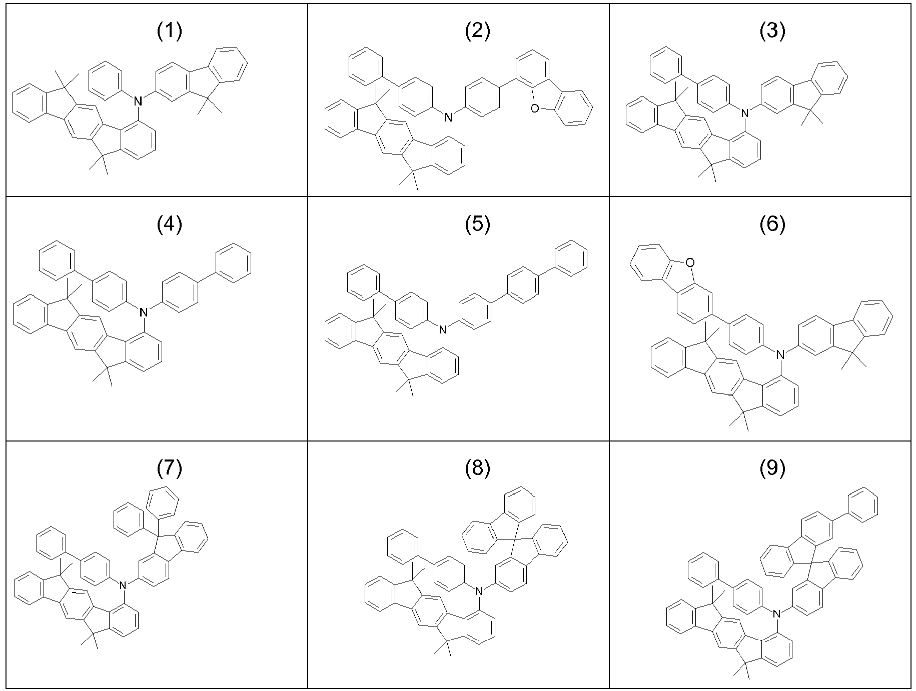

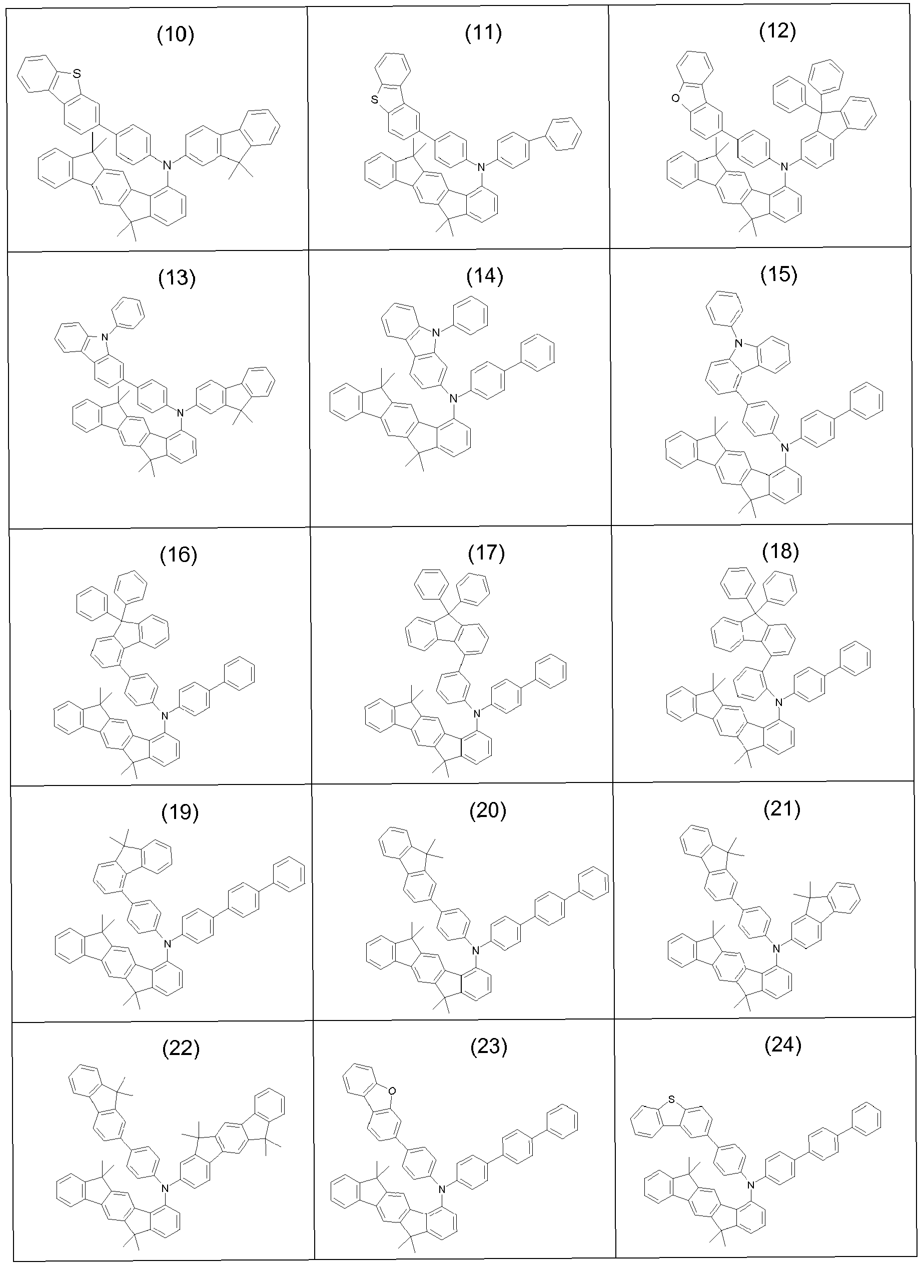

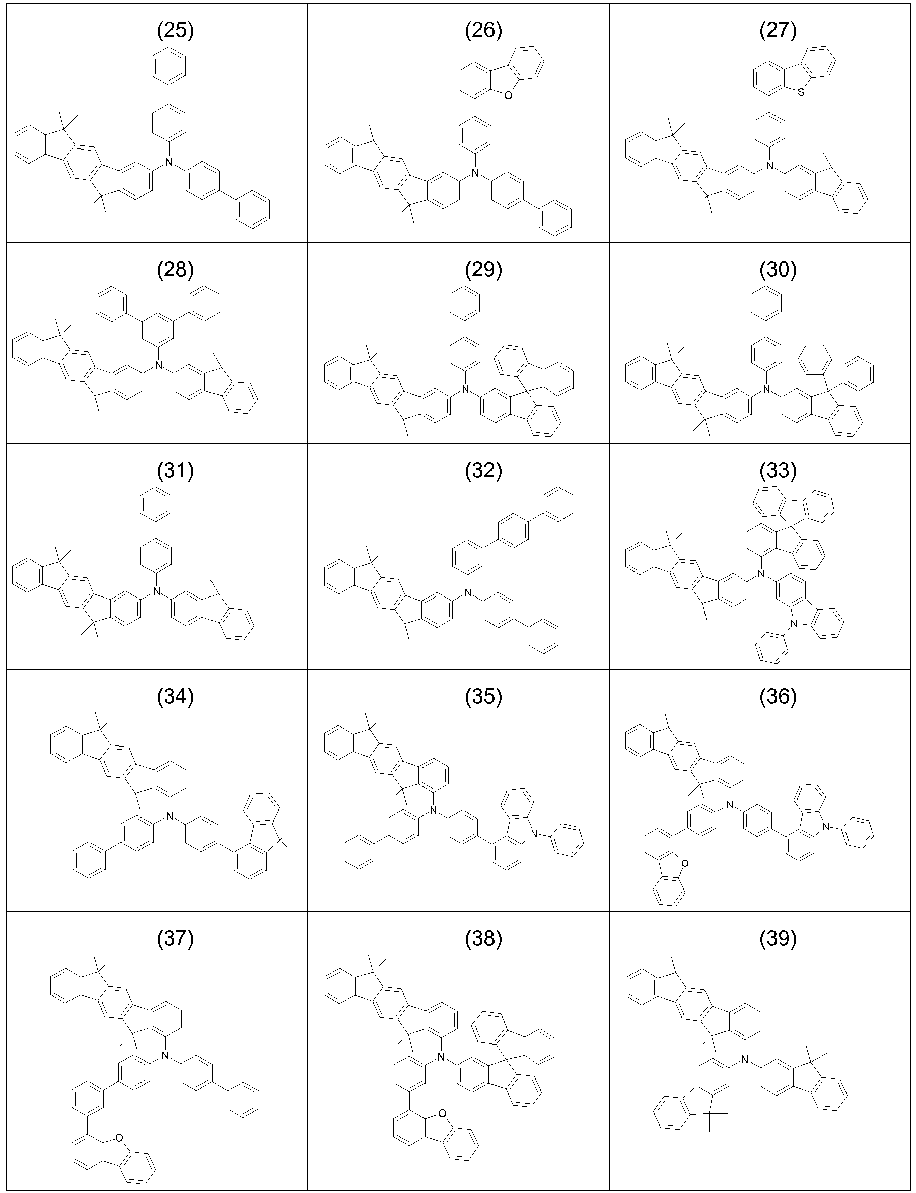

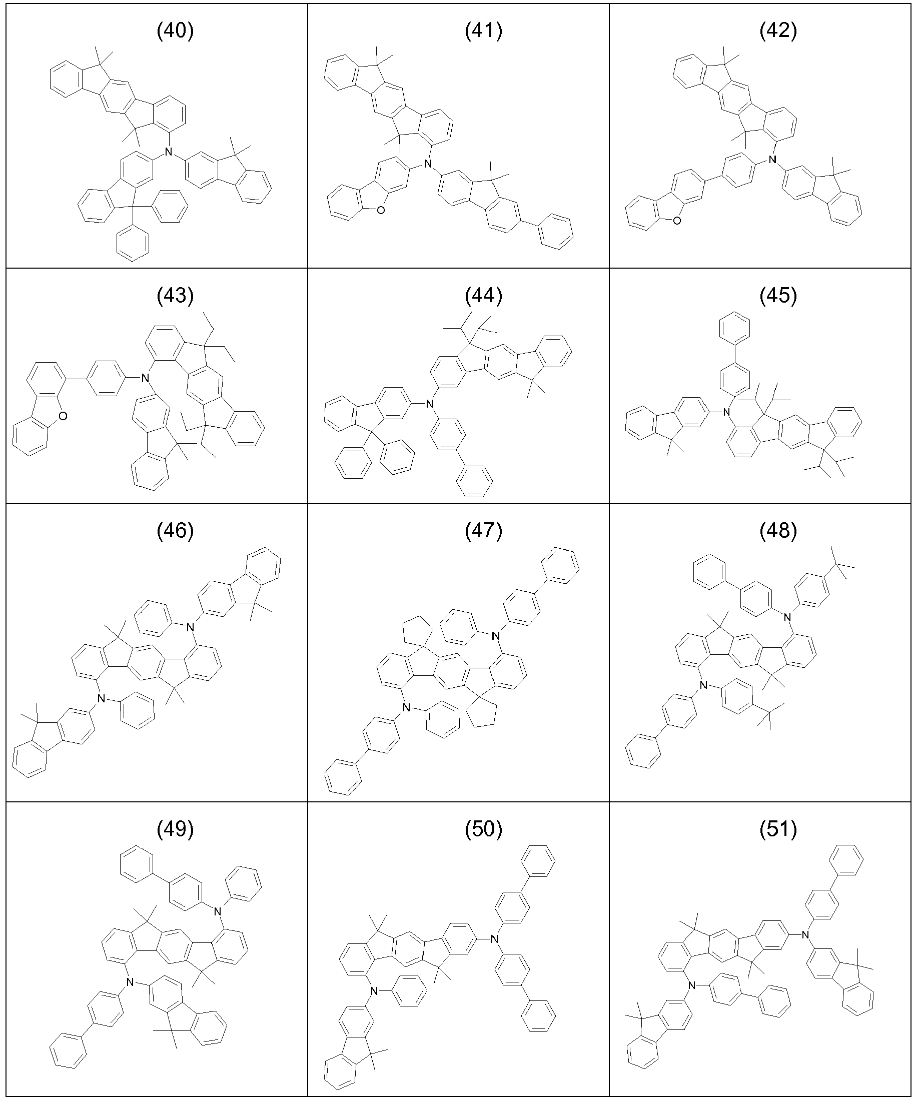

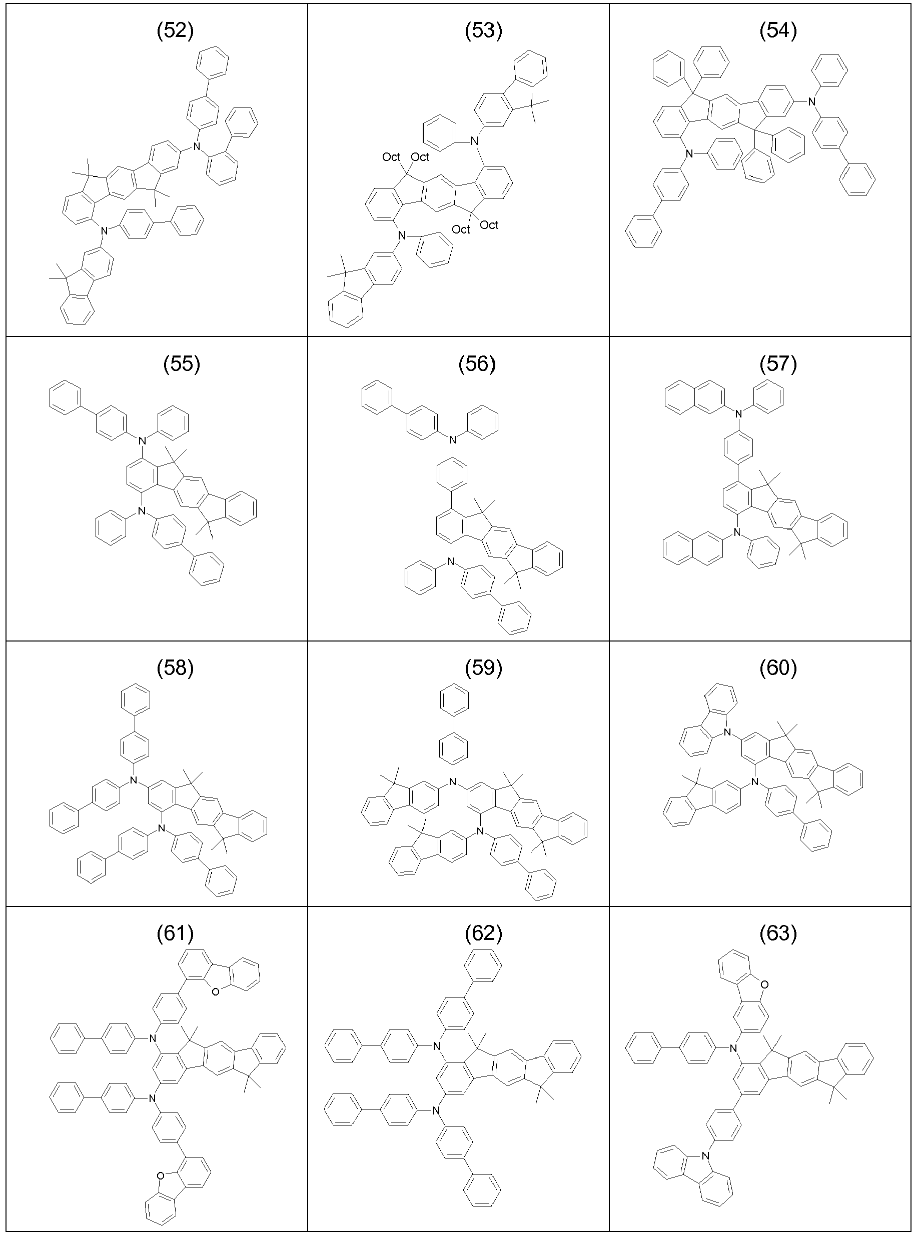

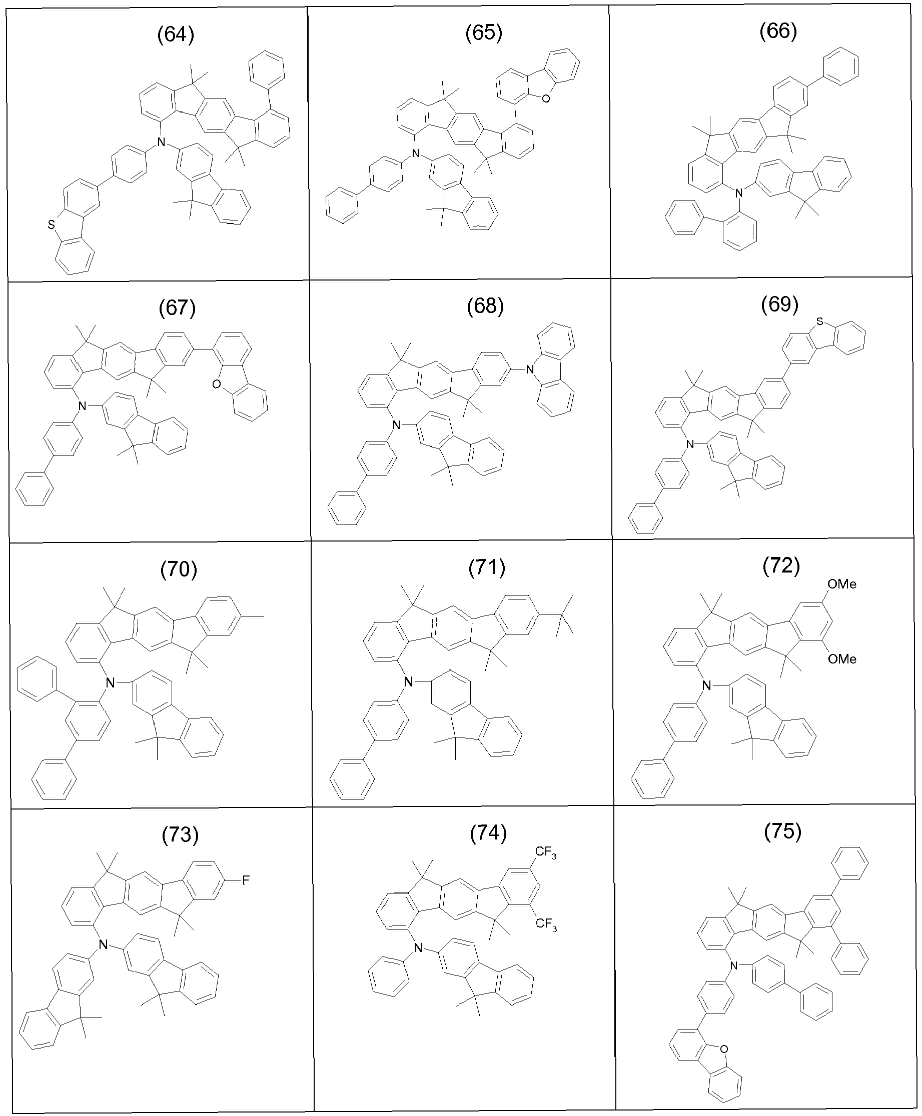

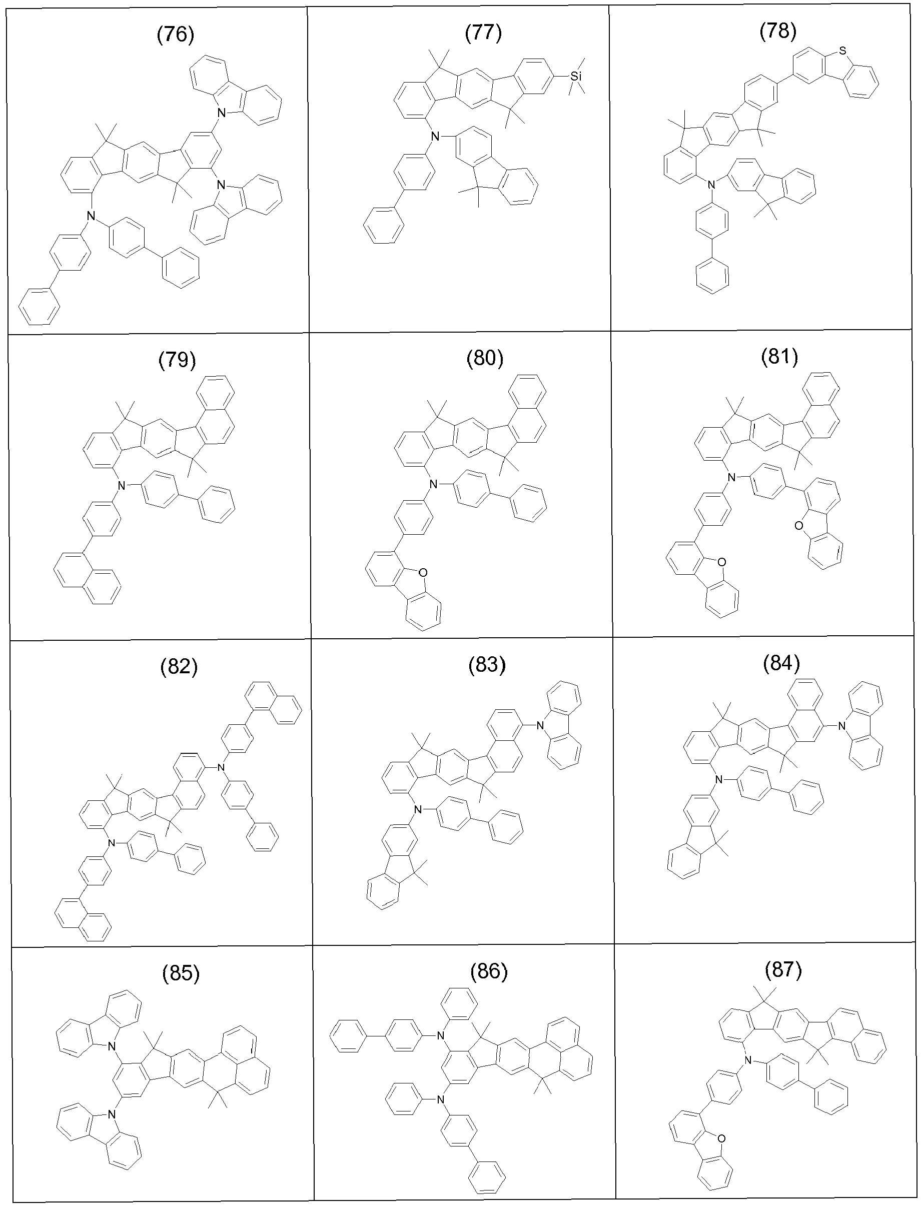

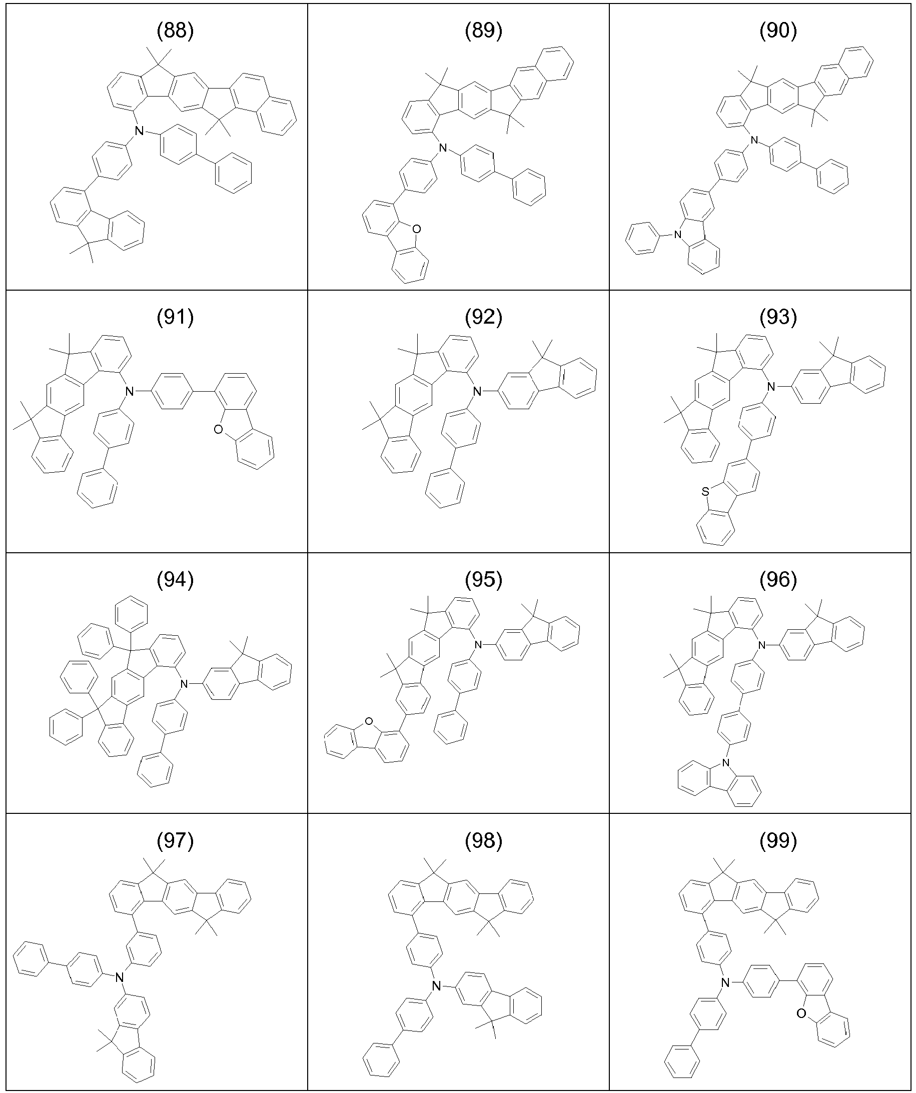

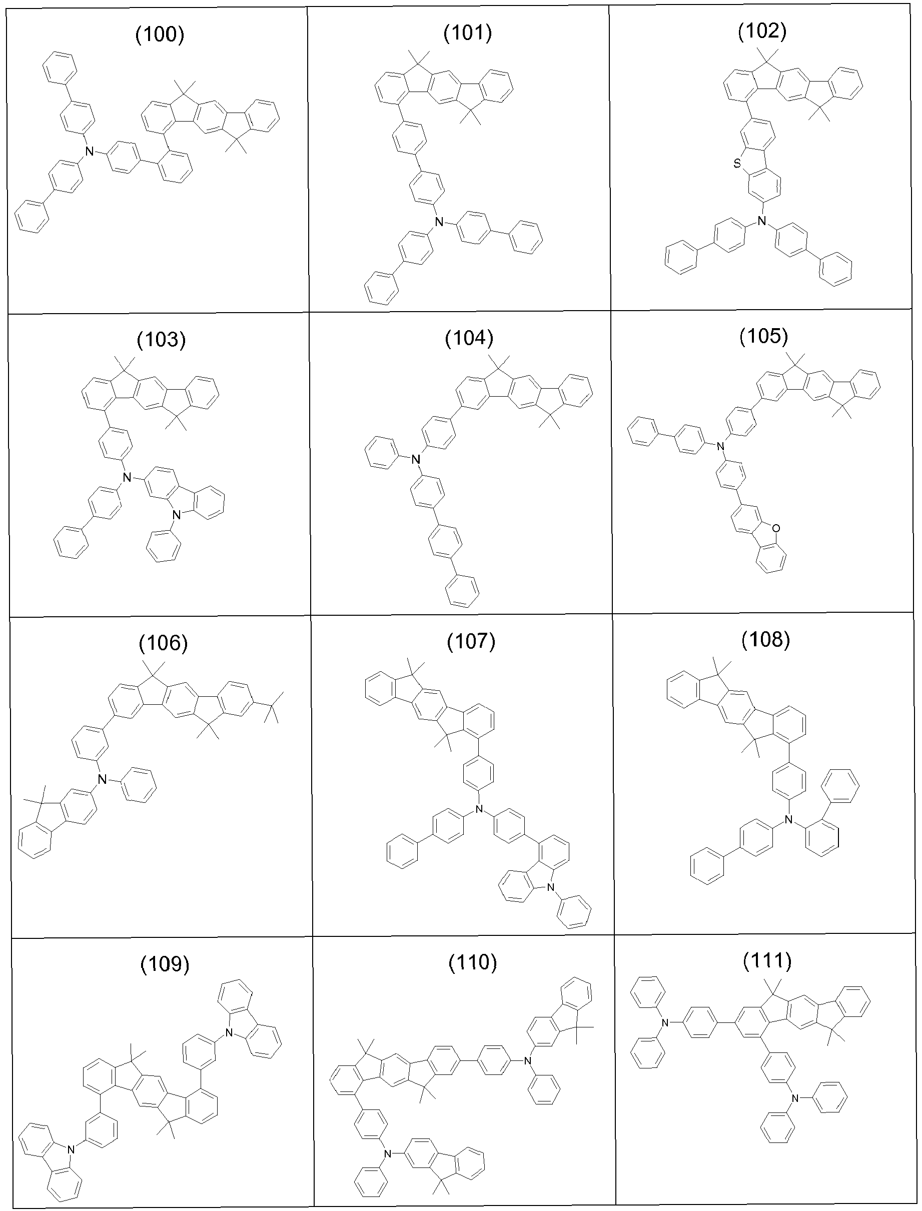

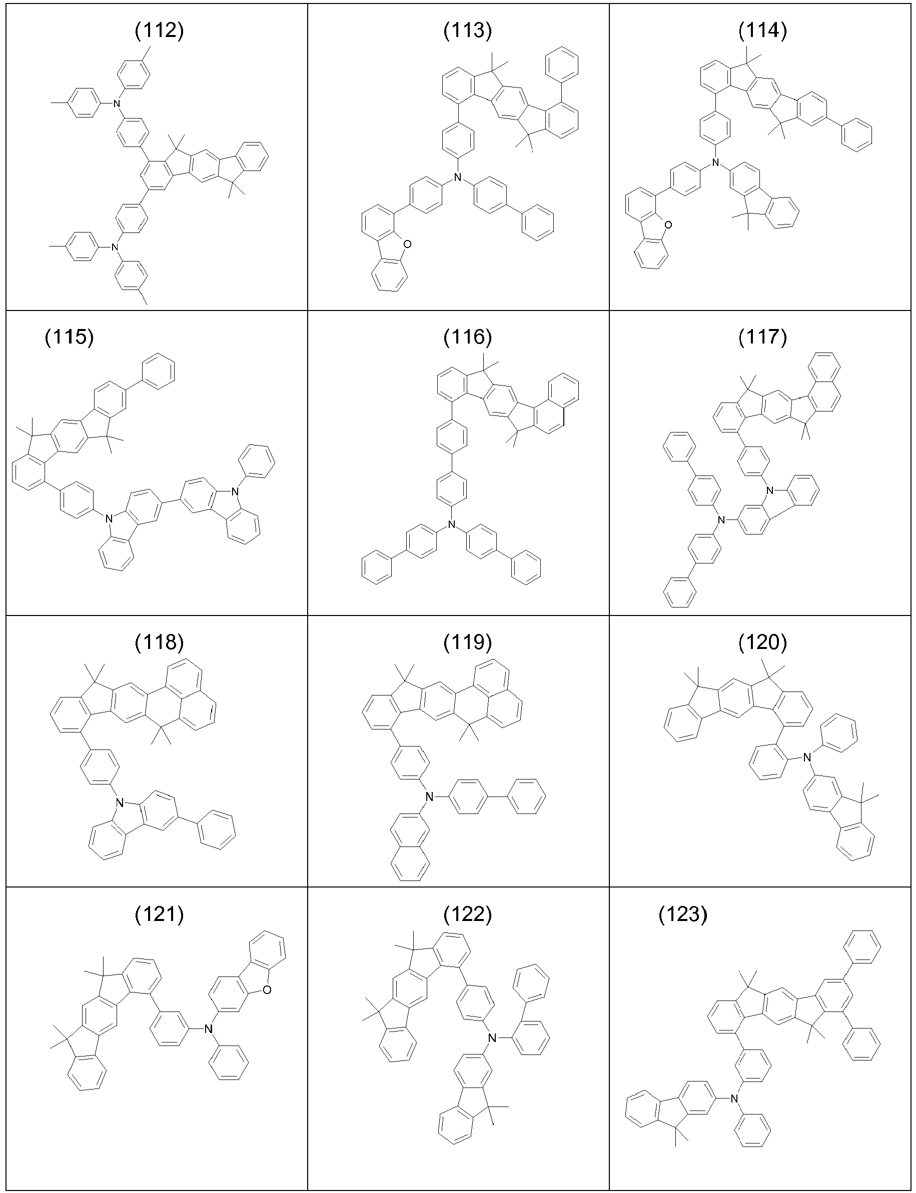

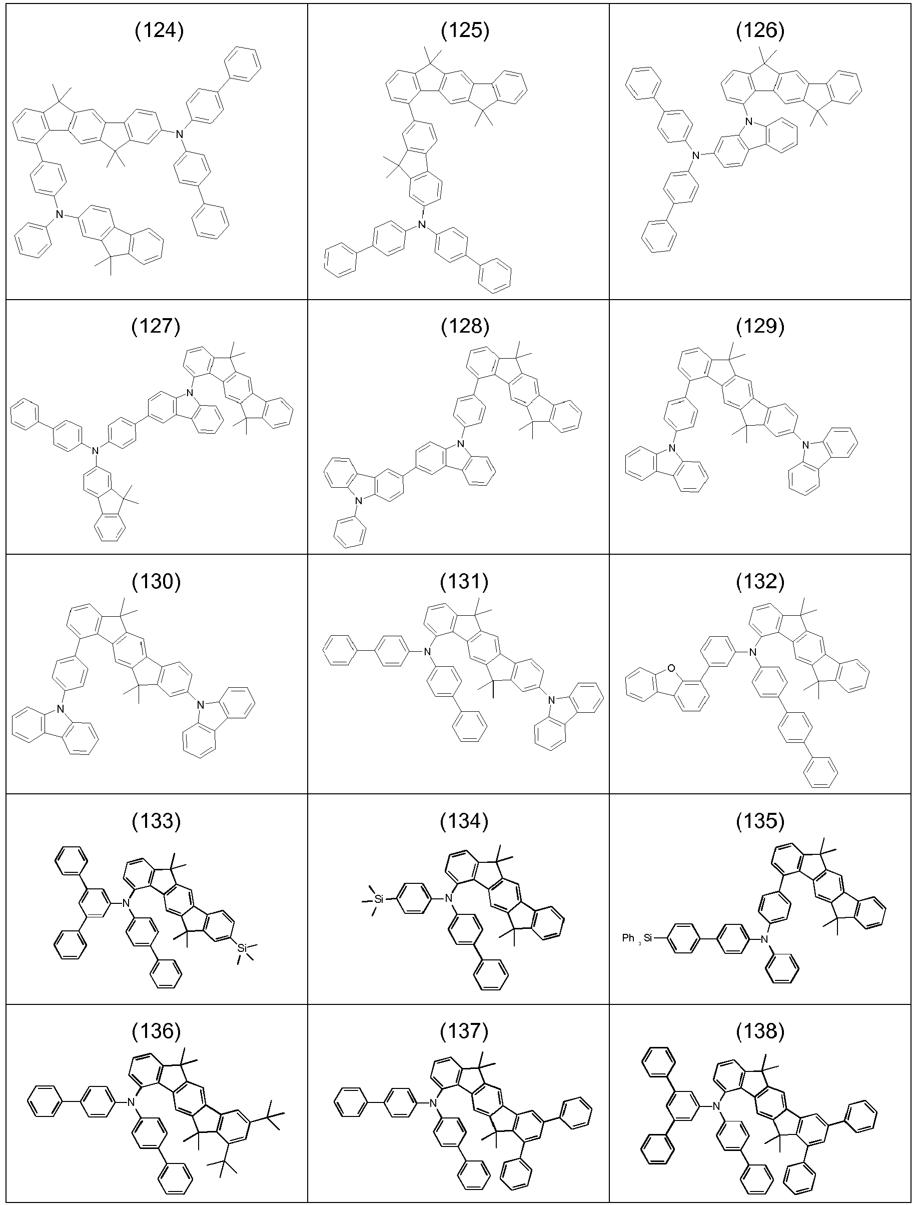

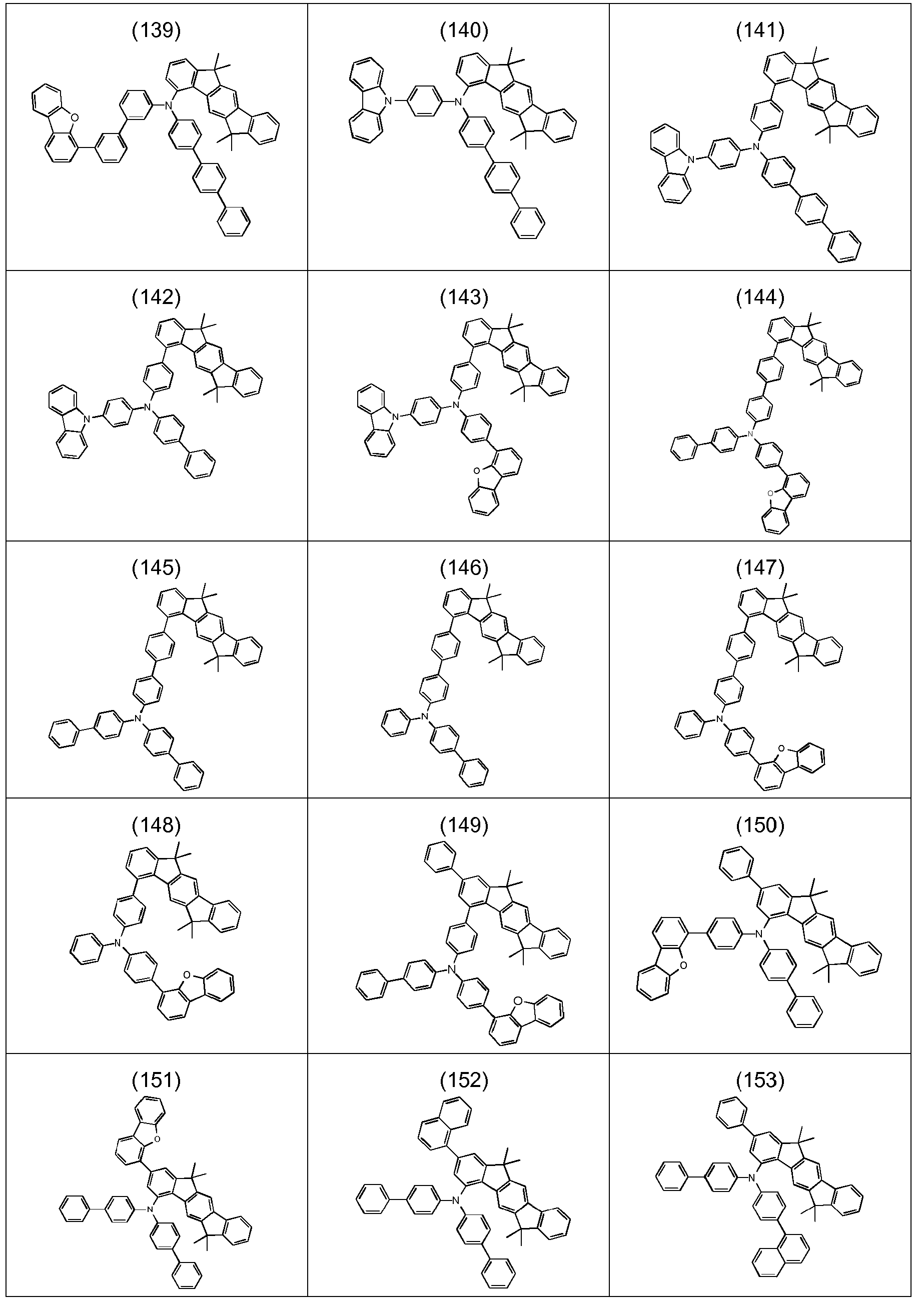

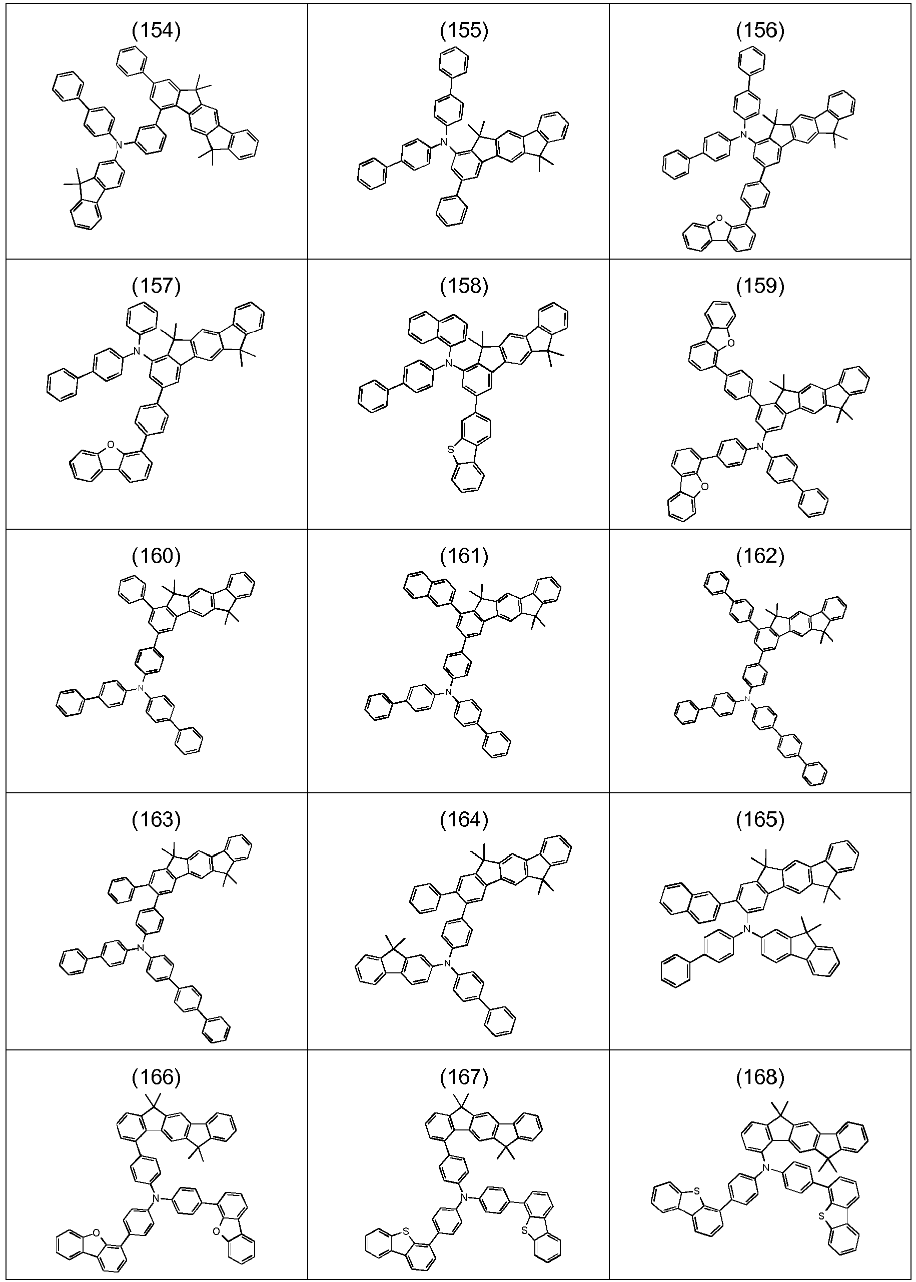

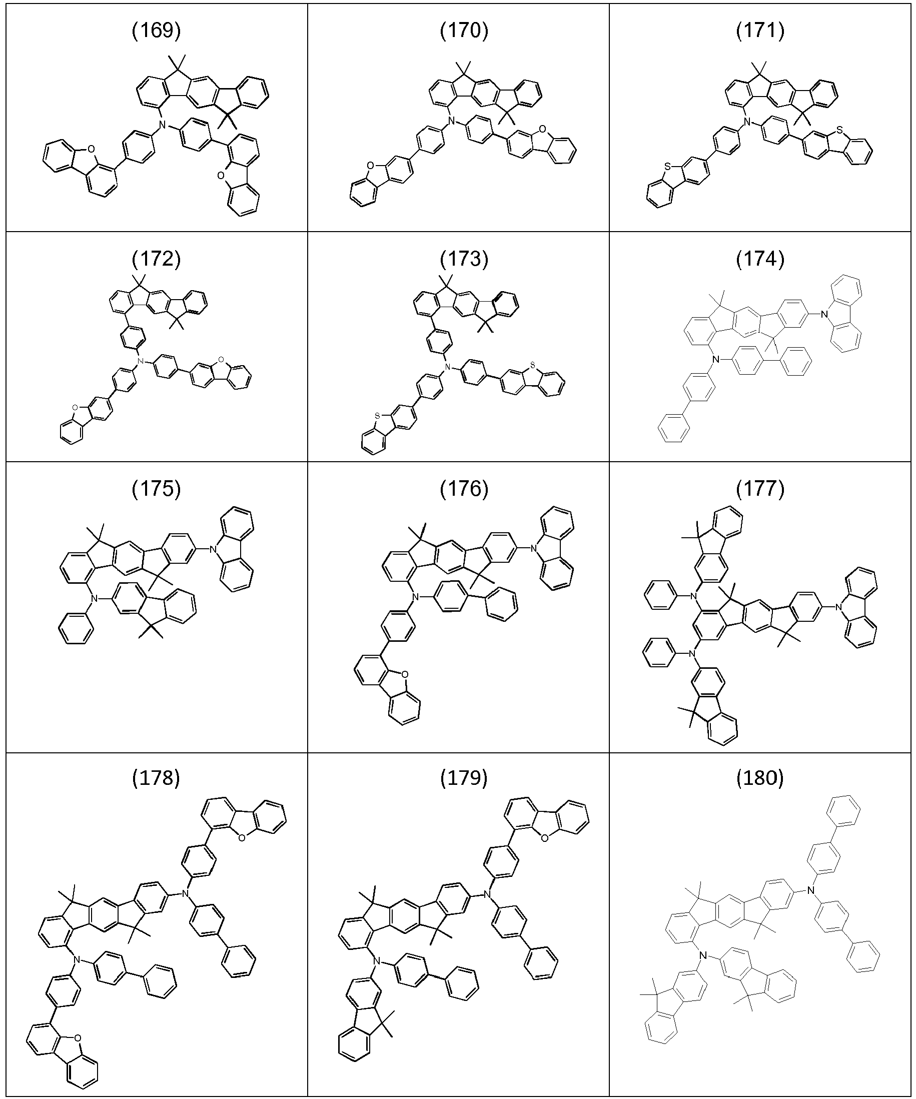

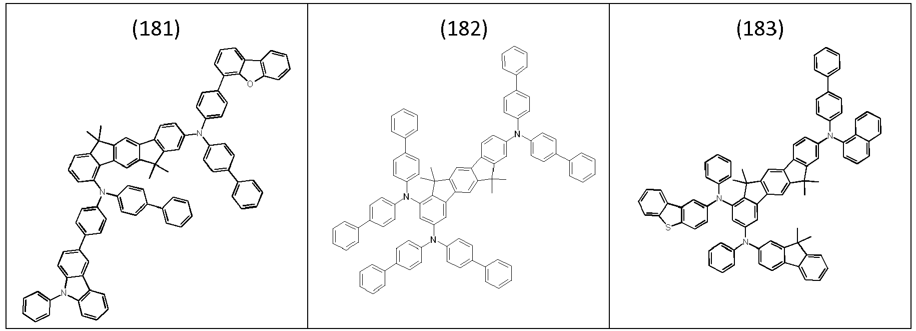

Die Verbindungen gemäß Formel (I) können mittels bekannten organischen Reaktionen hergestellt werden, insbesondere mittels Suzuki-Reaktionen, Hartwig-Buchwald-Reaktionen, und Zyklisierungsreaktionen.

Gemäß einem bevorzugten Verfahren (Schema 1) wird ausgehend von einer Benzolverbindung, die zwei reaktive Gruppen X und zwei Carbonsäureestergruppen trägt, über zwei sequenzielle Suzuki-Kupplungen eine Verbindung hergestellt, die eine Kette von drei Arylgruppen aufweist (Gruppe Ar und die beiden Phenylgruppen), wobei die endständige Phenylgruppe eine reaktive Gruppe X aufweist. Die reaktive Gruppe X steht in ortho- oder in meta-Stellung zur Bindung an die zentrale Phenylgruppe, bzw. steht in der reagierenden Phenyl-Boronsäure-Verbindung in ortho- oder in meta-Stellung zur Boronsäuregruppe. Anschließend werden die Carbonsäureestergruppen dieser Verbindung in tertiäre Alkoxygruppen überführt, durch Reaktion mit einer Metallalkylverbindung, bevorzugt einer Lithium-Alkylverbindung oder einer Grignard-Alkylverbindung. Diese tertiären Alkoxygruppen zyklisieren zu Ringen unter Einwirkung von Säure. Schließlich wird über eine Buchwald-Kupplung eine Aminogruppe eingeführt, oder es wird durch Suzuki-Reaktion eine Diarylamino-Aryl- oder eine Diarylamino-Heteroaryl-Gruppe eingeführt, so dass die Verbindung der Formel (I) erhalten wird.

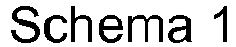

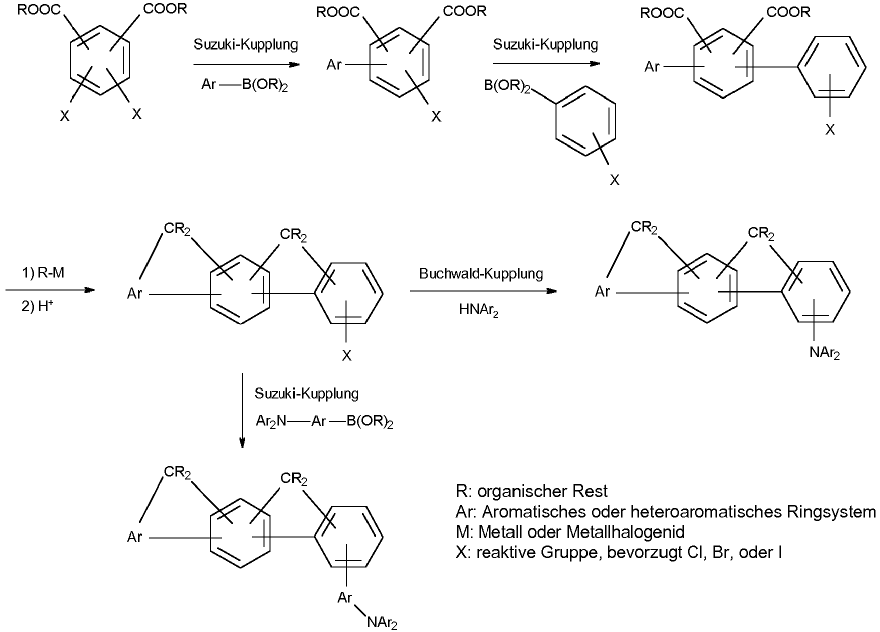

In einer Abwandlung des Reaktionswegs von Schema 1 (Schema 2) wird in einer der Suzuki-Kupplungen eine Phenylgruppe eingeführt, die anstelle der reaktiven Gruppe X eine Gruppe A, gewählt aus Gruppen -Ar-NAr₂ und -NAr₂, enthält. Die betreffende Gruppe A steht wie die Gruppe X in Schema 1 in ortho- oder in meta-Stellung zur Boronsäuregruppe. Bei diesem Syntheseweg entfällt die in Schema 1 im letzten Schritt stattfindende Suzuki- bzw. Buchwald-Kupplung.

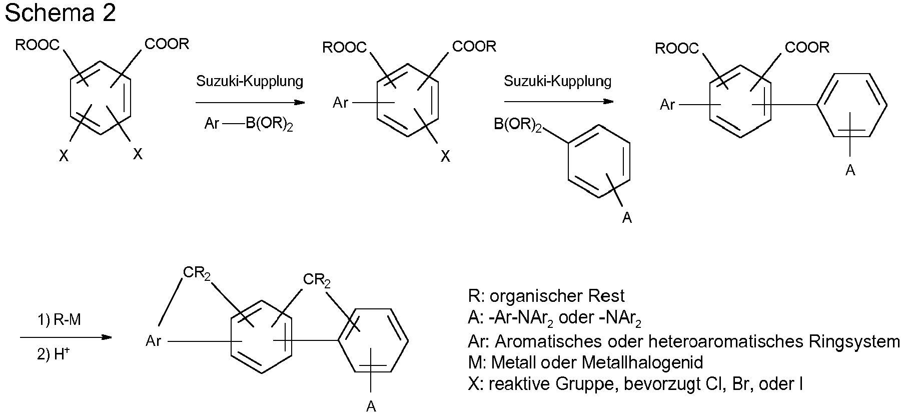

Weiterer Gegenstand der vorliegenden Anmeldung ist damit ein Verfahren zur Herstellung einer Verbindung gemäß Formel (I), dadurch gekennzeichnet, dass eine Benzolverbindung, die zwei Carbonsäureestergruppen, ein aromatisches oder heteroaromatisches Ringsystem und eine reaktive Gruppe trägt, in einer Suzuki-Reaktion mit einer Benzolverbindung umgesetzt wird, die eine Boronsäuregruppe und eine Gruppe gewählt aus reaktiven Gruppen, Diarylamino-Gruppen, Diarylamino-Aryl-Gruppen und Diarylamino-Heteroaryl-Gruppen enthält. Dabei stehen die Boronsäuregruppe und die Gruppe gewählt aus reaktiven Gruppen, Diarylamino-Gruppen, Diarylamino-Aryl-Gruppen und Diarylamino-Heteroaryl-Gruppen in ortho- oder in meta-Stellung am Benzolring zueinander.

Die reaktiven Gruppen sind dabei bevorzugt gewählt aus Cl, Br, I, Triflat, Mesylat und Tosylat.

Die oben beschriebenen Verbindungen der Formel (I), insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere, enthaltend eine oder mehrere Verbindungen gemäß Formel (I), wobei die Bindung(en) zum Polymer, Oligomer oder Dendrimer an beliebigen, in Formel (I) mit R¹, R², R³, R⁴ oder R⁵ substituierten Positionen lokalisiert sein können. Je nach Verknüpfung der Verbindung gemäß Formel (I) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nicht-konjugiert sein. Die erfindungsgemäßen Oligomere oder Polymere können linear, verzweigt oder dendritisch sein. In den linear verknüpften Strukturen können die Einheiten gemäß Formel (I) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (I) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (I) in Oligomeren, Dendrimeren und Polymeren gelten dieselben Bevorzugungen wie oben für Verbindungen gemäß Formel (I) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 2000/22026), Spirobifluorenen (z. B. gemäß EP 707020, EP 894107 oder WO 2006/061181), Paraphenylenen (z. B. gemäß WO 1992/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (I) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind die Suzuki-Polymerisation, die Yamamoto-Polymerisation; die Stille-Polymerisation; und die Hartwig-Buchwald-Polymerisation.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion, enthaltend mindestens eine Verbindung gemäß Formel (I) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen eignen sich für den Einsatz in elektronischen Vorrichtungen, insbesondere in organischen Elektrolumineszenzvorrichtungen (OLEDs). Abhängig von der Substitution werden die Verbindungen in unterschiedlichen Funktionen und Schichten eingesetzt.

Weiterer Gegenstand der Erfindung ist daher die Verwendung der Verbindung gemäß Formel (I) in einer elektronischen Vorrichtung. Dabei ist die elektronische Vorrichtung bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs).

Weiterer Gegenstand der Erfindung ist, wie bereits oben ausgeführt, eine elektronische Vorrichtung, enthaltend mindestens eine Verbindung gemäß Formel (I). Dabei ist die elektronische Vorrichtung bevorzugt ausgewählt aus den oben genannten Vorrichtungen.

Besonders bevorzugt ist sie eine organische Elektrolumineszenzvorrichtung (OLED), enthaltend Anode, Kathode und mindestens eine emittierende Schicht, dadurch gekennzeichnet, dass mindestens eine organische Schicht, die eine emittierende Schicht, eine lochtransportierende Schicht oder eine andere Schicht sein kann, mindestens eine Verbindung gemäß Formel (I) enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen.

Die Abfolge der Schichten der organischen Elektrolumineszenzvorrichtung enthaltend die Verbindung der Formel (I) ist bevorzugt die folgende: Anode-Lochinjektionsschicht-Lochtransportschicht-wahlweise weitere Lochtransportschicht(en)-wahlweise Elektronenblockierschicht-emittierende Schicht-wahlweise Lochblockierschicht-Elektronentransportschicht-Elektroneninjektionsschicht-Kathode. Es können zusätzlich weitere Schichten in der OLED vorhanden sein.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues, grünes, gelbes, orangefarbenes oder rotes Licht emittieren. Insbesondere bevorzugt sind Dreischichtsysteme, also Systeme mit drei emittierenden Schichten, wobei die drei Schichten blaue, grüne und orangefarbene oder rote Emission zeigen (für den prinzipiellen Aufbau siehe z. B. WO 2005/011013). Die erfindungsgemäßen Verbindungen sind dabei bevorzugt in einer Lochtransportschicht, Lochinjektionsschicht, Elektronenblockierschicht, und/oder emittierenden Schicht vorhanden, besonders bevorzugt in einer emittierenden Schicht als Matrixmaterial, und/oder in einer Elektronenblockierschicht.

Es ist erfindungsgemäß bevorzugt, wenn die Verbindung gemäß Formel (I) in einer elektronischen Vorrichtung enthaltend einen oder mehrere phosphoreszierende emittierende Verbindungen eingesetzt wird. Dabei kann die Verbindung in unterschiedlichen Schichten, bevorzugt in einer Lochtransportschicht, einer Elektronenblockierschicht, einer Lochinjektionsschicht, und/oder einer emittierenden Schicht enthalten sein. Besonders bevorzugt ist sie in einer Elektronenblockierschicht oder in einer emittierenden Schicht in Kombination mit einer phosphoreszierenden emittierenden Verbindung enthalten. In letzterem Fall ist die phosphoreszierende emittierende Verbindung bevorzugt gewählt aus rot oder grün phosphoreszierenden emittierenden Verbindungen. Ganz besonders bevorzugt ist sie in einer Elektronenblockierschicht enthalten.

Vom Begriff phosphoreszierende emittierende Verbindungen sind typischerweise Verbindungen umfasst, bei denen die Lichtemission durch einen spin-verbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende emittierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende emittierende Verbindungen Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten. Dabei werden im Sinne der vorliegenden Erfindung alle lumineszierenden Iridium-, Platin- oder Kupferkomplexe als phosphoreszierende emittierende Verbindungen angesehen.

Beispiele für die oben beschriebenen emittierenden Verbindungen können den Anmeldungen WO 00/70655, WO 01/41512, WO 02/02714, WO 02/15645, EP 1191613, EP 1191612, EP 1191614, WO 05/033244, WO 05/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind. Auch kann der Fachmann ohne erfinderisches Zutun weitere phosphoreszierende Komplexe in Kombination mit den Verbindungen gemäß Formel (I) in organischen Elektrolumineszenzvorrichtungen einsetzen. Weitere Beispiele sind in der folgenden Tabelle aufgeführt:

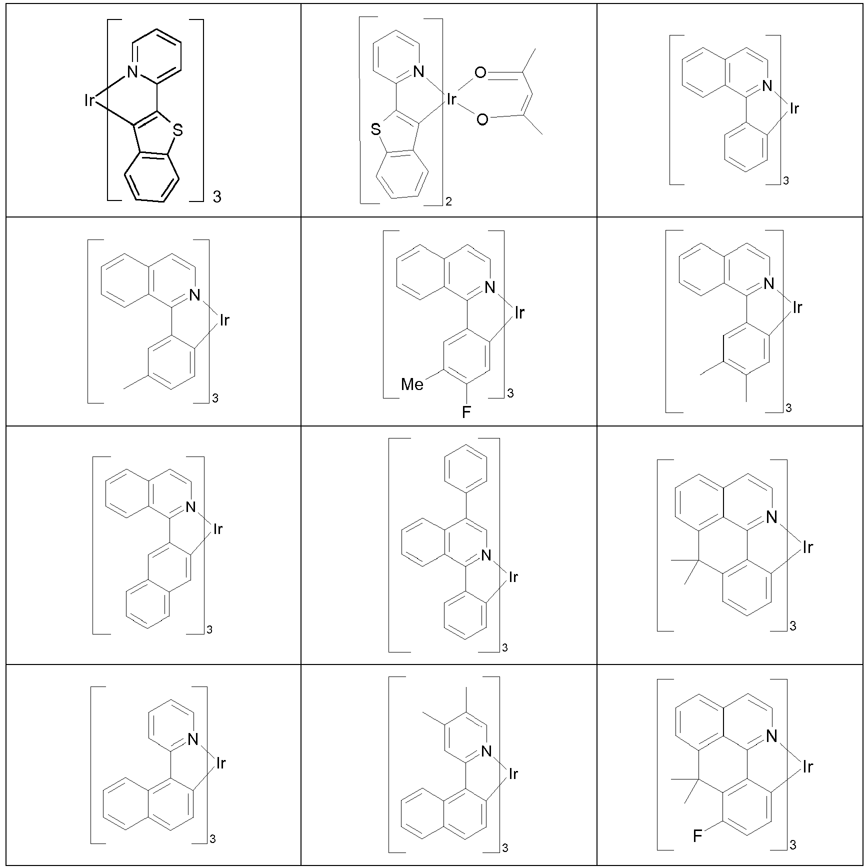

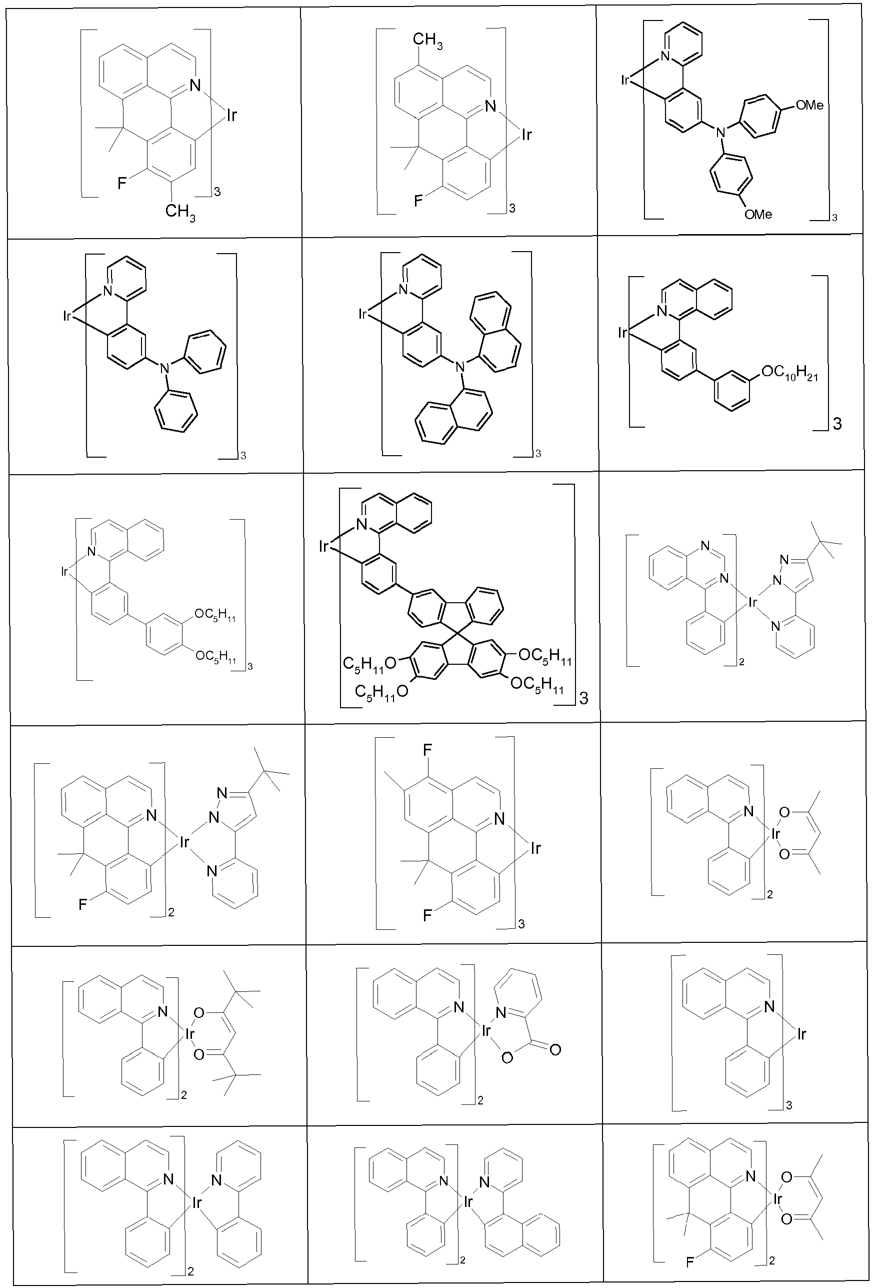

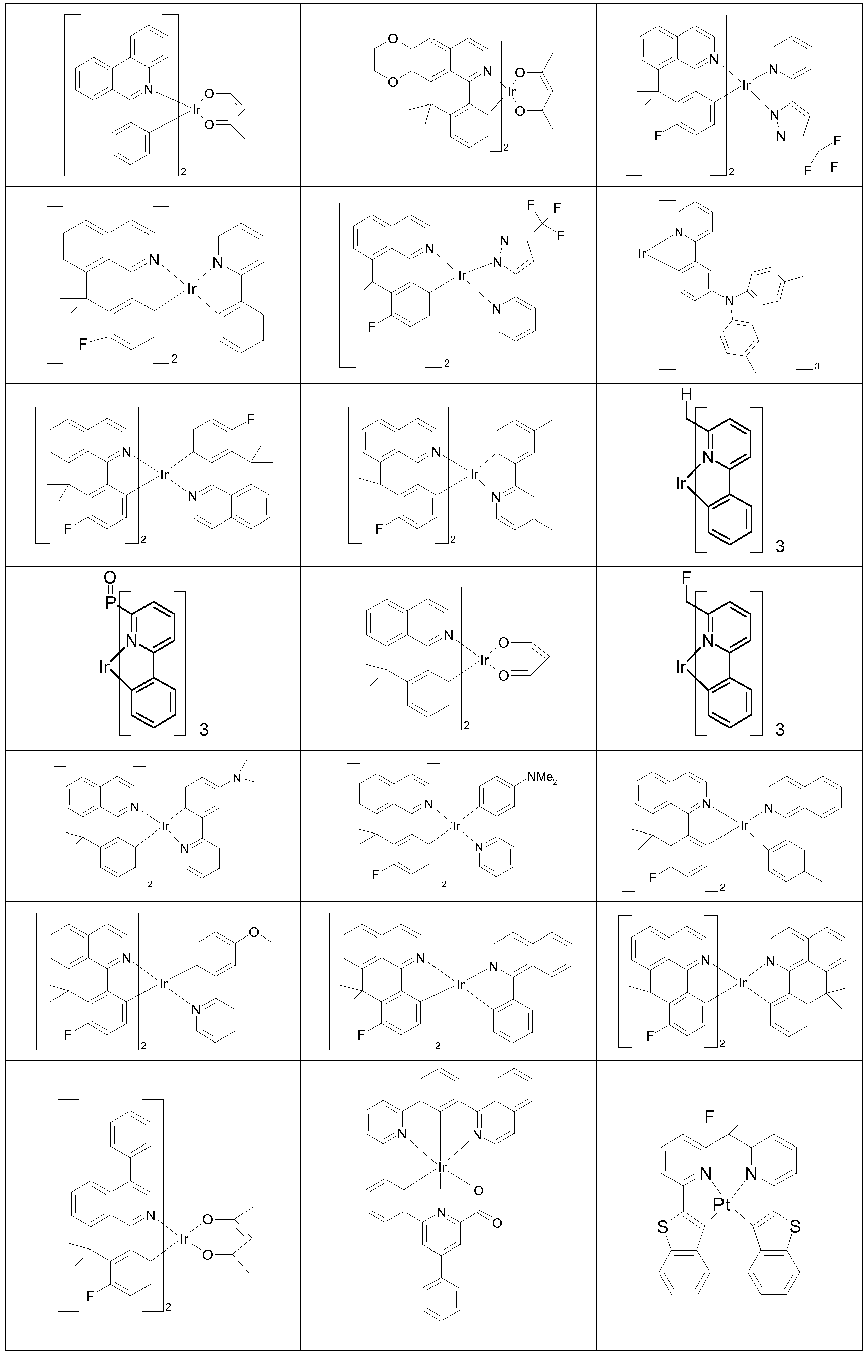

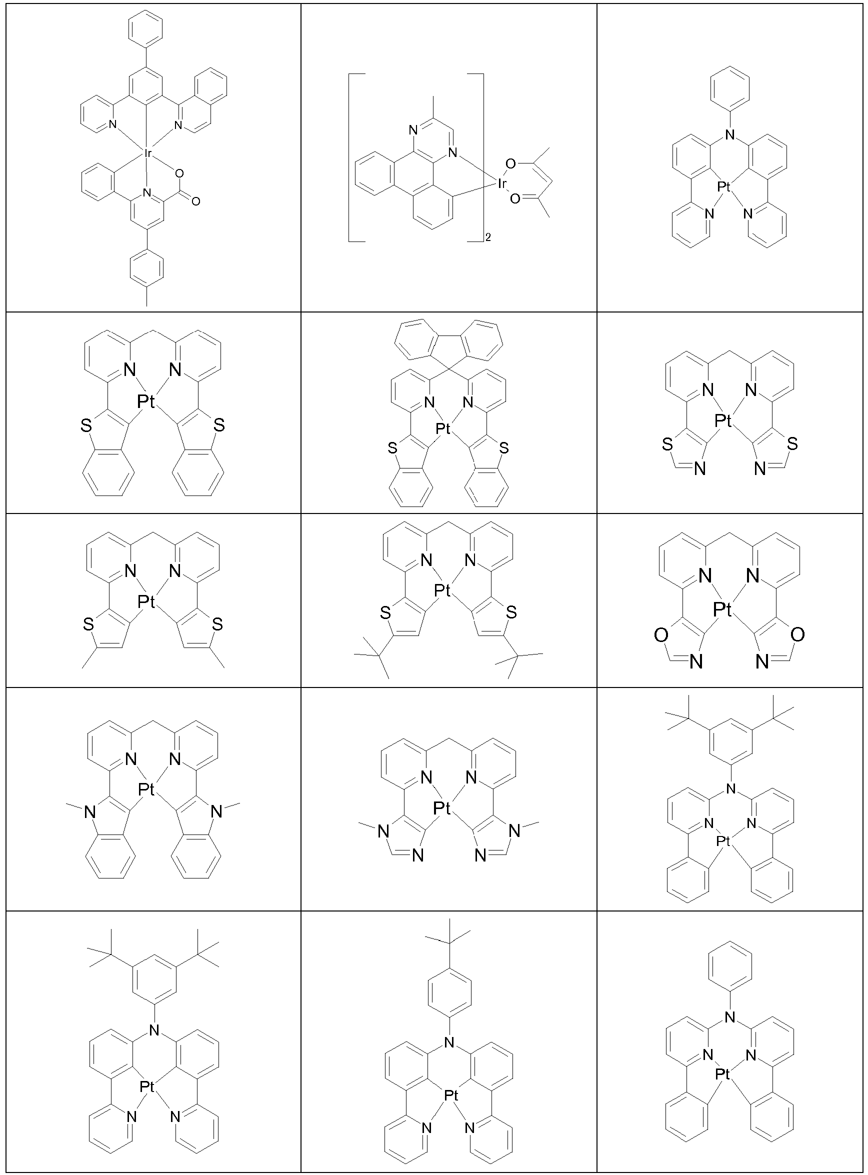

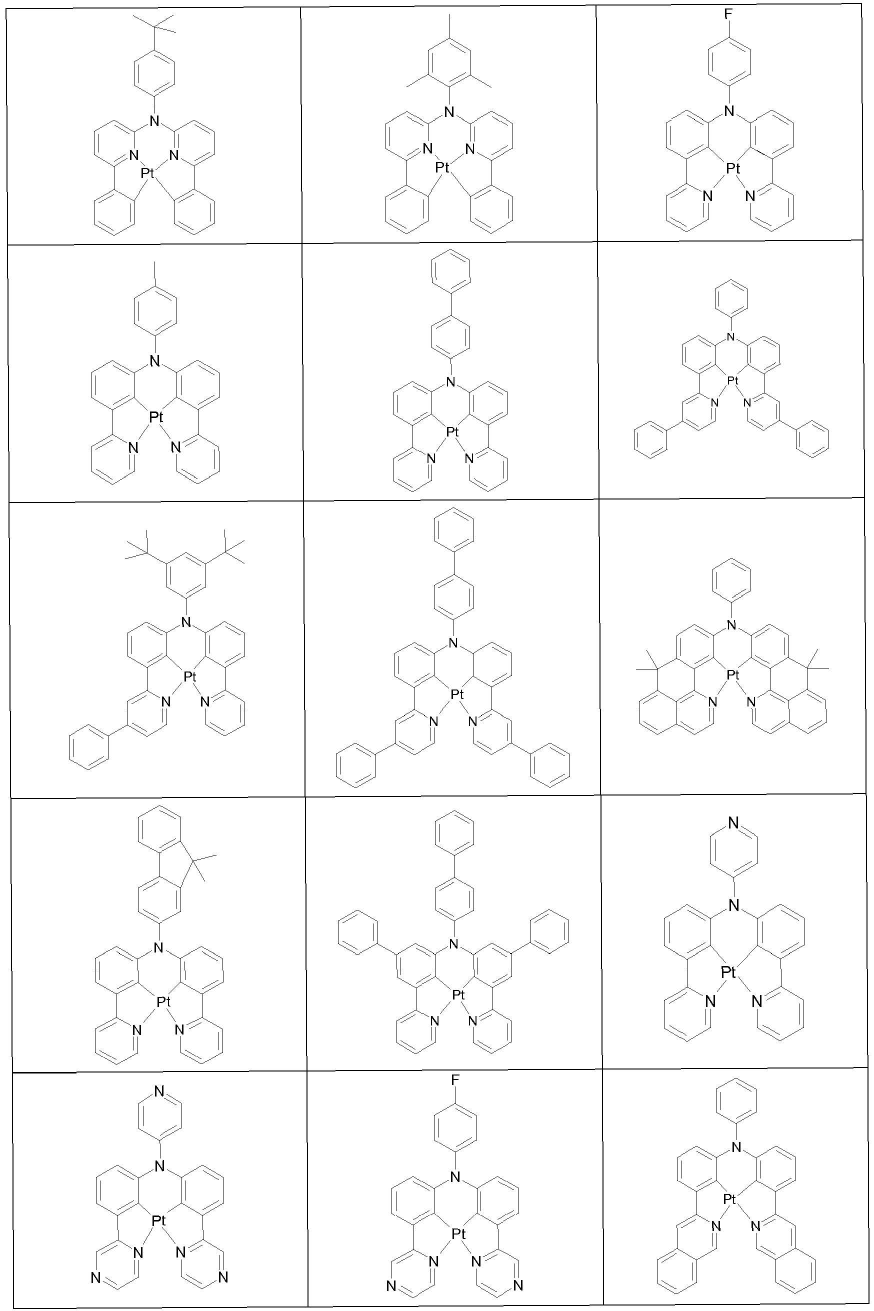

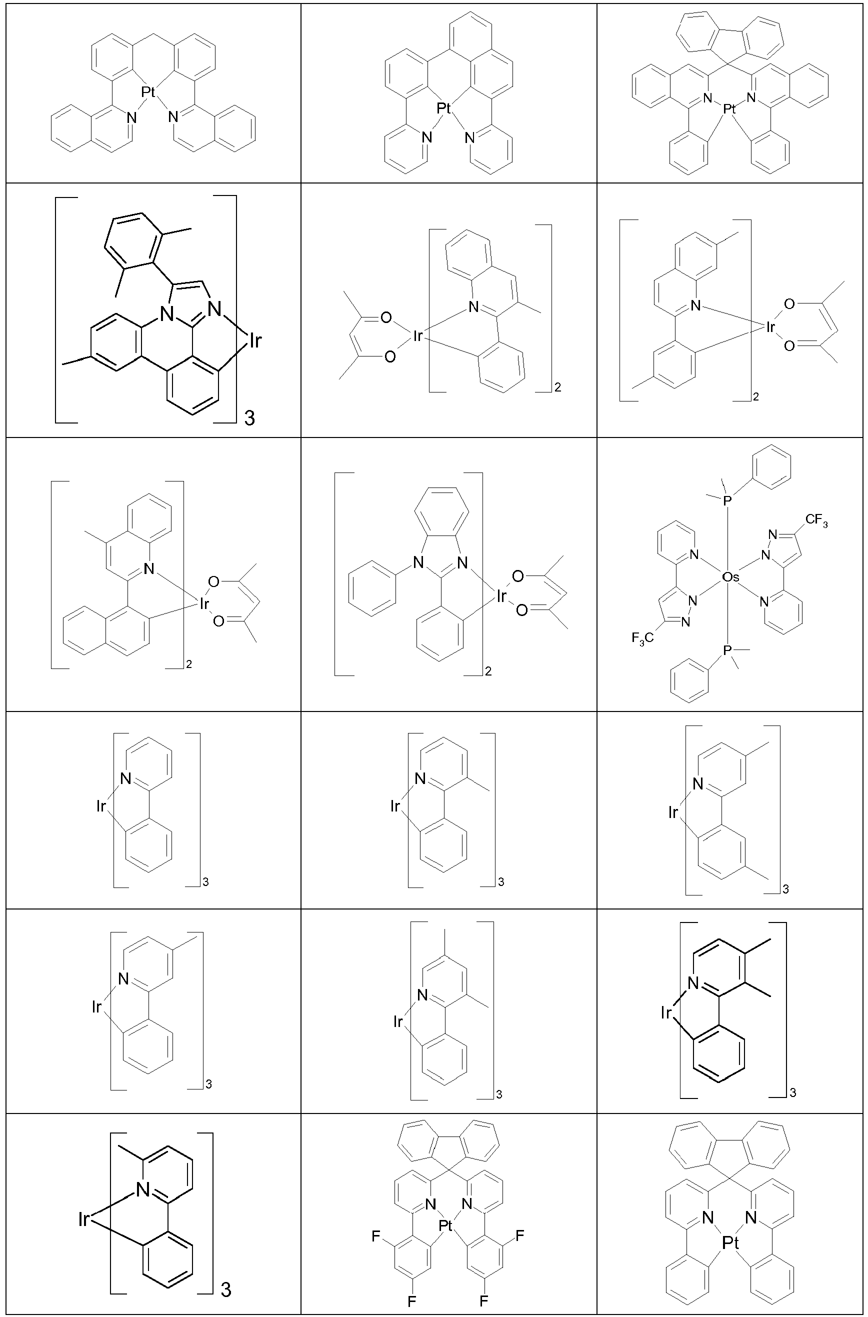

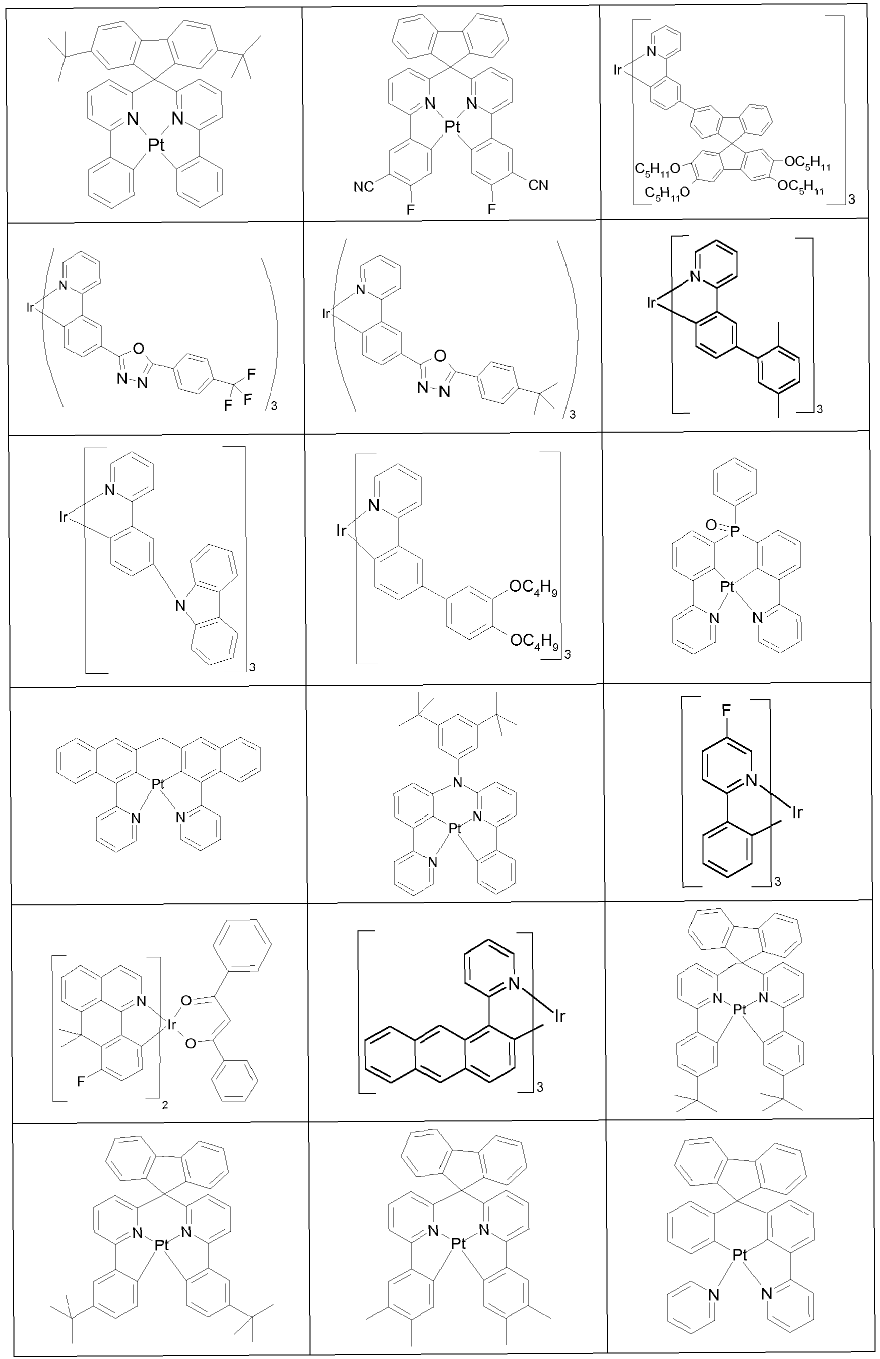

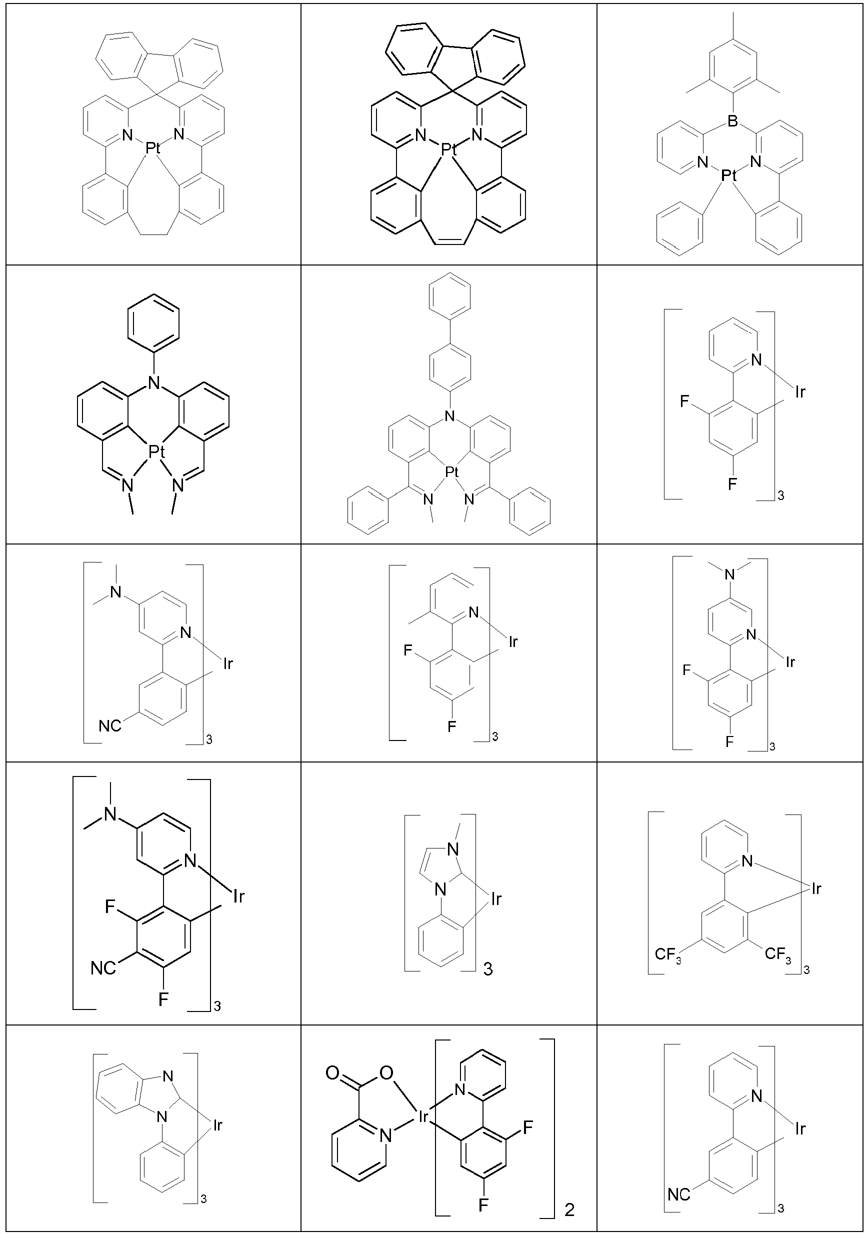

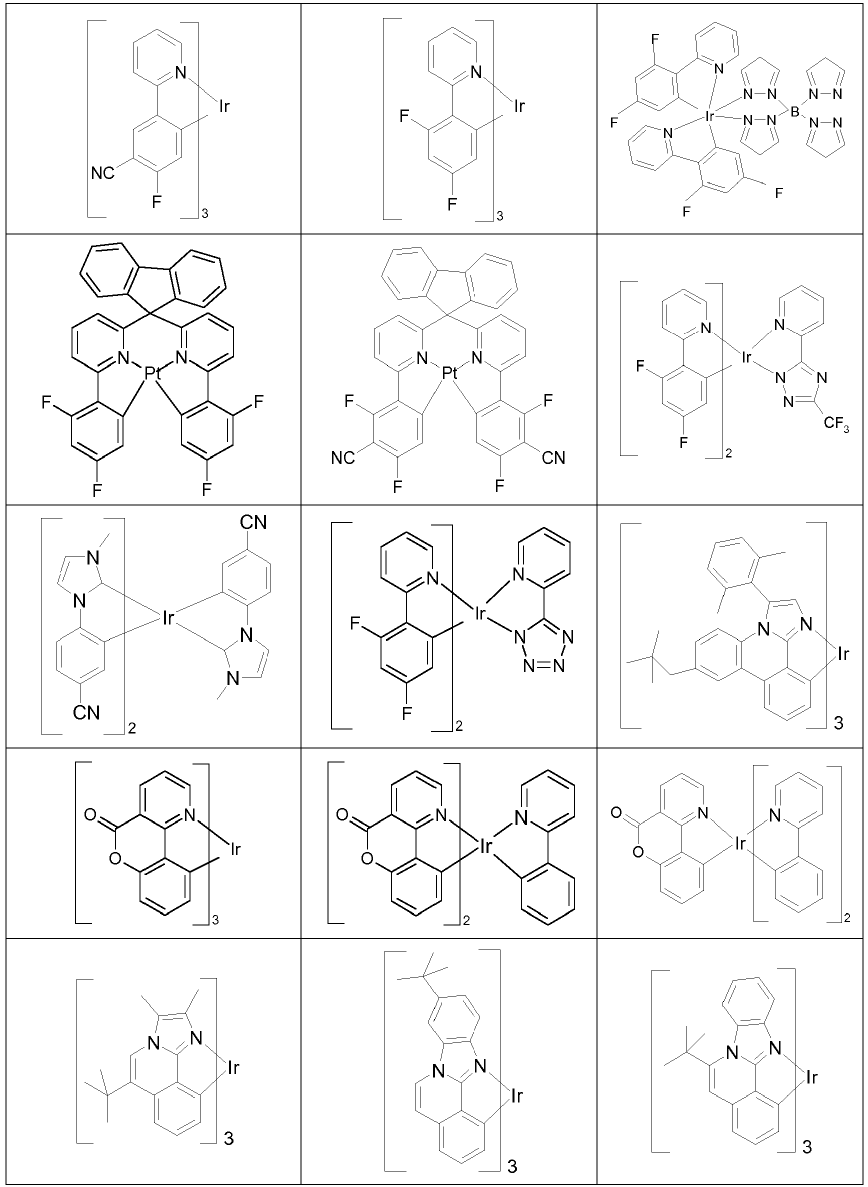

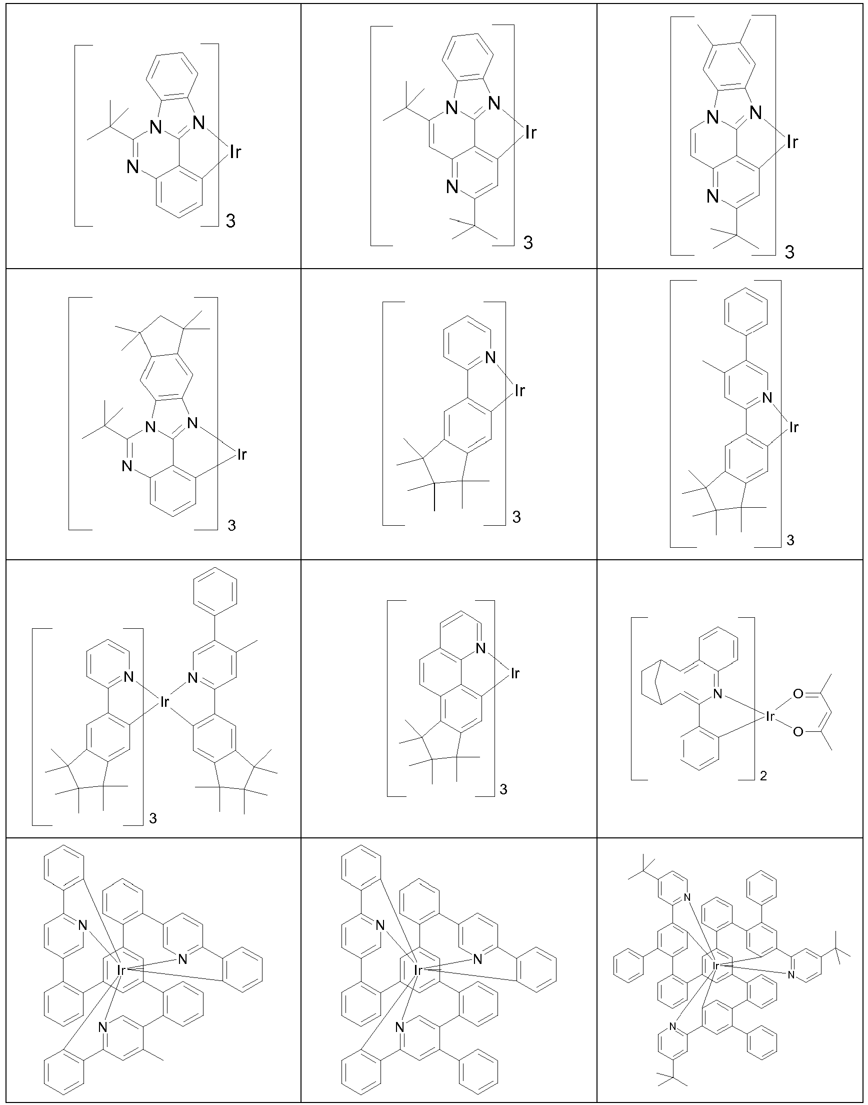

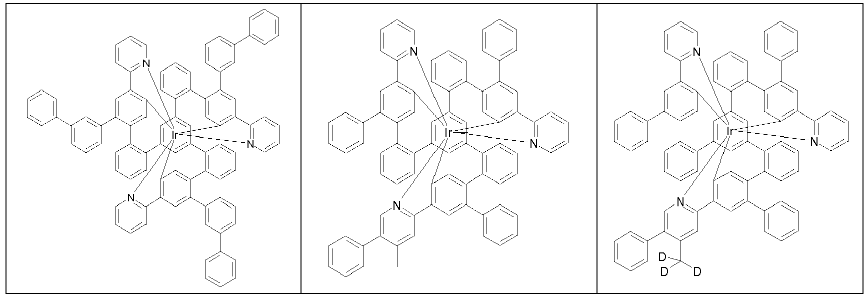

In einer bevorzugten Ausführungsform der Erfindung werden die Verbindungen gemäß Formel (I) als lochtransportierendes Material eingesetzt. Die Verbindungen liegen dann bevorzugt in einer lochtransportierenden Schicht vor. Bevorzugte Ausführungsformen von lochtransportierenden Schichten sind Lochtransportschichten, Elektronenblockierschichten und Lochinjektionsschichten. Wenn die Verbindung der Formel (I) in einer lochtransportierenden Schicht vorliegt, ist diese bevorzugt eine elektronenblockierende Schicht. Diese grenzt bevorzugt anodenseitig direkt an die emittierende Schicht an.

Eine Lochtransportschicht gemäß der vorliegenden Anmeldung ist eine Schicht mit lochtransportierender Funktion, welche sich zwischen Anode und emittierender Schicht befindet. Insbesondere ist es eine lochtransportierende Schicht, die keine Lochinjektionsschicht und keine Elektronenblockierschicht ist.

Lochinjektionsschichten und Elektronenblockierschichten werden im Sinne der vorliegenden Anmeldung als spezielle Ausführungsformen von lochtransportierenden Schichten verstanden. Eine Lochinjektionsschicht ist dabei im Fall von mehreren lochtransportierenden Schichten zwischen Anode und emittierender Schicht eine lochtransportierende Schicht, welche sich direkt an die Anode anschließt oder nur durch eine einzelne Beschichtung der Anode von ihr getrennt ist. Eine Elektronenblockierschicht ist im Fall von mehreren lochtransportierenden Schichten zwischen Anode und emittierender Schicht diejenige lochtransportierende Schicht, welche sich direkt anodenseitig an die emittierende Schicht anschließt. Bevorzugt enthält die erfindungsgemäße OLED zwei, drei oder vier lochtransportierende Schichten zwischen Anode und emittierender Schicht, von denen bevorzugt mindestens eine eine Verbindung gemäß Formel (I) enthält, besonders bevorzugt genau eine oder zwei eine Verbindung gemäß Formel (I) enthalten.

Wird die Verbindung gemäß Formel (I) als Lochtransportmaterial in einer Lochtransportschicht, einer Lochinjektionsschicht oder einer Elektronenblockierschicht eingesetzt, so kann die Verbindung als Reinmaterial, d.h. in einem Anteil von 100 %, in der Lochtransportschicht eingesetzt werden, oder sie kann in Kombination mit einer oder mehreren weiteren Verbindungen eingesetzt werden. Gemäß einer bevorzugten Ausführungsform enthält die organische Schicht enthaltend die Verbindung der Formel (I) dann zusätzlich einen oder mehrere p-Dotanden. Als p-Dotanden werden gemäß der vorliegenden Erfindung bevorzugt solche organischen Elektronenakzeptorverbindungen eingesetzt, die eine oder mehrere der anderen Verbindungen der Mischung oxidieren können.

Besonders bevorzugte Ausführungsformen von p-Dotanden sind die in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709, US 2010/0096600, WO 2012/095143 und DE 102012209523 offenbarten Verbindungen.

Besonders bevorzugt als p-Dotanden sind Chinodimethanverbindungen, Azaindenofluorendione, Azaphenalene, Azatriphenylene, I₂, Metallhalogenide, bevorzugt Übergangsmetallhalogenide, Metalloxide, bevorzugt Metalloxide enthaltend mindestens ein Übergangsmetall oder ein Metall der 3. Hauptgruppe, und Übergangsmetallkomplexe, bevorzugt Komplexe von Cu, Co, Ni, Pd und Pt mit Liganden enthaltend mindestens ein Sauerstoffatom als Bindungsstelle. Bevorzugt sind weiterhin Übergangsmetalloxide als Dotanden, bevorzugt Oxide von Rhenium, Molybdän und Wolfram, besonders bevorzugt Re₂O₇, MoO₃, WO₃ und ReO₃.

Die p-Dotanden liegen bevorzugt weitgehend gleichmäßig verteilt in den p-dotierten Schichten vor. Dies kann beispielsweise durch Co-Verdampfung des p-Dotanden und der Lochtransportmaterial-Matrix erreicht werden.

Bevorzugt sind als p-Dotanden insbesondere die folgenden Verbindungen:

| | | |
|---|---|---|
| 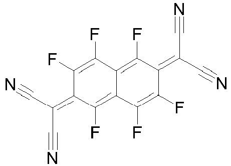 | 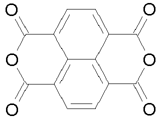 | 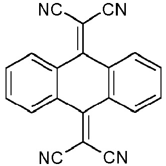 |
| (D-1) | (D-2) | (D-3) |
| 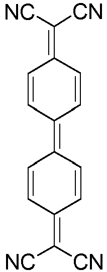 | 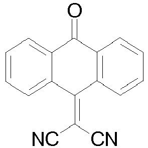 | 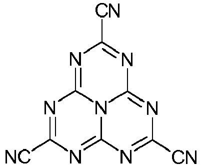 |
| (D-4) | (D-5) | (D-6) |
| 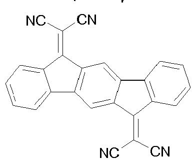 | 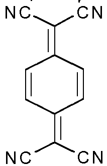 | 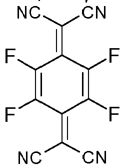 |
| (D-7) | (D-8) | (D-9) |
| 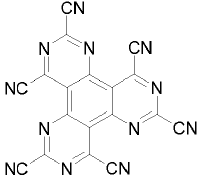 | 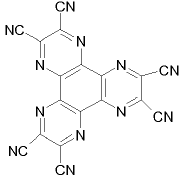 | 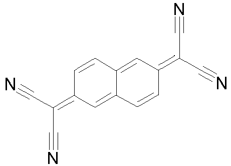 |
| (D-10) | (D-11) | (D-12) |
| 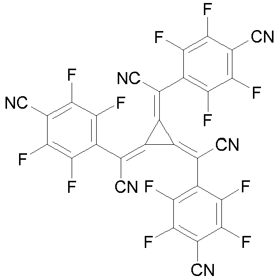 | | |
| (D-13) | | |

In einer weiteren bevorzugten Ausführungsform der Erfindung wird die Verbindung gemäß Formel (I) als Lochtransportmaterial in Kombination mit einem Hexaazatriphenylenderivat, wie in US 2007/0092755 beschrieben, in einer OLED verwendet. Besonders bevorzugt wird das Hexaazatriphenylenderivat dabei in einer separaten Schicht eingesetzt.

In einer bevorzugten Ausführungsform der vorliegenden Erfindung wird die Verbindung der Formel (I) in einer emittierenden Schicht als Matrixmaterial in Kombination mit einer oder mehreren emittierenden Verbindungen, vorzugsweise phosphoreszierenden emittierenden Verbindungen, eingesetzt. Die phosphoreszierenden emittierenden Verbindungen sind dabei bevorzugt gewählt aus rot phosphoreszierenden und grün phosphoreszierenden Verbindungen.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt zwischen 85.0 und 97.0 Vol.-%.

Entsprechend beträgt der Anteil der emittierenden Verbindung zwischen 0.1 und 50.0 Vol.-%, bevorzugt zwischen 0.5 und 20.0 Vol.-% und besonders bevorzugt zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere emittierende Verbindungen enthalten. Auch in diesem Fall sind die emittierenden Verbindungen im Allgemeinen diejenigen Verbindungen, deren Anteil im System der kleinere ist und die Matrixmaterialien sind diejenigen Verbindungen, deren Anteil im System der größere ist. In Einzelfällen kann jedoch der Anteil eines einzelnen Matrixmaterials im System kleiner sein als der Anteil einer einzelnen emittierenden Verbindung.

Es ist bevorzugt, dass die Verbindungen gemäß Formel (I) als eine Komponente von Mixed-Matrix-Systemen, bevorzugt für phosphoreszierende Emitter, verwendet werden. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die Verbindung der Formel (I) stellt dabei bevorzugt das Matrixmaterial mit lochtransportierenden Eigenschaften dar. Entsprechend ist, wenn die Verbindung der Formel (I) als Matrixmaterial für einen phosphoreszierenden Emitter in der emittierenden Schicht einer OLED eingesetzt wird, eine zweite Matrixverbindung in der emittierenden Schicht vorhanden, die elektronentransportierende Eigenschaften aufweist. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Genauere Angaben zu Mixed-Matrix-Systemen sind unter anderem in der Anmeldung WO 2010/108579 enthalten, deren entsprechende technische Lehre an in diesem Zusammenhang mit einbezogen ist.

Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen.

Die Mixed-Matrix-Systeme können einen oder mehrere emittierende Verbindungen umfassen, bevorzugt eine oder mehrere phosphoreszierende emittierende Verbindungen. Allgemein werden Mixed-Matrix-Systeme bevorzugt in phosphoreszierenden organischen Elektrolumineszenzvorrichtungen eingesetzt.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende emittierende Verbindungen, darunter insbesondere aus denjenigen, die elektronentransportierende Eigenschaften aufweisen.

Im Folgenden werden bevorzugte Ausführungsformen für die verschiedenen Funktionsmaterialien der elektronischen Vorrichtung aufgeführt.

Bevorzugte fluoreszierende emittierende Verbindungen sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte emittierende Verbindungen sind Indenofluorenamine bzw. - diamine, beispielsweise gemäß WO 2006/108497 oder WO 2006/122630, Benzoindenofluorenamine bzw. -diamine, beispielsweise gemäß WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen. Ebenfalls bevorzugt sind die in WO 2012/048780 und die in WO 2013/185871 offenbarten Pyren-Arylamine. Ebenfalls bevorzugt sind die in WO 2014/037077 offenbarten Benzoindenofluoren-Amine, die in WO 2014/106522 offenbarten Benzofluoren-Amine, die in WO 2014/111269 und in WO 2017/036574 offenbarten erweiterten Benzoindenofluorene, die in WO 2017/028940 und WO 2017/028941 offenbarten Phenoxazine, und die in WO 2016/150544 offenbarten Fluoren-Derivate, die mit Furan-Einheiten oder mit Thiophen-Einheiten verbunden sind.

Als Matrixmaterialien, bevorzugt für fluoreszierende emittierende Verbindungen, kommen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind. Bevorzugt sind weiterhin die in WO 2006/097208, WO 2006/131192, WO 2007/065550, WO 2007/110129, WO 2007/065678, WO 2008/145239, WO 2009/100925, WO 2011/054442, und EP 1553154 offenbarten Anthracenderivate, die in EP 1749809, EP 1905754 und US 2012/0187826 offenbarten Pyren-Verbindungen, die in WO 2015/158409 offenbarten Benzanthracenyl-Anthracen-Verbindungen, die in WO 2017/025165 offenbarten Indeno-Benzofurane, und die in WO 2017/036573 offenbarten Phenanthryl-Anthracene.

Bevorzugte Matrixmaterialien für phosphoreszierende emittierende Verbindungen sind neben den Verbindungen der Formel (I) aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109, WO 2011 /000455 oder WO 2013/041176, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinckomplexe, z. B. gemäß EP 652273 oder WO 2009/062578, Diazasilol- bzw. Tetraazasilol-Derivate, z. B. gemäß WO 2010/054729, DiazaphospholDerivate, z. B. gemäß WO 2010/054730, überbrückte Carbazol-Derivate, z. B. gemäß US 2009/0136779, WO 2010/050778, WO 2011/042107, WO 2011/088877 oder WO 2012/143080, Triphenylenderivaten, z. B. gemäß WO 2012/048781, oder Lactame, z. B. gemäß WO 2011/116865 oder WO 2011/137951.

Geeignete Ladungstransportmaterialien, wie sie in der Lochinjektions- bzw. Lochtransportschicht bzw. Elektronenblockierschicht oder in der Elektronentransportschicht der erfindungsgemäßen elektronischen Vorrichtung verwendet werden können, sind neben den Verbindungen der Formel (I) beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden. Bevorzugte Materialien für lochtransportierende und lochinjizierende Schichten sind insbesondere gewählt aus den folgenden Materialien:

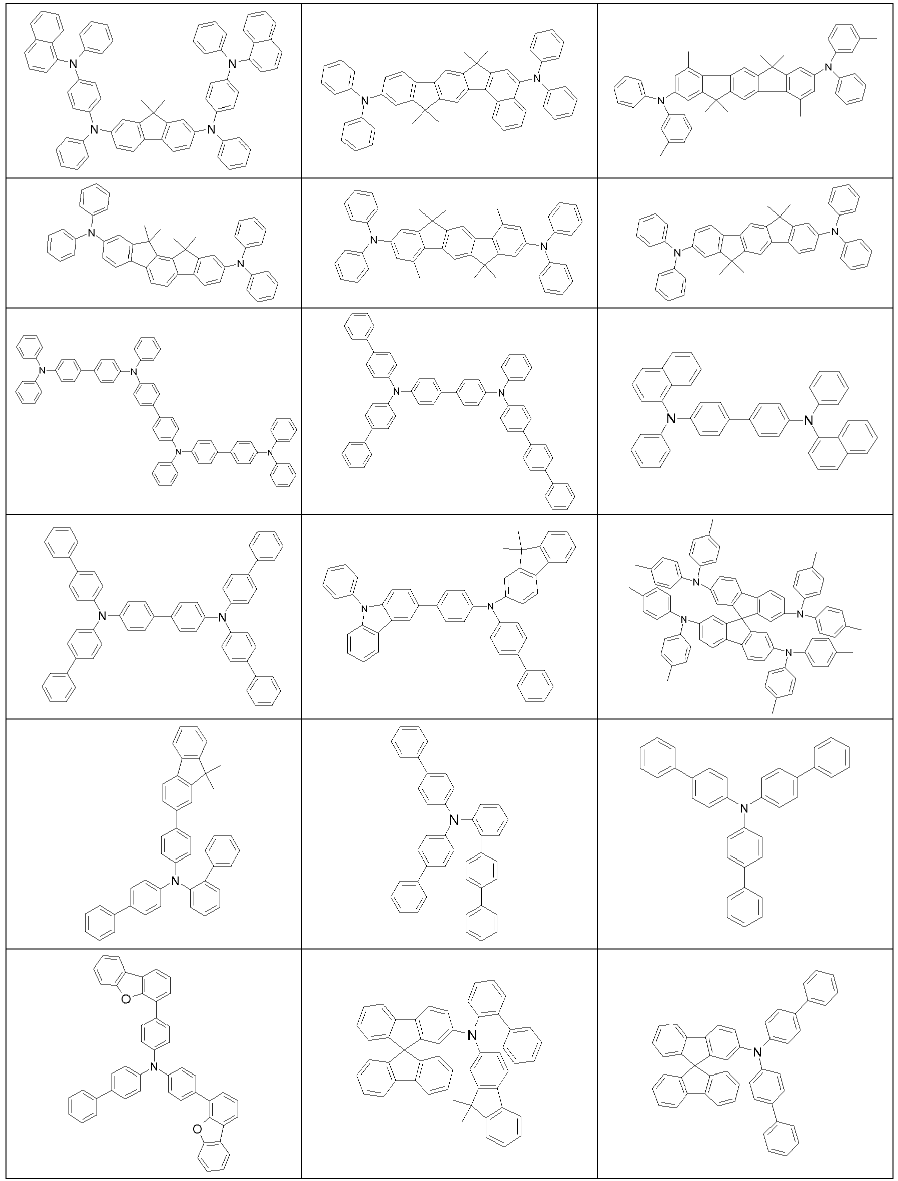

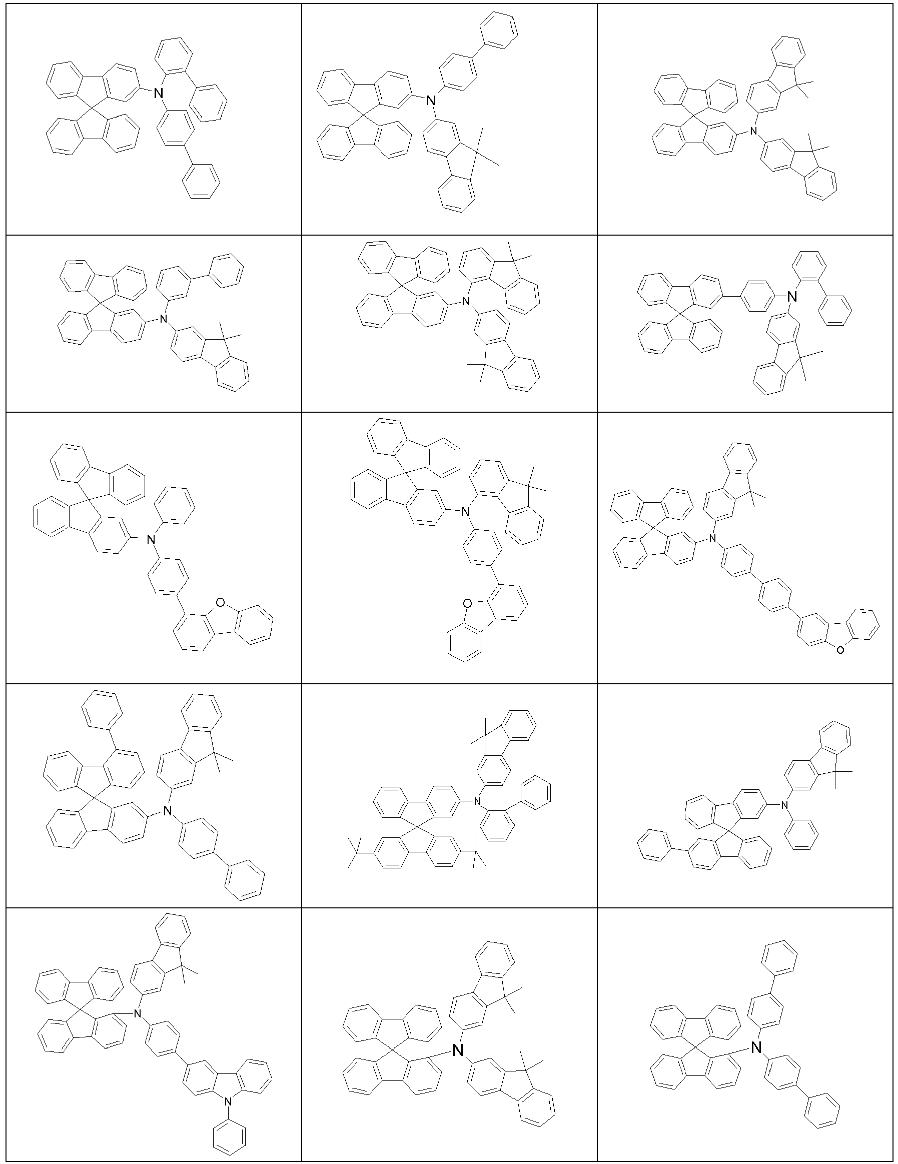

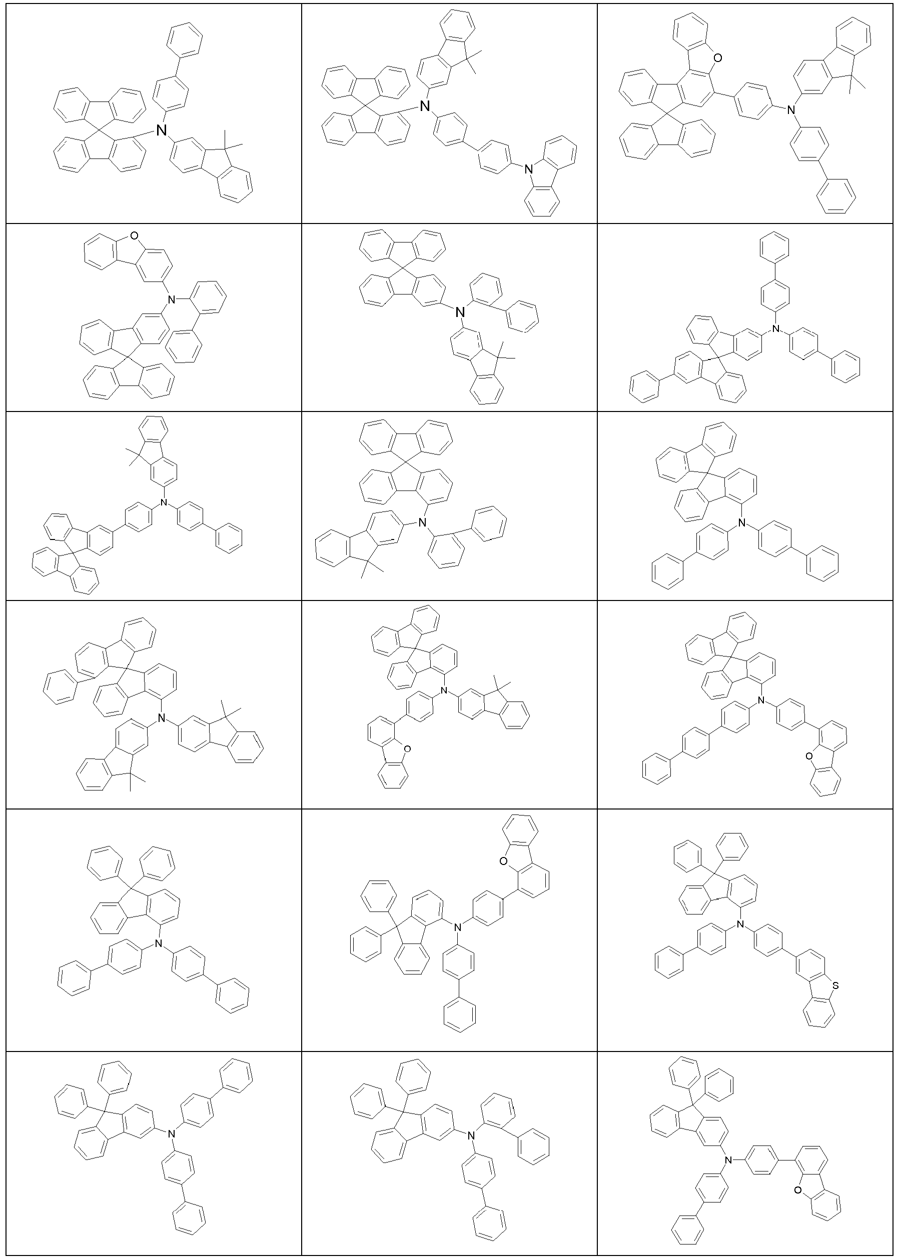

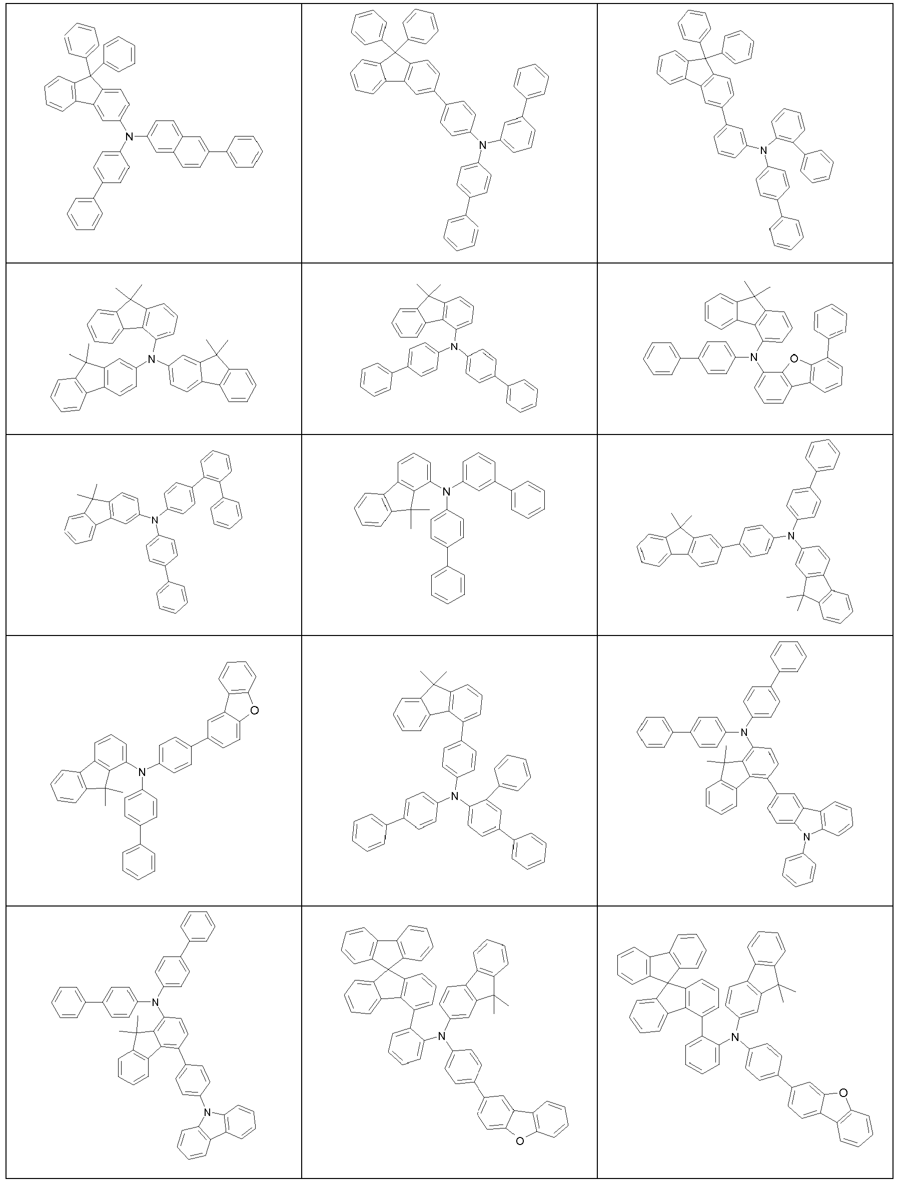

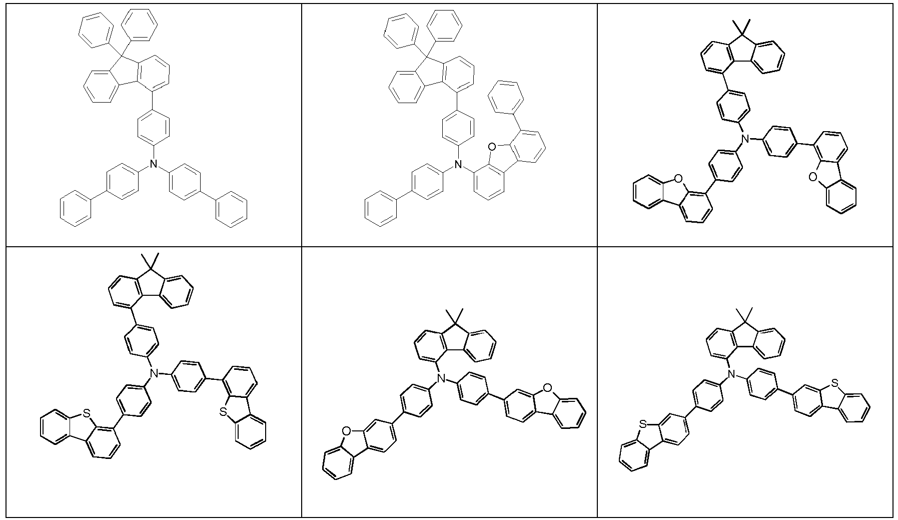

Bevorzugt umfasst die erfindungsgemäße OLED zwei oder mehr unterschiedliche lochtransportierende Schichten. Die Verbindung der Formel (I) kann dabei in einer oder in mehreren oder in allen lochtransportierenden Schichten eingesetzt werden. Gemäß einer bevorzugten Ausführungsform wird die Verbindung der Formel (I) in genau einer oder genau zwei lochtransportierenden Schichten eingesetzt, und in den weiteren vorhandenen lochtransportierenden Schichten werden andere Verbindungen eingesetzt, bevorzugt aromatische Aminverbindungen. Weitere Verbindungen, die neben den Verbindungen der Formel (I) bevorzugt in lochtransportierenden Schichten der erfindungsgemäßen OLEDs eingesetzt werden, sind insbesondere Indenofluorenamin-Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627 oder WO 2013/120577), Fluoren-Amine (z. B. gemäß WO 2014/015937, WO 2014/015938, WO 2014/015935 und WO 2015/082056), Spiro-Dibenzopyran-Amine (z. B. gemäß WO 2013/083216), Dihydroacridin-Derivate (z. B. gemäß WO 2012/150001), Spirodibenzofurane und Spirodibenzothiophene, z.B. gemäß WO 2015/022051, WO 2016/102048 und WO 2016/131521, Phenanthren-Diarylamine, z.B. gemäß WO 2015/131976, Spiro-Tribenzotropolone, z.B. gemäß WO 2016/087017, Spirobifluorene mit meta-Phenyldiamingruppen, z.B. gemäß WO 2016/078738, Spiro-Bisacridine, zB. gemäß WO 2015/158411, Xanthen-Diarylamine, z.B. gemäß WO 2014/072017, und 9,10-Dihydroanthracen-Spiroverbindungen mit Diarylaminogruppen gemäß WO 2015/086108.

Als Materialien für die Elektronentransportschicht können alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise Alq₃, Zirkoniumkomplexe, beispielsweise Zrq₄, Lithiumkomplexe, beispielsweise Liq, Benzimidazolderivate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Weiterhin geeignete Materialien sind Derivate der oben genannten Verbindungen, wie sie in JP 2000/053957, WO 2003/060956, WO 2004/028217, WO 2004/080975 und WO 2010/072300 offenbart werden.

Als Kathode der elektronischen Vorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die Vorrichtung wird entsprechend (je nach Anwendung) strukturiert, kontaktiert und schließlich versiegelt, um schädigende Effekte von Wasser und Luft auszuschließen.

In einer bevorzugten Ausführungsform ist die elektronische Vorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine elektronische Vorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronische Vorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I) nötig. Hohe Löslichkeit lässt sich durch geeignete Substitution der Verbindungen erreichen.

Weiterhin bevorzugt ist es, dass zur Herstellung einer erfindungsgemäßen elektronischen Vorrichtung eine oder mehrere Schichten aus Lösung und eine oder mehrere Schichten durch ein Sublimationsverfahren aufgetragen werden.

Erfindungsgemäß können die elektronischen Vorrichtungen enthaltend eine oder mehrere Verbindungen gemäß Formel (I) in Displays, als Lichtquellen in Beleuchtungsanwendungen sowie als Lichtquellen in medizinischen und/oder kosmetischen Anwendungen (z.B. Lichttherapie) eingesetzt werden.

### Beispiele

### A) Synthesebeispiele

### Beispiel 1-1: Synthese der erfindungsgemäßen Verbindung 1-1 und Varianten

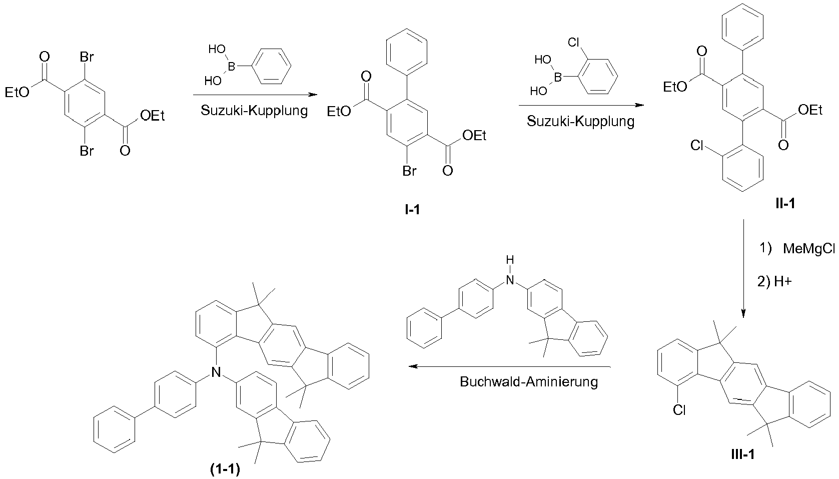

### Zwischenstufe I-1

10 g Phenylboronsäure (81 mmol) und 30 g Di-Brom-dicarbonsäureester (CAS-Nr. 18013-97-3) (77mmol) werden in 750 mL THF suspendiert. 160 ml 2 M Kaliumcarbonat-Lösung werden langsam zugetropft. Die Lösung wird entgast und mit N₂ gesättigt. Danach wird sie mit 0,89 g (0,8 mmol) Pd(Ph₃P)₄ versetzt. Die Reaktionsmischung wird 16 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Der verbleibende Rückstand wird mittels Säulenchromatographie aufgereinigt. Die Ausbeute beträgt 15,3 g (52% d. Th).

### Zwischenstufe II-1

7 g 4-Chlorphenylboronsäure (44,6 mmol) und 15,3 g des Brom-Derivats I-1 (40,56 mmol) werden in 300 mL THF suspendiert. 81 mL 1 M Kaliumcarbonat-Lösung werden langsam zugetropft. Die Lösung wird entgast und mit N₂ gesättigt. Danach wird sie mit 0,45 g (0,4 mmol) Pd(Ph₃P)₄ versetzt. Die Reaktionsmischung wird 12 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Nach Filtration des Rohproduktes über Kieselgel mit Toluol wird der verbleibende Rückstand aus MeOH umkristallisiert. Die Ausbeute beträgt 14,5 g (80% d. Th).

Analog dazu werden folgende Verbindungen hergestellt (Ausbeute 30-90 % d. Th.):

| | **Edukt 1** | **Boronsäure Derivat 1** | **Boronsäure Derivat 2** | **Produkt** |
|---|---|---|---|---|
| **II-2** | 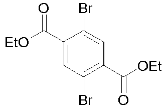 | 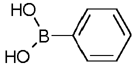 | 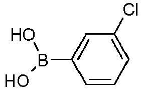 | 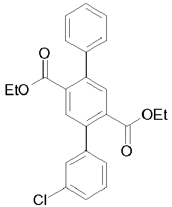 |
| **II-3** | 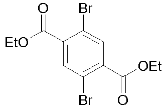 | 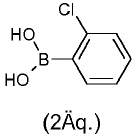 | | 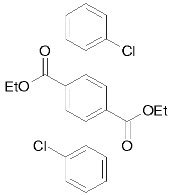 |
| **II-4** | 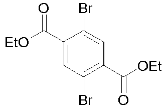 | 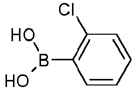 | 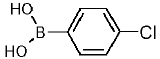 | 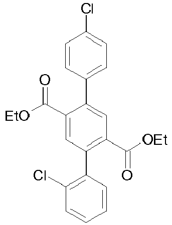 |
| **II-5** | 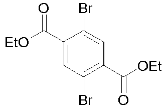 | 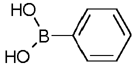 | 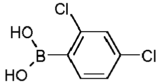 | 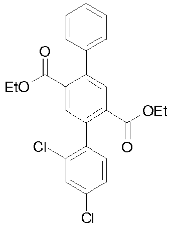 |
| **II-6** | 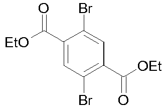 | 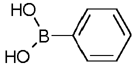 | 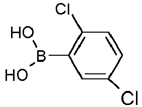 | 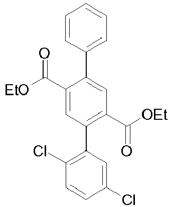 |
| **II-7** | 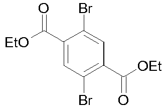 | 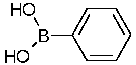 | 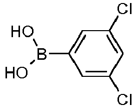 | 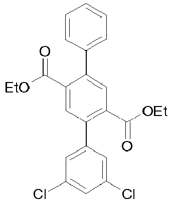 |
| **II-8** | 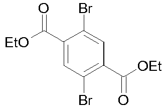 | 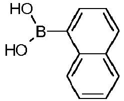 | 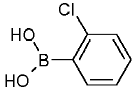 | 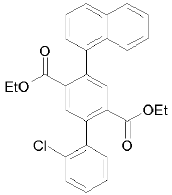 |
| **II-9** | 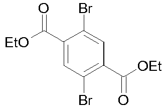 | 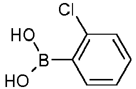 | 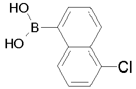 | 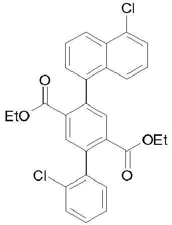 |
| **II-10** | 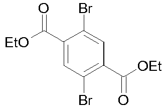 | 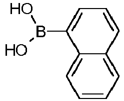 | 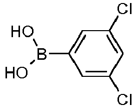 | 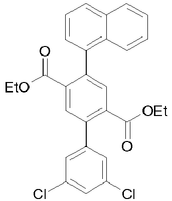 |
| **II-11** | 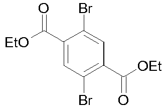 | 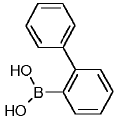 | 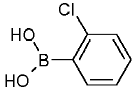 | 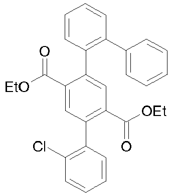 |
| **II-12** | 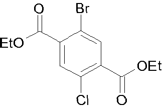 | 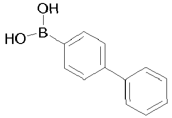 | 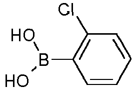 | 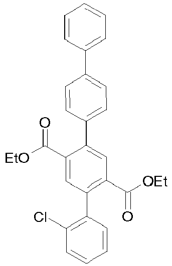 |
| | (340148-60-9) | | | |
| **II-13** | 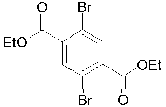 | 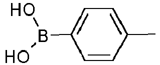 | 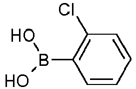 | 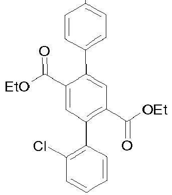 |
| **II-14** | 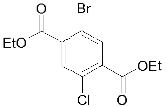 | 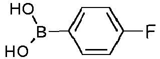 | 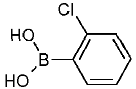 | 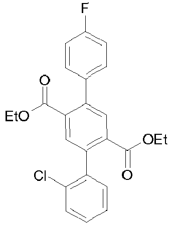 |
| | (340148-60-9) | | | |
| **II-15** | 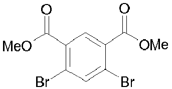 | 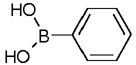 | 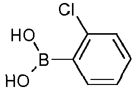 | 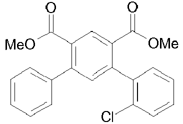 |
| | (90766-77-1) | | | |
| **II-16** | 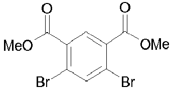 | 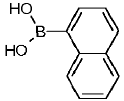 | 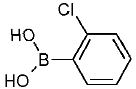 | 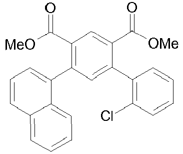 |
| **II-17** | 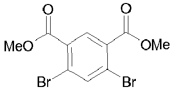 | 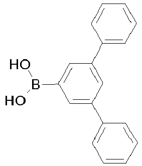 | 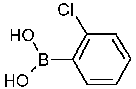 | 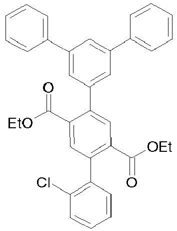 |
| **II-18** | 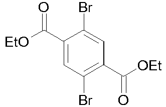 | 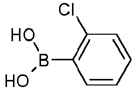 | 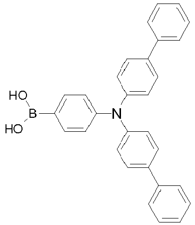 | 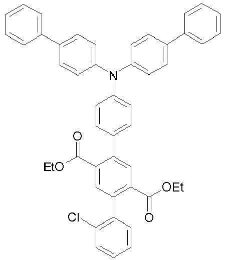 |
| **II-19** | 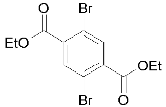 | 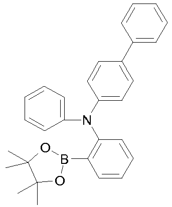 | 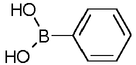 | 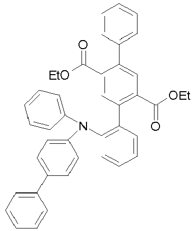 |
| **II-20** | 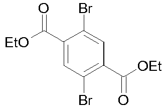 | 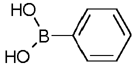 | 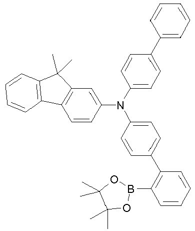 | 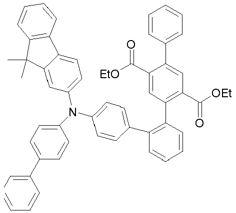 |
| **II-21** | 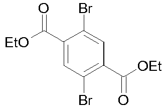 | 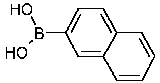 | 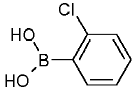 | 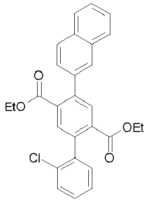 |
| **II-22** | 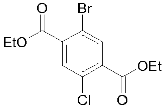 | 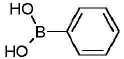 | 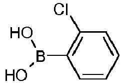 | 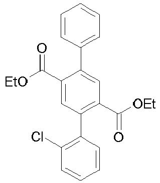 |
| | (340148-60-9) | | | |
| **II-23** | 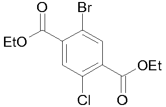 | 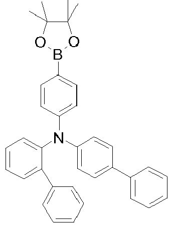 | 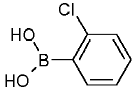 | 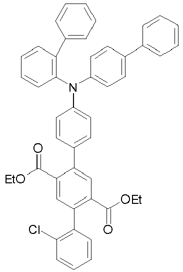 |
| | (340148-60-9) | | | |
| **II-24** | 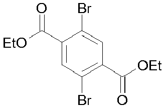 | 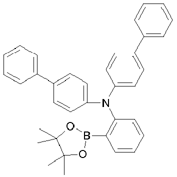 | 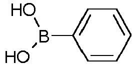 | 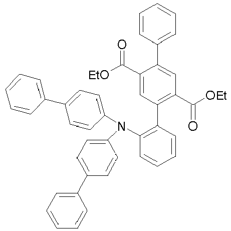 |
| **II-25** | 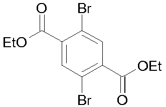 | 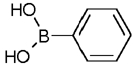 | 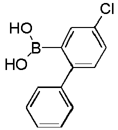 | 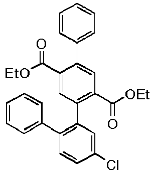 |
| **II-26** | 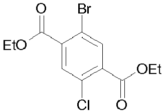 | 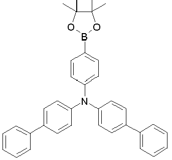 | 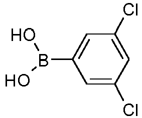 | 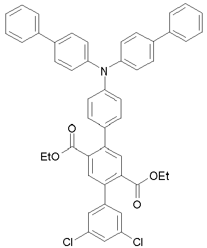 |

### Zwischenstufe III-1

14,0 g (34,2 mmol) Zwischenstufe II-1 werden in einem ausgeheizten Kolben in 250 mL getrocknetem THF gelöst. Die Lösung wird mit N₂ gesättigt. Die klare Lösung wird auf -5°C abgekühlt und dann werden 68,5 mL (205 mmol) einer 3M Methylmagnesiumchlorid-Lösung zugegeben. Die Reaktionsmischung wird langsam auf Raumtemperatur erwärmt und dann mit Ammoniumchlorid gequencht. Das Gemisch wird im Anschluss zwischen Essigester und Wasser verteilt, die organische Phase dreimal mit Wasser gewaschen, über Na₂SO₄ getrocknet und einrotiert.

Die einrotierte Lösung wird in Toluol gelöst und mit 6,6 g Amberlyst 15 versetzt. Der Ansatz wird auf 110°C erhitzt und 8 h auf dieser Temperatur gehalten. Dabei fällt ein weißer Feststoff aus. Der Ansatz wird dann auf Raumtemperatur abgekühlt, der ausgefallene Feststoff wird abgesaugt und mit Heptan nachgewaschen. Der Rückstand wird im Vakuum bei 40°C getrocknet. Nach Filtration des Rohproduktes über Kieselgel mit (Heptan:Essigsester 1:1) erhält man 10,2 g (86% der Theorie) des Produkts III-1.

Analog dazu werden folgende Verbindungen hergestellt (Ausbeuten 50-95% d. Th.):

| | **Edukt 1** | **Edukt 2** | **Produkt** |
|---|---|---|---|
| **III-2** | 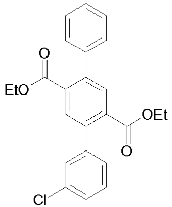 | MeMgBr | 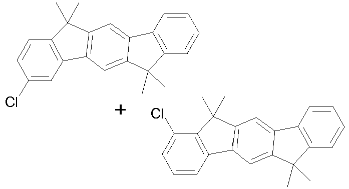 # |
| **III-3** | 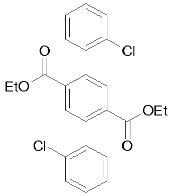 | MeMgBr | 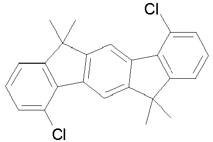 |
| **III-4** | 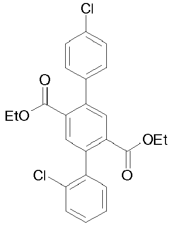 | MeMgCl | 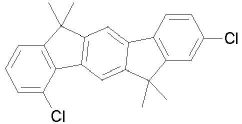 |
| **III-5** | 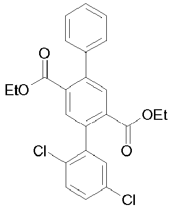 | MeMgCl | 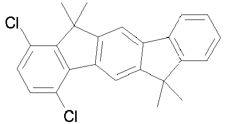 |
| **III-6** | 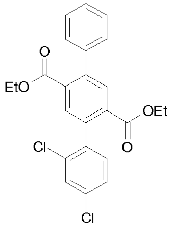 | MeMgCl | 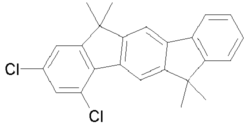 |
| **III-7** | 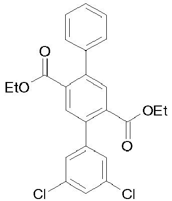 | MeMgBr | 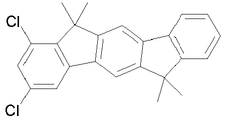 |
| **III-8** | 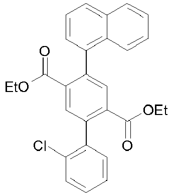 | MeMgCl | 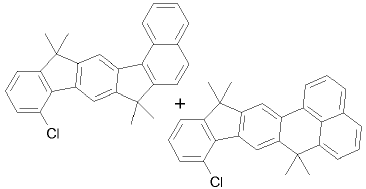 # |
| **III-9** | 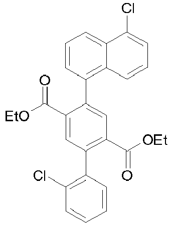 | MeMgCl | 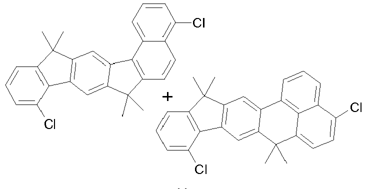 # |
| **III-10** | 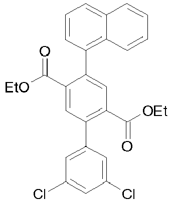 | MeLi | 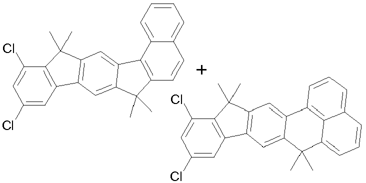 # |
| **III-11** | 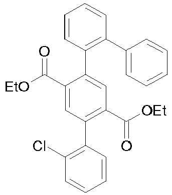 | MeMgBr | 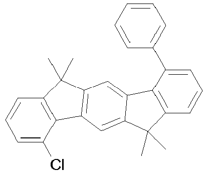 |
| **III-12** | 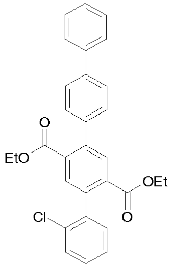 | MeMgCl | 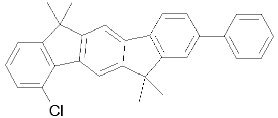 |
| **III-13** | 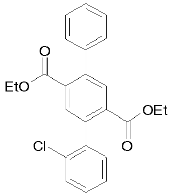 | MeMgCl | 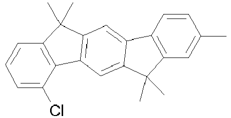 |
| **III-14** | 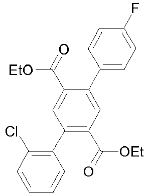 | MeMgCl | 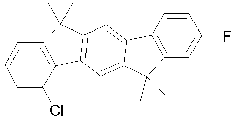 |
| **III-15** | 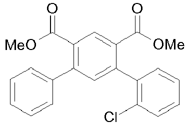 | MeMgCl | 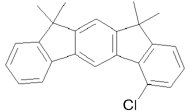 |
| **III-16** | 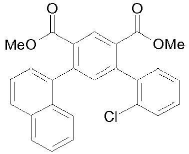 | MeMgBr | 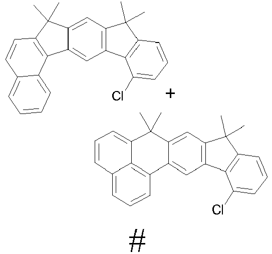 |
| **III-17** | 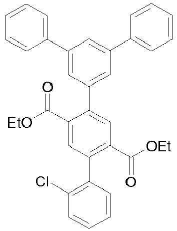 | MeLi | 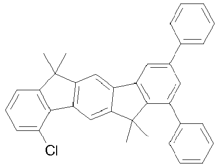 |
| **III-18** | 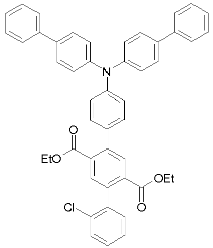 | MeMgCl | 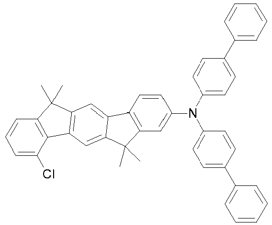 |
| **III-19** | 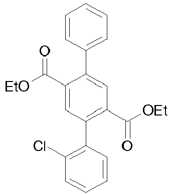 | EtMgBr | 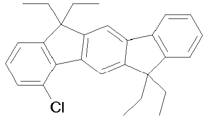 |
| **III-20** | 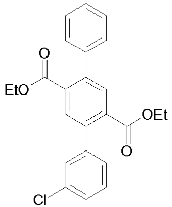 | i-PrMgBr | 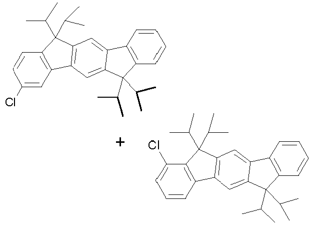 # |
| **III-21** | 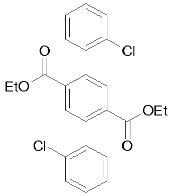 | i-OctMgBr | 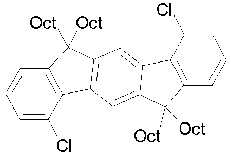 |
| **III-22** | 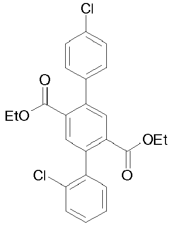 | PhLi | 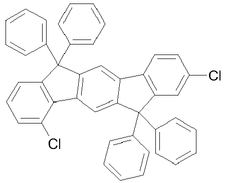 |
| **III-23** | 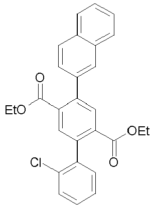 | MeMgBr | 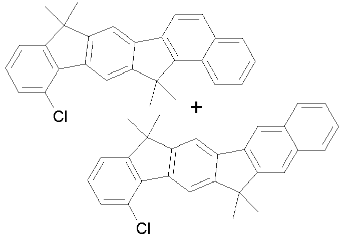 # |
| **III-24** | 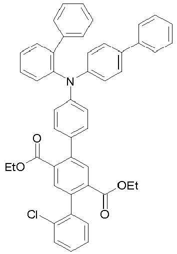 | MeMgBr | 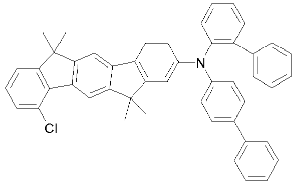 |
| **III-25** | 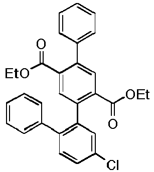 | MeMgCl | 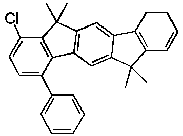 |
| **1-33** | 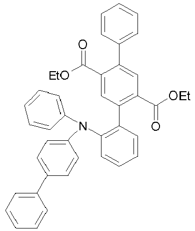 | MeMgBr | 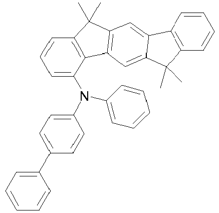 |
| **1-34** | 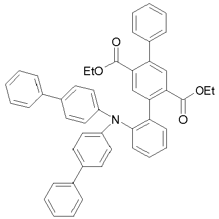 | MeMgBr | 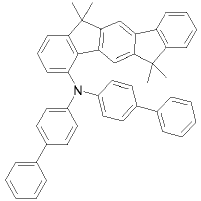 |
| **2-18** | 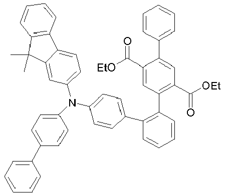 | MeMgCl | 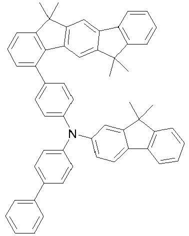 |
| **2-19** | 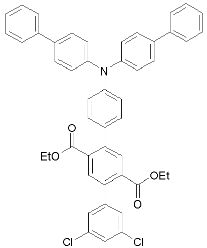 | MeMgBr | 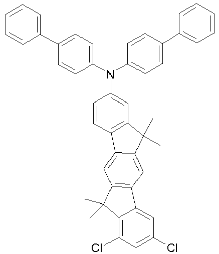 |

| | | | |
|---|---|---|---|
| #: Verbindungen können chromatographisch oder mittels Umkristallisation getrennt werden | | | |

### Verbindung 1-1

14,6 g 4-Biphenyl-(9,9-dimethyl-9H-fluoren-2-yl)-amin (40,6 mmol) und 14 g der Zwischenstufe III-1 (40,6 mmol) werden in 400 mL Toluol gelöst. Die Lösung wird entgast und mit N₂ gesättigt. Danach wird sie mit 0,33 g (0,81 mmol) S-Phos und 0,46 g (1,75 mmol) Pd₂(dba)₃ versetzt und anschließend werden 5,85 g Natrium-tert-butylat (80,9 mol) zugegeben. Die Reaktionsmischung wird 6 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Nach Filtration des Rohproduktes über Kieselgel mit Toluol wird der verbleibende Rückstand aus Heptan/Toluol umkristallisiert. Die Ausbeute beträgt 20 g (75% d. Th). Abschließend wird das Material im Hochvakuum sublimiert. Die Reinheit beträgt 99.9%.

Analog dazu werden folgende Verbindungen hergestellt (Ausbeuten 20-80% d. Th.):

| | **Edukt 1** | **Edukt 2** | **Produkt** |
|---|---|---|---|
| **1-2** | 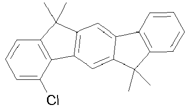 | 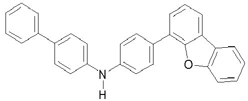 | 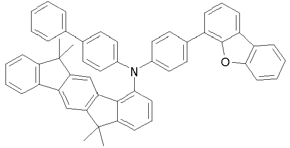 |
| **1-3** | 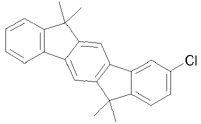 | 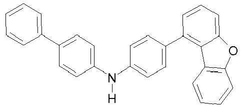 | 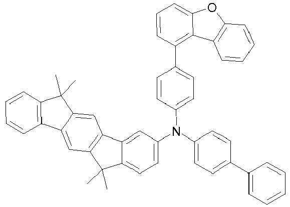 |
| **1-4** | 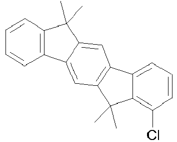 | 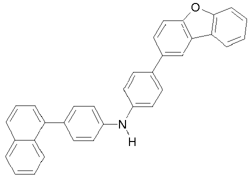 | 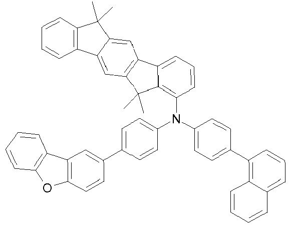 |
| **1-5** | 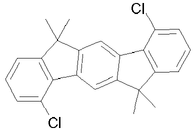 | 2 Äq. | 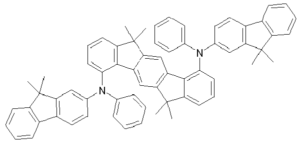 |
| | | 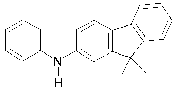 | |
| **1-6** | 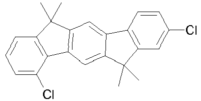 | 2 Äq. | 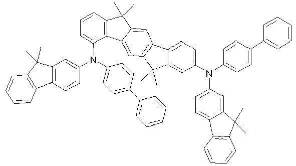 |
| | | 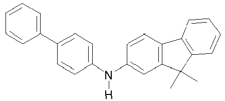 | |
| **1-7** | 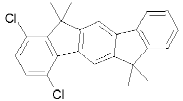 | 2 Äq. | 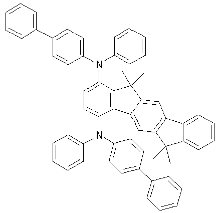 |
| | | 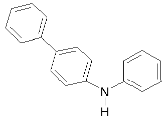 | |
| **1-8** | 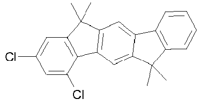 | 2 Äq. | 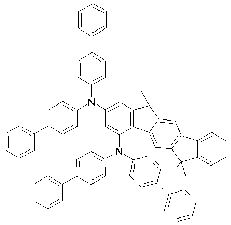 |
| | | 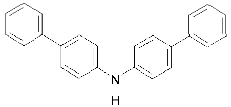 | |
| **1-9** | 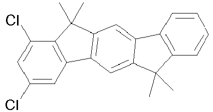 | 2 Äq. | 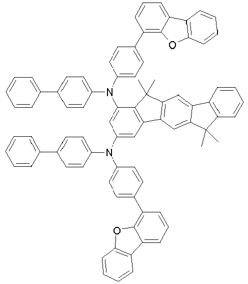 |
| | | 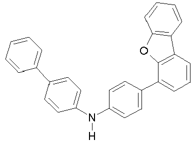 | |
| **1-10** | 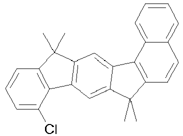 | 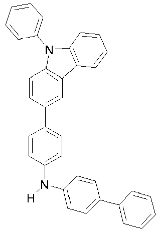 | 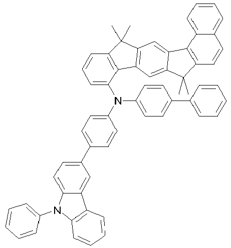 |
| **1-11** | 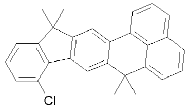 | 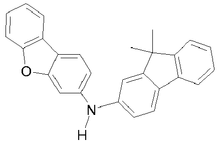 | 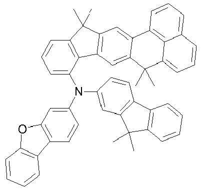 |
| **1-12** | 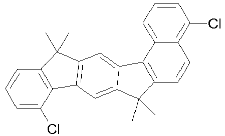 | 2 Äq. | 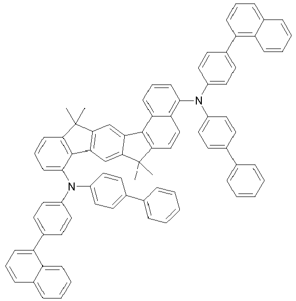 |
| | | 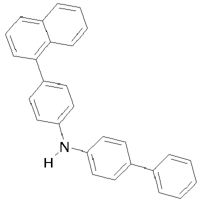 | |
| **1-13** | 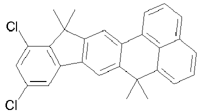 | 2 Äq. | 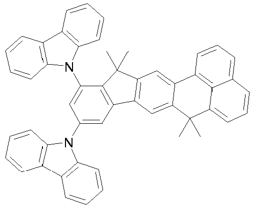 |
| | | 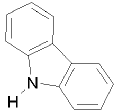 | |
| **1-14** | 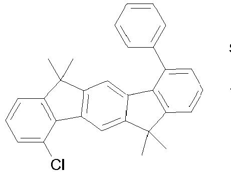 | 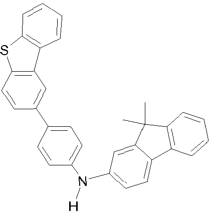 | 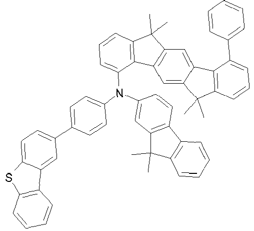 |
| **1-15** | 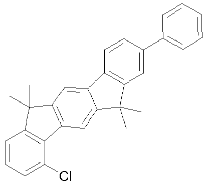 | 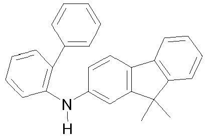 | 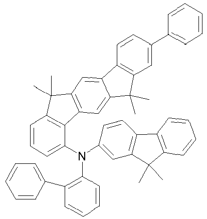 |
| **1-16** | 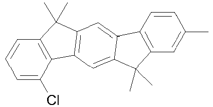 | 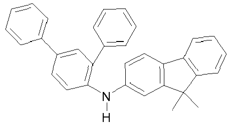 | 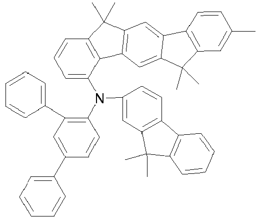 |
| **1-17** | 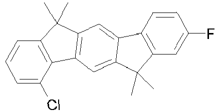 | 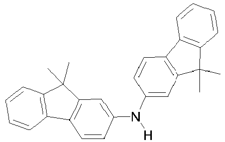 | 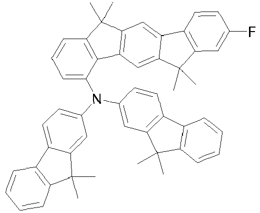 |
| **1-18** | 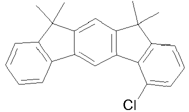 | 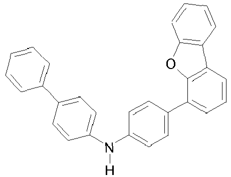 | 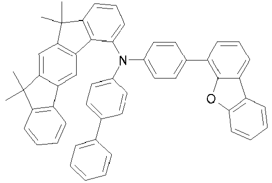 |
| **1-19** | 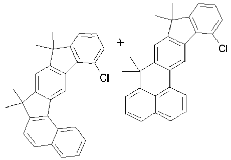 | 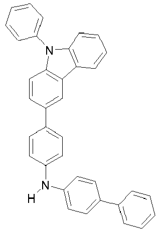 | # |
| | | | 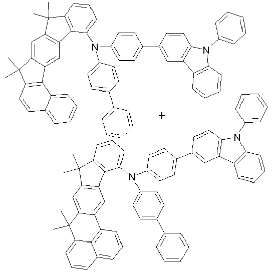 |
| **1-20** | 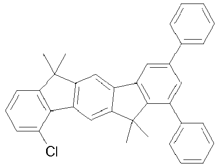 | 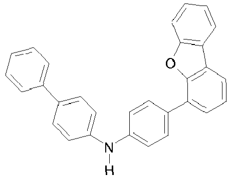 | 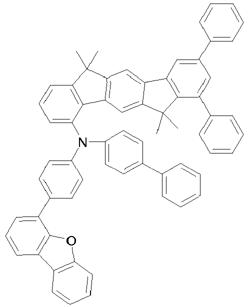 |
| **1-21** | 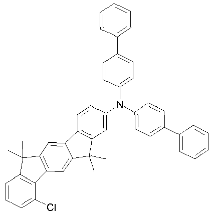 | 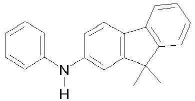 | 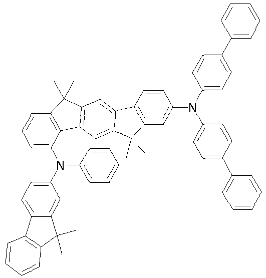 |
| **1-22** | 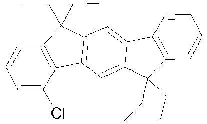 | 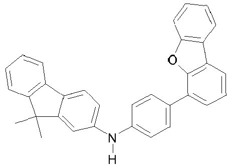 | 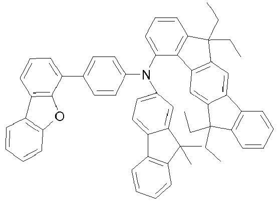 |
| **1-23** | 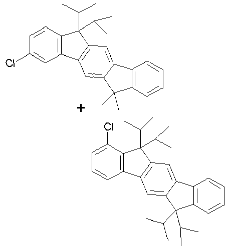 | 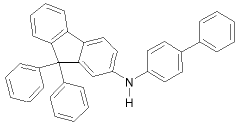 | # |
| | | | 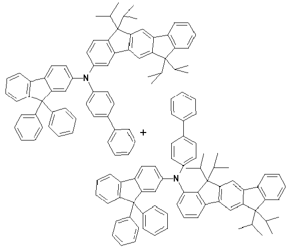 |
| **1-24** | 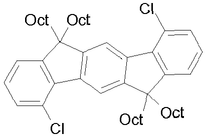 | 2 Äq. | 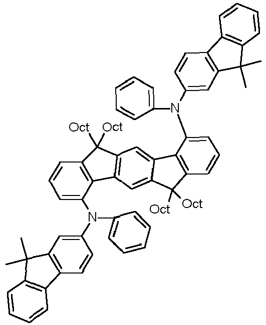 |
| | | 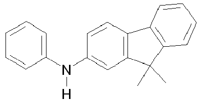 | |
| **1-25** | 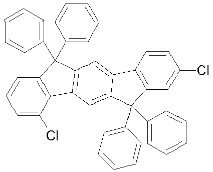 | 2 Äq. | 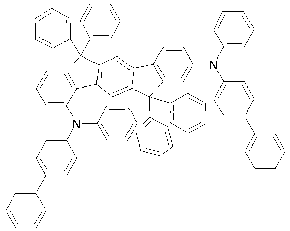 |
| | | 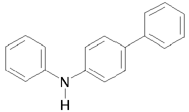 | |
| **1-26** | 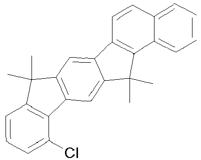 | 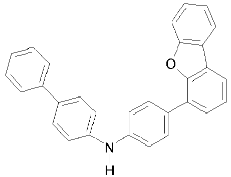 | 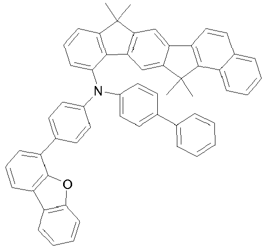 |
| **1-27** | 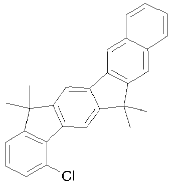 | 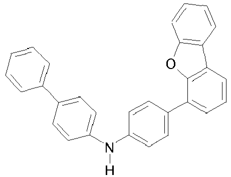 | 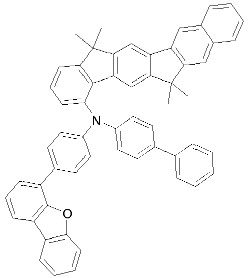 |
| **1-28** | 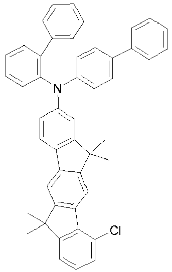 | 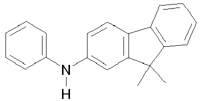 | 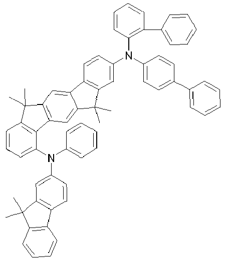 |
| **1-29** | 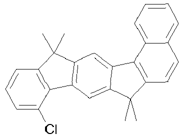 | 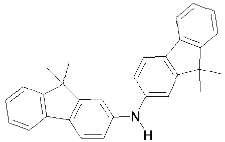 | 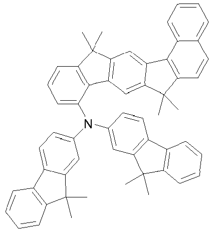 |
| **1-30** | 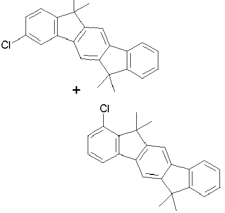 | 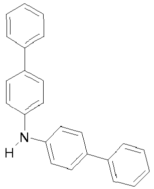 | 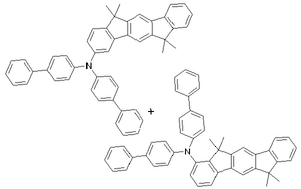 |
| | | | # |
| **1-31** | 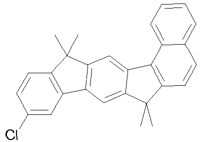 | 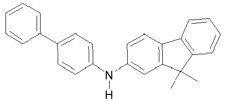 | 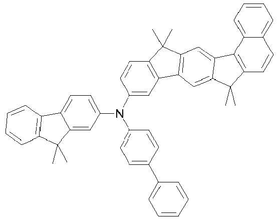 |
| **1-32** | 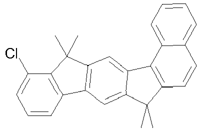 | 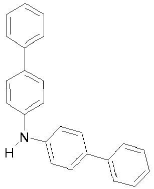 | 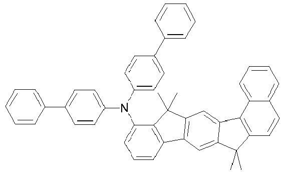 |
| **1-35** | 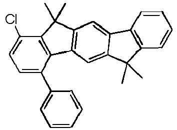 | 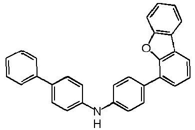 | 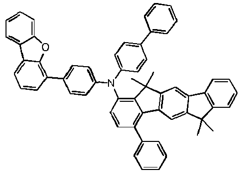 |
| **1-36** | 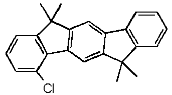 | 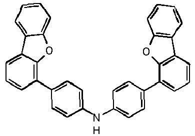 | 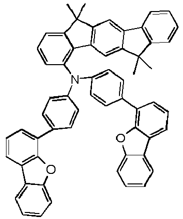 |
| **1-37** | 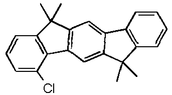 | 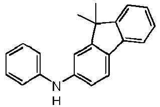 | 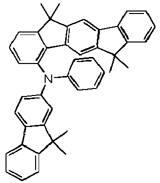 |
| **1-38** | 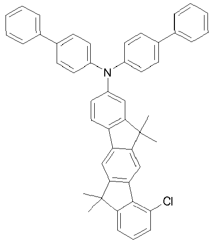 | 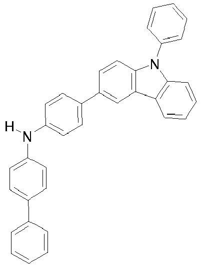 | 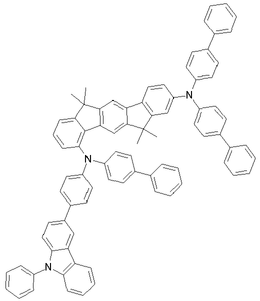 |
| **1-39** | 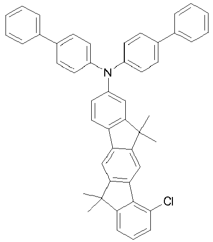 | 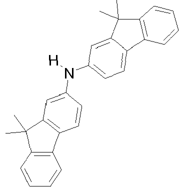 | 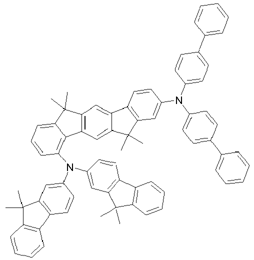 |
| **1-40** | 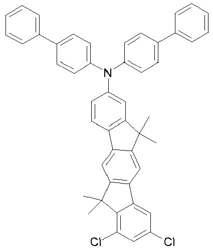 | 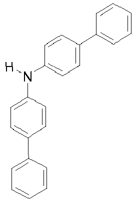 | 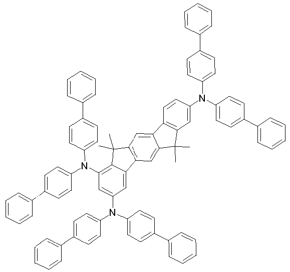 |
| **1-41** | 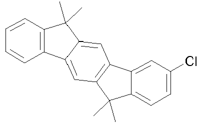 | 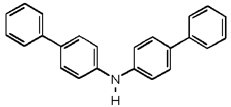 | 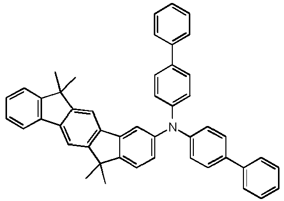 |
| **1-42** | 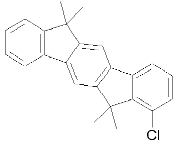 | 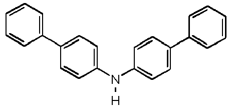 | 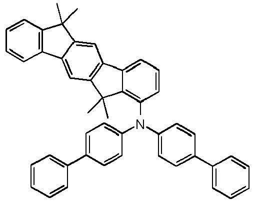 |

| | | | |
|---|---|---|---|
| #: Verbindungen können chromatographisch oder mittels Umkristallisation getrennt werden. | | | |

### Beispiel 2-1: Synthese der erfindungsgemäßen Verbindung 2-1 und Varianten

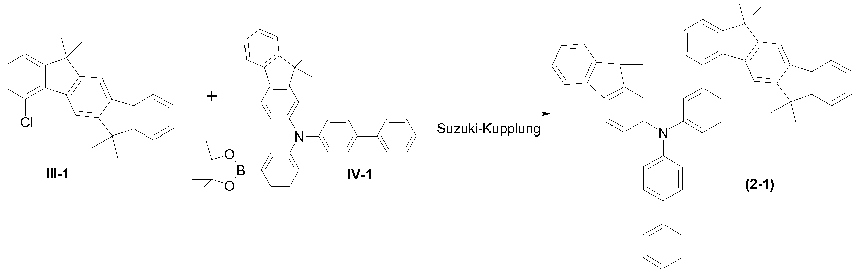

### Verbindung 2-1

19,6 g (34,8 mmol) des Pinacolboronester-Derivats (CAS-Nr.: 1616632-73-5) und 12,05 g (45 mmol) Zwischenstufe III-1 werden in 350 mL Dioxan und 10.6 g Caesiumfluorid (69,9 mmol) suspendiert. Zu dieser Suspension werden 1,02 g (1,39 mmol) Palladium-dichlorid-bis(tricyclohexylphosphin) gegeben, und die Reaktionsmischung wird 18 h unter Rückfluss erhitzt. Nach Erkalten wird die organische Phase abgetrennt, über Kieselgel filtriert, dreimal mit 80 mL Wasser gewaschen und anschließend zur Trockene eingeengt. Nach Filtration des Rohproduktes über Kieselgel mit Toluol wird der verbleibende Rückstand aus Heptan/Toluol umkristallisiert. Die Ausbeute beträgt 20 g (78% d. Th). Abschließend wird das Material im Hochvakuum sublimiert. Die Reinheit beträgt 99.9%.

Analog dazu werden folgende Verbindungen hergestellt (Ausbeuten 60-85% d. Th.):

| | **Edukt 2** | **Edukt 2** | **Produkt** |
|---|---|---|---|
| **2-2** | 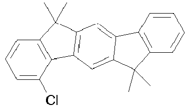 | 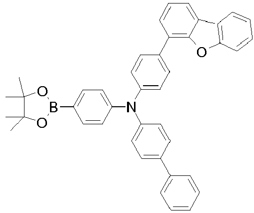 | 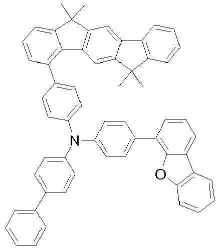 |
| **2-3** | 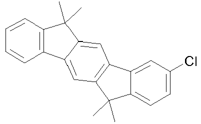 | 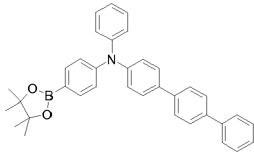 | 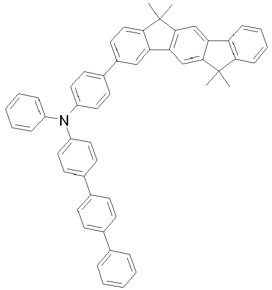 |
| **2-4** | 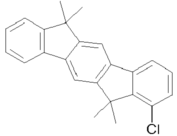 | 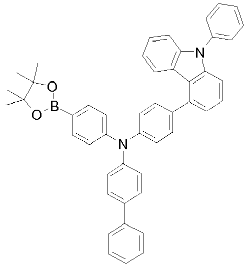 | 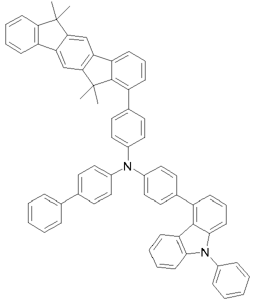 |
| **2-5** | 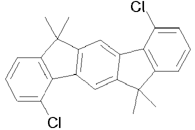 | 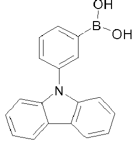 | 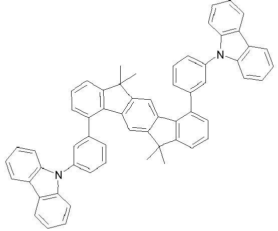 |
| **2-6** | 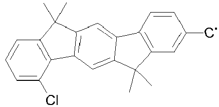 | 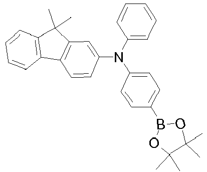 | 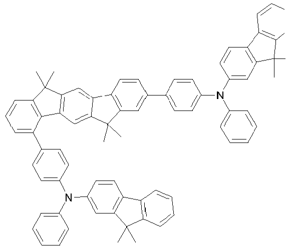 |
| **2-7** | 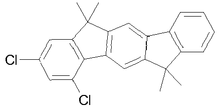 | 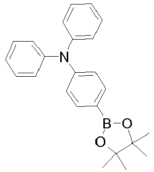 | 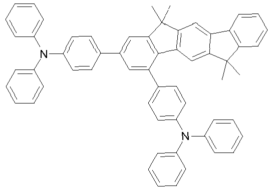 |
| **2-8** | 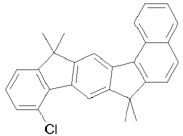 | 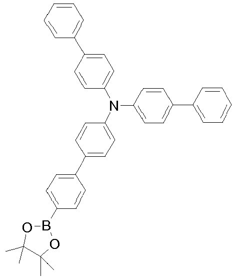 | 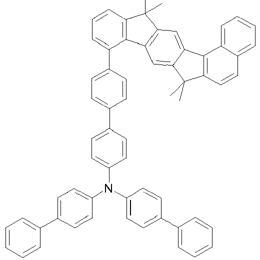 |
| **2-9** | 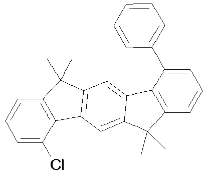 | 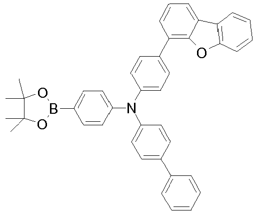 | 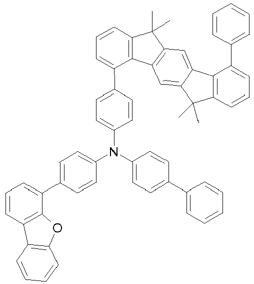 |
| **2-10** | 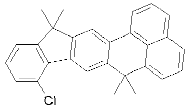 | 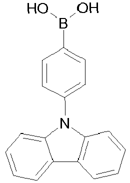 | 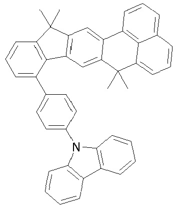 |
| **2-11** | 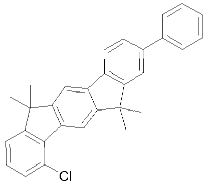 | 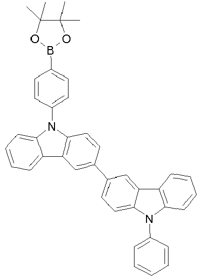 | 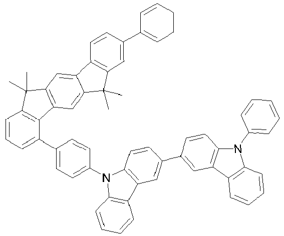 |
| **2-12** | 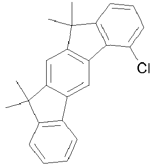 | 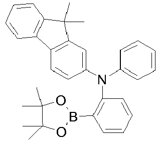 | 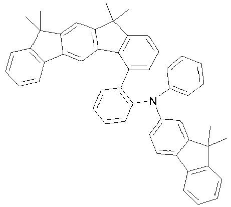 |
| **2-13** | 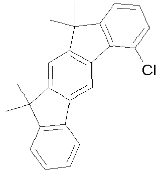 | 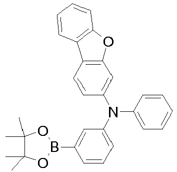 | 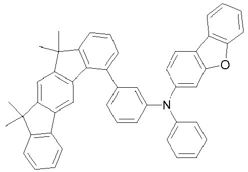 |
| **2-14** | 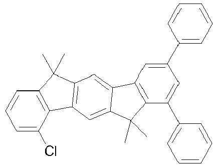 | 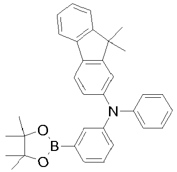 | 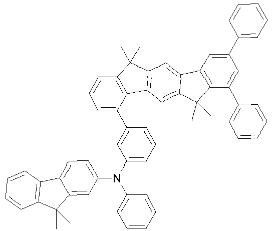 |
| **2-15** | 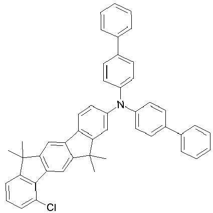 | 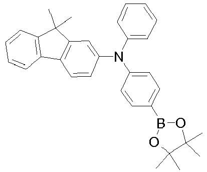 | 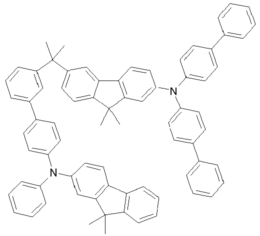 |
| **2-16** | 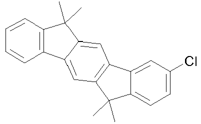 | 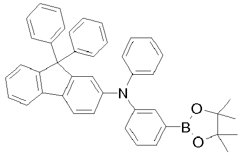 | 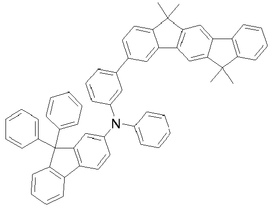 |
| **2-17** | 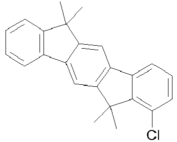 | 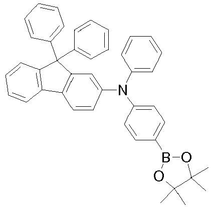 | 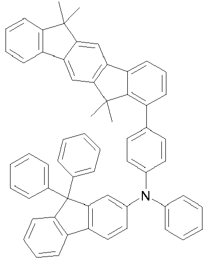 |
| **2-19** | 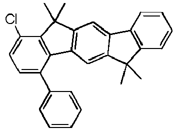 | 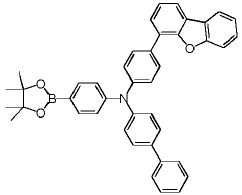 | 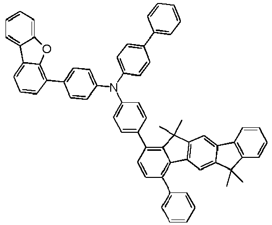 |

### B) Devicebeispiele

Es werden Beispiel-OLEDs gemäß der folgenden allgemeinen Vorschrift hergestellt:
Als Substrate werden Glasplättchen, die mit einer 50 nm dicken Schicht von strukturiertem ITO (Indium-Zinn-Oxid) beschichtet sind, verwendet. Darauf wird die folgende Schichtstruktur aufgebracht: Lochinjektionsschicht (HIL) / Lochtransportschicht (HTL) / Elektronenblockierschicht (EBL) / Emissionsschicht (EML) / Elektronentransportschicht (ETL) / Elektroneninjektionsschicht (EIL) / Kathode. Die Kathode besteht aus einer Aluminiumschicht einer Dicke von 100 nm. Die Materialien, die in den entsprechenden Schichten der Beispiel-OLEDs verwendet werden, sind in Tabelle 1 genannt, und die chemischen Strukturen dieser Materialien sind in Tabelle 3 aufgeführt.

Die Materialien werden mittels thermischer Gasphasenabscheidung in einer Vakuumkammer aufgetragen. Dabei besteht die Emissionsschicht immer aus wenigstens einem Matrixmaterial (Hostmaterial) und einem emittierenden Dotanden (Emitter), der dem Matrixmaterial bzw. den Matrixmaterialien in einem bestimmten Volumenanteil durch Co-Evaporation beigemischt wird. Der Ausdruck TMM:TEG (12%) bedeutet dabei, dass das Material TMM in der Schicht in einem Volumenanteil von 88% vorhanden ist, und dass das Material TEG in einem Volumenanteil von 12% vorhanden ist. Entsprechendes gilt für andere Schichten als die emittierende Schicht. Diese können ebenfalls entsprechend zwei oder mehr Materialien enthalten.

Die OLEDs werden standardmäßig charakterisiert. Hierfür werden die Elektrolumineszenzspektren und die externe Quanteneffizienz (EQE, gemessen in %), als Funktion der Leuchtdichte, berechnet aus Stromfluss- /Spannung-/Leuchtdichte-Kennlinien (IUL-Kennlinien), bestimmt. Dabei werden Lambert'sche Emissionscharakteristika angenommen. Weiterhin wird die Betriebsspannung bestimmt (U, in V).

EQE @ 10mA/cm² stellt die externe Quanteneffizienz bei einer Betriebs-Stromdichte von 10 mA/cm² dar. LT80 @ 40mA/cm² ist die Zeitdauer, bis zu der die initiale Leuchtdichte von 5000 cd/m² einer OLED auf 80% dieser Leuchtdichte, d.h. auf 4000cd/m², abgefallen ist, ohne dass ein Beschleunigungsfaktor berücksichtigt wurde.

### B-1) Verwendung der erfindungsgemäßen Verbindungen in grün fluoreszierenden OLEDs

OLED-Beispiele E-0 bis E-17 weisen den in Tabelle 1 gezeigten Schichtaufbau auf, wobei in der EBL jeweils eine der erfindungsgemäßen Verbindungen EBL-0 bis EBL-17 (Tabelle 3) vorliegt.

In allen Fällen werden mit den erfindungsgemäßen OLEDs gute Ergebnisse bzgl. Betriebsspannung, Lebensdauer und EQE erzielt (Tabelle 2).

| **Tabelle 1: Deviceaufbau** | | | | | | |
|---|---|---|---|---|---|---|
| **Bsp.** | **HIL** | **HTL** | **EBL** | **EML** | **ETL** | **EIL** |
| | Dicke / nm | Dicke / nm | Dicke / nm | Dicke / nm | Dicke / nm | Dicke / nm |
| E-0 | HTM:p-Dotand (5%) 20 nm | HTM 220 nm | EBL-0 10 nm | TMM-1:TMM-2 (28%): TEG (12%) 30 nm | ETM:LiQ(50%) 30 nm | LiQ 1 nm |
| E-1 | s.o. | s.o. | EBL-1 10 nm | s.o. | s.o. | s.o. |
| E-2 | s.o. | s.o. | EBL-2 10 nm | s.o. | s.o. | s.o. |
| E-3 | s.o. | s.o. | EBL-3 10 nm | s.o. | s.o. | s.o. |
| E-4 | s.o. | s.o. | EBL-4 10 nm | s.o. | s.o. | s.o. |
| E-5 | s.o. | s.o. | EBL-5 10 nm | s.o. | s.o. | s.o. |
| E-6 | s.o. | s.o. | EBL-6 10 nm | s.o. | s.o. | s.o. |
| E-7 | s.o. | s.o. | EBL-7 10 nm | s.o. | s.o. | s.o. |
| E-8 | s.o. | s.o. | EBL-8 10 nm | s.o. | s.o. | s.o. |
| E-9 | s.o. | s.o. | EBL-9 10 nm | s.o. | s.o. | s.o. |
| E-10 | s.o. | s.o. | EBL-10 10 nm | s.o. | s.o. | s.o. |
| E-15 | s.o. | s.o. | EBL-15 10 nm | s.o. | s.o. | s.o. |
| E-16 | s.o. | s.o. | EBL-16 10 nm | s.o. | s.o. | s.o. |
| E-17 | s.o. | s.o. | EBL-17 10 nm | s.o. | s.o. | s.o. |

| **Tabelle 2: Daten der OLEDs** | | | |
|---|---|---|---|
| Beispiel | U | EQE @ 10 mA/cm² | LT80 @ 40 mA/cm² |
| | [V] | [%] | [h] |
| E-0 | 3.9 | >16.5 | >250 |
| E-1 | 3.7 | >16.0 | >300 |
| E-2 | 4.1 | >17.0 | >300 |
| E-3 | 4.1 | >18.0 | >200 |
| E-4 | 4.1 | >16.0 | >250 |
| E-5 | 4.0 | >16.5 | >200 |
| E-6 | 4.0 | >17.0 | >250 |
| E-7 | 3.9 | >15.5 | >250 |
| E-8 | 3.8 | >15.0 | >250 |
| E-9 | 4.1 | >15.5 | >300 |
| E-10 | 4.0 | >17.0 | >250 |
| E-15 | 4.2 | >16.0 | >350 |
| E-16 | 4.1 | >16.0 | >250 |
| E-17 | 4.1 | >14.0 | >300 |

| **Tabelle 3: Verwendete Materialien** | | |
|---|---|---|
| 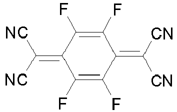 | 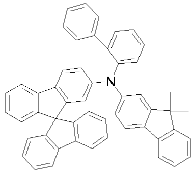 | 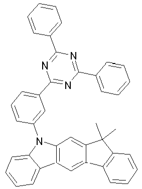 |
| p-Dotand | HTM | TMM-1 |
| 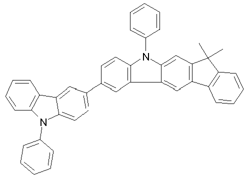 | 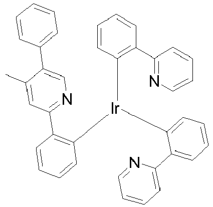 | 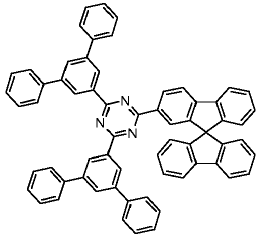 |
| TMM-2 | TEG | ETM |
| 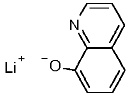 | 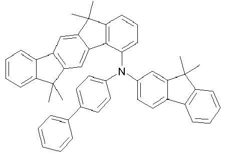 | 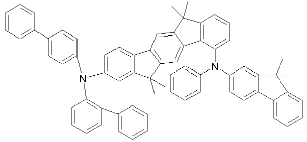 |
| 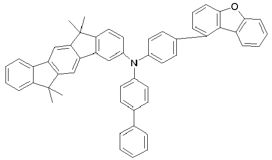 LiQ | 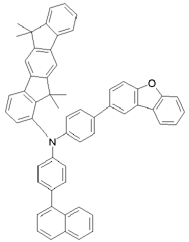 EBL-0 | 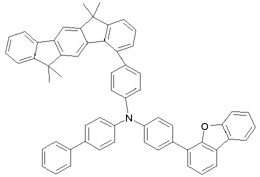 EBL-1 |
| EBL-2 | EBL-3 | EBL-4 |
| 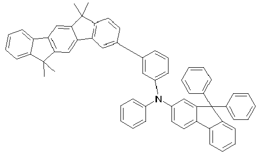 | 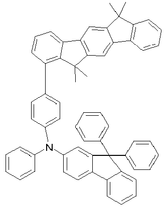 | 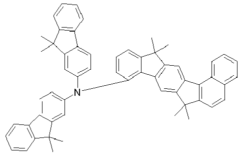 |
| EBL-5 | EBL-6 | EBL-7 |
| 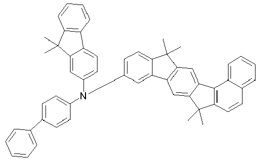 | 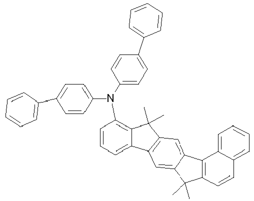 | 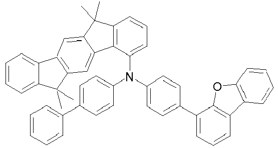 |
| EBL-8 | EBL-9 | EBL-10 |
| 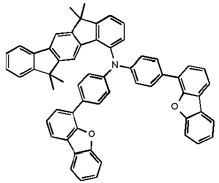 | 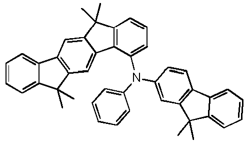 | 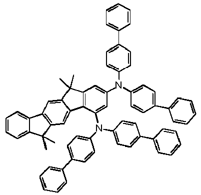 |
| EBL-15 | EBL-16 | EBL-17 |

### B-2) Vergleich der erfindungsgemäßen Verbindungen EBL-12, EBL-13 und EBL-14 mit der Verbindung EBL-11 gemäß dem Stand der Technik

OLED-Beispiele E-12, E-13 und E-14 enthalten in der Elektronenblockierschicht jeweils eine der erfindungsgemäßen Verbindungen EBL-12, EBL-13 und EBL-14. Das Vergleichsbeispiel OLED E-11 enthält in der Elektronenblockierschicht die Verbindung EBL-11. Es treten bei den OLEDs E-12, E-13 und E-14 höhere Werte für die EQE auf, als bei der Vergleichs-OLED E11. Insbesondere wird bei der erfindungsgemäßen OLED E-14 eine EQE@ 10 mA/cm² von mehr als 16% erhalten, während beim Vergleichsbeispiel E-11 eine EQE@ 10 mA/cm² von weniger als 15% erhalten wird, bei einer Spannung von in beiden Fällen 4.1 V.

| **Tabelle 1b: Deviceaufbau** | | | | | | |
|---|---|---|---|---|---|---|
| **Bsp.** | **HIL** | **HTL** | **EBL** | **EML** | **ETL** | **EIL** |
| | Dicke / nm | Dicke / nm | Dicke / nm | Dicke / nm | Dicke / nm | Dicke / nm |
| E-11 (Vgl.) | HTM:p-Dotand (5%) 20 nm | HTM 220 nm | EBL-11 10 nm | TMM-1:TMM-2 (28%): TEG (12%) 30 nm | ETM:LiQ(50%) 30 nm | LiQ 1 nm |
| E-12 | s.o. | s.o. | EBL-12 10 nm | s.o. | s.o. | s.o. |
| E-13 | s.o. | s.o. | EBL-13 10 nm | s.o. | s.o. | s.o. |
| E-14 | s.o. | s.o. | EBL-14 10 nm | s.o. | s.o. | s.o. |

| **Tabelle 3b: Verwendete Materialien** | | |
|---|---|---|
| 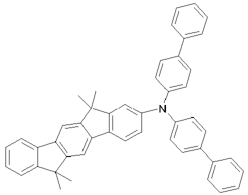 | 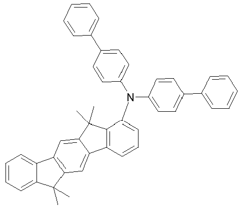 | 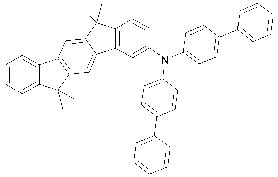 |
| EBL-11 | EBL-12 | EBL-13 |
| 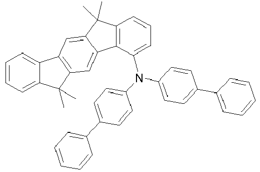 | | |
| EBL-14 | | |

### B-3) Verwendung der erfindungsgemäßen Verbindungen in blau fluoreszierenden OLEDs

OLED-Beispiele E-19 und E-20 weisen den in Tabelle 1c gezeigten Schichtaufbau auf, wobei in der EBL jeweils eine der erfindungsgemäßen Verbindungen EBL-15 bzw. EBL-16 (s. Tabelle 3c) vorliegt.

In allen Fällen werden mit den erfindungsgemäßen OLEDs gute Ergebnisse bzgl. Betriebsspannung, Lebensdauer und EQE erzielt (Tabelle 2c).

| **Tabelle 1c: Deviceaufbau** | | | | | | |
|---|---|---|---|---|---|---|
| **Bsp.** | **HIL** | **HTL** | **EBL** | **EML** | **ETL** | **EIL** |
| | Dicke / nm | Dicke / nm | Dicke / nm | Dicke / nm | Dicke / nm | Dicke / nm |
| E-19 | HTM:p-Dotand (5%) 20 nm | HTM 180 nm | EBL-15 10 nm | SMB-1:SEB-1 (5%) 20 nm | ETM:LiQ(50%) 30 nm | LiQ 1 nm |
| E-20 | s.o. | s.o. | EBL-16 10 nm | s.o. | s.o. | s.o. |

| **Tabelle 2c: Daten der OLEDs** | | | |
|---|---|---|---|
| Beispiel | U | EQE @ 10 mA/cm² | LT80 @ 60 mA/cm² |
| | [V] | [%] | [h] |
| E-19 | 4.0 | ca. 8.5 | ca. 350 |
| E-20 | 4.0 | ca. 8.5 | ca. 350 |

| **Tabelle 3c: Verwendete Materialien** | | |
|---|---|---|
| 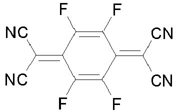 | 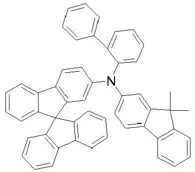 | 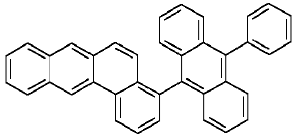 |
| p-Dotand | HTM | SMB-1 |
| 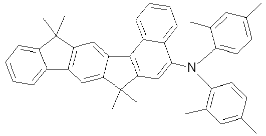 | 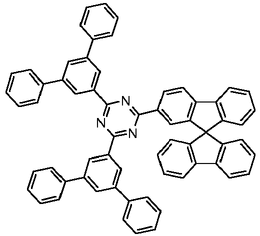 | 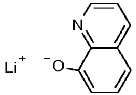 |
| SEB-1 | ETM | LiQ |
| 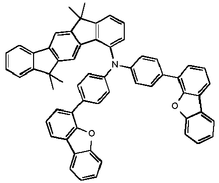 | 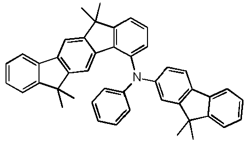 | |
| EBL-15 | EBL-16 | |

## Patentansprüche

1. Verbindung der Formel (I) 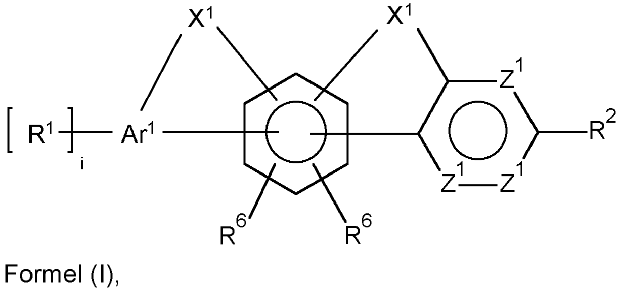 wobei für die auftretenden Variablen gilt:
Z¹ ist bei jedem Auftreten gleich oder verschieden gewählt aus CR¹ und CR³;
Ar¹ ist eine Arylgruppe mit 6 bis 20 aromatischen Ringatomen, die mit einem oder mehreren Resten R³ substituiert sein kann, oder eine Heteroarylgruppe mit 5 bis 20 aromatischen Ringatomen, die mit einem oder mehreren Resten R³ substituiert sein kann;
X¹ ist bei jedem Auftreten gleich oder verschieden eine divalente Gruppe gewählt aus -C(R⁴)₂-, -C(R⁴)₂-C(R⁴)₂-, -CR⁴=CR⁴- und -Si(R⁴)₂-;
R¹ ist eine Gruppe der Formel (N) 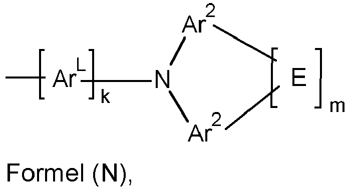
Ar^{L} ist gewählt aus aromatischen Ringsystemen mit 6 bis 40 aromatischen Ringatomen, die mit einem oder mehreren Resten R⁵ substituiert sein können, und heteroaromatischen Ringsystemen mit 5 bis 40 aromatischen Ringatomen, die mit einem oder mehreren Resten R⁵ substituiert sein können;
Ar² ist bei jedem Auftreten gleich oder verschieden gewählt aus aromatischen Ringsystemen mit 6 bis 40 aromatischen Ringatomen, die mit einem oder mehreren Resten R⁵ substituiert sein können, und heteroaromatischen Ringsystemen mit 5 bis 40 aromatischen Ringatomen, die mit einem oder mehreren Resten R⁵ substituiert sein können;
E ist eine Einfachbindung oder eine divalente Gruppe gewählt aus C(R⁵)₂, Si(R⁵)₂, N(R⁵), O, und S;
R² ist gewählt aus H, D, F, C(=O)R⁷, CN, Si(R⁷)₃, N(R⁷)₂, P(=O)(R⁷)₂, OR⁷, S(=O)R⁷, S(=O)₂R⁷, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁷ substituiert sein können; und wobei eine oder mehrere CH₂-Gruppen in den genannten Alkyl-, Alkoxy-, Alkenyl- und Alkinylgruppen durch -R⁷C =CR⁷-, -C=C-, Si(R⁷)₂, C=O, C=NR⁷, -C(=O)O-, -C(=O)NR⁷-, P(=O)(R⁷), -O-, -S-, SO oder SO₂ ersetzt sein können;
R³, R⁴, R⁵ bei jedem Auftreten gleich oder verschieden gewählt aus H, D, F, C(=O)R⁷, CN, Si(R⁷)₃, N(R⁷)₂, P(=O)(R⁷)₂, OR⁷, S(=O)R⁷, S(=O)₂R⁷, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R³ bzw. R⁴ bzw. R⁵ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁷ substituiert sein können; und wobei eine oder mehrere CH₂-Gruppen in den genannten Alkyl-, Alkoxy-, Alkenyl- und Alkinylgruppen durch - R⁷C=CR⁷-, -C≡C-, Si(R⁷)₂, C=O, C=NR⁷, -C(=O)O-, -C(=O)NR⁷-, NR⁷, P(=O)(R⁷), -O-, -S-, SO oder SO₂ ersetzt sein können;
R⁶ ist bei jedem Auftreten gleich oder verschieden gewählt aus H, D, F, C(=O)R⁷, CN, Si(R⁷)₃, P(=O)(R⁷)₂, OR⁷, S(=O)R⁷, S(=O)₂R⁷, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R⁶ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁷ substituiert sein können; und wobei eine oder mehrere CH₂-Gruppen in den genannten Alkyl-, Alkoxy-, Alkenyl- und Alkinylgruppen durch -R⁷C=CR⁷-, -C=C-, Si(R⁷)₂, C=O, C=NR⁷, -C(=O)O-, -C(=O)NR⁷-, NR⁷, P(=O)(R⁷), -O-, -S-, SO oder SO₂ ersetzt sein können;
R⁷ ist bei jedem Auftreten gleich oder verschieden gewählt aus H, D, F, C(=O)R⁸, CN, Si(R⁸)₃, N(R⁸)₂, P(=O)(R⁸)₂, OR⁸, S(=O)R⁸, S(=O)₂R⁸, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R⁷ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁸ substituiert sein können; und wobei eine oder mehrere CH₂-Gruppen in den genannten Alkyl-, Alkoxy-, Alkenyl- und Alkinylgruppen durch -R⁸C=CR⁸-, -C=C-, Si(R⁸)₂, C=O, C=NR⁸, -C(=O)O-, -C(=O)NR⁸-, NR⁸, P(=O)(R⁸), -O-, -S-, SO oder SO₂ ersetzt sein können;
R⁸ ist bei jedem Auftreten gleich oder verschieden gewählt aus H, D, F, CN, Alkyl- oder Alkoxygruppen mit 1 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁸ miteinander verknüpft sein können und einen Ring bilden können; und wobei die genannten Alkyl-, Alkoxy-, Alkenyl- und Alkinylgruppen, aromatischen Ringsysteme und heteroaromatischen Ringsysteme mit F oder CN substituiert sein können;
k ist gleich 0 oder 1, wobei im Fall k=0 die Gruppe Ar^{L} wegfällt und das Stickstoffatom der Gruppe der Formel (N) die Anbindungsposition darstellt;
m ist gleich 0 oder 1, wobei im Fall m=0 die Gruppe E wegfällt und die Gruppen Ar² nicht miteinander verbunden sind;
i ist gleich 0 oder 1, wobei im Fall i=0 die Gruppe R¹ wegfällt; und
wobei mindestens eine Gruppe Z¹ vorliegt, die CR¹ ist.

2. Verbindung nach Anspruch 1, **dadurch gekennzeichnet, dass** entweder i) eine Gruppe Z¹ gleich CR¹ ist, und die beiden anderen Gruppen Z¹ gleich CR³ sind, oder ii) zwei Gruppen Z¹ gleich CR¹ sind, und die andere Gruppe Z¹ ist gleich CR³.

3. Verbindung nach Anspruch 1 oder 2, **dadurch gekennzeichnet, dass** diejenige Gruppe Z¹, die in meta-Position zur Bindung zu X¹ steht, gleich CR¹ ist.

4. Verbindung nach einem oder mehreren der Ansprüche 1 bis 3, **dadurch gekennzeichnet, dass** Ar¹ gewählt ist aus Phenyl- und Naphthylgruppen, die jeweils mit einem oder mehreren Resten R³ substituiert sein können.

5. Verbindung nach einem oder mehreren der Ansprüche 1 bis 4, **dadurch gekennzeichnet, dass** X¹ gleich C(R⁴)₂ ist.

6. Verbindung nach einem oder mehreren der Ansprüche 1 bis 5, **dadurch gekennzeichnet, dass** die direkt an das Stickstoffatom bindende Gruppe von Ar² ein aromatisches Ringsystem ist.

7. Verbindung nach einem oder mehreren der Ansprüche 1 bis 6, **dadurch gekennzeichnet, dass** m = 0 ist.

8. Verbindung nach einem oder mehreren der Ansprüche 1 bis 7, **dadurch gekennzeichnet, dass** m = 1 ist, und die Einheit 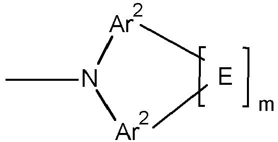 aus der Gruppe der Formel (N) gewählt ist aus den Gruppen:
| | | |
|---|---|---|
| 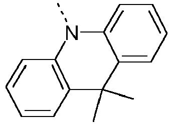 | 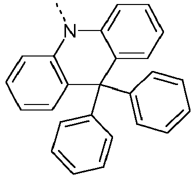 | 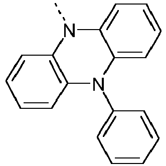 |
| N-1 | N-2 | N-3 |
| 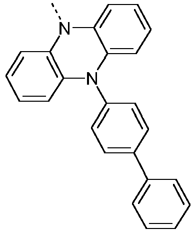 | 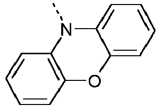 | 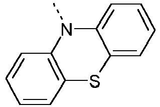 |
| N-4 | N-5 | N-6 |
| 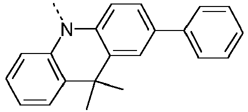 | 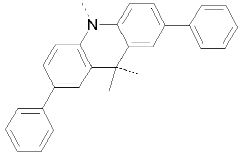 | 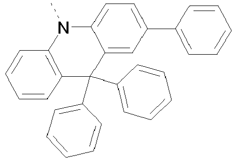 |
| N-7 | N-8 | N-9 |
| 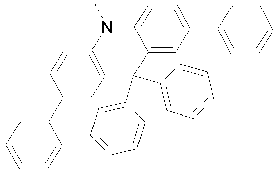 | 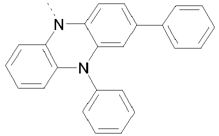 | 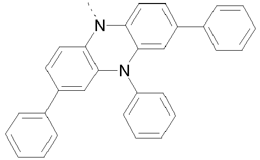 |
| N-10 | N-11 | N-12 |
| 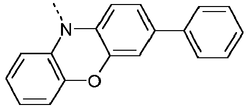 | 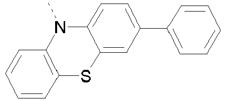 | 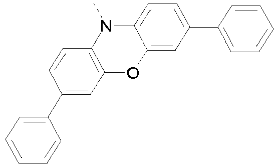 |
| N-13 | N-14 | N-15 |
| 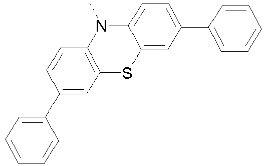 | 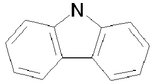 | 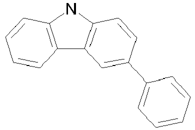 |
| N-16 | N-17 | N-18 |
| 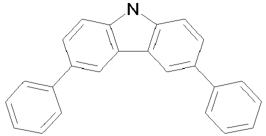 | 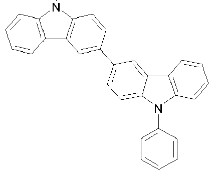 | 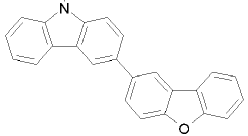 |
| N-19 | N-20 | N-21 |
| 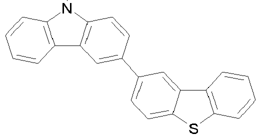 | 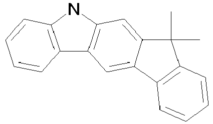 | 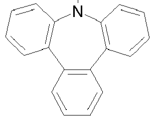 |
| N-22 | N-23 | N-24 |
| 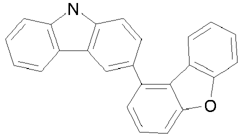 | 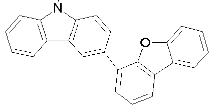 | 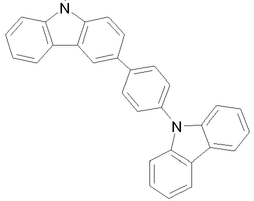 |
| N-25 | N-26 | N-27 |
| 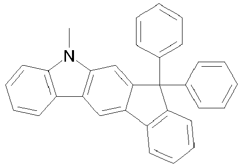 | 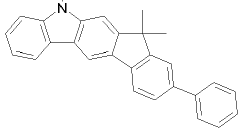 | 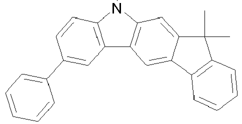 |
| N-28 | N-29 | N-30 |
| 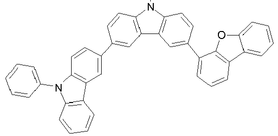 | 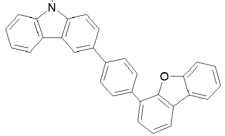 | |
| N-31 | N-32 | |
wobei die Gruppen an ihren freien Positionen jeweils mit einem Rest R⁵ substituiert sein können, und wobei die gestrichelten Bindungen die Bindungen an den Rest der Formel darstellen.

9. Verbindung nach einem oder mehreren der Ansprüche 1 bis 8, **dadurch gekennzeichnet, dass** R² gleich H ist.

10. Verbindung nach einem oder mehreren der Ansprüche 1 bis 9, **dadurch gekennzeichnet, dass** R⁶ gleich H ist.

11. Verbindung nach einem oder mehreren der Ansprüche 1 bis 10, **dadurch gekennzeichnet, dass** sie einer der folgenden Formeln (I-1) bis (I-9) entspricht
| |
|---|
| 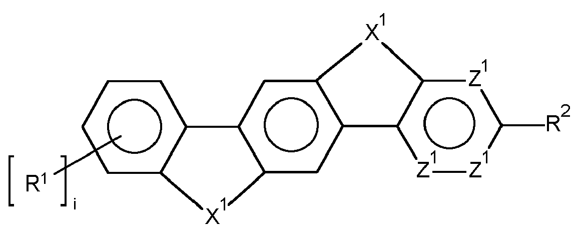 |
| Formel (I-1) |
| 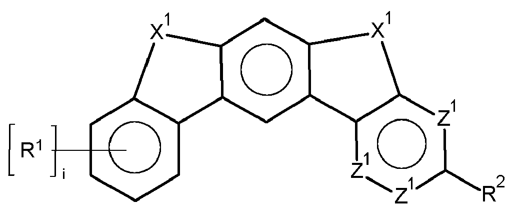 |
| Formel (I-2) |
| 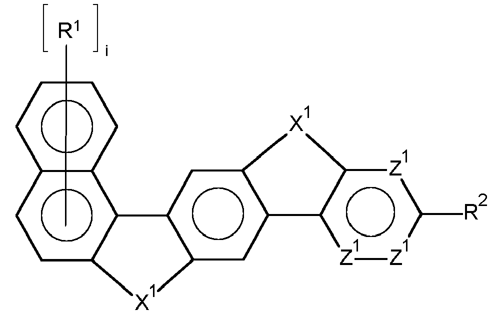 |
| Formel (I-3) |
| 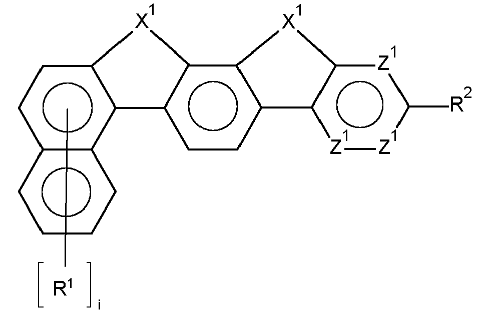 |
| Formel (I-4) |
| 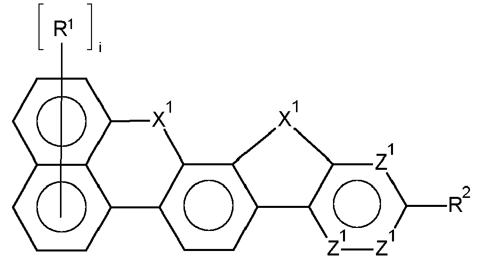 |
| Formel (I-5) |
| 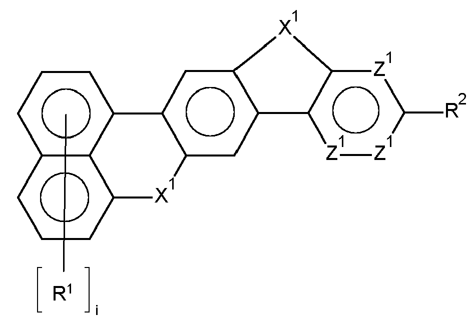 |
| Formel (I-6) |
| 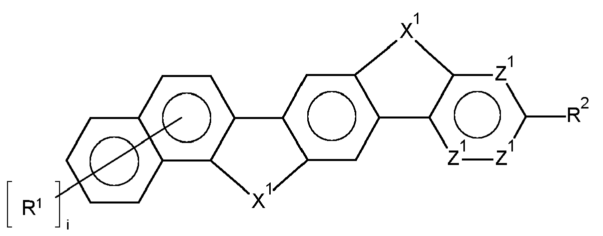 |
| Formel (I-7) |
| 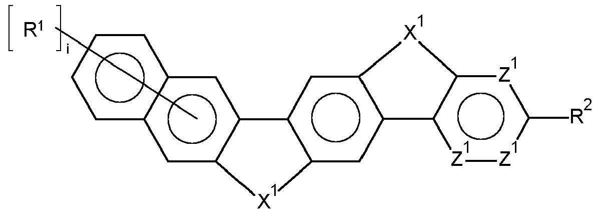 |
| Formel (I-4) |
| 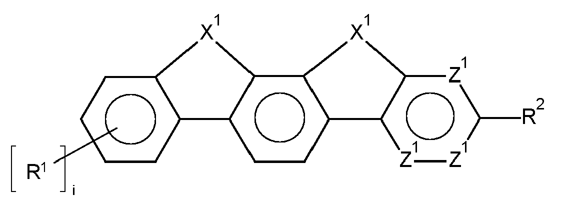 |
| Formel (I-5) |
wobei die auftretenden Variablen wie in einem oder mehreren der Ansprüche 1 bis 10 definiert sind, und wobei die Verbindungen an den freien Positionen an den aromatischen Ringen jeweils mit einem Rest R³ bzw. R⁶ substituiert sein können.

12. Verfahren zur Herstellung einer Verbindung gemäß Formel (I) nach einem oder mehreren der Ansprüche 1 bis 11, **dadurch gekennzeichnet, dass** eine Benzolverbindung, die zwei Carbonsäureestergruppen, ein aromatisches oder heteroaromatisches Ringsystem und eine reaktive Gruppe trägt, in einer Suzuki-Reaktion mit einer Benzolverbindung umgesetzt wird, die eine Boronsäuregruppe und eine Gruppe gewählt aus reaktiven Gruppen, Diarylamino-Gruppen, Diarylamino-Aryl-Gruppen und Diarylamino-Heteroaryl-Gruppen enthält.

13. Oligomer, Polymer oder Dendrimer, enthaltend eine oder mehrere Verbindungen gemäß Formel (I) nach einem oder mehreren der Ansprüche 1 bis 11, wobei die Bindung(en) zum Polymer, Oligomer oder Dendrimer an beliebigen, in Formel (I) mit R¹, R², R³ , R⁴ oder R⁵ substituierten Positionen lokalisiert sein können.

14. Formulierung, enthaltend mindestens eine Verbindung nach einem oder mehreren der Ansprüche 1 bis 11 oder ein Polymer, Oligomer oder Dendrimer nach Anspruch 13, sowie mindestens ein Lösungsmittel.

15. Elektronische Vorrichtung, enthaltend mindestens eine Verbindung nach einem oder mehreren der Ansprüche 1 bis 11, oder ein Polymer, Oligomer oder Dendrimer nach Anspruch 13.

16. Elektronische Vorrichtung nach Anspruch 15, **dadurch gekennzeichnet, dass** sie eine organische Elektrolumineszenzvorrichtung ist, enthaltend Anode, Kathode und mindestens eine emittierende Schicht, wobei mindestens eine organische Schicht der Vorrichtung, die gewählt ist aus emittierenden Schichten und lochtransportierenden Schichten, die mindestens eine Verbindung enthält.

17. Organische Elektrolumineszenzvorrichtung nach Anspruch 16, enthaltend Anode, Kathode und mindestens eine emittierende Schicht, **dadurch gekennzeichnet, dass** die mindestens eine Verbindung in einer Elektronenblockierschicht enthalten ist.

18. Verwendung einer Verbindung nach einem oder mehreren der Ansprüche 1 bis 11 in einer elektronischen Vorrichtung.

## Claims

1. Compound of the formula (I) 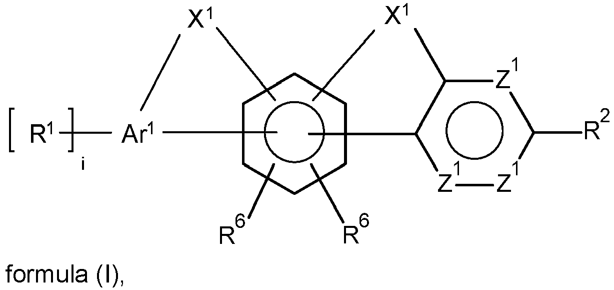 where the following applies for the variables occurring:
Z¹ is selected on each occurrence, identically or differently, from CR¹ and CR³;
Ar¹ is an aryl group having 6 to 20 aromatic ring atoms, which may be substituted by one or more radicals R³, or a heteroaryl group having 5 to 20 aromatic ring atoms, which may be substituted by one or more radicals R³;
X¹ is on each occurrence, identically or differently, a divalent group selected from -C(R⁴)₂-, -C(R⁴)₂-C(R⁴)₂-, -CR⁴=CR⁴- and -Si(R⁴)₂-;
R¹ is a group of the formula (N) 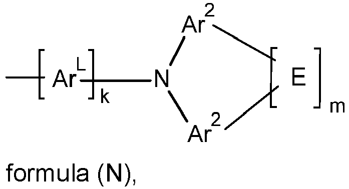
Ar^{L} is selected from aromatic ring systems having 6 to 40 aromatic ring atoms, which may be substituted by one or more radicals R⁵, and heteroaromatic ring systems having 5 to 40 aromatic ring atoms, which may be substituted by one or more radicals R⁵;
Ar² is selected on each occurrence, identically or differently, from aromatic ring systems having 6 to 40 aromatic ring atoms, which may be substituted by one or more radicals R⁵, and heteroaromatic ring systems having 5 to 40 aromatic ring atoms, which may be substituted by one or more radicals R⁵;
E is a single bond or a divalent group selected from C(R⁵)₂, Si(R⁵)₂, N(R⁵), O and S;
R² is selected from H, D, F, C(=O)R⁷, CN, Si(R⁷)₃, N(R⁷)₂, P(=O)(R⁷)₂, OR⁷, S(=O)R⁷, S(=O)₂R⁷, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where the said alkyl, alkoxy, alkenyl and alkynyl groups and the said aromatic ring systems and heteroaromatic ring systems may in each case be substituted by one or more radicals R⁷; and where one or more CH₂ groups in the said alkyl, alkoxy, alkenyl and alkynyl groups may be replaced by -R⁷C=CR⁷-, -C=C-, Si(R⁷)₂, C=O, C=NR⁷, -C(=O)O-, -C(=O)NR⁷-, P(=O)(R⁷), -O-, -S-, SO or SO₂;
R³, R⁴, R⁵ are selected on each occurrence, identically or differently, from H, D, F, C(=O)R⁷, CN, Si(R⁷)₃, N(R⁷)₂, P(=O)(R⁷)₂, OR⁷, S(=O)R⁷, S(=O)₂R⁷, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R³ or R⁴ or R⁵ may be linked to one another and may form a ring; where the said alkyl, alkoxy, alkenyl and alkynyl groups and the said aromatic ring systems and heteroaromatic ring systems may in each case be substituted by one or more radicals R⁷; and where one or more CH₂ groups in the said alkyl, alkoxy, alkenyl and alkynyl groups may be replaced by -R⁷C=CR⁷-, -C=C-, Si(R⁷)₂, C=O, C=NR⁷, -C(=O)O-, -C(=O)NR⁷-, NR⁷, P(=O)(R⁷), -O-, -S-, SO or SO₂;
R⁶ is selected on each occurrence, identically or differently, from H, D, F, C(=O)R⁷, CN, Si(R⁷)₃, P(=O)(R⁷)₂, OR⁷, S(=O)R⁷, S(=O)₂R⁷, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R⁶ may be linked to one another and may form a ring; where the said alkyl, alkoxy, alkenyl and alkynyl groups and the said aromatic ring systems and heteroaromatic ring systems may in each case be substituted by one or more radicals R⁷; and where one or more CH₂ groups in the said alkyl, alkoxy, alkenyl and alkynyl groups may be replaced by -R⁷C=CR⁷-, -C≡C-, Si(R⁷)₂, C=O, C=NR⁷, -C(=O)O-, -C(=O)NR⁷-, NR⁷, P(=O)(R⁷), -O-, -S-, SO or SO₂;
R⁷ is selected on each occurrence, identically or differently, from H, D, F, C(=O)R⁸, CN, Si(R⁸)₃, N(R⁸)₂, P(=O)(R⁸)₂, OR⁸, S(=O)R⁸, S(=O)₂R⁸, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R⁷ may be linked to one another and may form a ring; where the said alkyl, alkoxy, alkenyl and alkynyl groups and the said aromatic ring systems and heteroaromatic ring systems may in each case be substituted by one or more radicals R⁸; and where one or more CH₂ groups in the said alkyl, alkoxy, alkenyl and alkynyl groups may be replaced by -R⁸C=CR⁸-, -C=C-, Si(R⁸)₂, C=O, C=NR⁸, -C(=O)O-, -C(=O)NR⁸-, NR⁸, P(=O)(R⁸), -O-, -S-, SO or SO₂;
R⁸ is selected on each occurrence, identically or differently, from H, D, F, CN, alkyl or alkoxy groups having 1 to 20 C atoms, alkenyl or alkynyl groups having 2 to 20 C atoms, aromatic ring systems having 6 to 40 aromatic ring atoms and heteroaromatic ring systems having 5 to 40 aromatic ring atoms; where two or more radicals R⁸ may be linked to one another and may form a ring; and where the said alkyl, alkoxy, alkenyl and alkynyl groups, aromatic ring systems and heteroaromatic ring systems may be substituted by F or CN;
k is equal to 0 or 1, where, in the case where k=0, the group Ar^{L} is omitted and the nitrogen atom of the group of the formula (N) represents the bonding position;
m is equal to 0 or 1, where, in the case where m=0, the group E is omitted and the groups Ar² are not connected to one another;
i is equal to 0 or 1, where, in the case where i=0, the group R¹ is omitted; and
where at least one group Z¹ that is CR¹ is present.

2. Compound according to Claim 1, **characterised in that** either i) one group Z¹ is equal to CR¹ and the two other groups Z¹ are equal to CR³, or ii) two groups Z¹ are equal to CR¹ and the other group Z¹ is equal to CR³.

3. Compound according to Claim 1 or 2, **characterised in that** the group Z¹ in the meta position to the bond to X¹ is equal to CR¹.

4. Compound according to one or more of Claims 1 to 3, **characterised in that** Ar¹ is selected from phenyl and naphthyl groups, which may in each case be substituted by one or more radicals R³.

5. Compound according to one or more of Claims 1 to 4, **characterised in that** X¹ is equal to C(R⁴)₂.

6. Compound according to one or more of Claims 1 to 5, **characterised in that** the group of Ar² that is bonded directly to the nitrogen atom is an aromatic ring system.

7. Compound according to one or more of Claims 1 to 6, **characterised in that** m = 0.

8. Compound according to one or more of Claims 1 to 7, **characterised in that** m = 1, and the unit 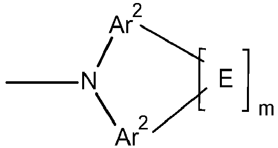 from the group of the formula (N) is selected from the groups:
| | | |
|---|---|---|
| 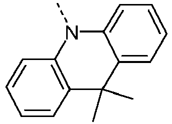 | 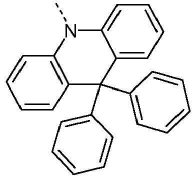 | 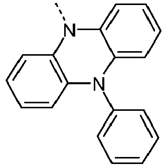 |
| N-1 | N-2 | N-3 |
| 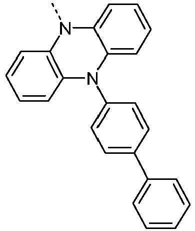 | 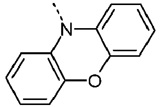 | 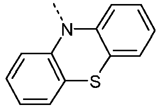 |
| N-4 | N-5 | N-6 |
| 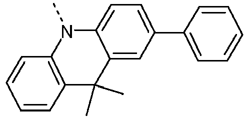 | 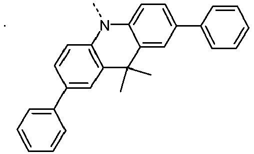 | 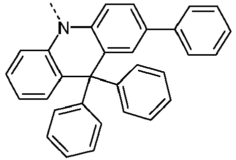 |
| N-7 | N-8 | N-9 |
| 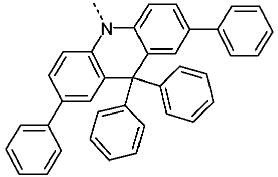 | 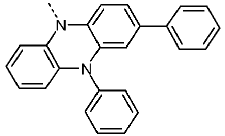 | 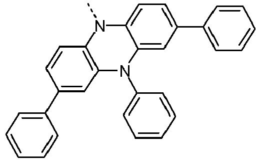 |
| N-10 | N-11 | N-12 |
| 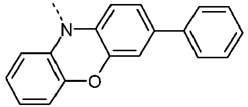 | 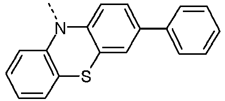 | 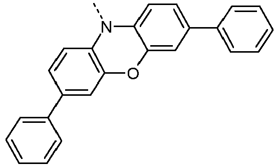 |
| N-13 | N-14 | N-15 |
| 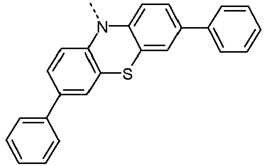 | 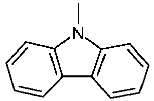 | 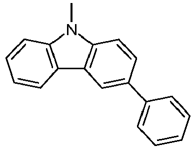 |
| N-16 | N-17 | N-18 |
| 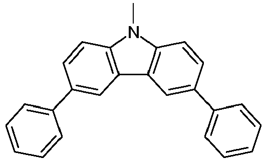 | 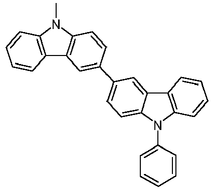 | 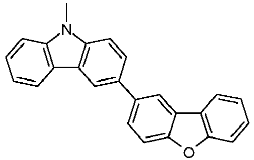 |
| N-19 | N-20 | N-21 |
| 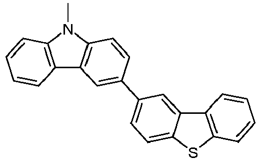 | 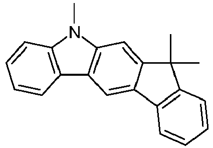 | 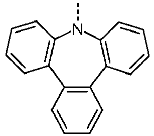 |
| N-22 | N-23 | N-24 |
| 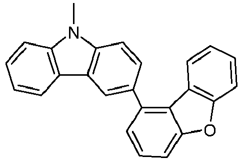 | 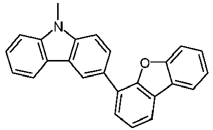 | 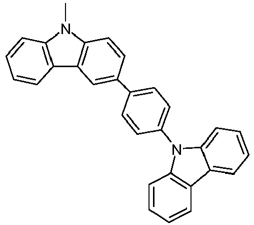 |
| N-25 | N-26 | N-27 |
| 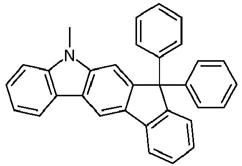 | 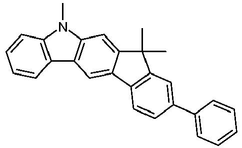 | 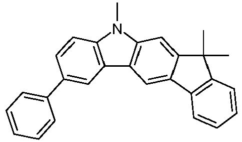 |
| N-28 | N-29 | N-30 |
| 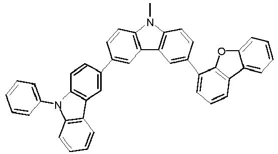 | 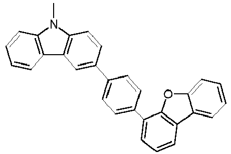 | |
| N-31 | N-32 | |
where the groups may be substituted by a radical R⁵ at each of their free positions, and where the dashed bonds represent the bonds to the remainder of the formula.

9. Compound according to one or more of Claims 1 to 8, **characterised in that** R² is equal to H.

10. Compound according to one or more of Claims 1 to 9, **characterised in that** R⁶ is equal to H.

11. Compound according to one or more of Claims 1 to 10, **characterised in that** it corresponds to one of the following formulae (I-1) to (I-9)
| |
|---|
| 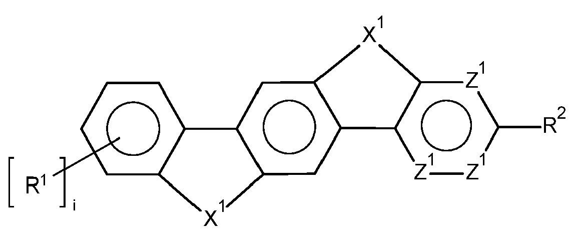 |
| formula (I-1) |
| 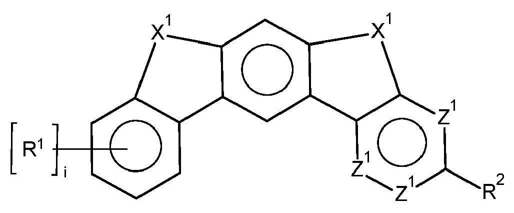 |
| formula (I-2) |
| 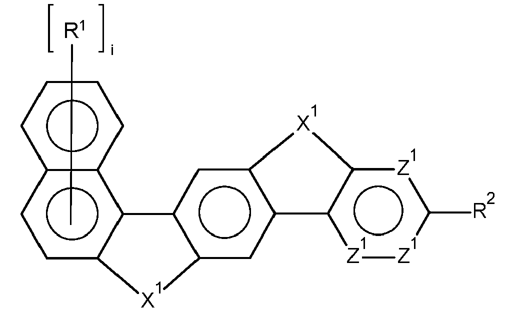 |
| formula (I-3) |
| 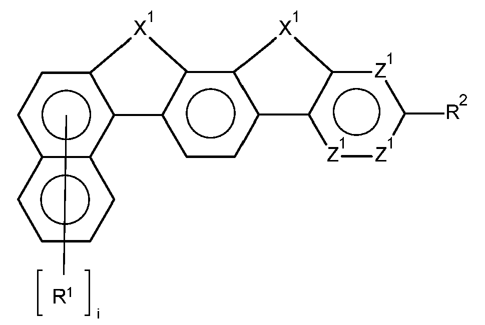 |
| formula (I-4) |
| 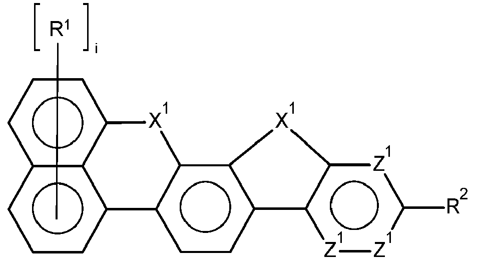 |
| formula (I-5) |
| 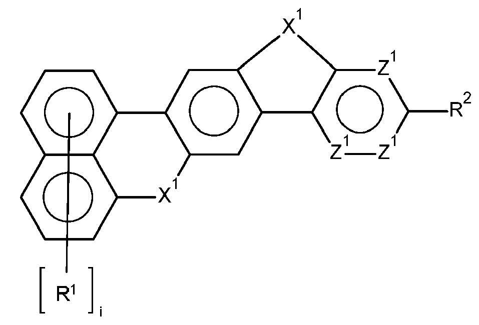 |
| formula (I-6) |
| 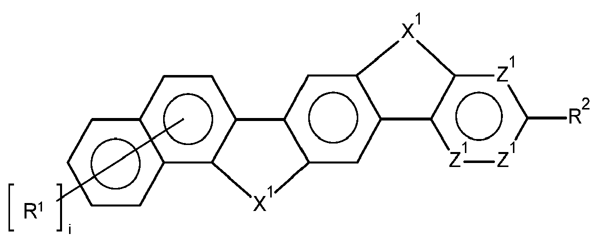 |
| formula (I-7) |
| 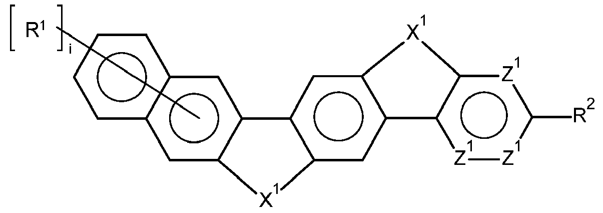 |
| formula (I-8) |
| 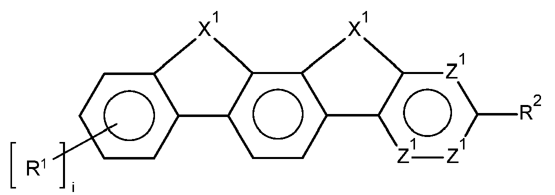 |
| formula (I-9) |
where the variables occurring are defined as in one or more of Claims 1 to 10, and where the compounds may be substituted by a radical R³ or R⁶ at each of the free positions on the aromatic rings.

12. Process for the preparation of a compound of the formula (I) according to one or more of Claims 1 to 11, **characterised in that** a benzene compound carrying two carboxylate groups, an aromatic or heteroaromatic ring system and a reactive group is reacted in a Suzuki reaction with a benzene compound containing a boronic acid group and a group selected from reactive groups, diarylamino groups, diarylamino-aryl groups and diarylamino-heteroaryl groups.

13. Oligomer, polymer or dendrimer containing one or more compounds of the formula (I) according to one or more of Claims 1 to 11, where the bond(s) to the polymer, oligomer or dendrimer may be localised at any desired positions in formula (I) that are substituted by R¹, R², R³, R⁴ or R⁵.

14. Formulation comprising at least one compound according to one or more of Claims 1 to 11 or a polymer, oligomer or dendrimer according to Claim 13, and at least one solvent.

15. Electronic device containing at least one compound according to one or more of Claims 1 to 11 or a polymer, oligomer or dendrimer according to Claim 13.

16. Electronic device according to Claim 15, **characterised in that** it is an organic electroluminescent device comprising anode, cathode and at least one emitting layer, where at least one organic layer of the device selected from emitting layers and hole-transporting layers comprises the at least one compound.

17. Organic electroluminescent device according to Claim 16 comprising anode, cathode and at least one emitting layer, **characterised in that** the at least one compound is present in an electron-blocking layer.

18. Use of a compound according to one or more of Claims 1 to 11 in an electronic device.

## Revendications

1. Composé de formule (I) 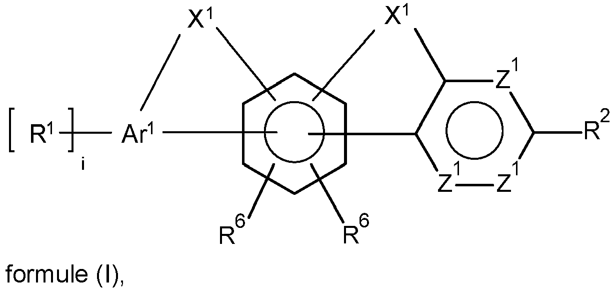 dans laquelle ce qui suit s'applique aux variables présentes :
Z¹ est choisi à chaque occurrence, de manière identique ou différente, parmi CR¹ et CR³ ;
Ar¹ est un groupement aryle ayant de 6 à 20 atomes de cycle aromatique, qui peut être substitué par un ou plusieurs radicaux R³, ou un groupement hétéroaryle ayant de 5 à 20 atomes de cycle aromatique, qui peut être substitué par un ou plusieurs radicaux R³ ;
X¹ est à chaque occurrence, de manière identique ou différente, un groupement divalent choisi parmi -C(R⁴)₂-, -C(R⁴)₂-C(R⁴)₂-, -CR⁴=CR⁴- et -Si(R⁴)₂- ;
R¹ est un groupement de formule (N) 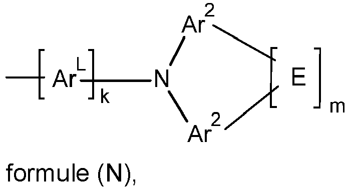
Ar^{L} est choisi parmi des noyaux aromatiques ayant de 6 à 40 atomes de cycle aromatique, qui peuvent être substitués par un ou plusieurs radicaux R⁵, et des noyaux hétéroaromatiques ayant de 5 à 40 atomes de cycle aromatique, qui peuvent être substitués par un ou plusieurs radicaux R⁵ ;
Ar² est choisi à chaque occurrence, de manière identique ou différente, parmi des noyaux aromatiques ayant de 6 à 40 atomes de cycle aromatique, qui peuvent être substitués par un ou plusieurs radicaux R⁵, et des noyaux hétéroaromatiques ayant de 5 à 40 atomes de cycle aromatique, qui peuvent être substitués par un ou plusieurs radicaux R⁵ ;
E est une liaison simple ou un groupement divalent choisi parmi C(R⁵)₂, Si(R⁵)₂, N(R⁵), O et S ;
R² est choisi parmi H, D, F, C(=O)R⁷, CN, Si(R⁷)₃, N(R⁷)₂, P(=O)(R⁷)₂, OR⁷, S(=O)R⁷, S(=O)₂R⁷, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où lesdits groupements alkyle, alcoxy, alcényle et alcynyle et lesdits noyaux aromatiques et noyaux hétéroaromatiques peuvent dans chaque cas être substitués par un ou plusieurs radicaux R⁷ ; et où un ou plusieurs groupements CH₂ dans lesdits groupements alkyle, alcoxy, alcényle et alcynyle peuvent être remplacés par -R⁷C=CR⁷-, -C=C-, Si(R⁷)₂, C=O, C=NR⁷, -C(=O)O-, -C(=O)NR⁷-, P(=O)(R⁷), -O-, -S-, SO ou SO₂ ;
R³, R⁴, R⁵ sont choisis à chaque occurrence, de manière identique ou différente, parmi H, D, F, C(=O)R⁷, CN, Si(R⁷)₃, N(R⁷)₂, P(=O)(R⁷)₂, OR⁷, S(=O)R⁷, S(=O)₂R⁷, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R³ ou R⁴ ou R⁵ peuvent être liés les uns aux autres et peuvent former un cycle ; où lesdits groupements alkyle, alcoxy, alcényle et alcynyle et lesdits noyaux aromatiques et noyaux hétéroaromatiques peuvent dans chaque cas être substitués par un ou plusieurs radicaux R⁷ ; et où un ou plusieurs groupements CH₂ dans lesdits groupements alkyle, alcoxy, alcényle et alcynyle peuvent être remplacés par -R⁷C=CR⁷-, -C=C-, Si(R⁷)₂, C=O, C=NR⁷, -C(=O)O-, -C(=O)NR⁷-, NR⁷, P(=O)(R⁷), -O-, -S-, SO ou SO₂ ;
R⁶ est choisi à chaque occurrence, de manière identique ou différente, parmi H, D, F, C(=O)R⁷, CN, Si(R⁷)₃, P(=O)(R⁷)₂, OR⁷, S(=O)R⁷, S(=O)₂R⁷, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R⁶ peuvent être liés les uns aux autres et peuvent former un cycle ; où lesdits groupements alkyle, alcoxy, alcényle et alcynyle et lesdits noyaux aromatiques et noyaux hétéroaromatiques peuvent dans chaque cas être substitués par un ou plusieurs radicaux R⁷ ; et où un ou plusieurs groupements CH₂ dans lesdits groupements alkyle, alcoxy, alcényle et alcynyle peuvent être remplacés par -R⁷C=CR⁷-, -C=C-, Si(R⁷)₂, C=O, C=NR⁷, -C(=O)O-, -C(=O)NR⁷-, NR⁷, P(=O)(R⁷), -O-, -S-, SO ou SO₂ ;
R⁷ est choisi à chaque occurrence, de manière identique ou différente, parmi H, D, F, C(=O)R⁸, CN, Si(R⁸)₃, N(R⁸)₂, P(=O)(R⁸)₂, OR⁸, S(=O)R⁸, S(=O)₂R⁸, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R⁷ peuvent être liés les uns aux autres et peuvent former un cycle ; où lesdits groupements alkyle, alcoxy, alcényle et alcynyle et lesdits noyaux aromatiques et noyaux hétéroaromatiques peuvent dans chaque cas être substitués par un ou plusieurs radicaux R⁸ ; et où un ou plusieurs groupements CH₂ dans lesdits groupements alkyle, alcoxy, alcényle et alcynyle peuvent être remplacés par -R⁸C=CR⁸-, -C=C-, Si(R⁸)₂, C=O, C=NR⁸, -C(=O)O-, -C(=O)NR⁸-, NR⁸, P(=O)(R⁸), -O-, -S-, SO ou SO₂ ;
R⁸ est choisi à chaque occurrence, de manière identique ou différente, parmi H, D, F, CN, des groupements alkyle ou alcoxy ayant de 1 à 20 atomes de C, des groupements alcényle ou alcynyle ayant de 2 à 20 atomes de C, des noyaux aromatiques ayant de 6 à 40 atomes de cycle aromatique et des noyaux hétéroaromatiques ayant de 5 à 40 atomes de cycle aromatique ; où deux, ou plus, radicaux R⁸ peuvent être liés les uns aux autres et peuvent former un cycle ; et où lesdits groupements alkyle, alcoxy, alcényle et alcynyle, lesdits noyaux aromatiques et noyaux hétéroaromatiques peuvent être substitués par F ou CN ;
k est égal à 0 ou 1, où, dans le cas où k=0, le groupement Ar^{L} est omis et l'atome d'azote du groupement de formule (N) représente la position de liaison ;
m est égal à 0 ou 1, où, dans le cas où m=0, le groupement E est omis et les groupements Ar² ne sont pas reliés les uns aux autres ;
i est égal à 0 ou 1, où, dans le cas où i=0, le groupement R¹ est omis ; et
où au moins un groupement Z¹ qui est CR¹ est présent.

2. Composé selon la revendication 1, **caractérisé en ce que** soit i) un groupement Z¹ est égal à CR¹ et les deux autres groupements Z¹ sont égaux à CR³, soit ii) deux groupements Z¹ sont égaux à CR¹ et l'autre groupement Z¹ est égal à CR³.

3. Composé selon la revendication 1 ou 2, **caractérisé en ce que** le groupement Z¹ en position méta par rapport à la liaison à X¹ est égal à CR¹.

4. Composé selon l'une ou plusieurs parmi les revendications 1 à 3, **caractérisé en ce que** Ar¹ est choisi parmi des groupements phényle et naphtyle, qui peuvent dans chaque cas être substitués par un ou plusieurs radicaux R³_{.}

5. Composé selon l'une ou plusieurs parmi les revendications 1 à 4, **caractérisé en ce que** X¹ est égal à C(R⁴)₂.

6. Composé selon l'une ou plusieurs parmi les revendications 1 à 5, **caractérisé en ce que** le groupement de Ar² qui est lié directement à l'atome d'azote est un noyau aromatique.

7. Composé selon l'une ou plusieurs parmi les revendications 1 à 6, **caractérisé en ce que** m = 0.

8. Composé selon l'une ou plusieurs parmi les revendications 1 à 7, **caractérisé en ce que** m = 1, et le motif 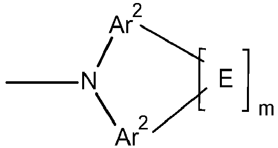 du groupement de formule (N) est choisi parmi les groupements :
| | | |
|---|---|---|
| 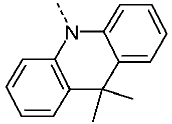 | 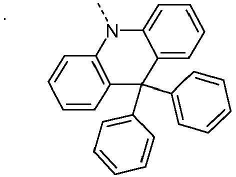 | 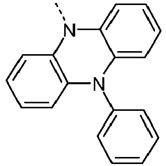 |
| N-1 | N-2 | N-3 |
| 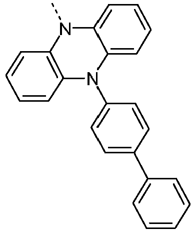 | 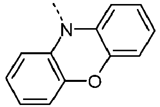 | 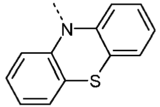 |
| N-4 | N-5 | N-6 |
| 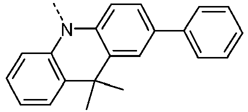 | 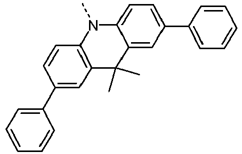 | 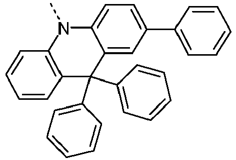 |
| N-7 | N-8 | N-9 |
| 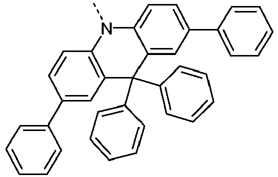 | 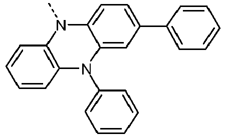 | 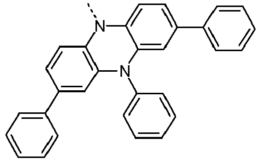 |
| N-10 | N-11 | N-12 |
| 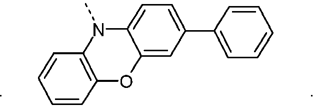 | 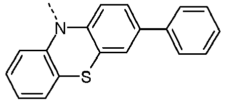 | 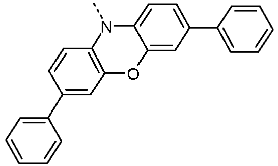 |
| N-13 | N-14 | N-15 |
| 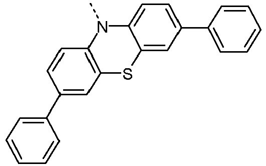 | 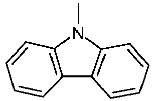 | 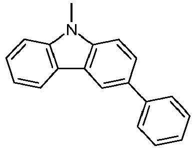 |
| N-16 | N-17 | N-18 |
| 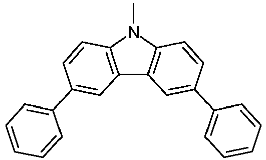 | 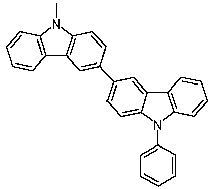 | 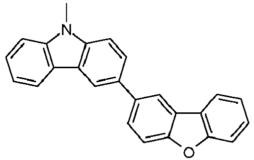 |
| N-19 | N-20 | N-21 |
| 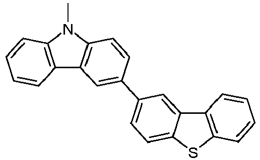 | 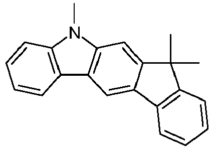 | 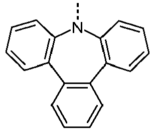 |
| N-22 | N-23 | N-24 |
| 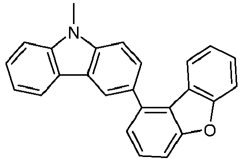 | 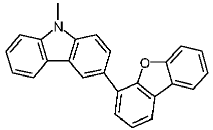 | 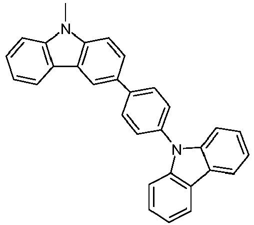 |
| N-25 | N-26 | N-27 |
| 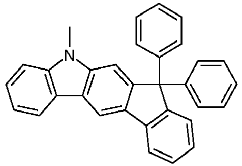 | 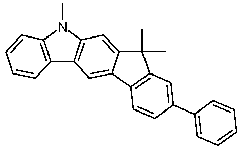 | 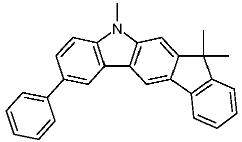 |
| N-28 | N-29 | N-30 |
| 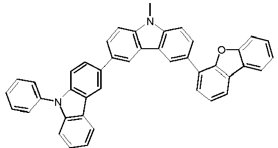 | 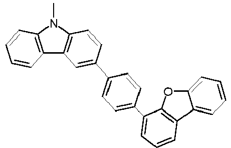 | |
| N-31 | N-32 | |
où les groupements peuvent être substitués par un radical R⁵ au niveau de chacune de leurs positions libres, et où les liaisons en pointillés représentent les liaisons au reste de la formule.

9. Composé selon l'une ou plusieurs parmi les revendications 1 à 8, **caractérisé en ce que** R² est égal à H.

10. Composé selon l'une ou plusieurs parmi les revendications 1 à 9, **caractérisé en ce que** R⁶ est égal à H.

11. Composé selon l'une ou plusieurs parmi les revendications 1 à 10, **caractérisé en ce qu'**il correspond à l'une des formules (I-1) à (I-9) suivantes
| |
|---|
| 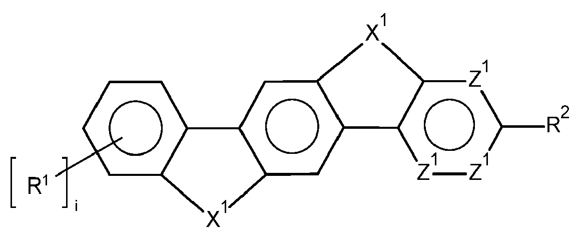 |
| formule (I-1) |
| 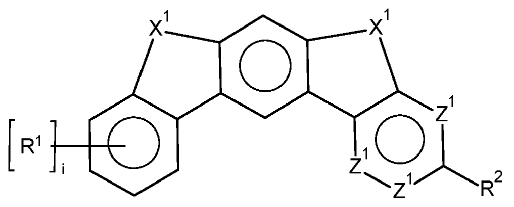 |
| formule (I-2) |
| 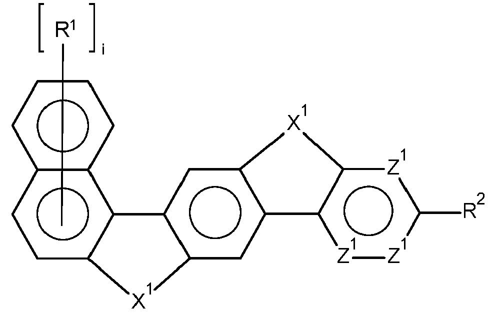 |
| formule (I-3) |
| 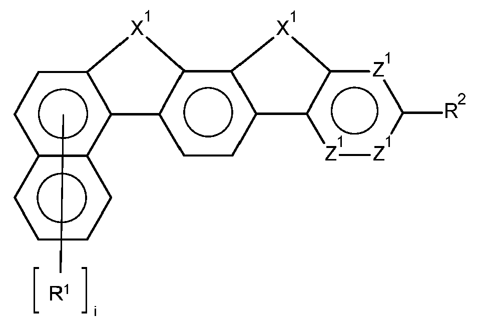 |
| formule (I-4) |
| 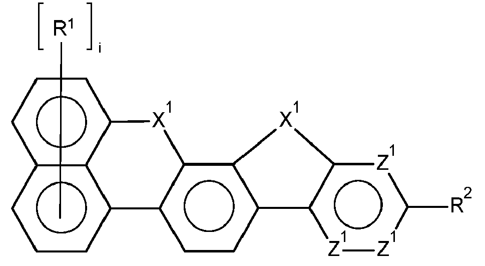 |
| formule (I-5) |
| 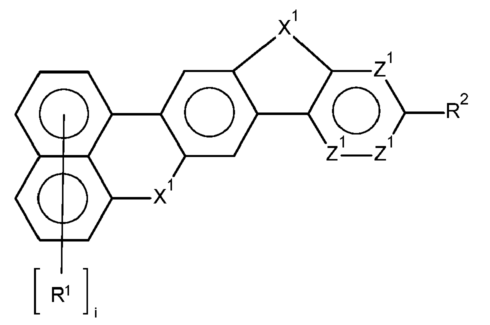 |
| formule (I-6) |
| 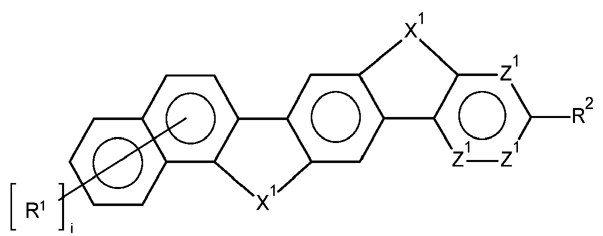 |
| formule (I-7) |
| 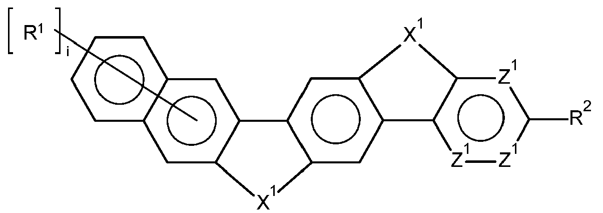 |
| formule (I-8) |
| 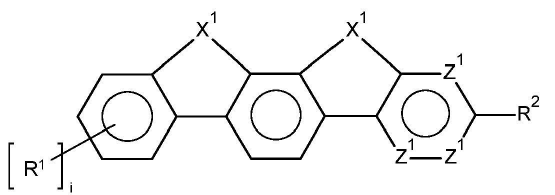 |
| formule (I-9) |
où les variables présentes sont telles que définies selon l'une ou plusieurs parmi les revendications 1 à 10, et où les composés peuvent être substitués par un radical R³ ou R⁶ au niveau de chacune des positions libres sur les cycles aromatiques.

12. Procédé de préparation d'un composé de formule (I) selon l'une ou plusieurs parmi les revendications 1 à 11, **caractérisé en ce qu'**un composé de benzène portant deux groupements carboxylate, un noyau aromatique ou hétéroaromatique et un groupement réactif est réagi dans une réaction de Suzuki avec un composé de benzène contenant un groupement acide boronique et un groupement choisi parmi des groupements réactifs, des groupements diarylamino, des groupements diarylamino-aryle et des groupements diarylamino-hétéroaryle.

13. Oligomère, polymère ou dendrimère contenant un ou plusieurs composés de formule (I) selon l'une ou plusieurs parmi les revendications 1 à 11, où la ou les liaisons au polymère, à l'oligomère ou au dendrimère peuvent être situées au niveau de positions désirées quelconques dans la formule (I) qui sont substituées par R¹, R², R³, R⁴ ou R⁵.

14. Formulation comprenant au moins un composé selon l'une ou plusieurs parmi les revendications 1 à 11 ou un polymère, oligomère ou dendrimère selon la revendication 13, et au moins un solvant.

15. Dispositif électronique contenant au moins un composé selon l'une ou plusieurs parmi les revendications 1 à 11 ou un polymère, oligomère ou dendrimère selon la revendication 13.

16. Dispositif électronique selon la revendication 15, **caractérisé en ce qu'**il s'agit d'un dispositif électroluminescent organique, comprenant une anode, une cathode et au moins une couche émettrice, où au moins une couche organique du dispositif choisie parmi les couches émettrices et les couches de transport de trous, comprend le au moins un composé.

17. Dispositif électroluminescent organique selon la revendication 16, comprenant une anode, une cathode et au moins une couche émettrice, **caractérisé en ce que** le au moins un composé est présent dans une couche de blocage d'électrons.

18. Utilisation d'un composé selon l'une ou plusieurs parmi les revendications 1 à 11 dans un dispositif électronique.
